# EUROPEAN PATENT APPLICATION

(11) **EP 2 586 791 A1**
(43) Date of publication of application: **01.05.2013**
(21) Application number: 11306400.0
(22) Date of filing: 28.10.2011
(51) Int. Cl.: C07K 14/36, C12R 1/465

(54) **Gene cluster for biosynthesis of griselimycin and methylgriselimycin**

(71) Applicant: SANOFI, 75008 Paris (FR)
(72) Inventor: The designation of the inventor has not yet been filed
(74) Representative: Schneider, Rudolf

(57) **Abstract**

The present invention refers to the gene cluster and genes comprised by the gene cluster which are involved in the biosynthesis of griselimycin and methylgriselimycin and to the use of the gene cluster, genes comprised thereby and proteins encoded thereby for the production of antibiotic agents.

## Description

The present invention relates to the field of antibiotics and more specifically to genes and proteins involved in the biosynthesis of (methyl)griselimycin. The present invention provides recombinant methods for producing (methyl)griselimycin by recombinant DNA technology.

Griselimycin is a naturally occurring antibiotic produced by microorganism of the genus *Streptomyces* which is the largest genus of the *Actinobacteria* and the type genus of the family *Streptomycetaceae.* Streptomycetes are Gram-positive and have genomes with high GC-content. *Streptomycetes* are anthus e am by a complex secondary metabolism. They produce over two-thirds of the clinically useful antibiotics of natural origin. Griselimycin is an antibiotic which was first described by Terlain and Thomas, 1971. It is composed of ten amino acids being in this order L-N-methylvaline, which is N-acetylated, L-trans-4-methylproline, L-N-methylthreonine, L-leucine, L-trans-4-methylproline, L-leucine, L-N-methylvaline, L-proline, D-N-methylleucine and glycine. The peptide chain is cyclised between glycine and L-N-methylthreonine via an ester bond resulting in a non-cyclised tail consisting of the amino acids L-N-methylvaline, which is N-acetylated, and L-trans-4-methylproline.

Griselimycin belongs to the group of non-ribosomally synthesized microbial peptides which group of peptides shows a remarkable structural diversity and comprises a widespread class of the most potent antibiotics and other important pharmaceuticals. Although structurally diverse, non-ribosomally synthesized microbial peptides share a common mode of synthesis, the multienzyme thiotemplate mechanism. Thereby, peptide bond formation takes place on large multienzyme complexes, which simultaneously represent template and biosynthesis machinery. These multienzyme complexes are designated non-ribosomal peptide synthetases (NRPSs). Sequencing of genes encoding NRPS revealed a modular organization. A module is a section of the NRPS polypeptide chain that is responsible for the incorporation of one defined monomer into the growing polypeptide chain. Thus, NRPSs are used simultaneously as template because the amino acid to be incorporated is determined by the module and as biosynthetic machinery because it is the module that harbors all necessary catalytic functions. Modules can be further dissected into catalytic domains. These domains catalyse at least the steps of substrate activation, covalent binding and peptide bond formation. Domains of equal function share a number of highly conserved sequence motifs. They can be modified for activity changes within the polypeptide chain which opens up prospects for manipulation of the NRPS machinery.

Within each module, the selection of a specific substrate (amino acid) is mediated by an adenylation domain (A-domain). This A-domain is responsible for the selection of the amino acids that make up the product and thus controls its primary sequence. A-domains activate amino and also some carboxy substrates as amino acyl adenylate while ATP is consumed. This reactive intermediate is further transported onto the terminal cysteamine thiol moiety of the Ppan prosthetic group that is attached to the peptidyl carrier protein (PCP) domain which is also referred to as thiolation (T) domain located downstream of the A-domain in the same module. It represents the transport unit that accepts the activated amino acid that is covalently tethered to its 4'PP cofactor thioester. This cofactor acts as a flexible arm to allow the bound amino acyl and peptidyl substrate to travel between different catalytic centers. The combination of A-domain and T-domain is defined as an initiation module since both domains are required to activate and covalently tether the first building block for subsequent peptide synthesis. The initiation modules of lipopeptide pathways harbor an additional N- terminal C domain for condensation of an acyl side chain with the amino group of the first amino acid. The condensation or C-domain is the central entity of non-ribosomal peptides synthesis because it is responsible for peptide bond formation between amino acyl substrates bound to the T-domain of adjacent modules. The enzyme catalyses the nucleophilic attack of the amino (or imino, hydroxyl) group of the activated amino acid bound to the downstream (with respect to the C-domain) module onto the acyl group or the amino acid tethered to the upstream module. The resulting peptidyl intermediate is then translocated down the assembly line for subsequent condensation and further modification steps. During synthesis, the growing peptide chain is handed over from one module to the next module until it reaches the final modul's T-domain. This module contains in most cases a TE (thioesterase)-domain that is important for the liberation of the product. Product release is achieved by a two-step process that involves an acyl-O-TE-enzyme intermediate that is subsequently attacked by either a peptide-internal nucleophile or water, which results in a macrocyclic product or a linear peptide, respectively.

An especially striking feature of non-ribosomal peptides is the occurrence of D-amino acids. One way to incorporate a D-amino acid is to use a D-amino acid selective A domain. The more common way is, however, through the use of E (epimerization)-domains that occur at the C-terminal end of modules responsible for the incorporation of D-amino acids. The enzyme catalyses the anthus e am of T-domain bound L-amino acid of the growing peptide chain. Discrimination of the T-domain bound L-amino acid through enantioselectivity of the downstream condensation domain donor site leads to a clean D-configurated product.

A number of NRPS contain methyltransferases (MT domain) that are responsible for the N- or C-methylation of amino acid residues thus making the peptide less susceptible to proteolytic breakdown. Both types of methyltransferases (N- or C-methyltransferases or abbreviated N-MT or C-MT) use S-adenosyl methionine as the methyl donor. The MT domains are often found as insertions in the A-domains (Finking et Marahiel, 2004; Marahiel, 2009). Even more striking is the incorporation of unnatural amino acids such as methyl-proline which are usually biosynthesized in advance to their use in the assembly line. Alternatively, incorporated amino acids can be modified enzymatically post assembly by the NRPS.

Griselimycin is naturally produced by microorganisms of the genus *Streptomyces.* However, although the production of griselimycin by *Streptomyces* has been known in the art, the genetic locus responsible for the biosynthesis of griselimycin has not been identified so far. Consequently, targeting and modification of the biosynthesis of griselimycin were still limited. Accordingly, there is a need for genetic information regarding the biosynthesis of griselimycin.

Besides griselimycin, *Streptomycetes* also produce a variant form of griselimycin, termed methylgriselimycin. Methylgriselimycin contains L-trans-4-methylproline instead of L-proline. Methylgriselimycin possesses some advantages, such as e.g different antibacterial specificity, in its use as an anti-bacterial agent over griselimycin, however, is produced by *Streptomycetes* as a side-component only. Accordingly, there is a need for genetic information regarding the biosynthesis of methylgriselimycin.

The production of the methylproline moiety as comprised by griselimycin and methylgriselimycin, namely L-trans-4-methylproline, was completely unknown. Accordingly, there is a need for genetic information regarding the biosynthesis of L-trans-4-methylproline.

The genetic locus encoding the polypeptides involved in the biosynthetic pathway of (methyl)griselimycin has now been identified. The present inventors succeeded in isolating and cloning the entire griselimycin biosynthetic gene cluster from the genomic DNA of *Streptomyces DSM 26643.* The present inventors also succeeded in elucidating the open reading frames (ORFs) that encode the polypeptides involved in the (methyl)griselimycin biosynthetic pathway including such responsible for the formation of L-trans-4-methylproline. This allows the identification and influencing of the distinct biosynthesis steps on the molecular level.

Thus, in one aspect, the present invention provides a nucleic acid comprising at least one nucleic acid selected from:
(a) a nucleic acid comprising at least one of the Open Reading Frames (ORFs) 1 to 26 as comprised by SEQ ID NO: 1 encoding proteins of SEQ ID Nos: 2 to 27 or a variant or fragment thereof whereby the variant or fragment encodes a functionally active variant or fragment of a protein of SEQ ID Nos: 2 to 27,
(b) a nucleic acid encoding at least one of the proteins of SEQ ID Nos: 2 to 27 or a functionally active variant or fragment thereof,
I a nucleic acid encoding a protein that is at least 70 %, 80 %, 90 %, 95 % or 97% identical in amino acid sequence to a protein or fragment thereof encoded by the nucleic acid of (a) or (b),
(d) a nucleic acid which hybridizes under stringent conditions with a nucleic acid of (a) to (c),
(e) a nucleic acid which hybridizes under stringent conditions with a nucleic acid of (a) to (d) and consists of 10 to 50 nucleotides in length, or
(f) a nucleic acid which is complementary to a nucleic acid of (a) to (f).

The nucleic acid as comprised by the present invention is, in one embodiment, a nucleic acid comprising or consisting of SEQ ID NO: 1 being the part of the genome of *Streptomyces DSM 26643* comprising the griselimycin biosynthesis gene cluster with a length of 66.868 nucleotides. This newly discovered gene cluster contains at least 26 ORFs anthunated ORF 1 to ORF 26 (ORF 1, 2, 3, 4, 5, 6, 7, 8, 9, 10, 11, 12, 13, 14, 15, 16, 17, 18, 19, 20, 21, 22, 23, 24, 25, 26) or designated with the gene names orf-1, orf-2, orf-3, orf-4, orf-5, orf-6, orf-7, nrps-1, int-1, tnp-1, int-2, tnp-2, tnp-3, dna, nrps-2, nrps-3, mbtH, mps-1, mps-2, mps-3, mps-4, orf-8, orf-9, orf-10, of-11, orf-12, respectively, from nucleotides 837 to 66.108 of SEQ ID NO: 1 with a length of 65.272 nucleotides. These ORFs encode proteins of SEQ ID Nos: 2 to 27 (SEQ ID NO: 2, 3, 4, 5, 6, 7, 8, 9, 10, 11, 12, 13, 14, 15, 16, 17, 18, 19, 20, 21, 22, 23, 24, 25, 26, 27), respectively, involved in the biosynthesis of (methyl)griselimycin. Structural and functional characterization of the proteins encoded by the griselimycin biosynthesis gene cluster is provided below.

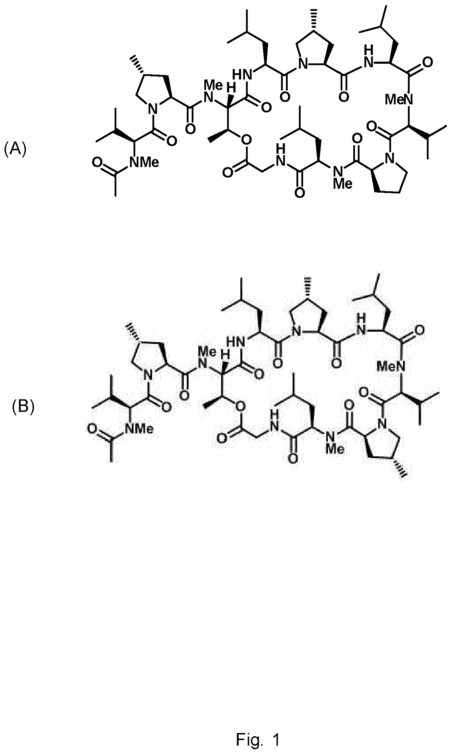

In the context of the present invention, the term "(methyl)griselimycin" refers to griselimycin as well as to methylgriselimycin. Griselimycin is an antibiotic which is composed of ten amino acids being L-N-methylvaline, which is acetylated (1), L-trans-4-methylproline (2), L-N-methylthreonine (3), L-leucine (4), L-trans-4-methylproline (5), L-leucine (6), L-N-methylvaline (7), L-proline (8), D-N-methyl-D-leucine (9) and glycine (10). The structural formula of griselimycin and methylgriselimycin is given in Fig. 1. Griselimycin and its variant methylgriselimycin are produced in different amounts by the same biosynthesis pathway involving the same genes of the (methyl)griselimycin biosynthesis gene cluster as identified herein. Methylgriselimycin differs from griselimycin by the presence of L-trans-4-methylproline instead of L-proline at position 8. The multidomain-enzyme responsible for the incorporation of L-trans-4-methylproline into methylgriselimycin and of L-proline into griselimycin is the same enzyme, namely NRPS-2, and is the protein product of the gene nrps-2, as identified herein.

In the context of the present invention the term "gene cluster" refers to a set of several genes which are located on a contiguous stretch of the genome and which direct the synthesis of (methyl)griselimycin. Some of the genes belonging to the gene cluster are genes which are related and are grouped to a gene family. They encode proteins which are similar in structure and function. The remainder of the genes are unrelated in structure and encode proteins which have different structure and function. The encoded proteins are either enzymes which catalyse reactions or are involved inter alia in regulation or transport. Altogether, the genes as comprised by the gene cluster encode proteins that serve the purpose of the biosynthesis of (methyl)griselimycin.

The term "nucleic acid" encompasses nucleic acids which are defined by ORFs 1 to 26 coding for the proteins of SEQ ID Nos: 2 to 27, respectively, as well as naturally occurring and non-naturally occurring variants or fragments thereof (as defined herein). The nucleic acid may be any macromolecule composed of chains of monomeric nucleotides carrying genetic information or form structures within cells. The most common (and therefore preferred) nucleic acids are deoxyribonucleic acid (DNA) and ribonucleic acid (RNA). The nucleic acid can be a DNA molecule such as a genomic DNA molecule such as SEQ ID NO: 1 or a cDNA molecule which can be single- or double-stranded such as a nucleic acid representing an ORF and encoding a protein as well as a synthetic DNA such as a synthesized single-stranded polynucleotide such as a primer or probe. The nucleic acid of the present invention can also be an RNA molecule. Preferably, the term also relates to non-coding regions of a gene, wherein these sections are of a relevant size in order to be specific for that gene. Examples of those regions are regulatory elements such as a promoter. Most preferably, the term "nucleic acid" relates to gene, ORF, promoter, DNA, cDNA or mRNA. The nucleic acid encoding the desired genetic information, preferably DNA, may comprise the gene of interest, a promoter region, a start codon and a stop codon and possibly further regions which may be used for regulation of expression of the gene. The regulatory regions may be heterologous to the respective gene or may be associated therewith in nature. The genetic information may be expressed permanently or under the control of a repressor and/or a promoter region in a cell into which the nucleic acid of the present invention is introduced. The obtained cells may be either used directly or used for tissue cultures or the cells may be harvested and samples comprising the respective protein are obtained by disrupting the cells. Alternatively, DNA or RNA may be used either in cells or in cell-free expression systems such as, e.g., microarray systems in which the DNA or RNA is immobilized and is translated and/or transcribed by the addition of functional cell lysate, comprising the factors required for transcription and/or translation (enzymes, ribosomes, tRNA, amino acids, nucleotides, ATP etc.). Also included are artificial nucleic acids which include peptide nucleic acid (PNA), morpholino and locked nucleic acid (LNA), as well as glycol nucleic acid (GNA) and threose nucleic acid (TNA). Each of these is distinguished from naturally-occurring DNA or RNA by changes to the backbone of the molecule.

The term " comprising" as used herein is meant to "include or encompass" the desired feature and further features which must not be specifically mentioned.. The term "comprising" is also meant to "consist of' the desired feature and not to include further features except the desired feature. Thus, the nucleic acid or protein referred to herein may be defined by additional features in addition to the definition as indicated, e.g. in addition to the definition by an ORF or SEQ ID number, or may consist of such indicated feature only.

The term "heterologous" as it relates to nucleic acid sequences such as coding or control sequences denotes sequences that are normally not associated with a region of a recombinant construct and/or a particular cell. A "heterologous" region is an identifiable segment of a nucleic acid within or attached to another nucleic acid that is not found in association with the other molecule in nature. For example, a heterologous region of a construct could be a regulatory region not found to be associated with a gene as identified herein in nature. Similarly, a heterologous sequence could be a coding sequence which is itself not found in nature as it contains e.g. synthetic sequences with codons different from the native gene. Moreover, a host cell transformed with a construct which is not normally present in the host cell would be considered heterologous for the purposes of the present invention. A homologous nucleic acid sequence is a variant sequence as defined herein. The term "homologous" may be used interchangeably with variant. The term "homologous" may also refer to an identical sequence.

In an embodiment of the present invention the nucleic acid comprises a nucleic acid molecule which encodes at least one distinct protein as comprised by the sequence of SEQ ID Nos: 2 to 27. In a particular embodiment, the entire sequence of the (methyl)griselimycin gene cluster as comprised by SEQ ID NO: 1 is comprised by a nucleic acid of the present invention. In another embodiment, the nucleic acids are variants or fragments of ORFs 1 to 26 encoding functionally active variants or fragments of at least one of the proteins of SEQ ID Nos: 2 to 27. The most preferred nucleic acid codes for a naturally occurring variant protein of ORFs 1 to 26 as detailed below, more preferably, a naturally occurring *Streptomyces* protein, still more preferably a protein of SEQ ID Nos: 2 to 27, which is involved in the synthesis of (methyl)griselimycin.

The term "nucleic acid" may comprise full length ORFs 1 to 26 as disclosed herein or may comprise fragments or partial sequences of ORFs 1 to 26. Such fragments or partial sequences of ORFs 1 to 26 encode fragments of the proteins with the amino acid sequences shown in SEQ ID Nos: 2 to 27, respectively. This may include fragmental proteins with short internal and/or C- and/or N-terminal deletions whereby the activity of the resulting proteins as identified herein is maintained to an extent of more than 5 %, more than 10 %, more than 20 %, more than 30 %, more than 40 %, more than 50 %, more than 60 %, more than 70 %, more than 80 %, more than 90 %, more than 95 %, more than 97 % or more than 100 % , e.g. more than 150 %, 200 %, 300 % 400 % or 500 %, of the activity of the wild-type proteins as encoded by the ORFs 1 to 26. Consequently, the respective nucleic acid encoding such fragments contains deletions within and/or at the 5' and/or 3' termini of ORFs 1 to 26, e. g., deletions of at the most 50%, 45%, 40%, 35%, 30%, 25%, 20%, 15%, 10 %, 5%, or less. In the context of the present invention, a fragmental nucleic acid sequence encoding a functionally active fragment of a protein of SEQ ID Nos: 2 to 27 means a sequence encoding a fragment which directs synthesis of (methyl)griselimycin and/or which can be substituted for the respective ORF encoding the protein of SEQ ID Nos: 2 to 27 to direct synthesis of (methyl)griselimycin.

The term "nucleic acid" may refer to variants of ORFs 1 to 26 as disclosed herein encoding functionally active variants of the proteins of SEQ ID Nos: 2 to 27 as defined herein. Such functionally active variants have a sequence identity with SEQ ID Nos: 2 to 27 of more than 50%, of more than 60%, preferably more than 70%, more preferably of more than 80%, still more preferably more than 85%, even more preferably more than 90%, even more preferably more than 95%, most preferably more than 97% and/or have an activity of more than 5%, more than 10%, more than 20%, more than 30%, more than 40%, more than 50%, more than 60%, more than 70%, more than 80%, more than 90%, more than 95%, more than 97% or more than 100 % , e.g. more than 150 %, 200 %, 300 % 400 % or 500 % of the activity of the proteins of SEQ ID Nos: 2 to 27. Consequently, the nucleic acid encoding such variants contains deletions, insertions, substitutions and/or additions within and/or at the 5' and/or 3' termini of ORFs 1 to 26 and show an identity to the sequences of ORFs 1 to 26 of more than 50%, more than 60%, more than 70%, preferably more than 80%, more preferably more than 85%, even more preferably more than 90%, even more preferably more than 95%, most preferably more than 97%. In the context of the present invention, a variant nucleic acid sequence encoding a functionally active variant of the protein of SEQ ID Nos: 2 to 27 means a sequence encoding a variant which directs synthesis of (methyl)griselimycin and/or which can be substituted for the respective ORF encoding the protein of SEQ ID Nos: 2 to 27 to direct synthesis of (methyl)griselimycin.

It is noted that the above mentioned modifications may be combined. For example, a nucleic acid as comprised by the present invention may be a fragment comprising one or more substitutions of ORFs 1 to 26. It should also be noted that fragments or variants include fragments or variants, as defined herein, of promoter or regulatory sequences with which ORFs 1 to 26 or fragments or variants thereof are associated in nature. These variants are functionally active in that they regulate the transcription or translation of the genes associated therewith.

An ORF is an open reading frame which is a DNA sequence that could potentially encode a protein. In the context of the present invention, the term "ORF" stands for open reading frame in the (methyl)griselimycin biosynthetic gene cluster as isolated from *Streptomyces DSM 22643* (ORFs 1 to 26). The present inventors succeeded in identifying the griselimycin biosynthesis gene cluster of *Streptomyces DSM 22643* and identified 26 ORFs over a stretch of 65.272 nucleotides. These ORFs were designated of-1, orf-2, orf-3, orf-4, orf-5, orf-6, orf-7, nrps-1, int-1, tnp-1, int-2, tnp-2, tnp-3, dna, nrps-2, nrps-3, mbtH, mps-1, mps-2, mps-3, mps-4, orf-8, orf-9, orf-10, orf 11 or orf-12, respectively. The whole griselimycin biosynthesis gene cluster of *Streptomyces DSM 22643* is 66868 nucleotides in length comprising ORFs 1 to 26 and additional nucleotides located 5' and 3' to ORF 1 and ORF 26, respectively.

The 26 ORFs comprised by SEQ ID NO: 1 extend from nucleotide 837 to nucleotide 66.108 of SEQ ID NO: 1 of *Streptomyces DSM 26643.* Table 1 shows a list of the specific putative ORFs, the designation of the corresponding genes, the start and stop nucleotides within SEQ ID NO: 1, the lengths of the genes in nucleotides (nt), the strandedness, the number of amino acids (aa) of the putative proteins encoded thereby, the protein designation and the SEQ ID numbers of the proteins. Table 2 shows proteins which are designated by their GenBank accession numbers as available from the NCBI (National Centre for Biotechnology Information; National Library of Medicine 38A, Bethesda, MD20894; USA; www.ncbi.nih.gov). These proteins have been identified, based on homology searches, as showing the highest identity/similarity to the proteins of SEQ ID Nos: 2 to 27. These proteins are defined by their function, their accession number and their origin. Table 2 moreover shows the e-value and the degree of identity and similarity between the proteins of SEQ ID Nos: 2 to 27 and the proteins as identified by the homology searches. The e-value relates to the expected number of chance alignments with an alignment score of at least equal to the observed alignment score. An e-value of 0.00 indicates a perfect homolog. The e-value is calculated as described in Altschul S.F. et al. 1997. The e-value assists in the determination of whether two sequences display sufficient similarity to justify an inference of homology. Identity indicates that identical amino acids are present in the compared proteins at the respective sites whereas similarity indicates that conservative amino acids substitutions as defined herein below are present at the respective sites. Moreover, the putative function of the preoteins encoded by ORFs 1 to 26 is indicated.

The substitution of a variant nucleic acid for ORFs 1 to 26 to direct synthesis of (methyl)griselimycin means that this variant nucleic acid can be inserted into the genome of *Streptomyces DSM 22643* instead of the ORF to which it is a variant thereby expressing a variant protein which takes over the function of the respective protein of SEQ ID Nos: 2 to 27 and directs, i.e. participates in, the synthesis of (methyl)griselimycin. The extent to which the variant takes over the function is as defined herein.

In another embodiment, the nucleic acid encodes a protein of SEQ ID Nos: 2 to 27. The nucleic acid may comprise the sequences of ORFs 1 to 26 as comprised by SEQ ID NO: 1 with the start and stop nucleotides (nt) as indicated in Table 1. The nucleic acid may also encode a protein with the same amino acids as the proteins of SEQ ID Nos: 2 to 27, however, which differs in its nucleotide composition due to the degeneracy of the genetic code.

In a further embodiment, the nucleic acid hybridizes under stringent conditions to a nucleic acid which is comprised by the (methyl)griselimycin gene cluster as identified by SEQ ID NO: 1 or which constitutes ORFs 1 to 26 or which encodes proteins with SEQ ID NO: 2 to 27 or functionally active variants or fragments thereof. In the present invention, the term " anthus e(s)(ing) under stringent conditions" refers to the formation of a hybrid between two nucleic acid molecules under conditions which allow the formation of a so-called specific hybrid, while a non-specific hybrid is substantially not formed. An example of such conditions includes conditions under which a complementary strand of a highly identical nucleic acid, namely, a DNA composed of a nucleotide sequence having 70 % or more, preferably 80% or more, more preferably 85% or more, still more preferably 90% or more and even more preferably 95% or more identity with the nucleotide sequence of SEQ ID NO. 1 or shown in ORFs 1 to 26, hybridizes, while a less complementary strand of a nucleic acid less identical than the above does not hybridize. More specifically, such conditions refer to conditions in which the sodium salt concentration is 15 to 750 mM, pref erably 50 to 750 mM, and more preferably 300 to 750 mM, the temperature is 25 to 70°C, preferably 50 to 70°C, and more preferably 55 to 65°C, and the formamide concentration is 0 to 50%, preferably 20 to 50%, and more preferably 35 to 45%. Furthermore, under stringent conditions, conditions for washing a filter after hybridization normally comprise the following: the sodium salt concentration is 15 to 600 mM, preferably 50 to 600 mM, and more preferably 300 to 600 mM, and the temperature is 50 to 70°C, preferably 55 to 70°C, and more preferably 60°C. Stringency, and thus specificity, can e.g. be increased by increasing the reaction temperature and/or lowering the ion strength of the reaction buffer. For example, low stringent conditions comprise hybridization in 3 x SSC at room temperature to 65°C and highly stringent conditions comprise hybridization in 0.1 x SSC at 68°C. Examplary moderately stringent conditions (nucleic acids hybridize under moderately stringent conditions, if they are maximally degenerate with respect to their codon composition) comprise 50% formamide, 5 x SSC and 1% SDS at 42°C and washing in 1 x SSC at 45°C. Highly stringent conditions comprise incubation at 42°C, 50% formamide, 5 x SSC and 1% SDS (e.g. 50% formamide, 5 x SSC and 1% SDS, 50 mM sodium phosphate, 5 x Denhardt's solution, 10 x dextran sulphate, 20 mg/ml sheared salmon sperm DNA) or 5 x SSC and 1% SDS at 65°C and washing in 0.2 x SSC and 0.1% SDS at about 65°C (1 x SSC stands for 0.15 M sodium chloride and 0.015 M trisodium citrate buffer). Preferred in the present invention are moderately or highly stringent conditions, most preferred are highly stringent conditions. In the context of the present invention, a "hybridizing" sequence means a sequence which encodes a protein which directs synthesis of (methyl)griselimycin and/or which can be substituted for the ORF to which it specifically hybridizes to direct synthesis of (methyl)griselimycin.

In a further preferred embodiment of the present invention, a nucleic acid which hybridizes under stringent conditions with a nucleic acid as identified above and consists of 5 to 100 nucleotides in length is provided. The sequence information provided in the present application enables the design of specific nucleotide probes and primers that are useful for identifying and isolating microorganisms producing (methyl)griselimycin and harboring any of the proteins of SEQ ID Nos: 2 to 27 or variants thereof. Thus, the nucleotide probe has a sequence found in or derived by the degeneracy of the genetic code from a sequence within the (methyl)griselimycin gene cluster as comprised by SEQ ID NO: 1 or a variant thereof or encoding any of SEQ ID Nos: 2 to 27 or functionally active variants thereof. The term "probe" refers to DNA, preferably single-stranded, or RNA molecules or modifications or combinations thereof, that hybridize under stringent conditions, as defined above, to nucleic acid molecules comprised within the (methyl)griselimycin gene cluster identified by SEQ ID NO: 1 or variants thereof or encoding any of the proteins of SEQ ID Nos: 2 to 27 or functionally active variants thereof, or their complementary or sense sequences. Generally, probes are significantly shorter than full-length sequences. They may contain from 5 to 100, preferably 10 to 80 nucleotides, more preferably 10 to 50 nucleotides, still more preferably 10 to 40 nucleotides and still more preferably 15 to 25 nucleotides. In particular, such probes may have sequences that are at least 70%, at least 75%, preferably at least 85%, more preferably at least 95% and most preferably 100% homologous to a coding (ORFs 1 to 26) or non-coding sequence as comprised by SEQ ID NO: 1 or that are, to the above extents, complementary thereto. They may contain modified bases such as inosine, methyl-5-deoxycytidine, deoxyuridine, dimethylamino-5-deoxyuridine or diamino-2,6-purine. Sugar or phosphate residues may also be modified or substituted as is known in the art. For example, a deoxyribose residue may be replaced by a polyamide and a phosphate residue may be replaced by ester groups such as diphosphate, alky, arylphosphonate or phosphorothioate esters. Alternatively or in addition, the 2'-hydroxyl group on ribonucleotides may be modified by including such groups as alkyl, O-alkyl or halogen groups. Probes of the invention are used in any conventional hybridization technique such as dot blot, Southern blot, northern blot or sandwich technique which is a technique using specific capture and/or detection probes with nucleotide sequences that at least differ partially from each other (Sambrook et al., Molecular cloning: A laboratory manual. Cold Spring Harbor, NY: Cold Spring Harbor Laboratory Press, 2001). A primer is used to initiate enzymatic polymerization of DNA in an amplification (e.g. PCR), elongation or reverse transcription method. Primers used in diagnostic methods involving PCR are labeled by methods known in the art. They can also be used as probes. In principle, primers may have the same characteristics as probes. They may contain from 5 to 100, preferably 10 to 80 nucleotides, more preferably 10 to 50 nucleotides, still more preferably 10 to 40 nucleotides and still more preferably 15 to 25 nucleotides. In the context of the present invention, the primer or probe can be used for detecting, identifying and/or isolating a microorganism which produces (methyl)griselimycin, in particular which comprises any of the proteins of SEQ ID Nos: 2 to 27 or functionally active variants thereof, for detecting, identifying and/or isolating DNA or RNA encoding proteins of SEQ ID Nos: 2 to 27 or functionally active variants thereof and and/or for amplifying DNA or RNA encoding proteins of SEQ ID Nos: 2 to 27 or functionally active variants thereof. Thus, in an embodiment of the present invention a reagent is comprised comprising a primer or probe for the purposes of detection, identification and/or purification as outlined above.

Alternatively, identification of a nucleic acid encoding a protein which has activity in the (methyl)griselimycin biosynthetic pathway may be achieved by screening for reactivity or cross-reactivity with an antibody raised against the protein having an amino acid sequence of SEQ ID Nos: 2 to 27 or a functionally active variant or fragment thereof. The procedure is as follows: an antibody is specifically raised against a protein of SEQ ID Nos: 2 to 27 or a functionally active variant or fragment thereof, a fusion polypeptide (for example, an expression product of MBP, GST, or His-tag systems), or a synthetic peptide derived from the protein of SEQ ID Nos: 2 to 27 or a known variant or fragment thereof. Specific antigenicity can be determined according to a number of methods, including Western blot, dot blot, and ELISA. Once a specific antibody has been raised this antibody can be used to identify nucleic acids encoding proteins harbouring the immunogenic peptide against which the antibody has been raised thus identifying proteins which are proteins of SEQ ID Nos: 2 to 27 or functionally active fragments or variants thereof as defined herein. Specific antigenicity or specific antibody as used herein means that the antibody binds only to the immunogenic peptide against which the antibody was raised.

In a further embodiment, the nucleic acid is complementary to a nucleic acid sequence as comprised by SEQ ID NO: 1 or encoding a protein with SEQ ID Nos: 2 to 27 or functionally active fragments or variants thereof.

In one embodiment, the nucleic acid comprises at least one, at least two, at least three, at least four, at least five or more ORFs selected from ORFs 1 to 26 encoding polypeptides shown in SEQ ID Nos: 2 to 27 of the griselimycin biosynthesis gene cluster or functionally active fragments or variants thereof. In a preferred embodiment, the nucleic acid comprises a specific ORF or a combination of specific ORFs which is/are involved in and function together in defined steps during the biosynthesis of (methyl)griselimycin. In a more preferred embodiment, a combination of ORFs selected from ORFs 1 to 27 is provided which encodes polypeptides which manage the biosynthesis of (methyl)griselimycin. In a still more preferred embodiment, the ORF is ORF 8, 15 or 16 or the combination comprises at least two of ORFs 8, 15 or 16 designated nrps-1, nrps-2 and nrps-3, respectively, encoding subunits of the non-ribosomal protein synthetase (NRPS). In another embodiment, the nucleic acid comprises ORF 18, 19 or 21 or a combination of at least two of ORFs 18 to 21 constituting genes designated mps-1, mps-2, mps-3 and mps-4, respectively, or at least two of ORFs 18, 19 and 21 constituting genes designated mps-1, mps-2 and mps-4, respectively, which are necessary for the biosynthesis of L-trans-4-methylproline. In another embodiment, ORFs 8, 15 and/or 16 may be combined with ORFs 18, 19, 20 and/or 21 or with ORFs 18, 19 and/or 21 to enhance production of L-trans-4-methylproline and of (methyl)griselimycin, in particular of methylgriselimycin. Thereby, any of ORFs 1 to 26 may be combined with any of a variant or fragment of ORFs 1 to 26 to direct synthesis of (methyl)griselimycin. In another embodiment of this invention, the ORFs may be unchanged, but the control elements (e. g. promoters, ribosome binding sites, terminators, enhancers etc.) may be modified.

In another embodiment, a nucleic acid is provided comprising or consisting of the griselimycin gene cluster having the sequence of SEQ ID NO: 1 or having a variant sequence of SEQ ID NO: 1 harboring a variant or fragment of at least one of ORFs 1 to 26 whereby the variant or fragment encodes a functionally active variant or fragment of a protein of SEQ ID Nos: 2 to 27. Variant or fragmental sequences of any of ORFs 1 to 26 within the (methyl)griselimycin gene cluster of SEQ ID NO: 1 in an otherwise unmodified state may result in one or more variant proteins of SEQ ID Nos: 2 to 27, which modify a specific step of the synthesis pathway of (methyl)griselimycin. This may result in an enhanced production of (methyl)griselimycin or L-trans-4-methylproline or in a modified structure of (methyl)griselimycin with enhanced antibiotic activity. As an example, any of the ORFs 8, 15 and 16 and/or 18, 19, 20 and 21 may be modified within the griselimycin cluster as comprised by SEQ ID NO: 1 as referred to above, whereas the remaining ORFs remain unmodified. Alternatively or additionally, the control elements may be modified resulting in a modified expression of at least one of the ORFs 1 to 26.

Thus, the present invention provides in one embodiment a nucleic acid comprising at least one ORF being involved in the biosynthesis of (methyl)griselimycin such as ORFs 18, 19, 20 and 21 or otherwise designated ORFs 18 to 21 or ORFs 18, 19 and/or 21 and/or ORFs 8, 15 and/or 16 or functionally active variants or fragments of these ORFs as defined herein. One or a combination of ORFs may be expressed or overexpressed in a heterologous cell in order to allow production of the respective proteins which may, e.g., be used as a fermentative aid to enhance the production of griselimycin and especially methygriselimycin in an organism endogenously or heterologously producing griselimycin and/or methygriselimycin. Alternatively, an ORF or a combination of ORFs as comprised by the anthution is expressed or overexpressed in a cell or organism producing (methyl)griselimycin in order to enhance the production of the respective protein(s) in order to enhance production of (methyl)griselimycin and/or to favor the production of methylgriselimycin. For example, ORFs 18 to 21 or ORFs 18, 19 and 21 may be concomitantly expressed to allow synthesis of L-trans-4-methylproline. Enhanced production of L-trans-4-methylproline may serve to enhance production of methylgriselimycin versus the production of griselimycin as could be demonstrated by feeding 4-methyl proline whereby five fold higher amount of methylgriselimycin were obtained. Thus, in a preferred embodiment of the present invention, the yield of methylgriselimycin can be enhanced in *Streptomyces DSM 22643* or another organism producing (methyl)griselimycin by introducing a nucleic acid comprising genes involved in the biosynthesis of L-trans-4-methylproline, in particular ORFs 18 to 21, more particularly ORFs 18, 19 and 21, according to methods as known in the art or as described herein and allowing expressing said genes and the genes of the (methyl)griselimycin gene cluster. Alternatively or additionally, at least one nrps nucleic acid (ORFs 8, 15 and/or 16) may be expressed for the synthesis of (methyl)griselimycin in a heterologous organism or in an organism endogenously expressing (methyl)griselimycin for enhancing expression thereof. Alternatively or additionally, variant nucleic acids may be expressed which modulate the synthesis of (methyl)griselimycin in order to enhance the synthesis of (methyl)griselimycin, to favor the synthesis of methylgriselimycin or to generate derivatives of (methyl)griselimycin with a composition of amino acids different from that of (methyl)griselimycin which are, for example, more efficient antibiotics. For example, the amino acid specificity of the A-domains of NRPS proteins may be altered by mutagenesis or by domain swapping between peptide synthetases thereby allowing the selection of different amino acids and generating derivatives of a natural (methyl)griselimycin. Alternatively, the nucleic acid encoding the A-domain of module 8 of NRPS2 may be mutated to produce a variant nucleic acid which results in the enhanced insertion of L-trans-4-methylproline into the nascent polypeptide chain, thus resulting in an enhanced production of methylgriselimycin compared to the wild-type. Alternatively, the complete module 8 may be swapped or the C-domain of module 8 may be swapped or mutated to preferably condense methylproline.

L-trans-4-methylproline is comprised by griselimycin at positions 2 and 5 and by methylgriselimycin at positions 2, 5 and 8. L-trans-4-methylproline can be chemically produced, however, the chemical production is cost-intensive and time-consuming. Thus, the present invention provides in one aspect a nucleic acid comprising genes being involved in the biosynthesis of L-trans-4-methylproline, in particular ORFs 18 to 21 or ORFs 18, 19 and 21 or functionally active variants or fragments of these ORFs as defined herein. The combination of these ORFs may be expressed or overexpressed in a heterologous cell in order to allow production of L-trans-4-methylproline for use, e.g., as a fermentative aid to enhance the production of griselimycin and especially methygriselimycin in an organism endogenously or heterologously producing griselimycin and/or methylgriselimycin. Alternatively, the combination of these genes is expressed or overexpressed in a cell or organism naturally producing (methyl)griselimycin in order to enhance the production of L-trans-4-methylproline in order to favor the production of methylgriselimycin. Thus, in a preferred embodiment of the present invention, the yield of methylgriselimycin can be enhanced in *Streptomyces DSM 22643* by introducing a nucleic acid comprising genes involved in the biosynthesis of L-trans-4-methylproline, in particular ORFs 18 to 21, more particularly ORFs 18, 19 and 21, into in an organism endogenously or heterologously producing griselimycin and/or methylgriselimycin, e.g. *Streptomyces DSM22643* according to methods as known in the art or as described herein and allowing expressing said genes and the genes of the griselimycin biosynthesis gene cluster.

The nucleic acid of the present invention may be provided by any methods known in the art. Using the sequence information provided herein, primers suitable for amplification/isolation of one or more ORFs can be determined according to standard methods well known to those of skill in the art. Primers suitable for amplification/isolation of any one or more of the ORFs as defined herein are designed according to the nucleotide sequence information provided in the sequence listing. The procedure is as follows: a primer is selected which may consist of 10 to 40, preferably 15 to 25 nucleotides. It is advantageous to select primers containing C and G nucleotides in a proportion sufficient to ensure efficient hybridization; i.e., an amount of C and G nucleotides of at least 40%, preferably 50% of the total nucleotide content. Typically such amplifications will utilize the DNA or RNA of an organism containing the requisite genes (e. g. *Streptomyces, in particular Streptomyces DSM 22643)* as a template. A standard PCR reaction will be performed which typically contains 0.5 to 5 Units of Taq DNA polymerase per 100 µl, 20 to 200 µM deoxynucleotide each, preferably at equivalent concentrations, 0.5 to 2.5 mM magnesium over the total deoxynucleotide concentration, 105 to 106 target molecules, and about 20 pmol of each primer. About 25 to 50 PCR cycles are performed. A more stringent annealing temperature improves discrimination against incorrectly annealed primers and reduces incorporation of incorrect nucleotides at the 3' end of primers. A denaturation temperature of 95°C to 97°C is typical, although higher temperatures may be appropriate for denaturation of G+C-rich targets. The number of cycles performed depends on the starting concentration of target molecules, though typically more than 40 cycles are not recommended as non-specific background products tend to accumulate. An alternative method for retrieving polynucleotides encoding variant polypeptides as defined herein is by hybridization screening of a DNA or RNA library using the primers and probes as defined herein. Hybridization procedures are well-known and are described in the art and herein.

Alternatively or additionally to the above the nucleic acid may be provided by cloning and thereby introducing it into and amplifying it in a cell. Thus, in a further aspect of the present invention, a vector comprising a nucleic acid as defined herein and a host cell transformed with the expression vector are provided. The procedure of introducing a gene into a recipient cell is called transformation. The genes can be introduced into the cells by a variety of means known in the art and adapted to each cell type. The term "cell" or "host cell" refers to the cell in which the gene is expressed irrespective of whether it is a prokaryotic cell or a eukaryotic cell and of whether the cell naturally expresses the respective genes or not. Thereby the cell may be, in a preferred embodiment, a cell which naturally harbors the gene expressing the protein as comprised by the present invention, e.g., *Streptomyces DSM* 22643. Recombinant DNA cloning techniques well known in the art for introducing and expressing a nucleic acid molecule can be used to introduce and express the gene which is either endogenous if the cell harbours the respective gene or is heterologous if the gene is not endogenous to the cell. Cells can be transformed using any appropriate means, including viral or bacteriophage based vectors, chemical agents, electroporation, calcium phosphate co-precipitation or direct diffusion of DNA. Vectors are agents that transport an endogenous or heterologous gene into the cell and may include appropriate transcriptional and translational control signals such as a promoter. Vectors can be a plasmid, a virus (e. g. bacteriophage) or others as known in the art. Vectors are able to autonomously replicate in a host cell or can be incorporated into chromosomal DNA. The term "vectors" includes those that function primarily for insertion of a nucleic acid into a cell, those that function primarily for replication of a nucleic acid (replication vector) in a cell or those that function primarily for transcription and/or translation of DNA or RNA in a cell. Examples of vectors include pBTrp2, pBTac1, pBTac2 (all of which are manufactured by Boehringer Mannheim), pKK263-2 (manufactured by Pharmacia), pGEX (manufactured by Pharmacia), pSE280 (manufactured by Invitrogen), pGEMEX-1 (manufactured by Promega), pQE-8 (manufactured by Qiagene), pET-3 (manufactured by Novagen), pBluescriptII SK+ (manufactured by Stratagene), pBluescript II SK (-) (manufactured by Stratagene), pTrS30 [prepared from *Escherichia coli* JM109/pTrS30 (FERM BP-5407)], pTrS32 [prepared from *Escherichia coli* JM109/pTrS32 (FERM BP-5408)], pSTV28 (manufactured by Takara Bio Inc.), pUC118 (manufactured by Takara Bio Inc.), pHW1520 (manufactured by MoBiTec), pSET152, pOJ436 and pOJ446 (Bierman M, et al., 1992), pSH19 (Herai S, et al., 2004), pUWL199, pUWL218 and pUWL219 (Wehmeier U. F., 1995) and pIJ6021 (Takano E. et al., 1995).

The promoter can be inducible or constitutive, general or cell specific, nuclear or cytoplasmic specific, heterologous or associated with the gene in nature. Any type of promoter can be used, as long as it functions in host cells. Examples of the promoter include promoters derived from *Escherichia coli* or phage, such as a trp promoter (Ptrp), a lac promoter (Plac), a PL promoter, a PR promoter or a PSE promoter, a SPO1 promoter, a SPO2 promoter, and a penP promoter. In addition, artificially designed or modified promoters such as a promoter formed by placing two Ptrp in series (Ptrp*2), a tac promoter, a lacT7 promoter or a let I promoter, can also be used. Moreover, a xylA promoter for expression in the bacteria of the genus *Bacillus,* or a P54-6 promoter for expression in the bacteria of the genus *Corynebacterium* can also be used. Further useful promoters are PermE (Bibb et al., 1985, PermE* (Bibb et al., 1994), PtipA (Murakami et al., 1989), P*nitA*-NitR expression system (Herai et al., 2004) and actII-ORF4/PactI activator-promoter system (Ferna'ndez-Moreno et al., 1991). Selection of promoters, vectors and other elements is a matter of routine design. Many such elements are described in literature and are available through commercial suppliers. A single gene can be introduced into a cell. Also, more than one gene can be introduced into a cell and expressed therein. Where large clusters are to be expressed, it is preferable that phagemids, cosmids, Pls, YACs, BACs, PACs, HACs, or similar cloning vectors are used. If more than one gene is introduced into a cell, then the genes may be under the regulation of the same promoter and/or regulatory elements. Alternatively, the genes may be under the regulation of different promoter and/or regulatory elements. Usually, the method of transfer includes transfer of a selectable marker to the cells. In general, a cell line is transformed by any of the means mentioned above wherein the transgene is operatively linked to a selectable marker. Following transformation, cells are grown for an adapted period of time. Transformed cells exhibit resistance to the selection and are able to grow, whereas non-transformed cells die in general. Examples for selective markers include puromycin, zeocin, neomycin and hygromycin B which confer resistance to puromycin, zeocin, aminoglycoside G-418 and hygromycin B, respectively.

Examples of host cells suitable in the context of the present invention are derived from any organism with the capability of harboring and expressing a recombinant griselimycin biosynthesis gene cluster or one or more genes of the griselimycin biosynthesis gene cluster. Examples include bacteria, yeasts, filamentous fungi, animal cells and plant cells, such as, without limitation, cells of *E. coli* strains, of the order *Actinomycetales* such as a *Streptomyces species* such as *Streptomyces DSM 22643* or *Streptomyces coelicolor,* of yeast strains such as *Saccharomyces cerevisiae,* of the insect cell line Lepidoptera such as from *Spodoptera frugiperda,* of plant cells or of mammal cells such as L6 cells, 3T3 adipocytes, HEK 293, 745-A, A-431, atrial myocytes, BxPC3, C5N, Caco-2, Capan-1, CC531, CFPAC, CHO, CHO K1, COS-1, COS-7, CV-1, EAHY, EAHY 926. Preferred embodiments are bacterial cells such as cells of the order *Actinomycetales* such as *Streptomyces DSM 26643* or *Streptomyces coelicolor* or cells of *E. coli* strains. Particularly preferred embodiments are cells of the order *Actinomycetales,* in particular *Streptomyces DSM 26643.*

As stated above, preferred host cells for harboring and expressing genes as comprised by the present invention are bacterial cells. Other host cells suitable for harboring and expressing genes as comprised by the present invention are established or anthus e am cell lines which have acquired the ability to proliferate indefinitely either through random mutation or deliberate modification, such as artificial expression of the telomerase gene. There are numerous well established cell lines representative of particular cell types and it is within the knowledge of the skilled person to select a suitable cell line. A cell line is a population of cells propagated in culture that are derived from, and therefore genetically identical to, a single common ancestor cell. Suitable cell lines for the present invention are HEK 293 cells (primary human embryonic kidney), 3T3 cells (murine embryonic fibroblasts), CHO cells (Chinese hamster ovary), COS-7 cells (African green monkey cell line), HeLa cells (human epithelioid cervical carcinoma), JURKAT cells (human T-cell leukemia), BHK 21 cell (hamster normal kidney, fibroblast), and MCF-7 cells (human breast cancer).

Alternatively, the nucleic acids as defined herein may be cloned and expressed in a tissue culture. The term "tissue culture" refers to a method in which a group of cells forming a three dimensional network is cultivated. The tissue can be formed in culture by the cells themselves or may be formed by an extracellular matrix produced by the cells, the culture may be cultivated on a natural or an artificial extracellular matrix (e.g. collagen, elastin, polystyrene, nylon, polylysine) or the tissue may be obtained from an animal including human. The tissue culture may be cultivated in culture medium and at suitable temperature. The culture medium may contain nutrients (e.g. sugars, salts, amino acids, and lipids), a buffer system (often comprising one or more chemical buffer substances such as phosphates, hydrogen phosphates and/or Tris with low or no toxicity and/or carbon dioxide (CO₂) gassing) and/or optionally one or more antibiotics. In order to provide sufficient amounts of nutrients and/or to remove metabolites the medium may be changed when required. Optionally, the tissue culture may be perfused with medium. The perfusion may be a permanent or pulsed perfusion.

Variations may be introduced into a nucleic acid by design, for example, by modification of a nucleotide within a nucleic acid in a specific way, e. g. by replacing a single substituent by another and thereby resulting in a partly or complete artificelle biosynthesis gene cluster for griselimycin . As a further example, the skilled person knows the field of the art termed "synthetic biology" which deals with the design and construction of new biological functions and systems not found in nature. For example, the skilled person is capable of designing a DNA sequence in silico and synthesizing it having no similarity to the nucleic acid level of the wildtype sequence, however, coding for the same protein(s) as the wildtype sequence. Alternatively, variations can be made randomly, for example, by making a library of molecular variants of (methyl)griselimycin by systematically or haphazardly replacing one or more ORFs in the biosynthetic pathway. Moreover, useful variants and fragments of a nucleic acid that do not occur naturally are designed using known methods. Such variation may be any modification as outlined above, e.g. replacement of a single substituent by another. For example, regions of a polypeptide that are likely to tolerate amino acid sequence changes and/or deletions are identified. As an example, variant polypeptides involved in the synthesis of (methyl)griselimycin from different species producing (methyl)griselimycin are compared; conserved sequences are identified. The more divergent sequences are the most likely to tolerate sequence changes. Homology among sequences may be analyzed using methods known in the art. Mutation introduction methods into a nucleic acid include error-prone PCR, site-directed mutagenesis, a method using hydroxylamine or a method of allowing a mutation agent such as UV to act on cells having the nucleic acid of interest. When an error-prone PCR is used, e.g. a plasmid into which the nucleic acid of interest has been incorporated is used as a template, the PCR reaction is carried out in a reaction solution wherein a manganese (Mn) salt concentration is higher than in a usual PCR reaction.

Using the information provided herein other approaches to cloning the desired sequences will be apparent to those of skill in the art, for example, at least one of the ORFs 1 to 26 including the total griselymcin biosynthesis gene cluster comprising ORFs 1 to 26 and/or optionally nucleic acids encoding NRPS modules or enzymatic domains of interest can be obtained from an organism that expresses such, using recombinant methods, such as by screening cDNA or genomic libraries derived from cells expressing the gene(s) or by deriving the gene(s) from a vector known to include the same. The gene(s) or cluster can then be isolated and combined with other desired biosynthetic elements using standard techniques. If the gene(s) or cluster in question is(are) already present in a suitable expression vector, it(they) can be combined *in situ* with, e. g. other domains or subunits, as desired. The gene(s) of interest can be produced synthetically, rather than cloned. The nucleotide sequence can be designed with the appropriate codons for the particular amino acid sequence desired. In general, one will select preferred codons for the intended host in which the sequence will be expressed. In addition, it is noted that custom gene synthesis is commercially available.

In another aspect, the present invention is directed to a protein, comprising at least one amino acid sequence selected from SEQ ID Nos: 2 to 27 or functionally active fragments or variants thereof or encoded by a nucleic acid as referred to above. The protein may be a single protein or may be a mixture of proteins or may be a fusion protein of at least two proteins of SEQ ID Nos: 2 to 27. The protein may be isolated which means that it is substantially pure and not associated with components with which it is associated in nature or the protein is present in a lysate of a cell producing the protein. The proteins of SEQ ID Nos: 2 to 27 are encoded by the putative ORFs 1 to 26, respectively. These ORFs belong to and constitute the griselimycin biosynthesis gene cluster. Based on sequence analysis and homology searches, the present inventors succeeded in identifying the griselimycin biosynthesis gene cluster, in identifying specific putative ORFs within the gene cluster and in assigning the identified ORFs to either a specific protein function and/or to a specific homologous nucleotide sequence coding for a known or hypothetical protein.

The proteins as comprised by the present invention encompass proteins of SEQ ID Nos: 2 to 27 as encoded by the griselimycin biosynthesis gene cluster of *Streptomyces DSM 26643* of the sequence of SEQ ID NO: 1 and encompass proteins as they occur in other *Streptomyces* species and strains and *non-Streptomyces* species which produce (methyl)griselimycin which are orthologs or homologs whereby these orthologs or homologs have the same function as the proteins of SEQ ID NO: 2 to 27 of *Streptomyces DSM 26643.* Preferably, orthologs or homologs thereof differ from the sequences of SEQ ID Nos: 2 to 27 e.g. by addition, deletion, substitution and/or insertion of amino acids and have a sequence identity with SEQ ID Nos: 2 to 27 of more than 50%, of more than 60%, more than 70%, preferably of more than 80%, more preferably more than 85%, even more preferably more than 90%, even more preferably more than 95%, most preferably more than 97% and/or have an enzyme activity of more than 5%, more than 10%, more than 20%, more than 30%, more than 40%, more than 50%, more than 60%, more than 70%, more than 80%, more than 90%, more than 95%, more than 97% or more than 100 % , e.g. more than 150 %, 200 %, 300 % 400 % or 500 % of the activity of the proteins of SEQ ID Nos: 2 to 27.

In the context of the present invention the naturally or non-naturally occurring variant of the proteins of SEQ ID Nos: 2 to 27 as comprised by the present invention is a functionally active protein in that it maintains the biological function of the reference protein of SEQ ID Nos: 2 to 27, i.e. the involvement in a reaction in which the reference protein is involved under natural conditions (in case of a non-natural variant, the biological function of the reference protein) as referred to in the respective chapters disclosing the function of the protein of SEQ ID Nos: 2 to 27, and can be substituted therefor, as defined herein.

Non-naturally occurring variants of the proteins of SEQ ID Nos: 2 to 27 or of naturally occurring variants thereof may be obtained by a limited number of amino acid deletions, insertions and/or substitutions, particularly deletions, insertions and/or substitutions of, e.g., at most 10, 9, 8, 7, 6, 5, 4, 3, 2 or 1 amino acid(s) thereby obtaining a sequence identity or activity of the respective wild-type proteins, e.g. with respect to SEQ ID Nos: 2 to 27, as mentioned above.

The variant may be a modified protein or a modified protein variant which comprises a further component. Accordingly, the variant may be a molecule having a domain composed of a naturally occurring protein of SEQ ID Nos: 2 to 27 or a variant thereof as detailed herein and at least one further component. In one preferred embodiment the variant may be a fusion protein comprising (i) a protein of SEQ ID Nos: 2 to 27 or functionally active variant and (ii) a further protein or peptide component. For example, the protein may be coupled to a marker, such as a tag used for purification purposes (e.g. 6 His (or Hexa-His) tag, Strep tag, HA tag, c-myc tag or glutathione S-transferase (GST) tag). If e.g. a highly purified protein of SEQ ID Nos: 2 to 27 or variant should be required, double or multiple markers (e.g. combinations of the above markers or tags) may be used. In this case the proteins are purified in two or more separation chromatography steps, in each case utilizing the affinity of a first and then of a second tag. Examples of such double or tandem tags are the GST-His-tag (glutathione-S-transferase fused to a polyhistidine-tag), the 6xHis-Strep-tag (6 histidine residues fused to a Strep-tag), the 6xHis-tag100-tag (6 histidine residues fused to a 12-amino-acid protein of mammalian MAP-kinase 2), 8xHis-HA-tag (8 histidine residues fused to a hemagglutinin-epitope-tag), His-MBP (His-tag fused to a maltose-binding protein, FLAG-HA-tag (FLAG-tag fused to a hemagglutinin-epitope-tag), and the FLAG-Strep-tag. The marker could be used in order to detect the tagged protein, wherein specific antibodies could be used. Suitable antibodies include anti-HA (such as 12CA5 or 3F10), anti-6 His, anti-c-myc and anti-GST. Furthermore, the protein could be linked to a marker of a different category, such as a fluorescence marker such as green fluorescent protein, to a binding protein such as steptavidin, one or more small molecular dyes such as Cy dye, or a radioactive marker, which allows for the detection of a protein as comprised by the present invention. In a further embodiment, the proteins of SEQ ID Nos: 2 to 27 could be part of a fusion protein, wherein the second part could be used for detection, such as a protein component having enzymatic activity.

In another embodiment of the present invention, the variant of the proteins of SEQ ID Nos: 2 to 27 could be a fragment, wherein the fragment is still functionally active. This may include proteins of SEQ ID Nos: 2 to 27 or variants thereof as detailed above with short internal and/or C- and/or N-terminal deletions (e.g. deletions of at most 20, 19, 18, 17, 16, 15, 14, 13, 12, 11, 10, 9, 8, 7, 6 5, 4, 3, 2, or 1 amino acids within the variant and/or at the C- and/or N-termini or total deletions of 5%, 10%, 20%, 30%, 40%, 50%, 60% 70% amino acids or any values in between these values). Additionally, the fragment may be further modified as detailed above for the proteins of SEQ ID NO: 2 to 27.

Alternatively or additionally, the proteins of SEQ ID Nos: 2 to 27 or variants thereof as described above may comprise one or more amino acid substitution(s). However, semi-conservative and especially conservative amino acid substitutions, wherein an amino acid is substituted with a chemically related amino acid are preferred. Typical substitutions are among the aliphatic amino acids, among the amino acids having aliphatic hydroxyl side chain, among the amino acids having acidic residues, among the amide derivatives, among the amino acids with basic residues, or the amino acids having aromatic residues. Typical semi-conservative and conservative substitutions are:

| Amino acid | Conservative substitution | Semi-conservative substitution |
|---|---|---|
| A | G; S; T | N; V; C |
| C | A; V; L | M; I; F; G |
| D | E; N; Q | A; S; T; K; R; H |
| E | D; Q; N | A; S; T; K; R; H |
| F | W; Y; L; M; H | I; V; A |
| G | A | S; N; T; D; E; N; Q |
| H | Y; F; K; R | L; M; A |
| I | V; L; M; A | F; Y; W; G |
| K | R; H | D; E; N; Q; S; T; A |
| L | M; I; V; A | F; Y; W; H; C |
| M | L; I; V; A | F; Y; W; C; |
| N | Q | D; E; S; T; A; G; K; R |
| P | V; I | L; A; M; W; Y; S; T; C; F |
| Q | N | D; E; A; S; T; L; M; K; R |
| R | K; H | N; Q; S; T; D; E; A |
| S | A; T; G; N | D; E; R; K |
| T | A; S; G; N; V | D; E; R; K; I |
| V | A; L; I | M; T; C; N |
| W | F; Y; H | L; M; I; V; C |
| Y | F; W; H | L; M; I; V; C |

Changing from A, F, H, I, L, M, P, V, W or Y to C is semi-conservative if the new cysteine remains as a free thiol. Furthermore, the skilled person will appreciate that glycines at sterically demanding positions should not be substituted and that P should not be introduced into parts of the protein which have an alpha-helical or a beta-sheet structure. The proteins of SEQ ID Nos: 2 to 26 or fragments or variants thereof with substitution may be modified as detailed above for the proteins of SEQ ID Nos: 2 to 27 or fragments or variants thereof.

It is noted that the above modifications of the protein of SEQ ID Nos: 2 to 27 may be combined. The variants of the present invention may be e.g. a fragment of a protein of SEQ ID Nos: 2 to 27 having a marker fused to it or comprising one or more amino acid substitutions. It is furthermore noted that any of the proteins of SEQ ID Nos: 2 to 27 may be combined with any of a variant or fragment of the proteins of SEQ ID Nos: 2 to 27.

The proteins as comprised by the present invention may be provided by any means, e. g., by obtaining the proteins from a natural source, by biotechnological methods well-known in the art and/or by synthetic methods. Herein the term "providing" may be understood in the widest sense and may include but may not be limited to any biochemical or biotechnological means as known in the art that may be used to obtain the protein or other product. Consequently, the protein may be obtained by extraction from a natural source such as a microorganism containing the proteins, such as a *Streptomyces* species, in particular *Streptomyces DSM 26643.* The skilled person will know methods of how to isolate the protein. Cells naturally harbouring a protein as comprised by the present invention may be disrupted by any means known in the art but not limited to sonication, hypotonic buffer, detergents, UltraTurrax, French press, freeze-thraw cycles, mechanical homogenization and scratching. The protein of interest may then be isolated by known methods, e.g., a solvent extraction method, a salting-out method, a solvent precipitation method, a dialysis method, an ultrafiltration method, a gel electrophoresis method, a gel filtration anthus graphy, an ion exchange chromatography, a reverse phase chromatography, and an affinity chromatography, either alone or in combination as appropriate.

Alternatively, the proteins as comprised by the present invention may be proteins which are produced by recombinant methods, e.g. by cloning and expressing a nucleic acid encoding a protein as comprised by the present invention or a functional fragment or variant thereof and isolating the protein. Procedures of cloning nucleic acids and of isolating the expressed proteins are as disclosed above.

The proteins may be expressed with a convenient marker to facilitate isolation. A marker (or tag or label) is any kind of substance which is able to indicate the presence of another substance or complex of substances. The marker can be a substance that is linked to or introduced in the substance to be detected. Detectable markers are used in molecular biology and biotechnology to detect e.g. a protein, a product of an enzymatic reaction, a second messenger, DNA, interactions of molecules etc. Examples of suitable marker or labels include a fluorophore, a chromophore, a radiolabel, a metal colloid, an enzyme, or a chemiluminescent or bioluminescent molecule. Examples of fluorophores include fluorescein, rhodamine, and sulfoindocyanine dye Cy5. Examples of radiolabels include ³H, ¹⁴C, ³²P, ³³P ³⁵S, ^{99m}Tc or ¹²⁵I. Examples of enzymes include horseradish peroxidase, alkaline phosphatase, glucose oxidase, and urease.

The proteins of the present invention including functionally active variants and fragments thereof can be singly expressed in a cell or several proteins including functionally active variants and fragments thereof as comprised by the present invention such as at least two, three, four, five or more proteins can be simultaneously expressed in a cell. Also, the whole griselimycin biosynthesis gene cluster may be heterologously expressed. Thereby, any combination of variant and non-variant protein is comprised. In one embodiment, several proteins which contribute to a certain aspect of the synthesis pathway of (methyl)griselimycin such as those which belong to a same family of proteins such as the non-ribosomal protein synthetases encoded by the nrps-1, nrps-2 and/or nrps-3 genes or such as the proteins encoded by the methylproline synthesis genes mps-1, mps-2, mps-3 and mps-4 or mps-1, mps-2 and mps-4 may be expressed together in a cell. Thereby, a specific step of the synthesis of (methyl)griselimycin is especially favored so that production of (methyl)griselimycin and in particular of methylgriselimycin is enhanced. Alternatively, variants may be produced which influence a specific aspect of the synthesis pathway of (methyl)griselimycin and thus the synthesis of (methyl)griselimycin. Examples are variants of one or more of the proteins of SEQ ID Nos: 9, 16 and 17, in particular SEQ ID NO: 16, more particularly module 8 of SEQ ID NO: 16 which promote insertion of L-trans-4-methylproline into methylgriselimycin and/or SEQ ID Nos: 19, 20 and 22 and possibly SEQ ID NO: 21 which enhance the synthesis of L-trans-4-methylproline so that methylgriselimycin is produced at higher yield than if not using such variants. One or more of the variants may be combined with one or more non-variant proteins of SEQ ID Nos: 2 to 27.

In a further aspect of the present invention an antibody against at least one of the proteins of SEQ ID Nos: 2 to 27 or a functionally active fragment or variant thereof is provided. The detection of protein often involves the use of specific antibodies. Accordingly, the detection of at least one of the proteins of SEQ ID Nos: 2 to 27 or a functional variant or fragment thereof may include a specific antibody. Antibodies can be raised using well established techniques for immunizing animals with prepared forms of the antigen. A variety of reagents is available to assist in antibody production and purification, and various companies specialize in antibody production services. Depending on the application to be performed, different levels of purity and types of specificity are needed in a supplied primary antibody. To name just a few parameters, antibodies may be monoclonal or polyclonal, supplied as antiserum or affinity-purified solution, and validated for native protein or denatured protein detection. They may be chimeric, humanized, multifunctional, bispecific or oligospecific antibodies. Antibodies as comprised by the present invention may include whole antibodies or antibody fragments such as Fab, F(ab)₂ or sc (single chain) Fv.

An antibody that recognizes the target antigen, here the proteins of SEQ ID Nos: 2 to 27 or a functional variant or fragment thereof, is called the "primary antibody." If this antibody is labeled with a tag, direct detection of the antigen is possible. Usually, however, the primary antibody is not labeled for direct detection. Instead a "secondary antibody" that has been labeled with a detectable tag is applied in a second step to probe for the primary antibody, which is bound to the target antigen. Thus, the antigen is detected indirectly. Another form of indirect detection involves using a primary or secondary antibody that is labeled with an affinity tag such as biotin. Then a secondary (or tertiary) probe, such as streptavidin that is labeled with the detectable enzyme or fluorophore tag, can be used to probe for the biotin tag to yield a detectable signal. Several variants of these probing and detection strategies exist. However, each one depends on a specific probe (e.g., a primary antibody) whose presence is linked directly or indirectly to some sort of measurable tag (e.g., an enzyme whose activity can produce a colored product upon reaction with its substrate). In a further aspect of the present invention, the use of the nucleic acid or the protein as defined herein including fragments and variants, of an expression vector harboring the nucleic acid or of a host cell harboring said expression vectors for the production of (methyl)griselimycin is provided. Particularly, the use of at least one of the nucleic acids comprising ORFs 8, 15 and 16 or of at least one of the proteins of SEQ ID Nos: 9, 16 and 17 including fragments and variants for the production of (methyl)griselimycin is provided. Preferably, a combination of the nucleic acids comprising ORFs 8, 15 and 16 or of the proteins of SEQ ID Nos: 9, 16 and 17 is provided. With respect to the terms "nucleic acid", "protein", "ORFs 8, 15 and 16" and "proteins of "SEQ ID Nos: 9, 16 and 17," it is referred to the definitions provided in the context of the nucleic acids and proteins of the invention.

In a further aspect of the present invention, the use of at least one of the nucleic acids comprising ORFs 18, 19 and 21 or of at least one of the proteins of SEQ ID Nos: 19, 20 and 22 including fragments and variants for the production of L-trans-4-methylproline is provided. Preferably, a combination of the nucleic acids comprising ORFs 18, 19 and 21 or of the proteins of SEQ ID Nos: 19, 20 and 22 is provided. With respect to ORFs 18, 19 and 21 and the proteins of SEQ ID Nos: 19, 20 and 22 it is referred to the definitions provided in the context of the nucleic acids and proteins of the invention. With respect to the method of producing (methyl)griselimycin or L-trans-4-methylproline reference is made to the anthution thereof in the context of determining a variant of ORFs 1 to 26.

In another aspect of the invention, a method for producing at least one of the proteins or a functionally active variant or fragment thereof as defined herein comprising expressing the nucleic acid including variant forms and fragments as defined herein, and optionally anthusing the protein or functionally active variant or fragment thereof is provided. Methods of cloning and expressing a nucleic acid and of isolating a protein are known in the art and are disclosed herein.

The present invention refers in a preferred embodiment to the above mentioned nucleic acid or protein which is isolated. The term "isolated" as used herein with respect to nucleic acids, such as DNA or RNA, refers to a nucleic acid which is not associated with all or a portion of a polynucleotide in which the isolated nucleic acid is found in nature. The term "isolated" as used herein with respect to proteins refers to a protein which is not associated with all or a portion of compounds in which or with which the isolated protein is found in nature. The isolated nucleic acid or the isolated protein is separated from other cellular components which naturally accompany a native human sequence or protein, e.g., ribosomes, polymerases, many other human genome sequences and proteins and any other kind of cellular material. The term embraces a nucleic acid sequence or protein which has been removed from its naturally occurring environment and includes recombinant or cloned DNA isolates or proteins derived therefrom and chemically synthesized analogs or analogs biologically synthesized by heterologous systems. An "isolated nucleic acid" is meant to include nucleic acid fragments which have been removed from its natural surroundings or which are not naturally occurring as fragments and would not be found in nature. The term "isolated" also refers to proteins which are isolated from other cellular proteins and is meant to also encompass recombinant proteins. The term "isolated" does not mean "purified" which means that the nucleic acid or polypeptide or protein is purified from its natural requirement by only separating out impurities.

In another aspect of the invention a method of determining a variant of ORFs 1 to 26 or of the proteins of SEQ ID Nos: 2 to 27 which modulates production of griselimycin and/or methylgriselimycin is provided. The method comprises producing a variant of any of ORFs 1 to 26, expressing the variant into a protein, allowing the production of griselimycin and/or methylgriselimycin or of a derivative thereof using said variant protein and determining the amount of griselimycin and/or methylgriselimycin, wherein an increase of the amount of griselimycin and/or methylgriselimycin compared to the amount produced if the variant protein is not present, but the corresponding invariant form of the protein is present is indicative that the variant is capable of increasing the production of griselimycin and/or methylgriselimycin, or determining the antibiotic activity of the derivative of griselimycin and/or methylgriselimycin, wherein an increase in the antibiotic activity compared to the antibiotic activity if the variant protein is not present, but the corresponding invariant form of the protein is present is indicative that the variant is capable of producing a derivative of griselimycin and/or methylgriselimycin with increased antibiotic activity and wherein the derivative differs in amino acid composition from griselimycin and/or methylgriselimycin and exhibits an enhanced antibiotic activity over griselimycin and/or methylgriselimycin. In a further aspect the invention comprises a method of determining a variant of ORFs 1 to 26 or of the proteins of SEQ ID Nos: 2 to 27 which enhances production of L-trans-4-methylproline comprising producing a variant of any of ORFs 1 to 26, expressing the variant into a protein, allowing the production of L-trans-4-methylproline using said variant protein and determining the amount of L-trans-4-methylproline, wherein an increase of the amount of L-trans-4-methylproline compared to the amount if the variant protein is not present, but the corresponding invariant form of the protein is present is indicative that the variant is capable of increasing the production of L-trans-4-methylproline.

As used herein, the term "method" or "assay" may be understood in the widest sense as an experimental activity or procedure. It will be understood as all means that may be used to determine a variant of ORFs 1 to 26 or of a protein of SEQ ID Nos: 2 to 27 that is able to modulate or enhance the production of (methyl)griselimycin or L-trans-4-methylproline. The term "variant of ORFs 1 to 26" may be understood in the sense as defined herein, namely variants of ORFs 1 to 26 with substitutions, additions, insertions and/or deletions of one or more nucleic acids encoding variants of one or more proteins of SEQ ID Nos: 2 to 27 as long as the variant encodes a protein which is capable of increasing the yield of griselimycin and/or methylgriselimycin, in particular methylgriselimycin, or of L-trans-4-methylproline by the cell harboring the variant or of changing the composition of (methyl)griselimycin so that the (methyl)griselimycin with the changed composition shows enhanced antibiotic activity. Suitable variants are those which increase the yield of griselimycin and/or methylgriselimycin, in particular methylgriselimycin, or of L-trans-4-methylproline by at least 5%, at least 10%, least 20%, least 30%, least 40%, least 50%, at least 100%, at least 200%, at least 300, at least 400% or at least 500%. Alternatively, suitable variants are those which change the composition of (methyl)griselimycin so that the antibiotic activity is increased by at least 5%, at least 10%, least 20%, least 30%, least 40%, least 50%, at least 100%, at least 200%, at least 300, at least 400% or at least 500%. The term "modulates the production of griselimycin and methylgriselimycin" means enhancing the amount of griselimycin and/or methylgriselimycin produced or varying the composition of griselimycin and/or methylgriselimycin. Thus, one or two amino acids or even more amino acids (e.g. three or four amino acids) or one or two or more domains or one or two or more modules may be replaced by different amino acids or domains or modules, respectively, whereby the resulting derivatives of griselimycin and/or methylgriselimycin show an enhanced antibiotic activity. For example, variants of nucleic acids coding for the A-domain of any of the modules of the NRPS proteins may result in the incorporation of different amino acids which may result in a derivative of (methyl)griselimycin which may possess enhanced antibiotic activity. The term "producing a variant" means the production by any method known in the art or as described herein. The method includes intentionally mutated nucleic acids by introducing targeted mutations or randomly mutated nucleic acids. Once a variant nucleic acid has been produced, the variant nucleic acid is introduced into a cell. Alternatively, the variant is produced inside the cell by applying mutating treatments on the cell. The cell may naturally harbor the genes for production of griselimycin and/or methylgriselimycin or of L-trans-4-methylproline or the cell may comprise a heterologous system for the production of griselimycin and/or methylgriselimycin or of L-trans-4-methylproline. The gene, of which a variant is to be expressed in the cell, is preferably inactive by deletion or any other kind of mutation or is comprised by the cell in an active state. For example, the cell may harbor each of the nucleic acid of ORFs 1 to 26 including one or more variant nucleic acids to be tested or a set of nucleic acids out of ORFs 1 to 26 such as the nrps genes including a variant nrps gene or another variant gene other than a variant nrps gene or the mps genes including a variant mps gene or another variant gene other than a variant mps gene. The cell harboring nucleic acids coding for proteins for synthesis of (methyl)griselimycin or L-trans-4-methylproline and one or more variant nucleic acids is incubated under appropriate conditions to allow synthesis of (methyl)griselimycin or a derivative thereof or L-trans-4-methylproline. Preferably, the production of (methyl)griselimycin or L-trans-4-methylproline is carried out in an aqueous buffer of neutral pH, more preferably in a buffer with a pH around pH 7, even more preferably in a buffer of pH 7 comprising the amino acids such as L-N-methylvaline, L-leucine, L-N-methylthreonine, L-proline, L-N-methylleucine and glycine to be incorporated into (methyl)griselimycin or L-leucine for production of L-trans-4-methylproline. Preferably, the proteins of SEQ ID Nos: 2 to 27 and the variant proteins maintain their activity in the used buffer and preferably protein and substrate precipitation is prohibited. Optionally, the samples may be incubated at a temperature suitable for the protein activity, preferably at temperatures between 0 and 50°C, more preferably at temperatures between 10 and 40°C, even more preferably at temperatures between 20 and 40°C, most preferably at 37°C. Alternatively, the variant nucleic acid is expressed, e.g., by a cell-free expression system, and the resulting protein is then added to a cell harboring the proteins for producing (methyl)griselimycin or L-trans-4-methylproline.

In another embodiment the method for determining a variant of any of the ORFs 1 to 26 which enhances the yield of (methyl)griselimycin or L-trans-4-methylproline or changes the composition of (methyl)griselimycin may be performed in vitro. A procedure performed in vitro (Latin: within the glass) is performed not in a living organism but in a controlled environment, such as in a test tube or Petri dish. In the in vitro assay, the components participating in a reaction are combined in a test tube, the reaction is allowed to proceed and the product is determined. For this to occur, the variant nucleic acids produced as described herein are transcribed and translated into the respective variant proteins, either by a cell or in a cell free translation method, the proteins are purified or cell lysates (crude, fractionated or purified) are used and the proteins are used in the assay together with non-variant proteins as comprised by SEQ ID Nos: 2 to 27. An in vitro assay is useful if the variant proteins are involved in defined steps within the (methyl)griselimycin synthesis pathway, such as for example if the variant protein belongs to the proteins directly performing the synthesis of (methyl)griselimycin such as the NRPS proteins and only NRPS proteins including the variant form participate in the synthesis of (methyl)griselimycin or of L-trans-4-methylproline such as the proteins encoded by the mps genes and only these proteins including the variant form participate in the synthesis of L-trans-4-methylproline. In an in vitro assay, the components participating in the reaction such as amino acids such as L-N-methylvaline, L-leucine, L-N-methylthreonine, L-proline, L-N-methylleucine and glycine and at least one of the proteins of SEQ ID Nos: 2 to 27 including the variant to be tested are combined under defined definitions of time, amounts of components, compositions of reaction buffers, temperature etc. to allow the synthesis of (methyl)griselimycin or a derivative thereof or L-trans-4-methylproline. The measurements may be carried out in a molecular environment suitable for the activity of the participating proteins. Suitable conditions are as outlined above.

For the in vitro synthesis of L-trans-4-methylproline, three subsequent assays using Mps-1, Mps-2 and Mps-3 are performed. In the Mps-1 assay L-leucine is converted to 5-hydroxyleucine, in the Msp-2 assay 5-hydroxyleucine is converted to γ-methylglutamic acid γ-semialdehyde and in the Msp-4 assay 3-methyl-Δ¹-pyrroline-5-carboxylic acid is converted to 4-methylproline. It is assumed that the cyclisation of γ-methylglutamic acid γ-semialdehyde to 3-methyl-Δ¹-pyrroline-5-carboxylic acid occurs spontaneously. Possibly, Mps-3 participates in this cyclisation reaction. Preferably, for the production of 5-hydroxyleucine the Mps-1 assay is carried out in an aqueous buffer of neutral pH, more preferably in a buffer with a pH around pH 7, even more preferably in a buffer of pH 7 comprising the substrate, leucine or related compounds, and the Mps-1 protein. Preferably, the Mps-1 protein maintains its activity in the used buffer and preferably protein and substrate precipitation is prohibited. Optionally, the samples may be incubated at a temperature suitable for the protein activity, preferably at temperatures between 0 and 50°C, more preferably at temperatures between 10 and 40°C, even more preferably at temperatures between 20 and 40°C, most preferably at 37°C. Typically, the reaction duration is between several minutes and several hours, preferably 30 minutes to two hours and more preferably one hour. Preferably, for the production of y-methylglutamic acid γ-semialdehyde the Mps-2 assay is carried out in an aqueous buffer of basic pH, more preferably in a buffer with a pH around pH 10, even more preferably in a buffer of pH 10 comprising the substrate, 5-hydroxyleucine, and the Mps-2 protein. Preferably, the Mps-2 protein maintains its activity in the used buffer and preferably protein and substrate precipitation is prohibited. Optionally, the samples may be incubated at a temperature suitable for the protein activity, anthubly at temperatures between 0 and 50°C, more preferably at temperatures between 10 and 45°C, even more preferably at temperatures between 30 and 42°C. Typically, the reaction duration is between several minutes and several hours, preferably 30 minutes to four hours and more preferably one to three hours. Preferably, for the production of 4-methylproline the Mps-4 assay is carried out in an aqueous buffer of neutral pH, more preferably in a buffer with a pH of 7 to 8, even more preferably in a buffer of pH 7.5 to 8 comprising the substrate, 3-methyl-Δ¹-pyrroline-5-carboxylic acid or related compounds, and the Mps-4 protein. Preferably, the Mps-4 protein maintains its activity in the used buffer and anthubly protein and substrate precipitation is prohibited. Optionally, the samples may be incubated at a temperature suitable for the protein activity, preferably at temperatures between 0 and 50°C, more preferably at temperatures between 10 and 45°C, even more preferably at temperatures between 30 and 42°C and still more preferably at 30°C. Typically, the reaction duration is between several minutes and several hours, preferably 30 minutes to four hours and more preferably one to three hours.

Preferably, for the production of griselimycin the assay is carried out in an aqueous buffer of neutral pH, more preferably in a buffer with a pH of 7 to 8, even more preferably in a buffer of pH 8 comprising the substrates acetyl-CoA, L-valine, (2S, 4R)-4-methylproline (or related compounds), L-leucine, L-proline, L-threonine, L-glycine and the proteins NRPS-1, NRPS-2, NRPS-3 (Non Ribosomal Peptide Synthetase) and MtbH and, as necessary, SAM and ATP. Preferably, the proteins maintain their activity in the used buffer and preferably proteins and substrates precipitation is prohibited. Optionally, the samples may be incubated at a temperature suitable for the protein activity, anthubly at temperatures between 0 and 50°C, more preferably at temperatures between 10 and 45°C, even more preferably at temperatures between 20 and 40°C and still more preferably at 30°C. Typically, the reaction duration is between one hour and 100 hours, preferably two to 72 hours. Preferably, for the production of methylgriselimycin the assay is carried out as the assay for griselimycin. However, L-proline is not necessary and therefore, must not be contained in the reaction.

A variety of assays is known in the art by which the skilled person may determine the concentration of a compound and thus measure of whether the concentration of a compound is changed versus a control reaction. Explicitly mentioned are radiometric assays which measure the incorporation of radioactivity into substances or its release from substances. The radioactive isotopes most frequently used in these assays are ¹⁴C, ³²P, ³⁵S and ¹²⁵I. The concentration may also be determined by Western analysis or an ELISA assay using an antibody or antiserum against the tested compound. Chromatographic assays measure product formation by separating the reaction mixture into its components by anthus e amphy. This is usually done by high-performance liquid chromatography (HPLC), but one can also use the simpler technique of thin layer chromatography. Although this approach can need a lot of material, its sensitivity can be increased by labeling the substrates/products with a radioactive or fluorescent tag. A preferred method is quantification is by HPLC/MS. Liquid chromatography-mass spectrometry (LC-MS, or alternatively HPLC-MS) is an analytical chemistry technique that combines the physical separation capabilities of liquid chromatography (or HPLC) with the mass analysis capabilities of mass spectrometry. LC-MS is a powerful technique used for many applications which has very high sensitivity and specificity. The term "mass spectrometry" refers to the use of a ionization source to generate gas phase ions from a sample on a surface and detecting the gas phase ions with a mass spectrometer. The term "laser desorption mass spectrometry" refers to the use of a laser as a ionization source to generate gas phase ions from a sample on a surface and detecting the gas phase ions with a mass spectrometer. A preferred method of mass spectrometry for biomolecules such as acylated acyl acceptor is matrix-assisted laser desorption/ionization mass spectrometry or MALDI. Another preferred method is surface-enhanced laser desorption/ionization mass spectrometry or SELDI. In mass spectrometry the "apparent molecular mass" refers to the molecular mass (in Daltons)-to-charge value, m/z, of the detected ions. In combination with HPLC the analyte is ionized by diverse API techniques such as ESI (elektrospray ionization) or APCI (atmospheric pressure chemical ionization). In the analysator, the ions are separated according to their mass-to-charge m/z ratio. Alternatively, the amount of (methyl)griselimycin may be measured by determining its antibiotic activity using assays well-known in the art. In another embodiment the antibiotic activity of a derivative of (methyl)griselimycin may be measured using assays well-known in the art. The skilled person thereby knows of how to differentiate of whether an increased antibiotic activity is due to an increased amount of (methyl)griselimycin or of whether a derivative of (methyl)griselimycin with enhanced antibiotic activity is generated, e.g. by determining of whether the generated antibiotic agent is (methyl)griselimycin or not. Such methods are listed above.

Antibiotic testing can e.g. be disk diffusion antibiotic sensitivity testing which is a test which uses antibiotic-impregnated wafers. A known quantity of bacteria is grown on agar plates in the presence of thin wafers containing the antibiotics. An area of clearing surrounds the wafer where bacteria are not capable of growing (called a zone of inhibition). This along with the rate of antibiotic diffusion is used to estimate the effectiveness of a particular antibiotic. In general, larger zones correlate with smaller minimum inhibitory concentration (MIC) of the antibiotic. This information can be used to evaluate the anthutiveness of a derivative of (methyl)griselimycin versus (methyl)griselimycin. MIC is the lowest concentration of an antimicrobial that will inhibit the visible growth of a microorganism after overnight incubation. Minimum inhibitory concentrations are important in diagnostic laboratories to confirm resistance of microorganisms to an antimicrobial agent and also to monitor the activity of new antimicrobial agents. MIC is generally regarded as the most basic laboratory measurement of the activity of an antimicrobial agent against an organism. MICs can be determined by agar or broth dilution methods usually following the guidelines of a reference body such as the Clinical and Laboratory Standards Institute (CLSI) or the British Society for Antimicrobial Chemotherapy (BSAC). There are several commercial methods available, including the well established Etest strips. The Etest system comprises a predefined and continuous concentration gradient of different antimicrobial agents, which when applied to inoculated agar plates and incubated, create ellipses of microbial inhibition. The MIC is determined where the ellipse of inhibition intersects the strip, and is easily read off the MIC reading scale on the strip.

Controls are a part of the test methods, since they can eliminate or minimize unintended influences (such as background signals). Controlled experiments are used to investigate the effect of a variable on a particular system. In a controlled experiment one set of samples has been (or is believed to be) modified and the other set of samples is either expected to show no change (negative control) or expected to show a definite change (positive control). The control can be determined in one test run together with the test substance. It may be determined before or after determining the effect of the test compound or it may be a known value. A possible control experiment may be an experiment in which the same conditions are used for the production of (methyl)griselimycin, a derivative thereof or L-trans-4-methylproline as in the test experiment, however, the variant nucleic acid is replaced by the nucleic acid which was the starting nucleic acid for producing the variant, e.g. the control nucleic acid is the wildtype nucleic acid. Examples are ORFs 1 to 26 being in an unmodified state in the control experiment, however, being modified in the test experiment.

The nucleic acid sequence of the variant may be determined by sequencing methods as known in the art whereby any mutation in any of ORFs 1 to 26 may be responsible for a modulation of the resulting protein resulting in an increased production of (methyl)griselimycin or L-trans-4-methylproline or in the production of a derivative of (methyl)griselimycin.

In another aspect of the invention, a method of producing griselimycin and/or methylgriselimycin is provided comprising providing at least one protein or a functionally active variant or fragment thereof as defined herein or a variant protein obtained by a method for identifying variants and incubating the at least one protein or variant or fragment thereof with L-N-methylvaline, L-leucine, L-N-methylthreonine, L-proline, L-N-methylleucine, glycine and possibly L-methylproline to produce griselimycin and/or methylgriselimycin. Preferably the at least one protein comprises at least NRPS-1, NRPS-2 and NRPS-3. In a further aspect, the present invention provides a method of producing L-trans-4-methylproline comprising providing at least one protein or a functionally active variant or fragment thereof as defined herein or a variant protein obtained by a method for identifying variants and incubating the at least one protein or variant or fragment thereof with L-leucine, 5-hydroxyleucine, γ-methylglutamic acid γ-semialdehyde or 3-methyl-Δ¹-pyrroline-5-carboxylic acid, preferably L-leucine, to produce L-trans-4-methylproline. Preferably, the at least one protein comprises at least Mps-1, Mps-2 and Mps-4. With respect to the terms "providing" and "protein or a functionally active variant or fragment thereof' it is referred to the definitions provided in the context of nucleic acids and proteins of the invention. With respect to the term "obtainable by the method" it is referred to the anthutions provided in the context of determining a variant of SEQ ID Nos: 2 to 27. The term "incubating" means maintaining the protein and other components at particular conditions in order to allow production of griselimycin and/or methylgriselimycin or L-trans-4-methylproline. With respect to the particular conditions reference is made to the definitions in the context of determining a variant of SEQ ID Nos: 2 to 27.

### FIGURES

Fig. 1 shows the structure of griselimycin (A) and methylgriselimycin (B).

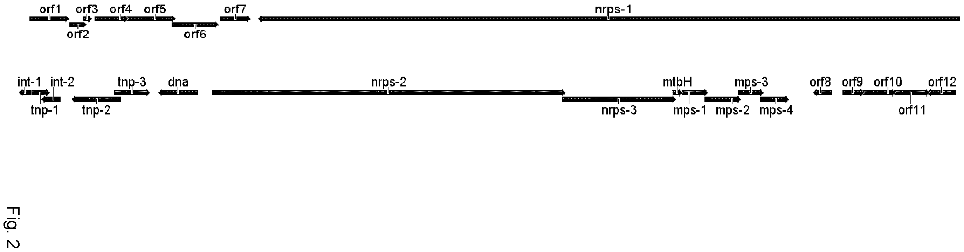

Fig. 2 shows the map and gene arrangement of the griselimycin biosynthesis gene cluster of *Streptomyces DSM 22643* as comprised by SEQ ID NO: 1. The putative ORFs are indicated by black block arrows designated by the respective gene names. Transcription directions and relative sizes are indicated.

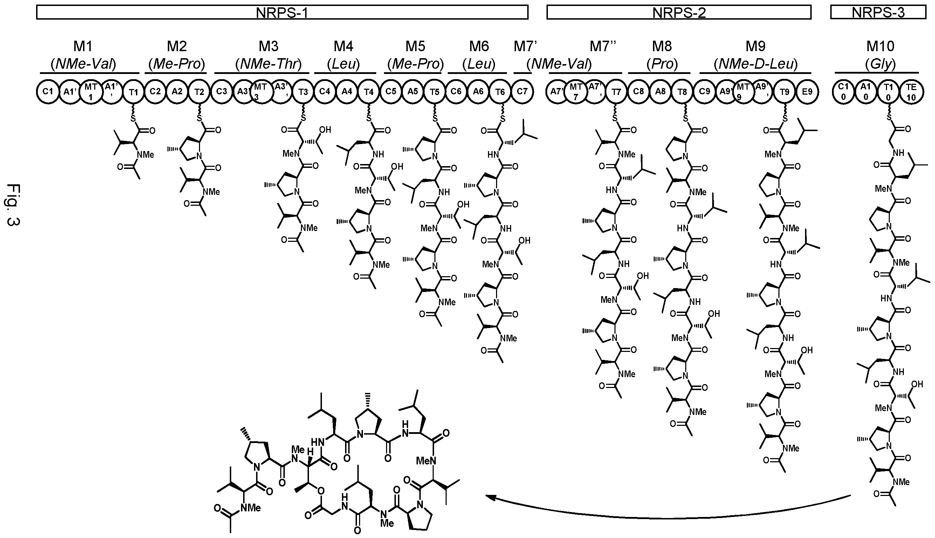

Fig. 3 shows a model of the griselimycin biosynthesis. The putative NRPS proteins, NRPS-1, NRPS-2, NRPS-3, are indicated by white blocks. The modules designated M1 to M10 are indicated below each block. Each module is dissected in domains designated C for condensation domain, A for adenylation domain, MT for methyltransferase domain, T for thiolation domain, E for epimerisation domain and TE for thioesterase domain, whereby the number behind each domain designation indicates the number of the module to which this domain is assigned. The arrangement A'-MT-A" indicates that the MT domains are integrated into the A domains. Below each module designation stands the amino acid which is introduced by the respective module into griselimycin. Nme-Val represents L-N-methylvaline, Me-Pro represents L-trans-4-methylproline, Nme-Thr represents L-N-methylthreonine, Leu represents L-leucine, Pro represents L-proline, Nme-D-Leu represents D-N-methylleucine and Gly represents glycine. Below the modules, the biosynthetic role of each module is presented by the presentation of the structure of the respective amino acids and their incorporation into griselimycin. By each module, one amino acid is condensed to the foregoing one resulting in an elongation of the growing peptide chain. Product release involves the TE10 domain with the subsequent cyclisation of the peptide to result in the macrocyclic product. The N-terminal C domain of module 1 is assumed to catalyze the acetylation of valine.

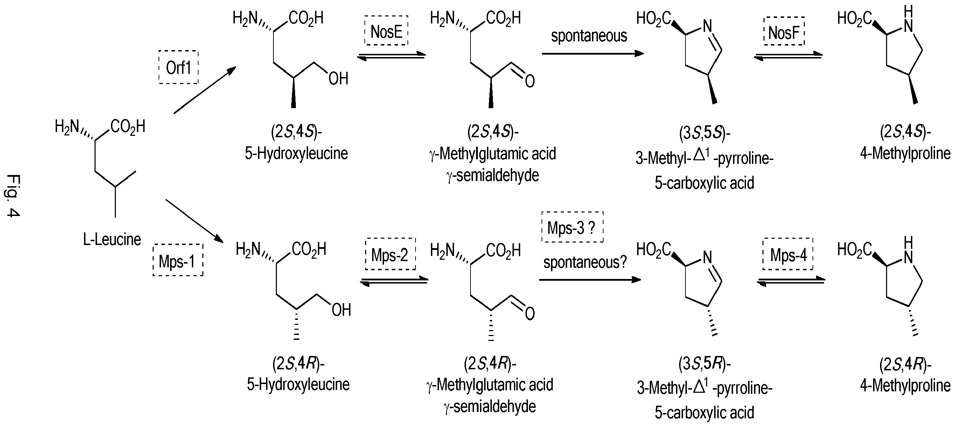

Fig. 4 shows the synthesis of 4-methylproline within the nostopeptolide biosynthesis gene cluster (Hoffmann et al., 2003; upper pathway) and the griselimycin biosynthesis gene cluster (lower pathway). In both biosynthesis pathways L-leucine is converted by the enzymes Orf1 (nostopeptolide) or Mps-1 ((methyl)griselimycin) to 5-hydroxyleucine, which is converted by NosE (nostopeptolide)) or Mps-2 ((methyl)griselimycin) to γ-methylglutamic acid γ-semialdehyde which spontaneously forms 3-methyl-Δ¹-pyrroline-5-carboxylic acid. This is converted to 4-methylproline by NosF (nostopeptolide) or Mps-4 ((methyl)griselimycin). The products of each step of the 4-methylproline synthesis of the (nostopeptolide) or ((methyl)griselimycin) pathways differ by their configuration with respect to the methyl group at position 4 of 5-hydroxyleucin, γ-methylglutamic acid γ-semialdehyde and 4-methylproline and at position 5 of 3-methyl-Δ¹-pyrroline-5-carboxylic acid, in that in the nostopeptolide biosynthesis the methyl group is in the S configuration, whereas in the ((methyl)griselimycin) biosynthesis the methyl group is in the R anthus etion. Possibly, Mps-3 plays a role in the conversion of γ-methylglutamic acid γ-semialdehyde to 3-methyl-Δ¹-pyrroline-5-carboxylic acid

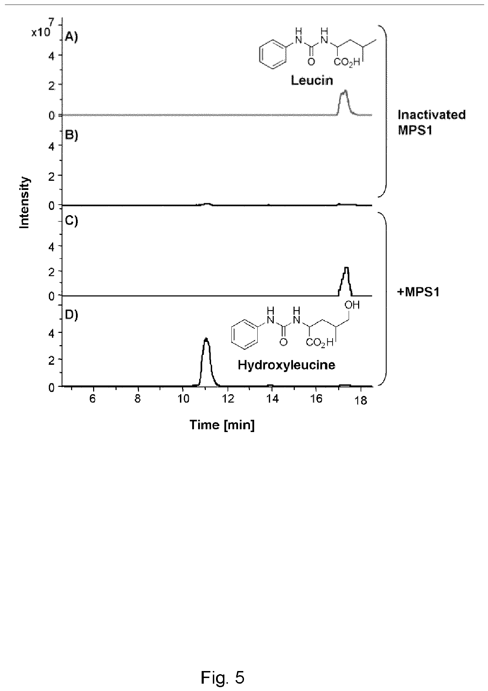

Fig. 5 shows the results of an HPLC-MS analysis of the Mps-1 or Leucine-hydroxylase assay. The diagram shows that in the enzyme assay performed with inactivated Mps-1 (chromatograms A) and B)) hydroxyleucine is not produced whereas in the assay performed with active Mps-1 (chromatograms C) and D)) hydroxyleucine is produced. A) and C): Extracted ion chromatograms for m/z= 273.1 ([M+Na]⁺ of PTC-leucine). B) and D): Extracted ion chromatograms for m/z= 289.1 ([M+Na]⁺ of PTC-hydroxyleucine).

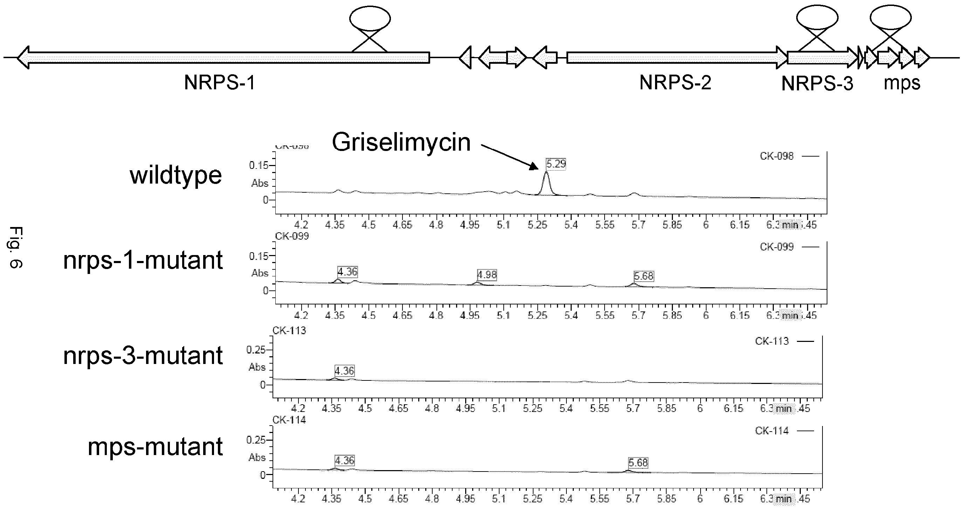

Fig. 6 shows results obtained with mutant nrps-1 and nrps-3 genes and a mutant mps operon. The nrps and mps genes are indicated by block arrows. The approximate location of the insertional mutations is indicated by the circle-cross symbol. Below the presentation of the nrps and mps genes are the relevant UHPLC chromatograms (UV 210 nm) of the supernatant from cultures of the wildtype, the nrps-1 mutant, the nrps-3 mutant and the mps-mutant. In the wildtype the peak at 5.29 min shows the presence of griselimycin. In contrast no griselimycin can be detected in the culture supernatants of the three mutants.

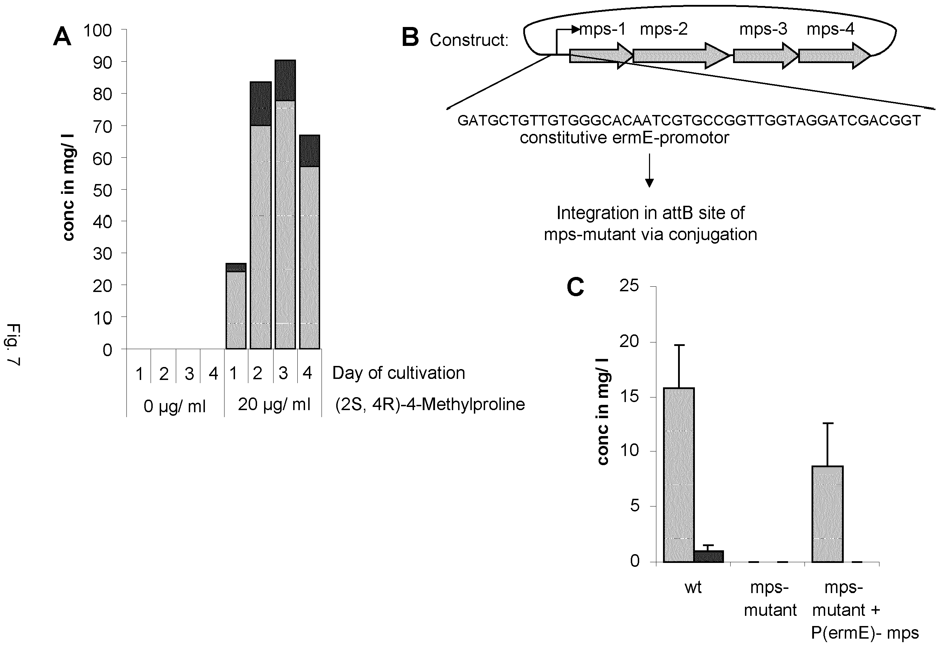

Fig. 7 shows complementation of the mps-mutant obtained in Example 5.
   A. Complementation of the mps mutant by feeding 20 µg/ml (2S, 4R)-4-methylproline once at day 0 to the production medium. At days 1 to 4 the (methyl)griselimycin production was analysed (grey bar: griselimycin production, black bar: methylgriselimycin production). Only in the complement medium the mps-mutant is able to produce (methyl)griselimycin. The experiment that is exhibiting an increase of methylgriselimycin versus griselimycin when methyl-prolin was feeded is mentioned in example 9 and on page 15.
   B. Schematical drawing of the relevant aspects for genetic complementation of the mps-mutant: The mps-operon (mps-1, mps-2, mps-3 and mps-4 genes) is under expression control of the 5' inserted constitutive ermE-promotor.
   C. Genetic complementation of the mps-mutant. After integration of the P(ermE)-mps-operon into the attB site of the mps mutant, the capability of griselimycin production is restored (grey bar: griselimycin production, black bar: methylgriselimycin production).

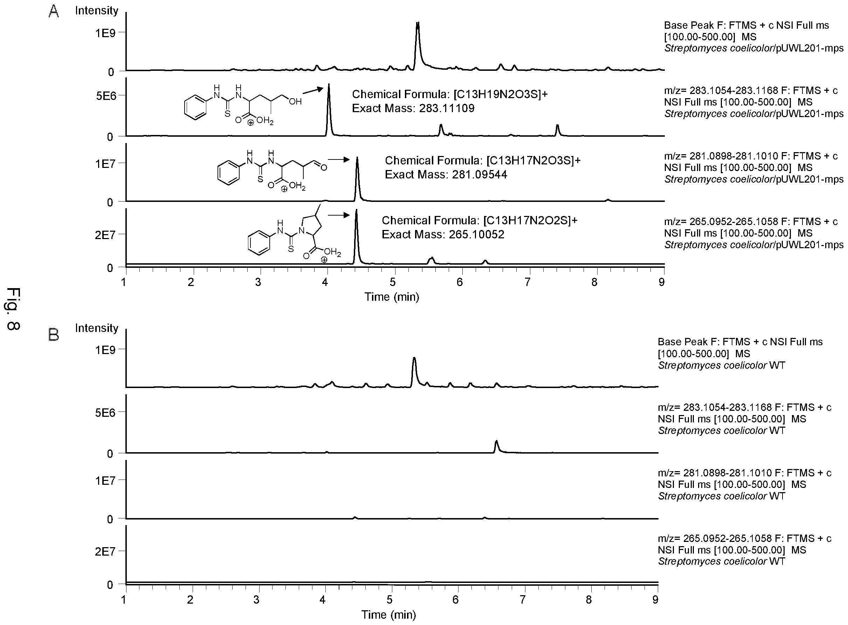

Fig. 8 shows an HPLC-MS analysis of derivatized extracts from an *mps* expression culture (*Streptomyces coelicolor*/*pUWL201-mps;* Fig. 8A) and the negative control (*Streptomyces coelicolor* WT; Fig. 8B). The PITC derivatives of the expected product (*2S*, *4R*)-4-methylproline (4^{th} chromatogram of Fig. 8A and B) as well as PITC derivatives of two biosynthetic intermediates 5-hydroxyleucine (2^{nd} chromatogram of Fig. 8A and B) and γ-methylglutamic acid γ-semialdehyde (3^{rd} chromatogram of Fig. 8A and B) could be detected in the extract of the *mps* expression culture, but not in *Streptomyces coelicolor* WT. The 1^{st} chromatogram of Fig. 8A and B corresponds to leucine

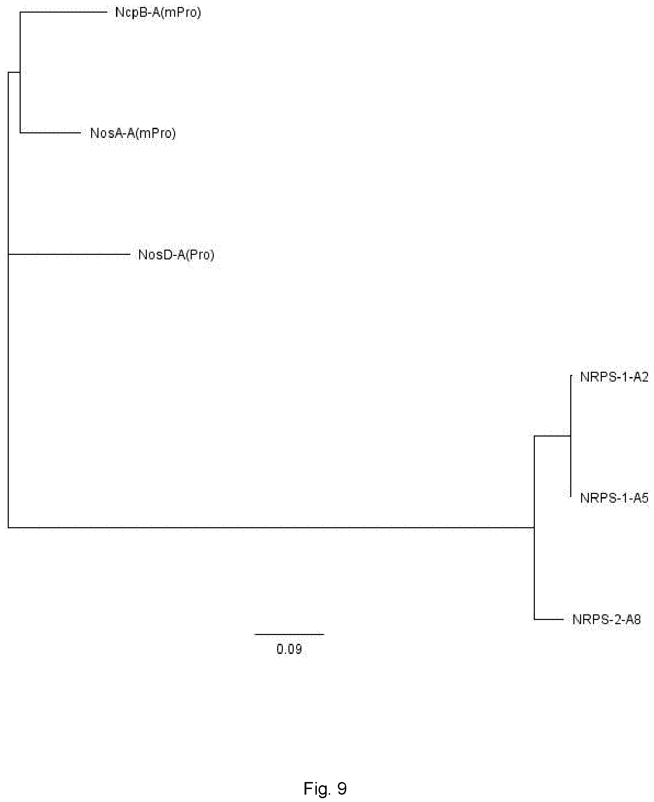

Fig. 9 shows a phylogenetic tree indicating the relationship between NosA-A(mPro), NcpB-A(mPro) and NosD-A(pro) and NRPS-1-A2, NRPS-1-A5 and NRPS-2-A8. The scale bar represents 0.09 substitutions per amino acid site.

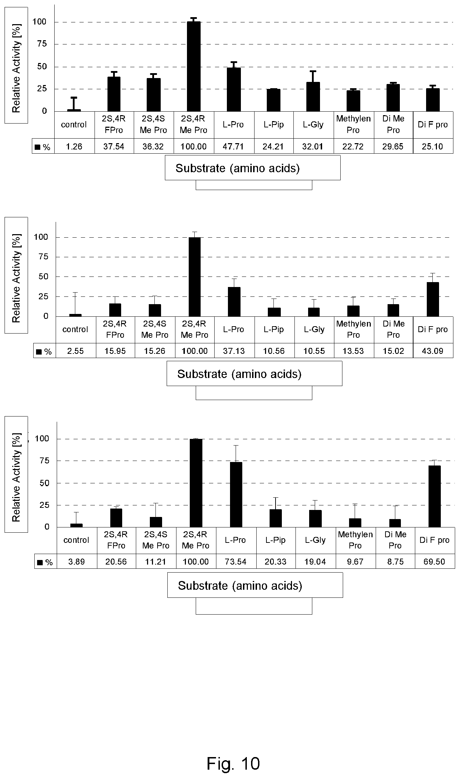

Fig. 10 shows an analysis of substrate specificity of the A domains of module 2 (A2; upper panel), module 5 (A5; middle panel) and module 8 (A8; lower panel) by in vitro ATP-Ppi-exchange experiments. For A5 and A8 experiments were performed in triplicate, for A2 in duplicate. Highest activities were scaled up to 100%. The following substrates were tested: (2S,4R)-4-fluoroproline (2S,4R Fpro), (2S,4S)-4-methylproline (2S,4S MePro), (2S,4R)-4-methylproline (2S,4R MePro), L-proline (L-Pro), L-pipecolic acid (L-Pip), L-glycine (L-Gly), 4-methylenproline (Methylen Pro), 4,4-Dimethylproline (Di Me Pro) and 4,4-difluoroproline (Di F Pro). Relative activities are indicated on the Y axis. Below each substrate activities as compared to highest activities which are given as 100% are indicated in percent. In the control experiment, no amino acid was present.

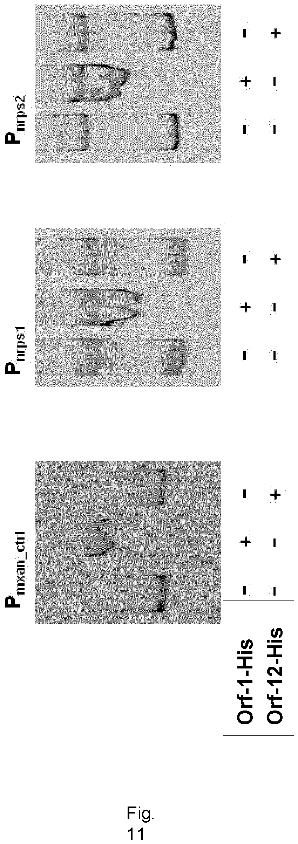

Fig. 11: Binding of full length regulators Orf-1 and Orf-12 to DNA fragments Pnrps1, Pnrps2 and Pmxan_ctrl. Scanned pictures of the EMSA assays are shown. Pnrps1 and Pnrps2: EMSA assay using DNA fragments comprising promoter regions of genes nrps1 or nrps2. Pmxan_ctrl: EMSA assay using Hex labeled DNA fragment comprising the dual promoter system in an intergenic region of *Myxococcus xanthus* DK1622 flanked by mxan_3950 and mxan_3951. Orf-1-His and Orf-12-His: C-terminal 6xHis tagged Orf-1 and Orf-12. +: With regulator. -: without regulator.

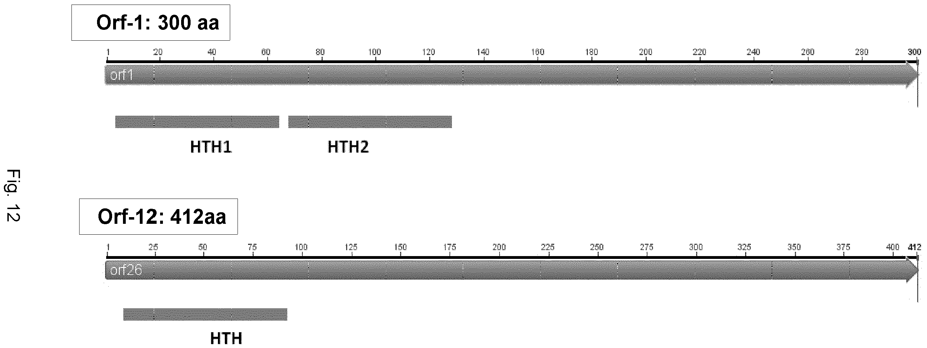

Fig. 12 shows the predicted domain organization of regulators Orf-1 and Orf-12. Domains predicted by the SMART tool at http://smart.embt-heidelberg.de/ are shown. HTH: helix-turn-helix motif, aa: amino acids.

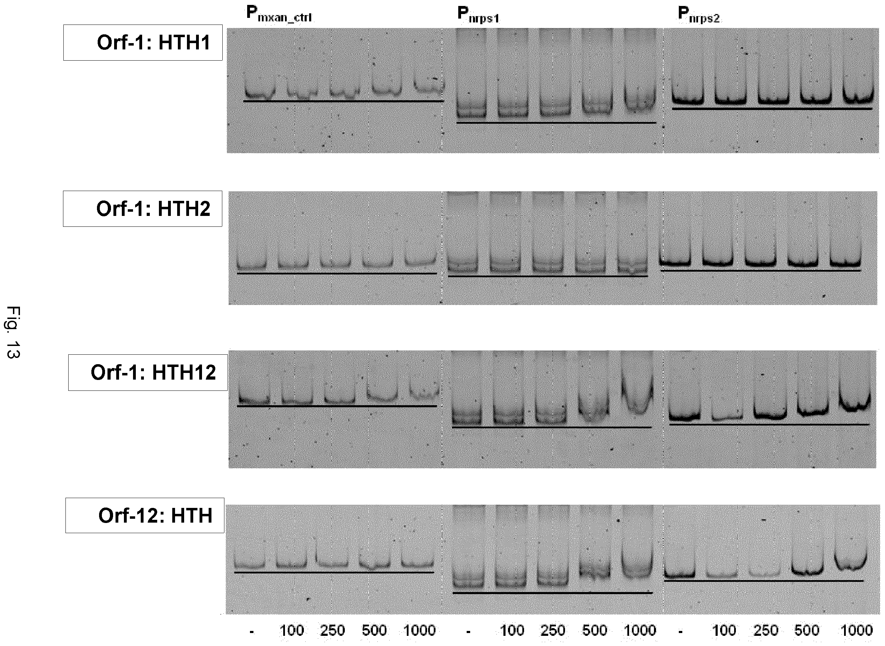

Fig. 13 shows the binding of shortened versions of regulators Orf-1 and Orf-12 to DNA fragments Pnrps1, Pnrps2 and Pmxan_ctrl. Scanned pictures of the EMSA assays are shown. In the assays derivatives of Orf-1 and Orf-12 were used only comprising the predicted HTH domains. Pnrps1 & Pnrps2: EMSA assay using DNA fragments comprising promoter regions of genes nrps1 or nrps2. Pmxan_ctrl: EMSA assay using Hex labeled DNA fragment comprising the dual promoter system in an intergenic region of *Myxococcus xanthus* DK1622 flanked by mxan-3950 and mxan-3951. Orf-1-HTH1, Orf-1-HTH2, Orf1-HTH12 and Orf-12-HTH: C-terminal 6xHis tagged Orf-1 and Orf-12 only comprising HTH domains. -: without regulator. Numbers depict the molar excess of regulator protein compared to the quantity of DNA fragment.

### EXAMPLES

### Example 1: Identification of the griselimycin biosynthesis locus in Streptomyces DSM 22643

*Streptomyces DSM 22643* naturally produces the antibiotics griselimycin and methylgriselimycin. However, the genetic locus involved in the biosynthesis of these antibiotic substances was not previously identified. For the identification of the griselimycin locus, *Streptomyces DSM 22643* was cultured according to standard microbiological techniques. DNA was extracted according to standard procedures and the total sequence was determined according to standard procedures. Analysis of the genes comprised by the DNA of *Streptomyces DSM 22643* and proteins encoded by the genes was performed in silico. Table 1 lists the genes identified by ORF numbers which were determined by homology searches. Specific gene designations were assigned to the ORF numbers. The location of the genes and their arrangement within the griselimycin biosynthesis gene cluster is schematically indicated in Fig. 2.

**Table 1**

| ORF number | gene designation | start nt | stop nt | gene length (nt) | strandedness | protein length (aa) | protein designation | SEQ ID NO: protein |
|---|---|---|---|---|---|---|---|---|
| 1 | orf-1 | 837 | 2075 | 1269 | positive | 412 | Orf-1 | 2 |
| 2 | orf-2 | 2185 | 2625 | 441 | positive | 146 | Orf-2 | 3 |
| 3 | orf-3 | 2625 | 2819 | 195 | positive | 64 | Orf-3 | 4 |
| 4 | orf-4 | 3033 | 4037 | 1005 | positive | 334 | Orf-4 | 5 |
| 5 | orf-5 | 4110 | 5630 | 1521 | positive | 506 | Orf-5 | 6 |
| 6 | orf-6 | 5627 | 7066 | 1440 | positive | 479 | Orf-6 | 7 |
| 7 | orf-7 | 7240 | 8133 | 894 | positive | 297 | Orf-7 | 8 |
| 8 | nrps-1 | 31885 | 8501 | 26385 | negative | 7794 | NRPS-1 | 9 |
| 9 | int-1 | 33102 | 32770 | 333 | negative | 110 | Int-1 | 10 |
| 10 | tnp-1 | 33187 | 33726 | 540 | positive | 179 | Tnp-1 | 11 |
| 11 | int-2 | 34127 | 33567 | 561 | negative | 186 | Int-2 | 12 |
| 12 | tnp-2 | 36300 | 34648 | 1653 | negative | 550 | Tnp-2 | 13 |
| 13 | tnp-3 | 36172 | 37314 | 1143 | positive | 380 | Tnp-3 | 14 |
| 14 | dna | 39032 | 37746 | 1287 | negative | 428 | Dna | 15 |
| 15 | nrps-2 | 39643 | 52149 | 12507 | positive | 4168 | NRPS-2 | 16 |
| 16 | nrps-3 | 52146 | 56120 | 3975 | positive | 1324 | NRPS-3 | 17 |
| 17 | mbtH | 56117 | 56335 | 219 | positive | 72 | MbtH | 18 |
| 18 | mps-1 | 56434 | 57246 | 813 | positive | 270 | Mps-1 | 19 |
| 19 | mps-2 | 57243 | 58418 | 1176 | positive | 391 | Mps-2 | 20 |
| 20 | mps-3 | 58445 | 59245 | 801 | positive | 266 | Mps-3 | 21 |
| 21 | mps-4 | 59242 | 60141 | 900 | positive | 299 | Mps-4 | 26 |
| 26 | orf-8 | 61680 | 61144 | 537 | negative | 178 | Orf-8 | 26 |
| 26 | orf-9 | 62170 | 62820 | 651 | positive | 216 | Orf-9 | 24 |
| 24 | orf-10 | 62824 | 63939 | 1116 | positive | 371 | Orf-10 | 25 |
| 25 | orf-11 | 63969 | 65165 | 1197 | positive | 398 | Orf-11 | 26 |
| 26 | orf-12 | 66108 | 65206 | 903 | negative | 300 | Orf-12 | 27 |

### Example 2: Genes and proteins involved in the biosynthesis of griselimyin and methylgriselimycin

By in silico analysis it was determined that the griselimycin biosynthesis gene cluster comprises 26 genes encoding 26 proteins. The biological function of the 26 (methyl)griselimycin biosynthetic proteins was assessed by computer comparison of each identified protein with proteins found at the NCBI. Table 2 identifies the proteins as comprised by the griselimycin biosynthesis gene cluster and the corresponding GenBank proteins found by the homology searches.

**Table 2**

| ORF number | best blast hit | | | | | |
|---|---|---|---|---|---|---|
| | Best blast hit | GenBank Accession number | nearest homologue in | e-Value | identity/ similarity (%) | putative function |
| 1 | TlmR2 | ABL74963.1 | *Streptoalloteichus hindustanus* | 2E-55 | 35/53 | Xre family transcriptional regulator |
| 2 | Hypothetical protein SSOG 02153 | ZP_07294072 | *Streptomyces hygroscopicus* ATCC 53653 | 5E-11 | 41/47 | Hypothetical protein |
| 3 | Conserved hypothetical protein | ZP_06583961 | *Streptomyces roseosporus* NRRL 15998 | 3E-15 | 61/86 | Conserved hypothetical protein |
| 4 | Conserved hypothetical protein | CBG69271.1 | *Streptomyces scabiei 87.26* | 1,00E-140 | 81/87 | Conserved hypothetical protein |
| 5 | Putative transporter | CBG69272.1 | *Streptomyces scabiei 87.26* | 0,0 | 91/94 | Transporter |
| 6 | Putative glutamine synthetase | CBG69273.1 | *Streptomyces scabiei 87.26* | 0,0 | 81/87 | Glutamine synthetase |
| 7 | Hypothetical protein | CBG69274.1 | *Streptomyces scabei* 87.26 | 8E-120 | 73 81 | Hypothetical protein |
| 8 | Amino acid adenylation domain-containing protein | YP_001539321.1 | *Salinispora arenicola* CNS-205 | 0,0 | 51/63 | NRPS |
| 9 | Integrase catalytic region | YP_004330163 | *Pseudonocardia dioxanivorans* CB 1190 | 1E-43 | 75/92 | Integrase catalytic region |
| 10 | Putative transposase for insertion sequence element | YP_001509642 | *Frankia sp.* EAN1pec | 3E-15 | 76/78 | Transposase for insertion sequence element |
| 11 | Integrase catalytic region | YP_004330974 | *Pseudonocardia dioxanivorans* CB1190 | 4E-33 | 67/81 | Integrase catalytic region |
| 12 | Rhodopirellula transposase family protein | ZP_07610914 | *Streptomyces violaceusniger* Tu 4113 | 0.0 | 74/80 | Transposase |
| 13 | Putative IS4 family Transposase | ZP_06418432.1 | *Frankia sp.* EUN 1f | 9E-60 E-60 | 43/55 | Transposase |
| 14 | DNA polymerase III, beta | ZP_06824689 | *Streptomyces sp.* SPB74 | 4E-110 | 53/70 | DNA polymerase III, beta |
| | subunit | | | | | subunit |
| 15 | putative pristinamycin I peptide synthase 3 and 4 | CBH31051.1 | *Streptomyces pristinaespiralis* | 0,0 | 51/62 | NRPS |
| 16 | Putative NRPS | ZP_04685020.1 | Streptomyces ghanaensis ATCC 14672 | 0,0 | 45/61 | NRPS |
| 17 | MbtH domain protein | ZP_06476953 | Frankia symbiont ofDatisca glomerata | 9E-26 | 72/81 | MbtH domain protein |
| 18 | Phytanoyl-CoA dioxygenase | YP_001539320.1 | *Salinispora arenicola* CNS-05 | 8E-91 | 60/76 | Leucine hydroxylase |
| 19 | Alcohol dehydrogenase | YP_707358.1 | *Rhodococcus jostii* RHA1 | 6E-102 | 51/67 | Alcohol dehydrogenase |
| 20 | Alpha/beta hydrolase fold protein | ZP_04702845.1 | *Streptomyces albus* J1074 | 3E-77 | 56/71 | Alpha/beta hydrolase |
| 21 | LmbY | ABX00626.1 | *Streptomyces lincolnensis* | 5E-131 | 76/88 | Oxidoreductase |
| 26 | Hypothetical protein SclaA2_13611 | ZP_08216838 | *Streptomyces clavuligerus* ATCC 27064 | 1E-42 | 65/78 | PNPOx-like superfamily protein |
| 26 | Hypothetical protein SAC-TEDRAFT_3210 | ZP_06272665.1 | *Streptomyces sp.* ACTE | 3E-38 | 64/74 | Hypothetical protein |
| 24 | Hypothetical protein SAC-TEDRAFT_3209 | ZP_06272664.1 | *Streptomyces sp.* ACTE | 2E-112 | 67/78 | Hypothetical protein |
| 25 | Amidohydrolase | ZP_06272663.1 | *Streptomyces sp.* ACTE | 1E-148 | 70/80 | Amidohydrolase |
| 26 | Hypothetical protein SACTEDRAFT 3207 | ZP_06272662.1 | *Streptomyces sp.* ACTE | 2E-64 | 52/69 | Xre family transcriptional regulator |

Between the individual modules and domains, there are linker regions, namely intermodule linker and inter-domain linker. At the N-terminus and C-terminus of the proteins, there are COM or docking domains which are necessary for interaction of the individual NRPS proteins. Communication-mediating (COM) domains facilitate the selective interaction between nonribosomal peptide synthetases by short communication-mediating domains (Hahn M. and Stachelhaus T.;. 2004).

Based on homology searches the genes designated nrps-1, nrps-2 and nrps-3 encode subunits of the nonribosomal protein synthetase (NRPS) synthesizing griselimycin and methylgriselimycin (see Fig. 3). The nrps-1 gene has a length of about 26.4 kb and encodes 6 modules that are necessary for the incorporation of (1) L-N-methylvaline (M1), (2) L-trans-4-methylproline (M2), (3) L-N-methylthreonine (M3), (4) L-leucine (M4), (5) L-trans-4-methylproline (M5) and (6) L-leucine (M6), respectively, into (methyl)griselimycin. Module 1 or M1 and module 3 or M3 each encode a C-domain, an A-domain which is interrupted by a MT-domain and a T-domain, whereas module 2 or M2, module 4 or M4, module 5 or M5 and module 6 or M6 each encode a C-domain, an A-domain and a T-domain. Furthermore it contains the condensation domain of module 7 or M7. The condensation domain of M1, C1, is responsible for N-acetylation. The nrps-2 gene has a length of about 12.5 kb and encodes part of module 7 and modules 8 and 9 that are necessary for the incorporation of (7) L-N-methylvaline (M7), (8) L-proline (griselimycin) or L-trans-4-methylproline (methylgriselimycin) (M8) and (9) D-N-methylleucine (M9). The part of module 7 comprises an A-domain which is interrupted by a MT-domain and a T-domain, module 8 or M8 comprises a C-domain, an A-domain and a T-domain and module 9 or M9 comprises a C-domain, an A-domain which is interrupted by a MT-domain, a T-domain and an E-domain. The presence of a D-amino acid at position 9 of (methyl)griselimycin coincides with the presence of an E-domain in module 9 which catalyses the insertion of D-N-methylleucine. The nrps-3 gene has a length of about 4 kb and codes for module 10 which incorporates glycine into the molecule and manages the cyclisation of the linear peptide chain between glycine and L-N-methylthreonine via an ester bond. Module 10 comprises a C-domain, an A-domain, a T-domain and a TE-domain.

Homology searches allowed the assignment of a biosynthesis role to the repeating units, the modules, of the NRPS proteins. Fig. 3 shows the assignment of modules 1 to 10 to the NRPS proteins, the dissection into the domains and the biosynthesis activity by indicating the amino acids and their incorporation into the growing griselimycin chain. Module 10 releases the chain from the NRPS-3 protein, followed by chain cyclisation. In Table 3, the approximate boundaries of the modules and domains on the protein and DNA level with respect to their location within the individual NRPS proteins are indicated. Thereby, C represents a condensation domain, A represents an adenylation domain, MT represents a methyltransferase domain, and T represents a thiolation domain. The MT domains are integrated into the A domains. This is indicated by the arrangement "A'-MT-A".

**Table 3**

| Protein | Modules/ Domains | Location within the protein sequence (aa) | Location within the gene cluster sequence (SEQ ID NO: 1) (nt) |
|---|---|---|---|
| NRPS-1 (SEQ ID NO: 9) | Module 1 | 9-1493 aa | 31861-27407 nt |
| | C1 | 9-336 aa | 31861-30878 nt |
| | A1' | 481-939 aa | 30445-29069 nt |
| | MT | 940-1365 aa | 29068-27791 nt |
| | A1" | 1366-1406 aa | 27790-27668 nt |
| | T1 | 1429-1493 aa | 27601-27407 nt |
| | Module 2 | 1517-2569 aa | 27337-24179 nt |
| | C2 | 1517-1858 aa | 27337-26312 nt |
| | A2 | 2003-2482 aa | 25879-24440 nt |
| | T2 | 2505-2569 aa | 24373-24179 nt |
| | Module 3 | 2591-4102 aa | 24115-19580 nt |
| | C3 | 2591-2932 aa | 24115-26090 nt |
| | A3' | 3077-3536 aa | 26657-21278 nt |
| | MT3 | 3537-3974 aa | 21277-19964 nt |
| | A3" | 3975-4015 aa | 19963-19841 nt |
| | T3 | 4038-4102 aa | 19774-19580 nt |
| | Module 4 | 4124-5170 aa | 19516-16376 nt |
| | C4 | 4124-4465 aa | 19516-18491 nt |
| | A4 | 4612-5083 aa | 18052-16637 nt |
| | T4 | 5106-5170 aa | 16570-16376 nt |
| | Module 5 | 5193-6245 aa | 16309-13151 nt |
| | C5 | 5193-5534 aa | 16309-15284 nt |
| | A5 | 5679-6158 aa | 14851-13412 nt |
| | T5 | 6181-6245 aa | 13345-13151 nt |
| | Module 6 | 6268-7312 aa | 13084-9950 nt |
| | C6 | 6268-6609 aa | 13084-12059 nt |
| | A6 | 6754-7265 aa | 11626-10211 nt |
| | T6 | 7248-7312 aa | 10144-9950 nt |
| | Module 7' | 7336-7677 aa | 9880-8855 nt |
| | C7 | 7336-7677 aa | 9880-8855 nt |
| NRPS-2 (SEQ ID NO: 16) | Module 7" | 42-1066 aa | 39766-42840 nt |
| | A7' | 42-500 aa | 39766-41142 nt |
| | MT7 | 501-938 aa | 41143-42456 nt |
| | A7" | 939-979 aa | 42457-42579 nt |
| | T7 | 1002-1066 aa | 42646-42840 nt |
| | Module 8 | 1088-2148 aa | 42904-46086 nt |
| | C8 | 1088-1430 aa | 42904-43932 nt |
| | A8 | 1575-2061 aa | 44365-45825 nt |
| | T8 | 2084-2148 aa | 45892-46086 nt |
| | Module 9 | 2172-4160 aa | 46156-52126 nt |
| | C9 | 2172-2513 aa | 46156-47181 nt |
| | A9' | 2658-3098 aa | 47614-48936 nt |
| | MT9 | 3099-3536 aa | 48937-50250 nt |
| | A9" | 3537-3577 aa | 50251-50373 nt |
| | T9 | 3600-3663 aa | 50440-50631 nt |
| | E9 | 3679-4160 aa | 50677-52126 nt |
| NRPS-3 (SEQ ID NO:. 17) | Module 10 | 13-1312 aa | 52182-56081 nt |
| | C10 | 13-343 aa | 52182-53174 nt |
| | A10 | 484-951 aa | 53595-54998 nt |
| | T10 | 974-1038 aa | 55065-55259 nt |
| | TE10 | 1060-1312 aa | 55326-56081 nt |

| | | | |
|---|---|---|---|
| nt: nucleotide, aa: amino acid | | | |

Based on homology searches and biochemical analysis the genes designated mps-1, mps-2-and mps-4 encode proteins necessary for the biosynthesis of L-trans-4-methylproline. L-trans-4-methylproline is contained at positions 2 and 5 of griselimycin and at positions 2, 5 and 8 of methylgriselimycin. The mps-1 gene encodes a protein with highest homology to 4-proline hydroxylases (41% identity/57% similarity) and phytanoyl-CoA dioxygenase but by biochemical analysis it could be shown that the mps-1 gene codes for a leucine hydroxylase which hydroxylates L-leucine to (2S, 4R)-5-hydroxyleucine. The gene mps-2 codes for an alcohol dehydrogenase that has 34% identity/55% similarity to the *nosE* gene of the nostopeptolide gene cluster which is involved in synthesis of L-cis-4-methylproline (Luesch et al. 2003). Mps-2 catalyses the dehydrogenation of (2S,4R)-5-hydroxyleucine to (2S, 4R)-γ-methylglutamic acid γ-semialdehyde. After spontaneous ring formation the protein encoded by mps-4 probably catalyses the reduction of (3S, 5R)-3-methyl-Δ¹-pyrroline-5-carboxylic acid to (2S,4R)-4-methylproline. The mps-4 gene shows the highest homology to Sib (55% identity/70% similarity) and LmbY, that both code for flavin dependent monoxygenases and catalyse a similar reaction (Li et al. 2009; Peschke et al. 1995). The mps-3 gene shows highest homology to α,β-hydrolase with highest homology to the *lipE* gene product (50% identity/68% similarity) in the friulimycin biosynthetic gene cluster (Müller et al. 2007). The encoded protein might act as type II thioesterase with a function in regeneration of misprimed NRPS. Possibly, the mps-3 gene product is involved in the cyclisation of the pyrroline derivative in the biosynthesis pathway of 4-methylproline. Based on these results, the present inventors succeeded to elucidate the biosynthetic pathway of L-trans-4-methylproline for the biosynthesis of griselimycin or methylgriselimycin. Fig. 4 shows a comparison of the biosynthesis pathways of 4-methylproline including the proteins involved therein within the nostopeptolide biosynthesis pathway of the cyanobacterium Nostoc sp. GSV264 (Hoffmann et al. 2003) and within the (methyl)griselimycin biosynthesis pathway as identified by the present invention. Based on sequence analysis the genes designated mps-1 to mps-4 are probably transcribed as an operon.

Furthermore, based on homology searches, the following genes were identified. There are two genes orf and orf-12 with homology to proteins of the Xre-family transcriptional regulators in the gene cluster. The encoded proteins play a role in regulation of (methyl)griselimycin biosynthesis. It could be shown that inactivation of both genes results in a significant loss of production of (methyl)griselimycin, which proves their function as direct or indirect regulators. Orf-5 encodes a protein with homology to a putative transport molecule and might be involved in export of (methyl)griselimycin. Orf-6 encodes a protein with the putative function of a glutamine synthetase. Int-1 and int-2 encode proteins with homology to integrase catalytic regions, so that it is assumed that these proteins have integrase functionality. Tnp-1, tnp-2 and tnp-3 between the genes nrps-1 and nrps-3 encode proteins with homology to putative transposase proteins and are assumed to have transposase activity. A further open reading frame has the putative function as a subunit 13 of the DNA polymerase III. This orf was designated *dna.* A small 219 bp ORF directly downstream of nrps-3 encodes a MbtH like protein and was therefore designated mbtH. The MbtH protein is needed for correct function of the NRPS (Barry & Challis, 2009). Orf 8 has homology to the hypothetical protein Scla2_13611 and is assumed to belong to the PNPOx-like superfamily. Orf-11 encodes a putative protein of the amidohydrolase superfamily. The proposed genes orf-2, orf-3, orf-4, orf-7, orf-9 and orf-10 all encode different conserved hypothetical proteins.

### Example 3: In vitro-synthesis of L-trans-4-methylprolin

In vitro-synthesis of L-trans-4-methylproline can be performed by coupling three subsequent assays, namely the Mps-1 assay, the Mps-2 assay and the Mps-3 assay. Cyclisation of γ-methylglutamic acid γ-semialdehyde to 3-methyl-Δ¹-pyrroline-5-carboxylic occurs spontaneously or may involve Mps-3. Fig. 4 shows the conversion of L-leucine to (2S,4R)-4-methylproline using Mps-1, Mps-2, possibly Mps-3 and Mps-4.

### (a) Leucine hydroxylase assay

Based on in silico analysis it was found that the mps-1 gene encodes a leucine hydroxylase. The following experimental procedure was performed which confirms that the Mps-1 protein encodes a leucine hydroxylase.

The putative leucine hydroxylase encoding gene mps-1 was amplified using the primers Gri3_for (5'- GCCGCCATATGATGCAGCTCACGGCCGAT-3') (SEQ ID NO: 28) and Gri3_rev (5'-GGTCAGGATCCTCATGCCAGCCTCGATTC-3') (SEQ ID NO: 29) from genomic DNA from *Streptomyces DSM 26643* with Phusion polymerase and cloned into the pJET 2.1 vector (Fermentas) by blunt end ligation. After sequence verification the DNA fragment was subcloned into pET28b(+) via the introduced *Nde*I/*Bam*HI restriction sites. The resulting construct pGris3 was transformed into *E. coli Rosetta* BL21 (DE3) pLysS/RARE for protein expression. Fresh transformants harboring the constructs were grown in LB-medium (11 batches started with 0.1% inocula from a 10-ml culture grown for 5 h at 37 °C) supplemented with kanamycin (50 µg/ml) and chloramphenicol (34 µg/ ml). All cells were grown at 37°C to an OD600 of approximately 0.8. The cells were then induced with 1 M isopropyl-b-D-thiogalactopyranoside (IPTG) to an end concentration of 0.2 mM and then grown at 16°C overnight. The cells were harvested by centrifugation (6000 rpm, 10 min, 4°C) and resuspended in buffer A (20 mM Tris-HCl, pH 7.8, 200 mM NaCl, and 10% [v/v] glycerol). The cells were then lysed (2 passes at 700 psi, French press, SLM Aminco), and the cell debris was removed by centrifugation (21,000 rpm, 10 min. 4°C). Prepacked HisTrap™ HP columns were used for preparative purification of histidine-tagged recombinant proteins by immobilized metal ion affinity chromatography (IMAC) on the Äkta prime^{™} plus system (Ge Healthcare). 15 ml protein lysates were filtered through a sterile filter and loaded onto a 1 ml HisTrap column. Purification was performed as recommended in the GE Healthcare manual (HisTrap HP, Instructions 71-5027-68 AF). The desired protein was eluted from the column in a stepwise imidazole gradient with buffer B (20 mM Tris-HCl, pH 7.8, 200 mM NaCl, and 10% [v/v] glycerol and 60, 100, 200, 300 and 500 mM imidazole, 10 ml fractions). Fractions containing the pure target protein, as determined by SDS-polyacrylamide gel electrophoresis (PAGE), were combined and concentrated to approximately 200 µl by using Amicon Ultra PL-10 centricons. Then 800 µl of storage buffer (50 mM Tris-HCl, pH 7.5, 50 mM NaCl, and 10% [v/v] glycerol) was added to the concentrated protein before flash-freezing in liquid nitrogen and storage at -80°C. Protein concentrations were determined using the Bradford assay (Bio-Rad, Hercules, CA, USA). 1-3 mg/ml purified protein were obtained per liter of culture.

Assays with leucine as substrate were conducted in MOPS (50 mM), pH7.0, and included substrate (1 mM), α-ketoglutarat (1 mM), FeSO₄ (25 µM), DTT (0.5 mM), ascorbate (0.1 mM) and Mps1 (1µM) in a total volume of 200 µl. Reactions were initiated by the addition of Mps1 and incubated at 30°C for 1 hour. Protein was precipitated with cold ethanol and the supernatant was decanted and used for derivatization. The solution was dried by rotary evaporation and resuspended in 200 µl coupling buffer (acetonitrile:pyridine:triethylamine:H₂O 10:5:2:3). 50 µl of PITC solution were added to this solution and after a 5 min reaction at room temperature 50 µl H₂O were added to stop the reaction. The solution was evaporated to dryness by speedvac evaporation. The resulting PTC amino acids were dissolved in 1 ml water:acetonitrile (7:2), centrifuged and the supernatant was dried by speed vac evaporation again. The amino acids were then dissolved in 100 µl water/acetonitrile and used for HPLC/MS analysis. PITC-leucine and PITC-hydroxyleucine were detected in the positive ionization mode (Leucine: [M+H]⁺ = 272.9 and hydroxyleucine: [M+H]⁺ = 288.9). It was shown that Msp-1 catalyses the reaction from leucine to 5-hydroxyleucine (Fig. 5).

### (b) Mps-2 assay

The conversion of 5-hydroxyleucine to γ-methylglutamic acid γ-semialdehyde can be performed by the Mps-2 or alcohol dehydrogenase assay according to Luesch et al., 2003 who performed the assay with the functionally similar protein NosE. Typical reaction mixtures with 5-hydoxyleucine or related compounds as substrate may contain 100 mM glycine (pH 10), 2 mM β-NAD, substrate (0.1 - 10 mM), 1 mM ZnSO₄, and approximately 3 µg of Mps-2. Typical reaction volumes may be 500 µl, reactions may be initiated by the addition of Mps-2 and incubated at 30-42°C for 1-3 h.

### (c) Mps-4 assay

The conversion of 3-methyl-Δ¹-pyrroline-5-carboxylic acid to 4-methylproline can be performed in the Mps-4 assay according to Luesch et al., 2003 who performed the assay with the functionally similar protein NosF. Typical reaction mixtures may contain 200 mM Tris (pH 8), substrate, 0.2 mM β-NADH or ß-NADPH, and approximately 5 µg of Mps-4. Reaction volumes are usually 1 ml, reactions may be initiated by the addition of Mps-4 and incubated at 30-42°C for 1-3 h. An alternative Mps-4 assay according to Becker et al., 1997 comprises 3-methyl-Δ¹-pyrroline-5-carboxylic acid or related compounds as substrate and may be conducted in 50 mM Tris/HCI, pH 7.5, and include substrate (0.1-10 mM), 0.2 mM FAD, 0.5 mM NADH and an appropriate dilution of Mps-4 (e.g. 1 µg final concentration in a total volume of 100-500 µl. Reactions may be initiated by the addition of Mps-4 and incubated at 30°C for 1-3 hours.

### Example 4: In vitro-synthesis of griselimycin and methylgriselimycin

In vitro-synthesis of griselimycin and methylgriselimycin can be performed according to reaction conditions as disclosed in Gaitatzis N. et al., 2001. Incubations of 1 ml each may contain 25 mM Tris HCl (pH 8.0), 50 mM NaCl, 1 mM acetyl-CoA (or 1 mM acetyl-SNAC), 2 mM L-valine, 2 to 3 mM (2S, 4R)-4-methylproline (or related compounds), 3 mM L-leucine, 1 mM L-proline (or no L-proline for the methylgriselimycin synthesis), 1 mM L-threonine, 4 mM S-adenosyl-L-methionine, 1 mM glycine, 10 mM ATP, 0.5-5 mM NRPS-1, NRPS-2, NRPS-3 and MtbH. The reactions may be incubated at 30°C after starting the reaction with ATP for 2-72 hours.

### Example 5: Inactivation of the genes nrps-1 and nrps-3 and of the mps operon

Insertional mutants of *Streptomyces DSM 22643* were created according to standard procedures. Fragments of about 3000 bp in length which are homologues to parts of the mps-operon, the nrps-1 and the nrps-3 gene, respectively, were amplified from chromosomal DNA of *DSM 26643* by PCR using the primers listed in Table 4. These fragments were cloned in the *E. coli* cloning vector pBluescript SK+ giving the plasmids pMPS, pNRPS-1, pNRPS-3. Subsequently, an AprR-oriT cassette, created as described in Gust et al., 2003, was amplified with the primers listed in Table 5 and integrated into the plasmids pMPS, pNRPS-1 and pNRPS-3 with the RedET technology (Genebridges) to give conjugational plasmids. The created plasmids were then transferred into *DSM 22643* and conjugants were analysed for correct genetic integration into the mps-operon, the nrps-1 and the nrps-3 gene, respectively, before being tested for (methyl)griselimycin production.

**Table 4: Primer for amplification of homologous DNA-fragments**

| Mutant | Primer | Sequence | SEQ ID NO |
|---|---|---|---|
| Nrps-1 | Nrps-1_for | GCTCTAGAGTCATGCAGATCAACTCG | 30 |
| | Nrps-1_rev | GCTCTAGATGGTCACGGACCTGTG | 31 |
| Nrps-3 | Nrps-3_for | GCTCTAGAATGGAGGGCATCGGAAT | 32 |
| | Nrps-3_rev | GCTCTAGAAGCTCTTCCGCCAGGAT | 33 |
| mps | Mps_for1 | CGGGGTACCCATCTGGTCCGTACCAT | 34 |
| | Mps_rev1 | TCGCGTCGTTTACCTCA | 35 |
| | Mps_for2 | GCCTTCACCGGAGCTCA | 36 |
| | Mps_rev2 | GCTCTAGATCCTGAACTCTCGCATA | 37 |

| | | | |
|---|---|---|---|
| Underlined: sequences for restriction sites | | | |

**Table 5: Primer for insertion of AprR-oriT-cassette**

| Mutant | Primer | Sequence | SEQ ID NO |
|---|---|---|---|
| Nrps-1 | Nrps-1_for2 | TGAGGTGTGTGCTGCGTCTGTTCCGGGGGC GACTGCGGTATTCCGGGGATCCGTCGACC | 38 |
| | Nrps-1_rev2 | TGACACGTCGTGGCGTGCACCGTCGTCTCC GTCGGCCCGTGTAGGCTGGAGCTGCTTC | 39 |
| Nrps-3 | Nrps-3_for2 | TCCGGATGCGACGGCAGTCGTCTTTGAGA CACGAGCGTATTCCGGGGATCCGTCGACC | 40 |
| | Nrps-3_rev2 | AGCATCACGTCGAACGATTGTCTGCGGTG TCCATGTCTTGTAGGCTGGAGCTGCTTC | 41 |
| mps | Mps_for3 | ATGAGCGTAGGTCTCCGTCTGTGGTAACC CCTGGTGTGATTCCGGGGATCCGTCGACC | 42 |
| | Mps_rev3 | TCAGGTATCACCGTGGGAAGGTGCCGGCT TTCCAGTACTGTAGGCTGGAGCTGCTTC | 43 |

| | | | |
|---|---|---|---|
| Underlined: sequences homologous to theAprR-oriT cassette | | | |

To verify griselimycin production in the wild type and to test the insertional mutants for the ability of griselimycin synthesis, the different strains were cultured in medium NL5645 (composition (amount in percentage): lactose 2.0, soy meal 2.5, NaCl 0.5, CaCO₃ 0.1 and yeast extract 0.5, pH 6.8) in shaking flasks. At different time points the culture supernatant was collected, filtered and mixed 1:1 with ethanol. After precipitation and separation of unsolved substances the supernatant was analysed by UHPLC (Ultra High Performance Liquid Chromatography) for (methyl)griselimycin production.

As can be taken from Fig. 6 the wildtype strain *DSM 22643* produces griselimycin (see "griselimycin" marked peak at 5.29 min in the UHPLC chromatogram (UV210 nm)), whereas the nrps-1, the nrps-3 and the mps-mutants of *DSM 22643* do not produce griselimycin (absence of the peark at 5.29 min). This experiment confirms the importance of the NRPS proteins and Mps proteins for the synthesis of griselimycin and methylgriselimycin.

### Example 6: Complementation of mps-mutants

To demonstrate that the genes of the mps-operon are relevant for synthesis of (2S, 4R)-4-methylproline the mps-mutant was complemented (1) by feeding (2S, 4R)-4-methylproline and (2) by genetic complementation due to the chromosomal integration of an expression construct, where the mps operon is expressed from the constitutive ermE-promoter.

In the feeding experiment 20 µg/ml (2S, 4R)-4-methylproline were fed to cultures of the mps-mutant and (methyl)griselimycin production was analysed in the culture supernatant as described in Example 5. Whereas in the normal culture medium without (2S, 4R)-4-methylproline feeding the mps-mutant does not produce any (methyl)griselimycin), feeding of 20 µg leads to (methyl)griselimycin production (Fig. 7A; also example 9).

For the genetic complementation of the mps-mutant, a construct, P(ermE)-mps, comprising the mps operon with the genes mps-1, mps-2, mps-3 and mps-4 under the control of the constitutive ermE promoter was created in a conjugational vector with an attP attachment site for integrase mediated integration into the chromosomal attB site (see Fig. 7B for the sequence of the ermE-promoter). After integration of this P(ermE)-mps construct into the mps-mutant created in Example 5 the complemented mps-mutant was tested for griselimycin production. As shown in Fig. 7C griselimycin production could be restored in the complemented mps-mutant.

Both experiments clearly indicate that the mps operon is necessary for the synthesis of (2S, 4R)-4-methylproline, that is then used for methylgriselimycin synthesis.

### Example 7: Heterologous production of methylproline by expression of the mpsl-4 gene set in Escherichia coli and Streptomyces coelicolor

To confirm that the gene set *mpsl-4* encodes the enzymatic machinery for 4-methylproline biosynthesis constructs for the expression in *E. coli* (pET28-mps) and *S*. *coelicolor* (pUWL201-mps) were generated.
(a) Generation of pUWL201-mps and heterologous expression in *Streptomyces coelicolor*
   A ∼ 5.8 kb *Eco*RI fragment from Cosmid B:L23 harbouring the 3' end from the griselimycin biosynthetic gene cluster (54283 - 60163 nt from SEQ ID NO:1) was subcloned into the replicative *E. coli*/*streptomyces* shuttle vector pUWL201 under control of the constitutive *PermE** promotor. The resulting expression construct pUWL201-mps was subsequently transformed into the host strain *Streptomyces coelicolor* A3(2) using the protoplast method (Kieser et al., 2000). The transformants (*S*. *coelicolor*/pUWL201-mps) were cultivated in parallel to the wildtype strain in 50 ml TSB medium for 4 days at 30°C in baffled shaking flasks (mutant cultures were supplemented with thiostreptone 20 µg/ml final concentration). Cells were harvested by centrifugation and extracted with 50 ml methanol (15 min sonication and 30 min stirring). After filtration the extract was evaporated to dryness and derivatized as described below.
(b) Generation of pET28-mps and heterologous expression in *Escherichia coli*
   A ∼3.7 kb *Xba*I/*Eco*RI fragment from the Cosmid B:L23-CmR was subcloned into the expression vector pET28b (Novagen) linearized with *Nhe*I/*Eco*RI under control of the inducible T7 promotor. The cloned 3.7 kb DNA fragment harbors the genes *mpsl-4* (56434 - 60163 nt from SEQ ID NO:1 extended with the sequence CTAGA at the 5' end). The resulting expression construct pET28-mps as well as the empty expression vector pET28b were subsequently transformed into the host strain *Escherichia coli* BL21 (DE3)/Codonplus/pL1SL2 by electroporation. The transformants (*E. coli* BL21 (DE3)/Codonplus/pL1SL2/pET28-mps or pET28b) were cultivated in 50 ml LB medium containing 0.2 % glucose supplemented with kanamycin (50 µg/ml final concentration), ampicillin (50 µg/ml final concentration) and chloramphenicol (25 µg/ml final concentration) at 30°C. Cultures were grown until OD₆₀₀ = 0.6, expression was induced with IPTG (0.3 mM final concentration) and incubation was continued for additional 4 hours at 30°C. Cells were harvested by centrifugation and extracted with 50 ml methanol (15 min sonication and 20 min stirring). After filtration the extract was evaporated to dryness and derivatized as described below.
(c) Derivatization of the extracts and HPLC-MS analysis
   The dried cell extracts as well as (2S,4R)-4-methylproline (as reference standard for retention time and fragmentation spectra) were resuspended in 200 µl coupling buffer (acetonitrile:pyridine:triethylamine:H₂O) 50 µl of PITC (phenylisothiocyanat) solution were added and after 5 min reaction at room temperature 50 µl H₂O were added to stop the reaction. The solution was evaporated to dryness by speedvac evaporation. The resulting PITC amino acids were dissolved in 1 ml water:acetonitrile (7:2), centrifuged and the supernatant was dried by speedvac evaporation. The derivatized extracts and the derivatized (*2S*,*4R*)-4-methylproline reference were then dissolved in 100 µl water/acetonitrile for HPLC/MS analysis.

All measurements were performed on a Dionex Ultimate 3000 RSLC system using a Waters BEH C18, 50 x 2.1 mm, 1.7 µm dp column. Two µl of sample was injected unless otherwise stated. Separation was achieved by a linear gradient with (A) H₂O + 0.1 % FA to (B) ACN + 0.1 % FA at a flow rate of 600 µl/min and 45 °C. The gradient was initiated by a 0.33 min isocratic step at 5 % B followed by an increase to 95 % B in 9 min to end up with a 1.6 min flush step at 95 % B before re-equilibration with initial conditions. UV and MS detection were performed simultaneously. Coupling the HPLC to a MS was supported by an Advion Triversa Nanomate nano-ESI system attached to a Thermo Fisher Orbitrap. Mass spectra were acquired in centroid mode ranging from 100-500 m/z at a resolution of R = 30000. Single reaction monitoring using collision induced dissociation was performed on 247.11, 249.12, 265.12, and 267.12 m/z. All parent ions were isolated within a 2 m/z window and fragmented at 35 % CID energy.

The expected derivative of 4-methylproline could be detected in the derivatized extracts from the cultures harboring the *mps* expression constructs (pUWL201-mps and pET28-mps), but not in the derivatized extracts of the negative controls as shown in Fig. 8 exemplified with the heterologous expression in *S*. *coelicolor* (*S*. *coelicolor*/puWL201-mps compared to *S*. *coelicolor* WT). By comparison of the retention times and high resolution mass data (including fragmentation data) with the derivatized PITC-(*2S*,*4R*)-4-methylproline reference substance, this compound could be clearly identified in the extracts of *mps* expression cultures.

This unambiguously proofs that the gene set *mpsl-4* encodes all necessary enzymatic activities required for the biosynthesis of the griselimycin precursor 4-methylproline. In addition, derivatives of two proposed intermediates of the pathway (5-hydroxyleucine and γ-methylglutamic acid γ-semialdehyde, see Fig. 8) could be detected providing further proof of the postulated enzymatic activities. The function of the putative leucine hydroxylase could be further verified by *in vitro* studies using the purified enzyme (as described above in Example 5). A derivative of the third postulated intermediate 3-methyl-Δ¹-pyrroline-5-carboxylic acid could not be detected as this compound cannot be derivatized with the applied method.

The successful heterologous expression of the *mps* genes in two different host strains (*E. coli* and *S*. *coelicolor*) also shows that this strategy can be applied to generate 4-methylproline, e.g. for organic synthesis approaches towards griselimycin or to feed the precursor to griselimycin/methylgriselimycin production cultures to enhance the methylgriselimycin yield.

### Example 8: In silico analysis of A domain specificity

An in silico analysis of the amino acid specificity of the A domains of the NRPS proteins was performed according to Challis et al., 2000. Briefly the A domain sequences were aligned with the sequence of the phenylalanine-activating A domain (PheA) of the gramicidin S synthetase GrsA. Based on this alignment, the eight amino acids lining the binding pocket were identified and compared with the specificity-conferring amino acids from A domains with known substrate specificity. In the following Table 6 the specificity of the A domains of NRPS-1, NRPS-2 and NRPS-3 for substrates as predicted in silico is presented. In comparison, the actual substrates as they are activated for introduction into (methyl)griselimycin are listed.

"Residue position" are the eight amino acids of the individual A domains, which are thought to be important for substrate recognition (`specificity-conferring amino acids' or 'specificity-conferring code'). These residues were compared with the 'specificity-conferring codes' of A domains with known substrate specificity (http://nrps.igs.umaryland.edu/nrps/blast.html).

**Table 6**

| A domain | Activated substrate(s)^{a} | In silico substrate prediction^{b} | Residue position^{c} | | | | | | | |
|---|---|---|---|---|---|---|---|---|---|---|
| | | | 265 | 266 | 269 | 278 | 299 | 301 | 326 | 330 |
| NRPS-1-A1 | Valine | Threonine | D | F | F | C | V | G | I | V |
| NRPS-1-A2 | (4R)Me-Proline | Proline | D | V | Q | F | A | G | H | A |
| NRPS-1-A3 | Threonine | Threonine | D | F | w | N | V | G | M | V |
| NRPS-1-A4 | Leucine | Phenylalanine/ Tryptophane | D | A | L | L | L | G | A | V |
| NRPS-1-A5 | (4R)Me-Proline | Proline | D | V | Q | F | A | G | H | A |
| NRPS-1-A6 | Leucine | Phenylalanine/ Tryptophane | D | A | L | L | L | G | A | V |
| NRPS-2-A7 | Valine | Threonine | D | F | F | C | V | G | I | V |
| NRPS-2-A8 | Proline/ (4R)Me-Pro | Proline | D | V | Q | F | A | A | H | V |
| NRPS-2-A9 | Leucine | Phenylalanine/ Tryptophane | D | A | L | L | L | G | A | V |
| NRPS-3-A10 | Glycine | Glycine | D | I | L | Q | L | G | V | I |
| NosA-A3^{d} | (4S)Me-Pro | Proline | D | V | Q | F | I | A | H | L |
| NcpB-A3^{e} | (4S)Me-Pro | Proline | D | V | Q | F | I | A | H | V |
| NosD-A2^{f} | Pro | Proline | D | V | Q | F | I | A | H | V |

| | | | | | | | | | | |
|---|---|---|---|---|---|---|---|---|---|---|
| ^{a} According to griselimycin and methylgriselimycin primary structures ^{b} According to the comparison of the specificity conferring code of other A domains (http://nrps.igs.umaryland.edu/nrps/blast.html) ^{c} The residues correspond to the gramicidin S synthetase PheA numbering (Challis et al., 2000) ^{d} Accession number: AF204805_1 (Hoffmann et al., 2003) ^{e} Accession number: AA026334 (Luesch et al., 2003) ^{f} Accession number: AAF17281 (Hoffmann et al., 2003) | | | | | | | | | | |

The results for the A3 domain, the A10 domain and partially the A8 domain are consistent with the amino acids which are actually incorporated into (methyl)griselimycin. The remaining results do not seem to be in alignment with the actually incorporated amino acids, which may be due to the method applied. Based on the eight amino acid residues of the synthetases which were believed to be essential for the specificity of the substrate amino acid to be incorporated into griselimycin, it is not possible to differ between proline and methylproline specific A domains.

Example 9: Phylogenetic analysis of methylproline and proline specific domains Phylogenetic analysis as presented in Fig. 9 of A domains which are specific for methylproline and proline demonstrate that A domains from methylproline incorporating NRPS modules (NRPS-1-A2 and NRPS-1-A5 and NepB8(mPro) and NosA-A(mPro)) are more closely related to each other than to the A domains of proline incorporating domains (NRPS-2-A8 and NosD(mPro)). This suggests, that the specificity is not only determined by the eight amino acid residues as presented in Table 6.

The analysis was performed using the Geneious Tree Builder function from the Geneious Pro 5.4.3 software (Tree Alignment Options: Cost Matrix (Blosum 62), Gap open penalty (12), Gap extension penalty (3) and Alignment type (Global alignment); Tree Builder Options: Genetic distance model (Jukes-Cantor), Tree build method (Neighbor-Joining), Outgroup (No outgroup)). The scale bar represents 0.09 substitutions per amino acid site.

### Example 10: Specificity of A domains of modules 2, 5 and 8

In order to gain insight into the specificity of the A domains of modules 2, 5 and 8, the domains were recombinantly produced and their specificity analysed via an ATP/PPi exchange assay in vitro. Results are shown in Fig. 10. As expected, specificity of the domains A2 and A5 is highest for (2S,4R)-4-methylproline. Interestingly, this is also the case for domain A8, although L-proline is preferably incorporated by module 8 during the biosynthesis of griselimycin. It is shown that there is also a high specificity for L-proline. It is assumed that the C domain of module 8 preferably condenses proline and introduces it into the growing peptide chain. Availability of methylproline during (methyl)griselimycin biosynthesis seems also to play a role as could be shown by feeding experiments.

This Example is concerned merely with analysing the specificity of the A domains in vitro. Interestingly, the A8 exhibits a higher specificity for MePro than for Pro in the in vitro experiment, although proline is preferably incorporated under standard culturing conditions. Consequently, we have speculated that this may be due to the specificity of the C domain and/or the availability of proline in the cell. Indeed, it was possible to shift the griselimycin/methylgriselimycin ratio toward methylgriselimycin by feeding Me-Pro to the WT. This experiment was carried out by Sanofi; we had then suggested the incorporation of these data by way of an Example, and as a result, additions were made to page 15/line 15 as well as to this Example (see the two sentences below). I think it would be good to state the result here once more, since it is helpful for interpreting the in vitro studies. However, we also think it is important to list the data again separately by way of an Example. The experiment was carried out at Sanofi, and we are unfortunately unable to provide any details here.

Feeding of 4-methylproline in concentrations from 0.025 to 1.0 g/l shows an increase of methylgriselimycin up to fivefold higher than the control.

The crucial experiment in this context is the feeding of MePro to the wild-type strain (not the complementation studies in Fig. 7). As already noted above, the experiment was carried out at Sanofi and is mentioned on page 15/line 15 and here on page 62, at the end of Example 10. It is not listed as an additional Example and would still have to be inserted by Sanofi (see also old comments 11 and 12 on page 40).

The experiments show also that the incorporation rate is not linear but reaches a saturation at about 0,5 g/l. Also the feeding with trans-hydroxyproline and trans-fluorproline showed significant incorporation.

### (a) Production of recombinant A2, A5 and A8 domains

In order to directly investigate A domain substrate specificity, we aimed to express A domains A2, A5 and A8 from the griselimycin pathway as N-terminally His₆-tagged proteins from pET28b (+). DNA fragments coding for the adenylation domains of A2, A5 and A8 were amplified from genomic DNA from the *Streptomyces DSM 22643* using the oligos A2_for 5'-CCGACCATATGGATCCGGATGTGACGGTGGG-3' (SEQ ID NO: 44) and A2_rev 5'-ACCGGGAATTCGCCGACGGCGGGCAGGCCGA-3' (SEQ ID NO: 45) (due to the high homology, all three A domains can be amplified by using the same primer pair), and cloned into pJET2.1 vector by blunt end ligation.

Sequencing revealed that all of the A domains were correct. According to the molecular structure of griselimycin and the *in silico* analysis A domains A2 and A5 were expected to incorporate 2S,4R-methylproline. A domain A8 was expected to show a broader substrate specificity and either incorporate L-proline or *2S,4R*-methylproline. To determine this *in vitro* we expressed the A domains in *E. coli* Rosetta pLys(RARE) at 16°C overnight. All three A domains could be obtained in the soluble fractions.

The proteins were then purified with Ni⁺-affinity chromatography using a stepwise imidazol gradient. Pure protein could be obtained in fraction 8 for all A domains. Although the amount of protein was very low (0.2 mg/ml), it could be used in the ATP-PPᵢ exchange assay to determine substrate specificity.

Substrate specificity of the 3 purified adenylation domains was evaluated using the established ATP-PPᵢ exchange assay (Mootz and Marahiel. 1997, Thomas et al., 2002). Briefly, each protein was incubated with a panel of different amino acids, including the anticipated substrate of each domain. As a control, each protein was incubated in the absence of added amino acid, to determine the radioactivity background reaction. Nine different substrates were tested (see Fig. 10).

### (b) Determination of substrate specificity by ATP-[³²P]PPᵢ exchange assay

To determine substrate specificity, ATP-[³²P]PPi reactions (100 µl) containing Tris-HCl (pH 7.5, 75 mM), MgCl₂ (10 mM), dATP (5 mM), amino acid (5 mM), and protein (2 µg) were performed at 30°C. ³²P-tetrasodium pyrophosphate was obtained from Perkin Elmer (NEN #NEX019). The reactions were started by the addition of [³²P]PPi (0.1 µCi final amount) for up to 30 min before quenching with charcoal suspensions (500 µl, 1.6% [w/v] activated charcoal, 0.1 M Na₄P₂O₇, and 0.35 M perchloric acid in H₂O). The charcoal was pelleted by centrifugation prior to being washed twice with the washing solution 0.1 M Na₄P₂O₇ and 0.35 M perchloric acid in H₂O), resuspended in H₂O (500 µl), and counted by liquid scintillation (Beckman LS6500).

### Example 11: Transcriptional regulators of the Xre-type encoded by orf-1 and orf-12 of the griselimycin biosynthetic cluster

To elucidate an involvement of two putative transcriptional regulators encoded by orf-1 and orf-12 in production of griselimycin inactivation mutants of the respective ORFs were generated. Subsequently, effects on production of griselimycin were determined by HPLC/MS analysis of extracts of the wild type strain ST105671 and the mutant strains ST105671-orf and ST105671-orf-26, respectively. In order to determine whether effects on production observed in the inactivation mutants are due to direct regulation by the transcription regulators encoded by orf-1 and orf-12, the regulators were heterologously expressed and purified. The purified proteins were used in electrophoretic mobility shift assays (EMSA) to examine direct interaction to putative promoter regions of the griselimycin biosynthetic cluster.

### (a) Inactivation of orf-1 and orf-12

The genes orf-1 and orf-12 were disrupted using derivatives of the pKC1132 plasmid integrating in orf-1 and orf-12 by homologous recombination. Two derivatives of pKC1132 (pKC1132-orf-1 and pKC1132-orf-26, respectively) were constructed carrying the central part of the coding region of either orf-1 or orf-12. Therefore, a 623 bp DNA fragment of orf-1 and a 1139 bp DNA fragment of orf-12 were amplified by PCR reactions using the primer combinations dxregrise_for/dxregrise_rev and dxreIIgrise_for/dxreIIgrise_rev, respectively (primer sequences see Table 8). *Eco*RI restriction sites were thereby attached to both ends of the fragments by the used primers. The DNA fragments and plasmid pKC1132 were digested using *Eco*RI and subsequently subjected to ligation. The ligations containing the desired plasmids pKC1132-orf-1 and pKC1132-orf-26, respectively, were transformed into electrocompetent *E.coli* DH10B cells. Transformants were selected on LB agar plates containing 60 µg/ml apramycin. Single colonies were grown in liquid LB medium in the presence of 60 µg/ml apramycin and plasmids were prepared. Plasmids carrying the correct inserts (pKC1132-orf-1 and pKC1132-orf-26) were identified by analytical digestion using *Eco*RI. *E.coli* ET12567 cells harboring the non-permissive plasmid pUZ8002 were then transformed using the correct plasmids pKC1132-orf-1 and pKC1132-orf-26. Transformants were selected on LB agar plates containing 60 µg/ml apramycin to yield strains *E.coli* ET12567pUZ8002pKC1132-orf-1 and *E.coli* ET12567pUZ8002pKC1132-orf-26. These strains were used to deliver the derivatives of plasmid pKC1132 into strain ST105671 by conjugation. The procedure was performed as follows. 2 ml of 2 day old liquid cultures of *Streptomyces* strain ST105671 grown in griselimycin production medium 5288 and in TSB medium at 30°C and 2 ml of the respective *E.coli* strains harboring plasmids pKC1132-orf-1 or pKC1132-orf-26 (grown in LB containing 25 µg/ml kanamycin, 25 µg/ml chloramphenicol and 60 µg/ml apramycin) were pelleted, washed with sterile water and resuspended in 500 µl TSB each. 250 µl of *Streptomyces* strain ST105671 and the respective *E.coli* strain were mixed and incubated overnight under aeration at 30°C. 1 ml of TSB containing 60 µg/ml apramycin and 25 µg/ml nalidixic acid was added and further incubated overnight at 30°C under aeration. After incubation cells were pelleted, washed once with TSB and plated out on MS-agar containing 60 µg/ml apramycin and 25 µg/ml nalidixic acid, followed by further incubation at 30°C until conjugants became visible. Correct integration of the plasmids pKC1132-orf-1 or pKC1132-orf-26 in the coding regions of orf-1 or orf-12, respectively, was verified by performing PCR using genomic DNA of the conjugants and combinations of primers lacZ1, lacZ2 and grixreI_g1, grixreI_g2 in case of disruption of orf-1 or grixreII_g1, grixreII_g2 in case of disruption of orf-12, respectively. The inactivation mutants of ST105671-orf-1 and ST105671-orf-26 generated by this procedure were used to determine effects on griselimycin production compared to the ST105671 predecessor.

### (b) HPLC/MS analysis of extracts of ST105671-orf-1 and ST105671-orf-26

Liquid cultures of four independent inactivation mutants of orf-1 and of two independent inactivation mutants of orf-12 were grown for two days in TSB liquid medium containing 60 µg/ml apramycin. Using these cultures, 100 ml of fresh TSB medium without antibiotic were inoculated 1:50 in duplicates each and grown for 3 days. Wild type ST105671 cells were treated equally without using apramycin in the preculture. Cells were pelleted by centrifugation and the supernatant was used for ethylacetate extraction. To monitor effects on griselimycin production the extracts were subsequently analyzed by HPLC/MS analysis. Quantities of griselimycin of each mutant and the wild type strain ST105671 relative to the total protein amount of each culture were determined. Therefore, the respective peak areas were integrated and the counts were set relative to the total protein amount of the pelleted cells. Mean values and standard deviations were calculated. Inactivation of orf-1 led to a more than 200 fold decrease in griselimycin production. No griselimycin could be detected in the inactivation mutant of orf-12 (see Table 7). The observations show an involvement of the Xre-type transcriptional regulators encoded by orf-1 and orf-12 in regulation of griselimycin production. Whether these effects are a result of direct regulation of promoters within the griselimycin biosynthetic cluster by binding of the Xre-type regulators or depict an indirect effect should be elucidated by performing DNA protein interaction studies (electrophoretic mobility shift assays, EMSA).

**Table 7**

| strain | no. of replicates | mean (rel. intensity) | std dev |
|---|---|---|---|
| ST105671 | 4 | 7,38*10⁹ | 4,59*10⁹ |
| ST105671-orf-1 | 8 | 3,19*10⁷ | 1,41*10⁷ |
| ST105671-orf-26 | 8 | - | - |

Table 7: Effects on griselimycin production in mutants ST105671-orf-1 and ST105671-orf 26. Quantities of griselimycin of each mutant and the wild type strain ST105671 are shown as mean values of counts of extracts obtained relative to the whole protein content from independently grown replicate cultures.

### (c) Verification of specific binding of the transcription regulators Orf-1 and Orf-12 to the promoter regions of nrps1 and nrps2

To examine a direct transcriptional regulation of griselimycin biosynthetic genes by regulators Orf-1 and Orf-12, possible binding to the putative promoter regions upstream of genes nrps1 and nrps2 was determined. Therefore, the coding regions of orf-1 and orf-12 were first amplified in PCR reactions with primer combinations xreI-NdeI-for/xreI-HindIII-rev and xreII-NdeI-for/xreII-HindIII-rev using genomic DNA of strain ST105671 as a template. *Nde*I restriction sites were thereby attached to the 5' ends and *Hind*III restriction sites attached to the 3' ends of the resulting DNA fragments. Plasmid pET22b and the amplified DNA fragments carrying genes orf-1 and orf-12 were digested with *Nde*I and *Hind*III. Subsequently, *Nde*I/*Hind*III digested orf-1 and orf-12 were ligated with *Nde*I/*Hind*III digested pET22b in two separate attempts. Ligations were transformed into *E.coli* DH10B and transformants selected on LB agar plates containing 200 µg/ml ampicillin. Plasmids were prepared and correct integration of the inserts (orf-1 and orf-12, respectively) into the plasmids was verified by *Hind*III/*Nde*I digests. Plasmids carrying the inserts were finally subjected to DNA sequencing in order to exclude mutations in the DNA sequence. Due to the cloning strategy, the resulting plasmids pET22b-orf and pET22b-orf-26 encoded for C-terminally 6 fold His tagged (6xHis) Orf-1 and Orf-12, respectively. Both plasmids were transformed into *E.coli* BL21(DE3) by electroporation and transformants were selected on LB agar plates containing 200 µg/ml ampicillin. Expression of the C-terminal 6xHis tag fusion proteins Orf-1-His and Orf-12-His was performed in LB liquid medium containing 200 µg/ml ampicillin in the presence of 0.1 mM IPTG at 16°C overnight. Cells were lysed by sonication and purification of Orf-1-His (∼33kDa) and Orf-12-His (∼47kDa) was achieved by using Ni²⁺ affinity columns.

Putative promoter region of genes nrps1 (581 bp region upstream of nrps1) and nrps2 (632 bp region upstream of nrps2) were amplified in PCR reactions using primer combinations prom4hex2_rev/prom4hex3_for and prom5hex2_rev/prom5hex3_for, respectively, to yield DNA fragments carrying 18 bp linker regions on both ends. In a second PCR reaction these DNA fragments were labeled with a Hex fluorophor at the 5' end of each strand. At this, primers (Hex2 and Hex3) carrying a 5' Hex fluorophor and being reverse complementary to the 18 bp linker regions attached in the first PCR reactions were used. Hex labeled DNA fragments spanning the above mentioned upstream regions of genes nrps1 and nrps2 were thereby generated and referred to as Pnrps1 and Pnrps2. In a parallel attempt, a 696 bp intergenic region flanked by genes mxan_3950 and mxan_3951 of the annotated genome of *Myxococcus xanthus* DK1622 comprising a dual promoter system was amplified with primers EH33951_rev/EH33950_rev using genomic DNA of the respective strain. As the primers carried the same 18 bp linker regions like mentioned above, the DNA fragment could subsequently be labeled with the Hex fluorophor at both 5' ends like described before. As specific binding of regulators Orf-1-His and Orf-12-His to this promoter region out of the genome of *Myxococcus xanthus* DK1622 cannot be expected, the resulting DNA fragment referred to as Pmxan_ctrl served as a negative control in the EMSA assays. These DNA fragments as well as both purified regulators (Orf-1-His and Orf-12-His) were subsequently used in EMSA assays. In brief, the assays were performed as follows. 4% native PAA-gels were prepared in 1x buffer HG (25 mM HEPES, 192 mM glycin, pH 7.3 with KOH). Pockets of the gel were rinsed and electrophoresis was performed for 30 min at 120 V without samples in 1x buffer HG. Samples (100 fMol of each DNA-fragment Pnrps1, Pnrps2 and Pmxan_ctrl with and without 500 fold molar excess of either Orf1-His or Orf-12-His and 2.5 µg high molecular weight salmon sperm DNA as competitor DNA) were prepared and incubated for 10 min at 30°C. Samples were loaded on the gel and electrophoresis was performed for 2 h at 120 V. Detection of the DNA fragments was done using a Typhoon 9410 imager (Amersham) at the respective wavelength. In the presence of Orf-1-His, a retardation of each Hex labeled DNA fragment compared to the attempts without Orf-1-His could be observed in electrophoresis indicating a non specific binding of regulator Orf-1-His to DNA comprising a promoter region (Fig. 11). On the other hand, Orf-12-His exhibited no ability to interact with any of the DNA fragments demonstrating no specificity towards the Hex labeled DNA used in the assays. Taken together these results suggested that neither Orf-1-His nor Orf-12-His are direct transcriptional regulators of the promoters Pnrps1 and Pnrps2.

Xre-type regulators Orf-1 and Orf-12 show remarkable aspects regarding their predicted domain organization which was determined using the SMART tool at http://smart.emblheidelberg.de/ (Fig. 12). As expected, both regulators possess an N-terminal Xre-type helix-turn-helix (HTH) motif whereby Orf-1 obviously contains a second domain of this kind (HTH2). In addition to this, both regulators are characterized by an extended C-terminus with unknown function.

As it was possible, that these extraordinary features are part of regulatory mechanisms which influence affinity and thereby specificity towards distinct DNA sequences of regulators Orf-1 and Orf-12 the EMSA assays were repeated using C-terminally shortened versions of the regulators. In case of Orf-1, these versions only comprised HTH1, HTH2 or both predicted HTH domains. The respective proteins were named ORF1-HTH1 (∼9.6kDa), ORF1-HTH2 (∼9.8kDa) and ORF1-HTH12 (16.4kDa). In case of Orf-12, the shortened version only contained the N-terminal HTH domain named ORF26-HTH (∼9.9kDa). Regulatory effects (e.g. binding of unknown cofactors or protein-protein interactions) resulting in conformational changes of the C-terminus or the whole protein and thereby influencing DNA binding affinity of the regulators should be excluded.

The shortened versions of Orf-1 and Orf-12 were obtained by cloning of the respective 5' regions of orf-1 and orf-12 as *Nde*I/*Hin*dIII fragments into *Nde*I/*Hind*III digested pET22b to yield the encoded C-terminal 6xHis tagged fusion proteins. Cloning was performed as described in case of the full length Orf-1 and Orf-12. Primer pairs used for amplification of the inserts to be cloned were xreI-NdeI-for/xreIhth1rev (Orf-1-HTH1), xreIhth2for/xreIhth12rev (Orf1-HTH2), xreI-NdeI-for/xreIhth12rev (Orf-1-HTH12) and xreII-NdeI-for/xreIIhthrev (Orf-12-HTH). Heterologous expression, purification and subsequent verification of binding to the above mentioned promoter regions of genes nrps1 and nrps2 was performed as described for the full length proteins. The EMSA assays performed using these shortened versions of the regulators revealed a specific binding of ORF1-HTH12 to promoter Pnrps1 and of Orf-12-HTH to both promoter regions Pnrps1 and Pnrps2 (Fig. 13).

Only by co-incubation of Orf1-HTH12 and Pnrps1 a significant retardation in electrophoresis of the DNA could be achieved. This retardation could not be observed using the negative control Pmxan_ctrl. Co-incubation with Pnrps2 gave a less clear result whereby no distinct statement can be made about direct binding of Orf1-HTH12 to promoter region Pnrps2. Orf-12-HTH co-incubated with Pnrps1 or Pnrps2 resulted in clear distinct retardations of the DNA fragments whereas in case of the negative control Pmxan_ctrl a shift was completely absent.

Based on theses experimental results, roles of the C-termini of both regulators remain to be elucidated but seem to be related to modification of DNA binding specificity of Orf-1 and Orf-12.

Nevertheless, based on the effects observed after inactivation of orf-1 or orf-12 on production of griselimycin, the direct specific binding of Orf1-HTH12 to promoter Pnrps1 and of Orf-12-HTH to promoters Pnrps1 and Pnrps2, the Xre-type transcriptional regulators encoded by orf-1 and orf-12 are clearly functionally related to griselimycin biosynthesis and can be considered as a part of the secondary metabolite cluster.

**Table 8**

| primer name | sequence 5' —> 3' | SEQ ID NO |
|---|---|---|
| dxregrise_for | cgGAATTCTGAGAAGCTGCCTGCAC | 46 |
| dxregrise_rev | cgGAATTCCTCATAGACCTGAGCGGC | 47 |
| dxreIIgrise_for | cgGAATTCCGTAGGACACGGAAACTGTC | 48 |
| dxreIIgrise_rev | cgGAATTCGCATCCATCCTGAGCAG | 49 |
| EH33950_rev | GGAATGGGCCGGGTACTGCAGCCTGCTATTT CTCAGAATC | 50 |
| EH33951_rev | GGAATGGGCCGGGTACTGAGAG- CACGGGGTGTG | 51 |
| grixreI_g1 | ATCGCCTTCGGCGACAG | 52 |
| grixreI_g2 | GCTGGGCGTATCCGACTC | 53 |
| grixreII_g1 | GAAGGGACAGGTCCACCG | 54 |
| grixreII_g2 | GGTGTCCTGCACGGACAG | 55 |
| hex2 | GCTCTCGGTGCCCTTGTG | 56 |
| hex3 | GGAATGGGCCGGGTACTG | 57 |
| lacZl | CTTGGGCTGCAGGTCGAC | 58 |
| lacZ2 | GTGTGGAATTGTGAGCGG | 59 |
| prom4hex2_rev | GCTCTCGGTGCCCTTGTGTTCAC- TATCCCCATGTTGTTTCC | 60 |
| prom4hex3_for | GGAATGGCCGGGTACTGCTGCTGACTGAAC- CAGATCTCAC | 61 |
| prom5hex2_rev | GCTCTCGGTGCCCTTGTGGATCGAA- CACGGCTCAGCTC | 62 |
| prom5hex3_for | GGAATGGGCCGGGTACTGGCAG- CACTTCATGGTCGC | 63 |
| xreI-HindIII-rev | cgtAAGCTTCTCGTTCGCGCCCGTTTC | 64 |
| xreIhth12rev | cgtAAGCTTGGCGATCGACCGGTCTTC | 65 |
| xreIhth1rev | cgtAAGCTTATCAGTCAGATCGGGCGG | 66 |
| xreIhth2for | gtaaggCATATGGATCTGTTCCCGCGGTCC | 67 |
| xreII-HindIII-rev | cgtAAGCTTGGTTGTGCTAATGCCCGTCC | 68 |
| xreIIhthrev | cgtAAGCTTCTCCGCTTCCGGCTCAC | 69 |
| xreII-NdeI-for | gtaaggCATAT- GAGTGAAAACCTGACGCTGGGAG | 70 |
| xreI-NdeI-for | gtaaggCATATGTCTGCTCGTCACTTCGACCG | 71 |

Table 8: List of primers used in the experiments. Name and DNA sequence in 5' to 3' orientation of oligonucleotides are shown. Letters with simple underlining: Restriction sites attached to the primers. Letters with double underlining: 5' linker regions attached to the respective sequence. Small letters: Nucleotides attached to enhance restriction efficiency.

### Example 12: Streptomyces DSM 22643

*Streptomyces DSM 22643* was deposited according to the Budapest Treaty on the International Recognition of the Deposit of Microorganisms for the Purposes of Patent Procedure with the Deutsche Sammlung von Mikroorganismen und Zellkulturen GmbH (DSMZ), Inhoffenstraße 7B, 38124 Braunschweig, Germany.

### REFERENCES

Altschul S.F. et al., Nucleic Acids Res., 1997, 25, 3389-3402
Barry S.M. and Challis G.L., Curr. Opin. Chem. Biol., 2009, 13, 205-215
Becker D. et al., Eur. J. Biochem. 1997, 249, 739-747
Bibb, M.J. et al., Gene. 1985, 38, 215-266
Bierman M. et al., Gene 1992,116, 43-49
Challis G. L. et al., Chem. Biol. 2000, 7, 211-264
Eriko T. et al., Gene, 1995,66, 133- 137
Ferna' ndez-Moreno M.A. et al., Cell, 1991, 66, 769-780
Finking R. and Marahiel M.A., Annu. Rev. Microbiol., 2004; 58, 453-488
Gaitatzis N. et al., Proc. Natl. Acad. Sci. USA, 2001, 98, 11136-11141
Gust B et al., Proc. Natl. Acad. Sci. USA, 2003, 100, 1541-1546
Hahn M. and Stachelhaus T.; Proc. Natl. Acad. Sci. USA, 2004, 101, 15585-15590;. Epub 2004 Oct 21
Herai S. et al., Proc..Natl. Acad. Sci. USA 2004, 101, 14031-14035
Hoffmann D. et al., Gene, 2003, 311, 171-180
Kieser et al., Practical Streptomyces Genetics, The John Innes Foundation, Norwich 2000
Li W. et al., Appl. Environ. Microbiol., 2009, 75, 2869-2878
Luesch H. et al., J. Org. Chem., 2003, 68, 83-91
Marahiel M.A., J. Pept. Sci., 2009, 15, 799-807
Mervyn J. et al., Molecular Microbiology, 1994, 14(3), 533-545
Mootz H.D. and Marahiel M. A., J. Bacteriol., 1997, 179, 6843-6850
Muller C. et al., Antimicrob. Agents Chemother., 2007, 51, 1028-1037
Murakami, T. et al., J. Bacteriol., 1989, 171, 1459-1466
Peschke U. et al., Mol. Microbiol., 1995, 16, 1137-1156
Sambrook et al., Molecular cloning: A laboratory manual. Cold Spring Harbor, NY: Cold Spring Harbor Laboratory Press, 2001
Terlain B. and Thomas J.-P., Bulletin de la Sociétié Chemique de France, 1971, 6, 2657-2662
Thomas M. G. et al., Chem. Biol. 2002, 9, 171-184.
Wehmeier U. F:, Gene, 1995, 165, 149-150
Yin X. et al., Chem. Biol. Chem. 2004, 5, 1274

## Claims

1. Nucleic acid comprising at least one nucleic acid selected from:
(a) a nucleic acid comprising at least one of the Open Reading Frames (ORFs) 1 to 26 as comprised by SEQ NO: 1 encoding proteins of SEQ NOs: 2 to 27 or a variant or fragment thereof whereby the variant or fragment encodes a functionally active variant or fragment of a protein of SEQ NOs: 2 to 27,
(b) a nucleic acid encoding at least one of the proteins of SEQ NOs: 2 to 27 or a functionally active variant or fragment thereof,
(c) a nucleic acid encoding a protein that is at least 70 %, 80 %, 90 %, 95 % or 97% identical in amino acid sequence to a protein or fragment thereof encoded by the nucleic acid of (a) or (b),
(d) a nucleic acid which hybridizes under stringent conditions with a nucleic acid of (a) to (c),
(e) a nucleic acid which hybridizes under stringent conditions with a nucleic acid of (a) to (d) and consists of 10 to 50 nucleotides in length, or
(f) a nucleic acid which is complementary to a nucleic acid of (a) to (f).

2. The nucleic acid of claim 1, wherein said nucleic acid comprises at least two ORFs, preferably at least three ORFs encoding any of the proteins of SEQ NOs: 2 to 27 or a functionally active variant or fragment thereof as referred to in items (a) to (d) of claim 1.

3. The nucleic acid of claim 1 or 2, wherein said nucleic acid comprises at least one of the ORFs 18, 19, 20 and 21 encoding proteins of SEQ ID NO: 19, 20, 21 and 22, respectively, or a functionally active variant or fragment thereof as referred to in items (a) to (d) of claim 1.

4. The nucleic acid of any one of claims 1 to 3, wherein said nucleic acid comprises at least one of the ORFs 8, 15 and 16 encoding proteins of SEQ ID NO: 9, 16 and 17, respectively, or a functionally active variant or fragment thereof as referred to in items (a) to (d) of claim 1.

5. The nucleic acid according to any one of claims 1 to 4 comprising or consisting of the griselimycin biosynthesis gene cluster having the sequence of SEQ ID NO: 1 or having a variant sequence of SEQ ID NO: 1 harboring a variant or fragment of at least one of ORFs 1 to 26 whereby the variant or fragment encodes a functionally active variant or fragment of a protein of SEQ ID NOs: 2 to 27.

6. An expression vector comprising a nucleic acid of any one of claims 1 to 5.

7. A host cell transformed with an expression vector of claim 6.

8. A protein comprising at least one amino acid sequence selected from SEQ ID NOs: 2 to 27 or a functionally active variant or fragment thereof or encoded by a nucleic acid of any one of claims 1 to 5.

9. An antibody specifically directed against at least one of the proteins or a functionally active variant or fragment thereof of claim 8.

10. Use of the nucleic acid of any one of claims 1 to 5, the expression vector of claim 6, the host cell of claim 7 or the protein of claim 8, in particular of at least one of the proteins of SEQ ID NOs: 9, 16 and 17, for the production of griselimycin and/or methylgriselimycin.

11. Use of the nucleic acid of claim 3, the expression vector of claim 6 comprising the nucleic acid of claim 3, the host cell of claim 7 transformed with said expression vector or of at least one of the proteins of SEQ ID NOs: 19, 20, 21 and 22 for the production of L-trans-4-methylproline.

12. A method of producing at least one of the proteins or a functionally active variant or fragment thereof of claim 8 comprising:
a) expressing the nucleic acid of any one of claims 1 to 5, and
b) optionally isolating the protein or a functionally active variant or fragment thereof.

13. A method of determining a variant of ORFs 1 to 26 as comprised by SEQ ID NO: 1 encoding the proteins of SEQ ID NOs: 2 to 27 or of the proteins of SEQ ID NOs: 2 to 27 which modulates production of griselimycin and/or methylgriselimycin comprising
a) producing a variant of any of ORFs 1 to 26,
b) expressing the variant into a protein,
c) allowing the production of griselimycin and/or methylgriselimycin or of a derivative thereof using the protein of b), and
d1) determining the amount of griselimycin and/or methylgriselimycin,
wherein an increase of the amount of griselimycin and/or methylgriselimycin compared to the amount produced if the protein of b) is not present, but the corresponding invariant form of the protein is present is indicative that the variant is capable of increasing the production of griselimycin and/or methylgriselimycin, or
d2) determining the antibiotic activity of the derivative of griselimycin and/or methylgriselimycin,
wherein an increase in the antibiotic activity compared to the antibiotic activity if the protein of b) is not present, but the corresponding invariant form of the protein is present is indicative that the variant is capable of producing a derivative of griselimycin and/or methylgriselimycin with increased antibiotic activity,
and wherein the derivative differs in amino acid composition from griselimycin and/or methylgriselimycin and exhibits an enhanced antibiotic activity over griselimycin and/or methylgriselimycin.

14. A method of determining a variant of ORFs 1 to 26 as comprised by SEQ ID NO: 1 encoding proteins of SEQ ID NOs: 2 to 27 or of the proteins of SEQ ID NOs: 2 to 27 which enhances production of L-trans-4-methylproline comprising
a) producing a variant of any of ORFs 1 to 26,
b) expressing the variant into a protein,
c) allowing the production of L-trans-4-methylproline using the protein of b), and
d) determining the amount of L-trans-4-methylproline,
wherein an increase of the amount of L-trans-4-methylproline compared to the amount produced if the protein of b) is not present, but the corresponding invariant form of the protein is present is indicative that the variant is capable of increasing the production of L-trans-4-methylproline.

15. A method of producing griselimycin and/or methylgriselimycin comprising
a) providing at least one protein of claim 8 or a functionally active variant or fragment thereof or providing a variant protein obtainable by the method of claim 13 or 14, and
b) incubating the at least one protein of a) with L-N-methylvaline, L-leucine, L-N-methylthreonine, L-proline, L-N-methylleucine and glycine to produce griselimycin and/or methylgriselimycin.

16. A method of producing L-trans-4-methylproline comprising
a) providing at least one protein of claim 8 or a functionally active variant or fragment thereof or providing a variant protein obtainable by the method of claim 14, and
b) incubating the at least one protein of a) with L-leucine, 5-hydroxyleucine, γ-methylglutamic acid γ-semialdehyde and/or 3-methyl-Δ¹-pyrroline-5-carboxylic acid to produce L-trans-4-methylproline.

17. *Streptomyces DSM 22643.*


SEQUENCE LISTING

<110>  Sanofi

<120>  Gene cluster for biosynthesis of griselimycin and
       methylgriselimycin

<130>  DE2011/234

<160>  31

<170>  PatentIn version 3.5

<210>  1
<211>  66868
<212>  DNA
<213>  Streptomyces DSM 22643


<220>
<221>  misc_feature
<222>  (837)..(2075)
<223>  ORF 1, positive strandedness

<220>
<221>  misc_feature
<222>  (2185)..(2625)
<223>  ORF 2, positive strandedness

<220>
<221>  misc_feature
<222>  (2625)..(2819)
<223>  ORF 3, positive strandedness

<220>
<221>  misc_feature
<222>  (3033)..(4037)
<223>  ORF 4,  positive strandedness

<220>
<221>  misc_feature
<222>  (4110)..(5630)
<223>  ORF 5,  positive strandedness

<220>
<221>  misc_feature
<222>  (5627)..(7066)
<223>  ORF 6,  positive strandedness

<220>
<221>  misc_feature
<222>  (7240)..(8133)
<223>  ORF 7,  positive strandedness

<220>
<221>  misc_feature
<222>  (31885)..(8501)
<223>  ORF 8, negative strandedness

<220>
<221>  misc_feature
<222>  (33102)..(32770)
<223>  ORF 9, negative strandedness

<220>

<221>  misc_feature
<222>  (33187)..(33726)
<223>  ORF 10, positive strandedness

<220>
<221>  misc_feature
<222>  (34127)..(33567)
<223>  ORF 11, negative strandedness

<220>
<221>  misc_feature
<222>  (36172)..(37314)
<223>  ORF 13, positive strandedness

<220>
<221>  misc_feature
<222>  (36300)..(34648)
<223>  ORF 12, negative strandedness

<220>
<221>  misc_feature
<222>  (39032)..(37746)
<223>  ORF 14, negative strandedness

<220>
<221>  misc_feature
<222>  (39643)..(52149)
<223>  ORF 15, positive strandedness

<220>
<221>  misc_feature
<222>  (52146)..(56120)
<223>  ORF 16, positive strandedness

<220>
<221>  misc_feature
<222>  (56117)..(56335)
<223>  ORF 17, positive strandedness

<220>
<221>  misc_feature
<222>  (56434)..(57246)
<223>  ORF 18, positive strandedness

<220>
<221>  misc_feature
<222>  (57243)..(58418)
<223>  ORF 19, positive strandedness

<220>
<221>  misc_feature
<222>  (58445)..(59245)
<223>  ORF 20, positive strandedness

<220>
<221>  misc_feature
<222>  (59242)..(60141)
<223>  ORF 21, positive strandedness

<220>
<221>  misc_feature
<222>  (61680)..(61144)
<223>  ORF 22, negative strandedness

<220>

<221>  misc_feature
<222>  (62170)..(62820)
<223>  ORF 23, positive strandedness

<220>
<221>  misc_feature
<222>  (62824)..(63939)
<223>  ORF 24, positive strandedness

<220>
<221>  misc_feature
<222>  (63969)..(65165)
<223>  ORF 25, positive strandedness

<220>
<221>  misc_feature
<222>  (66108)..(65206)
<223>  ORF 26, negative strandedness

<400>  1
tgccccacct caacgctgtt gtgtgctggt gcctccgtac ggctatggga atgacgctat      60

gtcacatagc ttgtatgaca cgggtggttg accgccagca agcggtgcgt acgtggccct     120

ccgagtagca ggatgcacca acggccgacc ggcatcagct ttggaagagg aattttcgac     180

ggtccaggct cagctgcgag tgttcaccac tgccatggtg cccaaacggg gtggctcgct     240

gcgaggtgtg ccgctgtggt cggcggctgc gatgctccaa ccgacgatgt gctgctccgc     300

ctccccggtg aggaagtgcg gtgggttcat cccgtggagc cgcggttctc ggcgatcagg     360

ggactctggt ggtgttcttc cgaggttcgc aacaacgcga gaggccccga tcaaggtcag     420

gcaggttcca tgcggaggcg gcgcccgcag cgagaggcac tccttctggc cagtatgtct     480

gtgaccggcg gaggcggtgg gattcgacca acgggaacgc ttccacgtcc ttagggcctt     540

ccggcgagtg caggattcgc gcggacaatc tgtccgtagg tacttccacg cccacctctt     600

gactggtggg cgatgacgct actcactctg cgcccgcttc gctacgacat gccctgcttc     660

ggccgtgcgg tcacaggtcc aggagggaac aacaaggtgc ctgtggcccg tgctgtagat     720

ccacctcgtg ggcgcggggc catcgactct gttaacagtt ggcgaccaag ggcgcaggct     780

tggacattat ccgtactctg atccaagtgc ttcatcgcga agggacaggt ccaccgatga     840

gtgaaaacct gacgctggga gaccgtctcc gtatcgctcg taggacacgg aaactgtctg     900

ccgcccaact ggcacagaag gtcgcggtct ccccgagcta cgtgcagaag ttggagtcgg     960

gcgcgcgcaa ggcttcaccc tcgttggtcc tggcgctcgc caaggcgctg cgcttcggtc    1020

ccgaggtcct gaccggccag ccgtactacg gtgagccgga agcggaggac ggcgtccacg    1080

ccgtcatacc cgagctccgt cgcctcctgc tctgctacga cagccccgac gacctggaga    1140

tcgctccgcg ggcgcttccc gtgctcgcct cggaggtcga ccaggtggcg gccctacgcc    1200

gcgacgcccg ctacgccccg atgggcccgc tgctgccgcc gatcatcacc gaactgaccc    1260

acgtcgccct cggcgggaac aacggcgacc ggggcaaggc gttctggcac ctggcacgcg    1320

cctatcgcgc cgtgaactcc ctggcccaca agatgggcca ccacgacctg tcgaacacgg    1380

ctctggagcg cgtccgttgg gcggccgacc gctccggcga cccgctgatg cagttcacgg 1440
ccggctacct cgtcgcaggg gcgatgctgc gccaaggcgc gtactcgccg gcccgccgca 1500
agctccttgg gctgcggacc gagttggagc ggttccagcc ggagcactcc ttcacggagg 1560
acgcgctcgc ggtcgacggc gcgcttctgc tgaagctggc ggttctcgaa gcccgcgaga 1620
acaactccga tcgcgccgac gcgtacctgc gggaggcgga gcaggtcgcg accatggcag 1680
gcaaccgtga ctccctcgca tacgagatgt cgttcgggcc gacgaacatc cgcatccatg 1740
aggtgcacgc gatgatcgac acgggtgaca ccgagcaggc cctcgcccgc ctcgtcgaat 1800
ggtctccggt ctccggcggc gagtgggcgc cgcccagtac gaccgtcggc gagcggtcga 1860
gccaccactt catcgacgtg gcgtccgcga agctcgcaga gggcgaccgg gacggtgcct 1920
tcgcggacct caagcgcgca cggaaggtcg cgcccaacca cacgcgattc cacccgtccg 1980
ttcgcgagac cactgcggca ctgctcagga tggatgcaca cccctccaac gagctgtctg 2040
cttttgggag ttggacgggc attagcacaa cctgagccct tgtcaagggc tttggcgatc 2100
ggagcccacg gacagactgt ccgtgcagga caccttgctg aatggaatgg tctcttcaca 2160
cgcagtggtg aggaggcctt ttccatgtcc gacgacaatc ccgcagctcg cgcgaacccg 2220
ccgcgctgga tgcccatcgg cgaggagccc gagctgtgct ccgcggggga gtggtgggac 2280
gccgtacgcg ccgtagaggc cgtgggccgg cgcgcgatcg agatcctcgg cgagggtgac 2340
gaaccggtcg gcccggtgat cctggaccac ggaggcccgg agcctcggct gtacttcctg 2400
gttcccgtgg gtaccgcggc tcgctgggag gagccgggga ccgtagcgct ggggcagaag 2460
tgccacgtcg tcgtgccgtc cgcggagagc acgacgcctc cggggatgca ctggcacgtc 2520
tttccccagg gtccgcgatc gctgacgcga cctgacgcgc tgcgccgcgc gctgagccag 2580
gctcgccggg agcggcgagg gcccgcagag gaagcagcct gctgatggcc ttgagcatct 2640
ccctcgtgct ggtactcggg atcgtcgtgg tgctcctggt ccgaagcaaa gccgtgaaac 2700
ccggacccgc gatcgtctgc gtgcttttcg ggttcttcct ggcgagcacg tccatcgccc 2760
cgaacatcaa ccggttcgtg accggcgtcg ccgacatgat cggacagatt agcttctagg 2820
cggcgcgacg cggcatccga atccccttta cctgccaggc cggcggtcca ccccgatcac 2880
ttcgcggccc cgcacccccc aacttgcctg aggcccctga tgacattcat gttcagctcg 2940
gagagcacat ccccccgtac agtgcgcgcg catcggacga acggccccga tcccgttcgc 3000
cggcggcgcc tgatccgcgc cgagggactc tgatgcgtct cgtctaccac ccggctggcc 3060
atgacgacga cctacgtacg gccctggtcg aactgcaggc ggggcgttgg aagtcggcgc 3120
gggggctgct ctgggagacg ggcacgcact ggccgttgcg aacgtcacgc actcagttgc 3180
tggccgtggc agccgcccga tcagacgtgg tcgacgtctg gctcgtcgag gagccggaca 3240
gctacgacgc gcagctgatg gctgttcgtg tagccgtcga gagagcgctg cgtgctcaac 3300

gtcaacaaca tcctcggacc ttggagttcg agacgagggc gcgacgtgcc gcgctcctgg 3360
cagctcgtcg tgcgccgcac gatcccgtgc cgtgggtgtg cctcgttgcg ctggcgcaga 3420
tcgacacgca gcaactgcgt caggaacaca ggatgaggcc caacgagccg atgctgccca 3480
gcggtccgtg ggggttgttg tacgaggtca accagcgtga tccgtacaac cgcgaggcct 3540
accaccgtgt gctgcagttc ctgctcgccc ttgaggggcc gtggggcgct tctttggcgg 3600
cggtcttcga cttcggccga tccgtggcct cgcagcgtcc cgtcggatcg cctctgctgc 3660
ttctgccggc gtacgcacag atcgagcagc gtcggcaatc gcgcgccgat ccactgtggc 3720
ggcggcagtg ggcgcaggag tcgacactcg actacacact ggccgccttt cacgactggt 3780
tccacaaggc gcccgtcggg cagcactcgg tggccgatct gaacctcctg gcctacgcgt 3840
tgtgggcagg agcccagtac ctggaggccg ccgaggtgtt cgaggtgatg ggcccgtacg 3900
cggcccgtga gccgtgggcg tcggtgcacg agggggccgc agggccggac cccggcgagg 3960
cactgctcct tcgggcgcgc gccgaatccc tctcgtacgc ccgtaaccat aggccgcgcg 4020
ccgggccgca cctgtgatcg gcatccgcca gcagccaagc taattggcat gaacctgtcc 4080
agatcgtctt gagttttgga ggctgacccg tgtctcgttt gagtccgaac catcgcgcga 4140
cgtcccggaa gcccgatgac gcgtacctgc gggagctggg ctacgagccc gtgctgaccc 4200
ggcgcatggg gccgttcggg aacttcgcga tttccttcag cgtcatcagt gtcttgtccg 4260
gctgcatgac cttgtacggc ttcggcctga acactggcgg cccgtcggtg atgctgtggg 4320
gctggctgat cgtcggcgcc atggtgacgt tcatcggcgc cgcactcgcc gaggtgacct 4380
ccgcctatcc gacctccgga gccctgtact accaggcaga gcagctcggc gggcggaagt 4440
ggggctggta cacaggctgg ctcaacttgt tgggcctgct cggcgcgatc gccggcatcg 4500
actacggggc cgcgctgttc acgggcgccc tgttaaacct gcagtggggc ttcgagccga 4560
cgccgggcgg gaccatggtg atcttcctgt gcatcctcgc cctgaacctg ttcggggtcc 4620
ggctggtcag cattctcaac agcatcagcg tctggtggca cctcggcggc gtcacggtga 4680
tcgttggggc actggcgatc gtgccctctc atcaccagtc cgcagacttc gtgttcggcg 4740
agttcgtcaa caacaccggc tggtccagcc ccttgtacgt ggcagtgctc ggtctgctgc 4800
tggcccagta cacgttctgc ggttacgacg cctccgcgca cctgtcggag gagaccaccg 4860
acgcccaggt ctccgcatcc cgcggcatca tccacgcgat cgggtggtcg tggctggccg 4920
ggttcgtcct gctggcaggg ctcacgttcg cgatccagga ctacgcgggc accgtgggca 4980
ccgcgacggg ggtgccgccg gcgcagatct tcctggatgc tctgggcgtg gcgggggcga 5040
aggcactgct cctggtggtg atcatcgcgc agttgtgctg tggcaacgcc gagaccgccg 5100
cggccagccg gatggtgttc gcgttctcgc gcgacggggc gctgccgggc tcgcacctgt 5160
ggcgtcaggt tgaccgacgc actggcaccc cgcgcatggc cgtgctgctg gcggtcgtgt 5220
gcgccgccgt gcttgccctg ccgagcctgt acagccccgt cgcgtacgcg gcgatcacca 5280

gcatcaacgt ggtcggcatc accccggcct acgcgatccc gatctacttg cgcatcaaga 5340

accatgatcg cttccggccc ggcccttgga acctcggcaa ctggggtgtg gccgtcggca 5400

cgatcgccgt tgtctgggtg gtgttcgtga cggtgctgtt ctgtctgccg cagacacgcc 5460

ccgcggaagg cggcctcgtg tccgtggaga ccttcaacta cgcgccgatc gctctgctcg 5520

tcgtcctggt ccttgcctgg gggtggtggc ggaagcaggg cagctcctat gaagtgccgg 5580

cccagaactt cgaccgctcg acggctacgt acgaagacga ggtcgtgtga tgacgagaac 5640

cacgaccggc aacggcaccg tggcccgatt ggccgccgca cctggccccg agcagggcgg 5700

gcgctccggg aagggcatct ccctgtccga cctgcggaac ctcgtaaagg cgggtgcgat 5760

cgacacagtg ttgctcgccg tccctgacct gcaggggagg ctgaagggga agcgttacga 5820

cgccaaccac ttcctcaagc gcgtcgcgca tgacggcgcc gaggtgtgcg cctacgtcct 5880

ggcaaccgac gtcgacatga gcccggcgga cggcttcgcc ctgacctcgt gggagaccgg 5940

ctaccaggac ctgtccgtgc aaccggccct gtcgaccctg tgcgtcgtgc cgtggctgcc 6000

gcgcaccgtt gccgtgctcg gcgatgcggt gcaccacgac gggacgctga tcgacatcgc 6060

gccccgccag atcctcctcc agcagttgac ccgactgtcg cggcacgggc tgcatcccaa 6120

ggtggggatc gagaccgagt tcgtcctcta caagggcacg tacgcggacg cggaacaggc 6180

tggctatcag ggtctacggc cgttgacgac ggagaacctg gactacgccc tcgaccacga 6240

ccccgtgtct gaccggtact tgcgccgtct tcagcgggcg ctcgccgggg cggggatgcc 6300

cgtggaagcg atcaagaccg aagcaggccc cggccaggtc gaggtgacct tcccgtacgg 6360

tggtgctctc accgcctgcg accggcaccc gctcttcaag cacgccgtgc gcaccctggg 6420

ctcgcgagcc ggtctggcac ccacgttcat ggcagccccg gagaccggcc gagccaatgg 6480

cctgcacctg cacgtgtcgc tgtggtcgaa ggccatcagc caactccacg agcccggcac 6540

cgagcacgaa ctgtcccagg tcggtcagca cgccatcgcg ggtctgctcg ccggcctgcc 6600

cgagctgggc ccgttctacg cacccagcgt caactcctac aagcggttca cgcccggctc 6660

cttcgcgccg accacgttca cctggggccg cgacaaccgc acctgcgccg tccgcgtcgt 6720

cggccgcggc gaagggctgc acctggagat ccgtgtgccg ggtgcggacg ccaatccgta 6780

tctggcgctg tcggcggtgc tggccgccat ggaccacggt ctggaacgga agcccgccct 6840

tggtcccgaa gcgaccggca acgcctaccg cgcgagtgga acagaagccc cagtgccatc 6900

caccctcggc ttggccctga ccgtattcca ggacagcgcg ctcgcgcggc aggccttcgg 6960

caccgaggtg gtcgagcact acgcccgcct cgccgggctc gaactcgccc acgacgagcg 7020

cctcgtcacc gatgccgagc gtcagcggtg gctggcccgt gcctgaccgg tccgcttccc 7080

aggagggaac tatcgccgtg agcgtccgca gatgaccagc tcaggccgca ttgtcagtgg 7140

cccttggcaa gctacccacc cacgacagga cactgagcgc gtccgatccg tcacccatcg 7200

tggcgtggtc gggcggccaa gcgagcggaa gggcgccctg tgaccgacat gcccagtgcg 7260

ctccaccaac tggtggagtc ggtgaccgac atgtacgagg tggtcgccga gcacgcccgg 7320

cccggagcca tccggccttc cgtctgggag gtcaacggcc cgggcggcca aaggtggttc 7380

gggaaggttc acgcgggccc gaagctccac cggcgcgagg tgacggcgta ccagaagtgg 7440

accgtggccc tgcgcgcgga ccatgcaccc gaactggtcg ccgcggacac gctgacgcgc 7500

accgttctgg tcaccgccgt gcccggacac ggtctcgaca cgctgcgcct gcccgccgag 7560

caggaacatg cggcttacgt ccaggccggc gaactgctcg cccggttcca caccgcggca 7620

gccgacgagc cgatgccgga aacggccgat gaggcatggg acgaggcggt cgcccgattg 7680

ctggaccgta cggcgacgca cgcgtcggaa cacgatctcg cgttggtgcg cacgctggcg 7740

aaggaggctc ctccacgcct gcccccggta tcccagcacg gcgactacat gcccaagaac 7800

tggatgtggg acgagacaga gcagcgccta cgggtcatcg acttcgaacg ggccgaactc 7860

cggacccccg cctaccggga cctgagtcgg ctgcgctacc ggatcctgtg ccaccgcccc 7920

gacctcgacg ccgccttcca ccacggatac ggccgtcccc tcaccgagga ggaacagatc 7980

gcgtgccggg cctacggggc gcttgatgcc ctggactccc tggactgggg gatcaagcac 8040

cgcgacatcg gactggtcga cgaggcgcag accatgctgg agaacctgcg ccgggagacc 8100

ggcaagaggg tgtggggcgg gtggcgcgcg tgaacgcctt cgccgtcctc acgacccgcc 8160

gtccgtccgt acacggcggc gacggaaaag ccgaaggaca tgcgctgccg caataaagcg 8220

actcgcatca gccgatgggg ccgaccaacg atcgaggtca aggtcctcca gggagcggcc 8280

acggtctccg agtcgaatca cctgccgatc gtcccacaga cctcaaacgc ggcctgacgc 8340

ggagcatgcg ggtgaagcct ccagtaaggc acatcctgga ggcttcacgg ggcaccgatc 8400

ggatgtcggt cccggactcc acctacggtt tgagcgggct tgaatggcgc tcacatcaac 8460

ggattcagcc cgccgagtgc cggttaccca aagggtgacg tcacgagtct cccccggacc 8520

acttgttgag gttgtcaaga agcttcatac gttcctgctg ggtcagcacc tcgatacgtc 8580

gcagacgctt gtcaggattc gcaatcacct ccctcagtac gcgcagtagg cgctcagtca 8640

gcttttctac tgtggccaca ctgaaaagag acgagtcata ctcaattact ccgttaattc 8700

cccgtgctga attattctcg gcgcgatact cgctgaaatt gaagaacagg tcgaacttcg 8760

cggtctgcgt gtgagtggtc tcaaagcgtg cttgcaggcc tggaagtttg aactgccctt 8820

cgggggtgtt ttggagggcg agcatgactt ggaagagggg gtggtgggcg agggagcgtt 8880

cggggttgag gatttcgacg aggtgttcga aggggacgtc ctggtgggcg taggcggcca 8940

ggtcggtctc gcgtacccgg gccaggagtt cacggaaggt ggggttgccg gaagtatccg 9000

tgcgcaggac gagggtgttg acgaagaacc cgaccaggtc gtcgagggcc tcgtcggtgc 9060

ggccggcgat cgggctgccc agggggatgt cgtcccccgc acccagacgg ctcatcaacg 9120

ccgccaagcc ggcctgcagc accatgaaca cggtcgtacc ggtggaacgt gccaggtcta 9180

tcaggccttg gtggagttcg gcgtcccatt tgaagtacag ggagtcgcct tggtaggtgg 9240

ctgtggcggg gcggggccgg tcggtgggca gggtgacctg ttcgggcagg ccggtgaggg 9300

tggtgcgcca gtactccagc tgccgggcga tcaggctgcc cgtgtcggag tggtcgccga 9360

ggagatcgtt ctgccagagg gtgtagtccg cgtactgcac cggcagcgcc gaccagcccg 9420

gcgtgcggcc gtcggcccgg gccgcatacg ccaccgtcag gtcacggaac aagggggaca 9480

gtgaccagcc gtctcctgcg atgtggtgca cgacgagcac cagcacgtgc tcctcggggg 9540

cgagtgcgaa cagccacgcc cgcactggca actccgcgga gagatcgaag gcatactgcg 9600

atgctgccga gaccgcttcg gccagcccct cctcgctcgt ttggacgacg tgcagcaccg 9660

gccgggcctc cacctcggca agaacgtgct gatagggaac gctgtcggtt tccgggaaga 9720

cggtccgcag gctttcgtgc cgctccacca cgtcggtgag tgccgtgtgc agggcctccc 9780

ggtccagccg ccccgacaac cgcagcgcca tcgggatgtt gtaggtcgca ctcggccctt 9840

ccaaacggtg gaggaaccac aggcggctct gcgcggagga caaaggtatc cgctccggac 9900

gcaccgcggg gaccagcgtc gagcgcaccg tctcaccgtt gctggtctcc agcagcacag 9960

tcagtgcggc gacggtgggg gcttcgaaga gggcgcggac ggggacttcg gtgttgaggg 10020

tggcgcggat gcggctgatg aggcgggtgg cgaggaggga gtgtccgccg aggtcgaaga 10080

agctgtcgtc gatgccgacg gcgggcaggc ccaggacttc ggcgaacagg ccggcgagga 10140

tctcctcgcg ggggctgcgt gcggtgcgtc cggcgggttc ggcagcgaac tccggtgtgg 10200

gcagggcccg gcggtcgagc ttcccgctcg cggtcagtgg cagccgctcc agcaccacca 10260

ccgcggcggg caccatgtag tccggcagcc tggtgcgcag gtactcccgg accgtacccg 10320

catccaccac accacccgag gcggggacgt caccggaggt gatgccggtg ggcacgatat 10380

agagggcgag ctgtttgccg cgggtgtcgt cctcatgcac ggtggccgcg gcctgcgcga 10440

cggcgggatg gccggcggcg gtggtctcga tctcgccgag ttcgatccgg aacccgcgga 10500

tcttgacctg gtcgtcggcg cgtccgacga actccagctc accggaggtg ttccagcgca 10560

ccacgtcccc ggtccggtac atgcgtgaac ccgccggccc gtacggatcc gccacgaacc 10620

gcgacgcggt cagaccagca cggttcacat acccacgcgc cagacccgca cccgccacat 10680

acaactcacc cgcaacaccc accggcacca cacgcaaccc cgcatccagg acgtagacgc 10740

gggtgttggg cagcggacgg ccgatggggg cggtgtcctg ggtgagggga tcgctccagg 10800

tggcgacgac gctggcttcg gtggggccgt aggcgttgat gaagcgcctg ccgggcgacc 10860

agcgggccac cagttccggc gggcaggctt ccgctccggt cagcagtgtc cgcaggtgcg 10920

gcaggtccgt ttcgggcagg gtggcgagcg cgctgggggt gatcagtgtg tgggtgatct 10980

cctggcgggc gaggagcgcg gccaggtcgt cgcccagcag ccggccttgt ggcgcgatga 11040

ccagggtggc gccgctgccg aacgccatgc tcagttccag caccgccgcg tcgaatccga 11100

ccgaggccag ttgcaggacc cggctgccgg cggacaggtc cagcgcagcc cgttccgctg 11160
ccgtcaggtt cgccagtccc gcatggggaa ccagcactcc cttcggcaca cccgtcgaac 11220
cggaggtgta gatcacatac gccggatgcg acccctccag caccacaccc cgctcggaat 11280
cggtgggatc cgacaccgca cacagcgcga cggtctcggt gacagccggg tcgtccagga 11340
ggacggaggt gacgccttcg gtgcggggca ggccggccgc gacatcggag gtggtcagca 11400
ggagtgtggg gcgggtgtcg gtgagcatgt gggtgatgcg ttcggccggg tagtccggat 11460
ccacgggcag atacgccgcg ccggccttga gtaccgccag gaccgcgatc accaggtcgg 11520
tgccgcgggg cagggccagc gccacgatcc gttccggtcc cgcgccctgc ccaatcagat 11580
agtgcgccag ccggttcgcc cgctcgttca gctcgcggta ggagagtcgc tcggtctcga 11640
agacgaccgc ggtcgcctcc ggcgcctcgg cagcctgggc ctggaacagt tccggcaggc 11700
tggccggcgg cacctccagg gcggtgtcgt tccactccgc caccagccgg tgctcctcct 11760
gcccgctcag caccggcaga gcacccaccg tcacatccgg atcggccgcc accgacacca 11820
gcacccgccg caaccgctcc agcaccacct ccaccgacac ccggtcaaac agatccgtac 11880
tgaactccac agaaccatgg agccccccag gcccaccgct gtctcccaca agcccgtaga 11940
agttcagggc cagatcgaat cgggacgtac gcgtgccgac tggctcaaca ctgatctcca 12000
ggccgtccag ttcaagccga tggccaggag tgttgtccag cgtcaccgat acctggaaga 12060
gggggtggtg ggcgagggag cgttcggggt tgaggatttc gacgaggtgt tcgaagggga 12120
cgtcctggtg ggcgtaggcg gccaggtcgg tctcgcgtac ccgggccagg agctcacgga 12180
aggtggggtt gccggaagta tccgtgcgca ggacgagggt gttgacgaag aacccgacca 12240
ggtcgtcgag ggcctcgtcg gtacggccgg cgatcgggct gcccaggggg atgtcgtccc 12300
ccgcacccag acggctcatc aacgccgcca agccggcctg cagcaccatg aacacggtcg 12360
taccggtgga acgtgccaga tccaccagac cctgatgcaa ctcagcatcc cagctgaact 12420
cgaacacctc acccctctgc gacgccatcg ccggccgcgg gcggtccgtc ggcaacgtga 12480
cctgctccgg caggccggtg agggtggtgc gccagtactc cagctgccgg gcgatcaggc 12540
tgccggtgtc ggagtggtcg ccgaggaggt cgttctgcca cagcgtgtag tccgcgtact 12600
gcaccggcag cggcgcccag cccggcacac ggccgtcggc ccgggccgcg taagcggtga 12660
ccaggtcacg ggagagaggt gccagcgacc agccgtcact cgcgatgtgg tgcaccacca 12720
gcacgagcac gtgctcctca cgggcgagtg cgaacagcca cgcccgcacc ggcaactcaa 12780
ccgacaggtc gaacgggtag cgggcagcac cggccagcgc ctgatccagc tggtcttctg 12840
caatgtcggc aacctccagc acgggctggg cttcggcccc ggccaggatc tgctgacagg 12900
gcaccccgtc ggtttcgggg aagacggtcc gcagactctc gtgccgctcc actacatcgg 12960
cgagtgccgt gtgcagggcc tcccggtcca gccgccccga caaccgcagc gccatcggca 13020
tgttgtaggt cgcactcggc ccctcaagcc ggtggaggaa ccacaggcgg ctctgcgccg 13080

acgacagcgg catccgctcc ggacgcgtca cggggaccag cgccgagcgc accgactcgc 13140

tgctggtctc caggagtgcg gcgaggccgg cgacggtggg ggtctcgaac agagcccgga 13200

tcggtatctc cgcaccaaga atggcgcgga tgcggctgat gaggcgggtg gcgaggaggg 13260

agtgtccgcc gaggtcgaag aagctgtcgt cgatgccgac ggcgggcagg ccgaggactt 13320

cggcgaacag gccggcgagg atctcctcgc gggggctgcg tgcggtgcgt ccggcgggtt 13380

cggcagcgaa ctccggtgtg ggcagggccc ggcggtcgag cttcccgctc gcggtcagtg 13440

gcagctgctc cagcaccacc accgccgccg gcaccatgta gtcgggcagt ttcgtgcgca 13500

ggtactcccg gaccgtaccc gcatccacca caccaccggt gccggtgggc acgatataga 13560

gggcgagctg tttgccgcgg gtgtcgtcct catgcacggt ggccgcggcc tgcgcgacgg 13620

cgggatggcc ggcggcggtg gtctcgatct cgccgagttc gatccggaac ccgcggatct 13680

tgacctggtc gtcggcgcgt ccgacgaact ccagctcacc ggaggtgttc cagcgcacca 13740

cgtccccggt ccggtacatg cgtgaacccg caggcccgta cggatccgcc acgaaccgcg 13800

acgcggtcag accagcacgg ttcacatacc cacgcgccag acccgcaccc gccacataca 13860

actcacccgc aacacccacc ggcaccgcac gtagagcagc atccaggaca tatacacggt 13920

cgttagctac cggaccgcct actggagccg ccgacaccgg ccagtcagcc acccgctccg 13980

gcaacacata cgcagtcacc gcatgcgtct ccgtaggccc gtagtggttg tgcagccgga 14040

tgtgcggacg ctcccggaag aaccggcgca tcgccgcccc cggcaccagc gcctcaccac 14100

cctgggccac atgccgcagc gcgggcagga ccagccccag ctcaccggcc gcctgcgcga 14160

ccgcgtcaat caccagcgcc ggagcgaaca actcctcgac cgcatgctcc tgcagccacc 14220

gcgccagccc ctccccactg cgccggacct cctcacccgg aacccacaac tccttgccga 14280

acaccagcgc cgacaagatc tcctgcgccg acacatcgaa actgatcgtc gtgaactgcg 14340

ccgtccgcgc acccgcccgc cccgcaatca ccgagggatg ccagtgcagc agattcacca 14400

gaccacccga cggcatcacc acccccttcg gcacacccgt cgaaccggag gtgtagatca 14460

catacgccgg atgcgacccc tccagcacca caccccgctc ggaatcggtg ggatccgaca 14520

ccgcacacag cgcgacggtc tcggtgacag ccgggtcgtc caggaggacg gaggtgacgc 14580

cttcggtgcg gggcaggccg gccgcgacat cggaggtggt cagcaggagt gtggggcggg 14640

tgtcggtgag catgtgggtg atgcgttcgg ccgggtagtc cggatccacg ggcagatacg 14700

ccgcgccggc cttgagtacc gccagcaccg cgatcaccag gtcggtgccg cggggcaggg 14760

ccagcgccac gatccgttcc ggtcccgcgc cctgcccaat cagatagtgc gccagccggt 14820

tcgcccgctc gttcagctcg cggtaggaga gtcgctcggt ctcgaagacg accgcggtcg 14880

cctccggtgt ggcggcagcc tgggcctgga acagttccgg caggctggcc ggcggcacct 14940

ccagggcggt gtcgttccac tccgccacca gccggtgctc ctcctgcccg ctcagcaccg 15000

gcagagcacc caccgtcaca tccggatccg ccgccaccga caccagcacc cgccgcaacc 15060
gctccagcac cacctccacc gacacccggt caaacagatc cgtactgaac tccacagaac 15120
catggagccc cccaggccca ccgctgtctc ccacaagccc gtagaagttc agggccagat 15180
cgaatcggga cgtacgcgtg ccgactggct caacactgat ctccaggccg tccagttcaa 15240
gccgatggcc aggagtgttg tccagcgtca ccgatacctg gaagaggggg tggtgggcga 15300
gggagcgttc ggggttgagg atttcgacga ggtgttcgaa ggggacgtcc tggtgggcgt 15360
aggcggccag gtcggtctcg cgtacccggg ccaggagttc acggaaggtg gggttgccgg 15420
aagtatccgt gcgcaggacg agggtgttga cgaagaaccc gaccaggtcg tcgagggcct 15480
cgtcggtgcg gccggcgatc gggctgccca gggggatgtc gtcccccgca cccagacggc 15540
tcatcaacgc cgccaagccg gcctgcagca ccatgaacac ggtcgtaccg gtggaacgtg 15600
ccaggtctat caggccttgg tggagttcgg cgtcccattt gaagtacagg gagtcgcctt 15660
ggtaggtggc tgtggcgggg cggggccggt cggtgggcag ggtgacctgt tcgggcaggc 15720
cggtgagggt ggtgcgccag tactccagct gccgggcgat caggctgccg gtgtcggagt 15780
ggtcgccgag gagatcgttc tgccagaggg tgtagtccgc gtactgcacc ggcagcgccg 15840
accagcccgg cgtgcggccg tcggcccggg ccgcatacgc caccgtcagg tcacggaaca 15900
agggggacag tgaccagccg tctcctgcga tgtggtgcac gacgagcacc agcacgtgct 15960
cctcgggggc gagtgcgaac agccacgccc gcaccggcaa ctccgcggag agatcgaacg 16020
ggtagcgggc ggcaccagcc agtgcttctt cgagtccgtc ctcaccgacc tcggcgacct 16080
ccagcactgg ccgggcctcg gccccggtca ggatgcgctg gtggggaatt ccgtcggttt 16140
cggggaagac ggtccgcagg ctttcgtgcc gctccaccac atcggcgagt gccagacgca 16200
ggacctcagg catgaccgga ccggtcagcc gcaacgccat cggaacgttg taggtcgcac 16260
tcggcccctc caaacggtgg aggaaccaca ggcggctctg cgccgacgac agcggcatcc 16320
gctccggacg cgtcacgggg accagcgccg agcgcaccga ctcgctgctg gtctccagga 16380
gtgcggcgag gccggcgacg gtgggggtct cgaacagagc ccggatcggt atctccgcac 16440
caagaatggc gcggatgcgg ctgatgaggc gggtggcgag gagggagtgt ccgccgaggt 16500
cgaagaagct gtcgtcgatg ccgacggcgg gcaggcccag gacttcggcg aacaggccgg 16560
cgaggatctc ctcgcggggg ctgcgtgcgg tgcgtccggc gggttcggca gcgaactccg 16620
gtgtgggcag ggcccggcgg tcgagcttcc cgctcgcggt cagtggcagc cgctccagca 16680
ccaccaccgc ggcgggcacc atgtagtccg gcagcctggt gcgcaggtac tcccggaccg 16740
tacccgcatc caccacacca cccgaggcgg ggacgtcacc ggaggtgatg ccggtgggca 16800
cgatatagag ggcgagctgt ttgccgcggg tgtcgtcctc atgcacggtg gccgcggcct 16860
gcgcgacggc gggatggccg gcggcggtgg tctcgatctc gccgagttcg atccggaacc 16920
cgcggatctt gacctggtcg tcggcgcgtc cgacgaactc cagctcaccg gaggtgttcc 16980

agcgcaccac gtccccggtc cggtacatgc gtgaacccgc cggcccgtac ggatccgcca 17040

cgaaccgcga cgcggtcaga ccagcacggt tcacataccc acgcgccaga cccgcacccg 17100

ccacatacaa ctcacccgca acacccaccg gcaccacacg caaccccgca tccaggacgt 17160

agacgcgggt gttgggcagc ggacggccga tgggggcggt gtcctgggtg aggggatcgc 17220

tccaggtggc gacgacgctg gcttcggtgg ggccgtaggc gttgatgaag cgcctgccgg 17280

gcgaccagcg ggccaccagt tccggcgggc aggcttccgc tccggtcagc agtgtccgca 17340

ggtgcggcag gtccgtttcg ggcagggtgg cgagcgcgct gggggtgatc agtgtgtggg 17400

tgatctcctg gcgggcgagg agcgcggcca ggtcgtcgcc cagcagccgg ccttgtggcg 17460

cgatgaccag ggtggcgccg ctgccgaacg ccatgctcag ttccagcacc gccgcgtcga 17520

atccgaccga ggccagttgc aggacccggc tgccggcgga caggtccagc gcagcccgtt 17580

ccgctgccgt caggttcgcc agtcccgcat ggggaaccag cactcccttc ggcacacccg 17640

tcgaaccgga ggtgtagatc acgtatgccg gatgcgaccc ctccagcacc acaccgcggt 17700

ccgtgtccga cggatcatcc gccgacaaca ccgccaccgc ctcgacgacg gccggagcat 17760

ccacgaggac ggaggtgacg ccttcggtgt ggggcaggcc ggcggccacg ccgctggtgg 17820

tgaccatgag ggccggggcg gcatcgtgca gcatgtgggt gatgcgttcg gcggggtagt 17880

ccgggtccac gggcagatac gccgctccgg ccttgagtac cgccaggacc gcgatcacca 17940

ggtcggtgcc gcggggcagg gccagcgcca cgatccgttc cggtcccgcg ccctgtttga 18000

tcaggtagtg cgccagccgg ttcgcccgct cgttcagctc gcggtaggag agtcgctcgg 18060

tctcgaagac gaccgcggtc gcctccggcg cctcggcagc ctgggcctgg aacagttccg 18120

gcaggctggc cggcggcacc tccagcacgg tgtcgttcca ctccgccacc agccggtgct 18180

cctcctgccc gctcagcacc ggcagagcac ccaccgtcac atccggatcc gccgccaccg 18240

acaccagcac ccgccgcaac cgctccagca ccacctccac cgacacccgg tcaaacagat 18300

ccgtactgaa ctccacgaag ccaccaagcc ccgaagccgc accatcctca cgctgccact 18360

cgacaaaatg cagcgacaca tcaaaccgcg acgtaccagt cggcgccacg ggtactgcac 18420

cgacgttcac cgtcccaggt agggcgaagc tccgctctgg ggtgttttgg agggcgagca 18480

tgacttggaa gagggggtgg tgggcgaggg agcgttcggg gttgaggatt tcgacgaggt 18540

gttcgaaggg gacgtcctgg tgggcgtagg cggccaggtc ggtctcgcgt acccgggcca 18600

ggagttcacg gaaggtgggg ttgccggaag tatccgtgcg caggacgagg gtgttgacga 18660

agaacccgac caggtcgtcg agggcctcgt cggtacggcc ggcgatcggg ctgcccaggg 18720

ggatgtcgtc ccccgcaccc agacggctca tcaacgccgc caagccggcc tgcagcacca 18780

tgaacacggt cgtaccggtg gaacgtgcca gatccaccag accctgatgc aactcagcat 18840

cccagctgaa ctcgaacacc tcacccctct gcgacgccat cgccggccgc gggcggtccg 18900

tcggcaacgt gacctgctcc ggcaggccgg tgagggtggt gcgccagtac tccagctgcc 18960
gggcgatcag gctgccggtg tcggagtggt cgccgaggag gtcgttctgc cacagcgtgt 19020
agtccgcgta ctgcaccggc agcggcgccc agcccggcac acggccgtcg gcccgggccg 19080
cgtaagcggt gaccaggtca cgggagagag gtgccagcga ccagccgtca ctcgcgatgt 19140
ggtgcaccac cagcacgagc acgtgctcct cacgggcgag tgcgaacagc cacgcccgca 19200
ccggcaactc aaccgacagg tcgaacgggt agcgggcagc accggccagc gcctgatcca 19260
gctggtcttc tgcaatgtcg gcaacctcca gcacgggctg ggcttcggcc ccggccagga 19320
tctgctgaca gggcaccccg tcggtttcgg ggaagacggt ccgcagactc tcgtgccgct 19380
ccactacatc ggcgagtgcc gtgtgcaggg cctcccggtc cagccgcccc gacaaccgca 19440
gcgccatcgg catgttgtag gtcgcactcg gcccctcaag ccggtggagg aaccacaggc 19500
ggctctgcgc ggaggacagg ggtattcgtt ccggacgggc tgcgggggta agtgtcgggc 19560
ggacggcccg gttctcgtcc aggagggtgg ccaggccggc gacggtgggg gcttcgaaga 19620
ggtcacggac ggggacttcg gtgttgaggg tggcgcggat gcggctgatg aggcgggtgg 19680
cgaggaggga gtgcccgccg aggtcgaaga agctgtcgtc gatgccgacg gcgggcaggc 19740
cgaggacttc ggcgaacagg ccggcgagga tttcctcgcg ggggctgcgg ggggcgcggc 19800
cggtggtgtg ggtgccgagg tcgggtgcgg gcagggcccg gcggtcgagt ttgccgctgg 19860
cggtcagcgg gagccgttcg aggacgacga tggcggcggg caccatgtgg tcggggagtt 19920
tggtgcgcag gtggtcacgg acctgtgtga tcagtgtgct ggtgtcccgg gaggcggccg 19980
ggttgttcgc cagtgcctgg aaggacgtct ccgcggtgtg gtccggggcg gaggtttccc 20040
ggagatcggt ggcggtggcg gtggcggtgg cggaccatgc cgtgccgtcg aaaatatcga 20100
ggggcaggag gagggtgttg taggtgtcgg gggcgggggc gggctggagc acggcgtggt 20160
agccggtggc ggcggcgagc tggtggaggt gttcggggtc gatgccgcgg gggccggcca 20220
gcgcggtgac ggcctcggtg agggtgccgc cgttctccag gacccgcagc gcggcgtact 20280
cgcccgccag ccgggcgttg ggtatacccg tcacccgcaa cggcgcagcc ccacgagacc 20340
gcagcaggtc atcgaggccg tccagaccac cggacagagt gttccagctg atccgttcga 20400
ccagtgccgg cgtttctgcc tctgccttcg tgggccgcgc tgcctctgct tcttcctctg 20460
ttgctgtgtc tgtttctgtg tttgtggtgt ggaggatgac gtcgtagcgg tggcgggtga 20520
gttcgttgtg ggcctggccg tgtttgaggc gcacctcgac cgccgacacc gcgggcagcg 20580
tgcggcccac ggcggtgaag aactccgggt ccagcagcag ttcgttctcc agcagcagtc 20640
cctgttcgac cgcccgccgc agtccgcccg cgtccatccc gtcaccagcc ctgccgccgg 20700
tggtgcgggt gagctggaca gcggtgtgga acgcgcgcag agtacgcagg tcacgcacgt 20760
caccgatgaa gacccggcca cccggcgcca gccgggacac cgcggcctcg atcaccgacg 20820
ccagatactg cgcaccgggg aagtactgca ccaccgagtt gatcaccacg gtgtcgaagt 20880

agccctccgg caggccccgc gtgtcatcgg cccgctggca ggcgagccgc acccgttccc 20940

gcagcaccgg atcggcctgc acatgcccgt ccagcgcatc gatcaccaca cccgacagat 21000

cggtggccca gtactcctca cacccggccg ccagacgcga catcagcaga ccggtcccca 21060

ccccgatctc cagaacccgc cgcccgccca gaccccggat ccgctccacc gtggcatcac 21120

gccactcccg catctcctcc agcgggatcg ccgcaccgtc ataactgctg tcccagcccg 21180

agaaatcctc cccgaacaca gaagaccccg gcccgccata caacgagtca taaatctccc 21240

gccactcacc cacctgctca tccaccacac ccgacacacc accgtcagga acagccccat 21300

caccggagac agagccggag acgtcaccgg aggtgagacc ggtgggcacg acatacaggg 21360

cgagctgttt gccgcgggtg tcgtcctcat gcacggtggc cgcggcctgc gcgacggcgg 21420

gatggccggc ggcggtggtc tcgatctcgc cgagttcgat ccggaacccg cggatcttga 21480

cctggtcgtc ggcgcgcccg acgaactcca gctcaccgga ggtgttccag cgcaccacgt 21540

ccccggtccg gtacatgcgt gaacccgccg gcccgtacgg atccgccacg aaccgcgacg 21600

cggtcagacc agcacggttc acatacccac gcgccagacc cgcacccgcc acatacaact 21660

cacccgcaac acccaccggc accacacgca accccgcatc caggacatag acacgaaggt 21720

cagggatcgc tgtaccaatt gcgctgctgc cgtcagaggc gaaagcagag ccacggttca 21780

attccctgta cgtcacgtgg accgtggtct cggtgatccc gtacatgttg accaggacag 21840

gctctgtgtc gagatgcgct tcgaaccagg gcttcaacct gcttgtgtcc agggcctcac 21900

cgccgaagac gactcggcgc aatgaaagac gccggtcgag agcagagttc tcctgggcag 21960

cctgctgcaa ttggctgaag gccgacgggg tctggttgag gacagtcaca ccttctgcca 22020

ccagcagacg caacaactcc accggcgacc gactcgtcag atgcgacacg atgaccaacc 22080

gcccaccgtg cagaagcgca ccccacaact cccacaccga gaaatcgaag gcataggaat 22140

gaaagagcgt ccatacgtca tcggctccga atccgaacca gtgatcagtt gcggcaaaca 22200

accgagtcac gttctgatgc ggtatcaaca ctccctttgg ctgccctgtg gagccggagg 22260

tgtagatcac gtaggcggga tgcgacgcgt ccagcacacc gcggtccgtg tccgacggat 22320

catccgccga caacaccgcc accgcctcga cgacgaccgg gtcgtccagg agaacctggg 22380

ggatcccttc gatgtggggt aggtcggctg tcgcgtcgga ggtggtcagc agcagtgtgg 22440

ggcgggtgtc ggtgagcatg tgggtgatgc gttcggccgg gtagtccgga tccaccggca 22500

gatacgccgc gcccgctttc agtaccgcca gcaccgcgat caccaggtcg gtgccgcggg 22560

gcagggccag cgccacgatc cgttccggtc ccgcgccctg tttgatcagg tagtgcgcca 22620

gccggttcgc ccgctcgttc aacacccgat acgagatgcg gacatcctcg aagacgaccg 22680

cggtcgcctc cggtgtggcg gcagcctggg cctggaacag ttccggcagg ctggccggcg 22740

gcacctccag ggcggtgtcg ttccactccg ccaccagccg gtgctcctcc tgcccgctca 22800

gcaccggcag agcacccacc gtcacatccg gatccgccgc caccgacacc agcacccgcc 22860

gcaaccgctc cagcaccacc tccaccgaca cccggtcaaa caaatccgta ctgaactcca 22920

cgaagccacc aagacctgcg ggcaggtcac ctagctcgtt ggactcaaag aagtggaaag 22980

acagatcaaa ccgcgaagtc ctggtctcgg cccgttcact tcgcatgttg atgtcaggga 23040

gggtgaagtt gcggggtggt gtgttgtgga gggcgagggc cacctggaag agggggtggt 23100

gtgccggaga gcgttcgggg ttgaggattt cgacgaggtg ttcgaagggg acgtcctggt 23160

gggcgtaggc ggccaggtcg gtctcgcgta cccgggccag gagttcacgg aaggtggggt 23220

tgccggaagt atccgtgcgc aggacgaggg tgttgacgaa gaacccgacc aggtcgtcga 23280

gggcctcgtc ggtgcggccg gcgatcggac tgccgagggg gatgtcgtcc cccgcaccca 23340

gacggctcat caacgccgcc aaccccgcct gcagcaccat gaacacggtc gtgccggtgg 23400

aacgtgccag gtctatcagg ccttggtgga gttcggcgtc ccaggcgaag tcgtgcaggg 23460

cgccttggta ggtggcggtg gccggccgcg gccggtcggt gggcagggtg acctgttcgg 23520

gcaggccggt gagggtggtg cgccagtact ccagctgccg ggcgaccaga ctgtccccat 23580

cggactggtc gccgaggagg tcgttctgcc agagggtgta gtcggcatac tgcaccggca 23640

gcgccgacca gcccggcgtg cggccgtcgg cccgggccgc atacgccacc gtcaggtcac 23700

ggaacaaggg ggacagtgac cagccgtctc ctgcgatgtg gtgcacgacg aggacgagca 23760

cgtgctcctc gggggcgagt gcgaacagcc acgcccgcac cggcaactcc gcggagagat 23820

cgaacgggta gcgggcagca ccggccagcg cctcgtccag ctggtcctct gcaatgtcgg 23880

caacctccag cacgggctgg gcttcggccc cggccaggat gtgctggcag ggcacgccgt 23940

cggtttcggg gaagacggtc cgcaggctct cgtgccgctc cgctacatcg gcgagtgccg 24000

tgtgcagggc ctcccggtcc agccgccccg acaaccgcag cgccatcggc atgttgtagg 24060

tcgcactcgg cccctccagc cggtggagga accacaggcg gttctgtgcg gaggacaggg 24120

gtattcgttc cggacgggct gcgggggtaa gtgtcgggcg gacggcccgg ttctcgtcca 24180

ggagggtggc caggccggcg acggtggggg cttcgaagag ggcgcggacg gggacttcgg 24240

tgttgagggt ggcgcggatg cggctgatga ggcgggtggc gaggagggag tgtccgccga 24300

ggtcgaagaa gctgtcgtcg atgccgacgg cgggcaggcc gaggacttcg gcgaacaggc 24360

cggcgaggat ctcctcgcgg gggctgcgtg cggtgcgtcc ggcgggttcg gcagcgaact 24420

ccggtgtggg cagggcccgg cggtcgagct tcccgctcgc ggtcagtggc agctgctcca 24480

gcaccaccac cgccgccggc accatgtagt cgggcagttt cgtgcgcagg tactcccgga 24540

ccgtacccgc atccaccaca ccaccggtgc cggtgggcac gatatagagg gcgagctgtt 24600

tgccgcgggt gtcgtcctca tgcacggtgg ccgcggcctg cgcgacggcg ggatggccgg 24660

cggcggtggt ctcgatctcg ccgagttcga tccggaaccc gcggatcttg acctggtcgt 24720

cggcgcgtcc gacgaactcc agctcaccgg aggtgttcca gcgcaccacg tccccggtcc 24780

ggtacatgcg tgaacccgcc ggcccgtacg gatccgccac gaaccgcgac gcggtcagac 24840

cagcacggtt cacataccca cgcgccagac ccgcacccgc cacatacaac tcacccgcaa 24900

cacccaccgg caccgcacgt agagcagcat ccaggacata tacacggtcg ttagctaccg 24960

gaccgcctac tggagccgcc gacaccggcc agtcagccac ccgctccggc aacacatacg 25020

cagtcaccgc atgcgtctcc gtaggcccgt agtggttgtg cagccggatg tgcggacgct 25080

cccggaagaa ccggcgcatc gccgcccccg gcaccagcgc ctcaccaccc tgggccacat 25140

gccgcagcgc gggcaggacc agccccagct caccggccgc ctgcgcgacc gcgtcaatca 25200

ccagcgccgg agcgaacaac tcctcgaccg catgctcctg cagccaccgc gccagcccct 25260

ccccactgcg ccggacctcc tcacccggaa cccacaactc cttgccgaac accagcgccg 25320

acaagatctc ctgcgccgac acatcgaaac tgatcgtcgt gaactgcgcc gtccgcgcac 25380

ccgcccgccc cgcaatcacc gagtgatgcc agtgcagcag attcaccaga ccacccgacg 25440

gcatcaccac ccccttcggc acacccgtcg aaccggaggt gtagatcaca tacgccggat 25500

gcgacccctc cagcaccaca ccccgctcgg aatcggtggg atccgacacc gcacacagcg 25560

cgacggtctc ggtgacagcc gggtcgtcca ggaggacgga ggtgacgcct tcggtgcggg 25620

gcaggccggc cgcgacatcg gaggtggtca gcaggagtgt ggggcgggtg tcggtgagca 25680

tgtgggtgat gcgttcggcc gggtagtccg gatccaccgg cagatacgcc gcgccggcct 25740

tgagtaccgc cagcaccgcg atcaccaggt cggtgccgcg gggcagggcc agcgccacga 25800

tccgttccgg acccgcgccc tgcccaatca gatagtgcgc cagccggttc gcccgctcgt 25860

tcagctcgcg gtaggagagt cgctcggtct cgaagacgac cgcggtcgcc tccggcgcct 25920

cggcagcctg ggcctggaac agttccggca ggctggccgg cggcacctcc agggcggtgt 25980

cgttccactc cgccaccagc cggtgctcct cctgcccgct cagcaccggc agagcaccca 26040

ccgtcacatc cggatccgcc gccaccgaca ccagcacccg ccgcaaccgc tccagcacca 26100

cctccaccga cacccggtca aacagatccg tactgaactc cacgaagact tcgaggccac 26160

ctgggagccc ggactcgcta cgacgttcgg tcaatccgag gaagagatca aagcgagcag 26220

tgtgggtcgc gatcggccat tccttcgcct ccagtccgct cagagcaaac cggcgctctg 26280

gggtgttttg gagggcgagc atgacttgga agagggggtg gtgtgcgagg gagcgttcgg 26340

ggttgaggat ttcgacgagg tgttcgaagg ggacgtcctg gtgggcgtag gcggccaggt 26400

cggtctcgcg tacccgggcc aggagttcac ggaaggtggg gttgccggaa gtatccgtgc 26460

gcaggacgag ggtgttgacg aagaacccga ccaggtcgtc gagggcctcg tcggtgcggc 26520

cggcgatcgg gctgccgagg gggatgtcgt cccccgcacc cagacggctc atcaacgccg 26580

ccaagccggc ctgcagcacc atgaacacgg tcgtaccggt ggaacgtgcc aggtctatca 26640

gaccctgatg caactcagca tcccagctga actcgaacac ctcacccctc tgcgacgcca 26700

tcgccggccg cgggcggtcc gtcggcaacg tgacctgctc cggcaggccg gtgagggtgg 26760

tgcgccagta ctccagctgc cgggcgacca gactgccggt gtcggactgg tcgccgagga 26820

ggtcgttctg ccacagcgtg tagtccgcgt actgcaccgg cagcgccgac cagcccggca 26880

ccccgccctc ggcccgggcc gcatacgcca ccgtcagatc acggaacaac ggggacagtg 26940

accagccgtc ccccgcgatg tgatgcacca ccagcaccag cacatgctcc tcgggagcaa 27000

gcgcgaacaa ccacgcccgc accggcaact cggtggagag atcgaaggca tgcctcgaaa 27060

cgatcaggat ggtgtctgct agttcttcct cactcgtctg cctcgtctcc agcacgggct 27120

gggcttcggc cccggccagg atgtgctgac agggcacccc gtcggtttcg gggaagacgg 27180

tccgcaggct ctcgtgccgc tccaccacat cggcgagtgc cgtctgcagg gcctcccggt 27240

ccagccgccc cgacaaccgc agcgccatcg ggatgttgta ggtcgcactc ggcccctcaa 27300

gccggtggag gaaccacagg cggctctgcg ccgacgacag cggtattcgt tccggacgag 27360

ctgcgggagt cagcttcctg cggaccacgt cgctgctgcc ggtctccagc agcacagcca 27420

gccccgccac agtcggcgct tcgaatagat cccggatcgg tatctccacg ccaagaatgg 27480

cgcggatgcg gctgatgagg cgggtggcga ggagggagtg cccgccgagg tcgaagaagc 27540

tgtcgtcgat gccgacggcg ggcaggccga ggacttcggc gaacaggccg gcgaggattt 27600

cctcgcgggg gctgcggggg gcgcggccgg tggtgtgggt gccgaggtcg ggtgcgggca 27660

gggcccggcg gtcgagtttg ccgctggcgg tcagcgggag ccgttcgagg acgacgatgg 27720

cggcgggcac catgtggtcg gggagtttgg tgcgcaggtg gtcacggacc tgtgtgatca 27780

gtgtgctggt gtcccgggag gcggccgggt tgttcgccag tgcctggaag gacgtctccg 27840

cggtgtggtc cggggcggag gtttcccgga gatcggtggc ggtggcggtg gcggtggcgg 27900

accatgccgt gccgtcgaaa atatcgaggg gcaggaggag ggtgttgtag gtgtcggggg 27960

cgggggcggg ctggagcacg gcgtggtagc cggtggcggc ggcgagctgg tggaggtgtt 28020

cggggtcgat gccgcggggg ccggccagcg cggtgacggc ctcggtgagg gtgccgccgt 28080

tctccaggac ccgcagcgcg gcgtactcgc ccgccagccg ggcgttgggt atacccgtca 28140

cccgcaacgg cgcagcccca cgagaccgca ggatctcctc cagccctgtg agctggcccg 28200

gaacactgtt ccagctgatc ccttccaccg gcgcaggcgt cggagctggt gcttctgtgt 28260

ctgtttctgt gtttgtggtg tggaggatga cgtcgtagcg gtggcgggtg agttcgttgt 28320

gggcctggcc gtgtttgagg cgcacctcga ccgccgacac cgcgggcagc gtgcggccca 28380

cggcggtgaa gaactccggg tccagcagca gttcgttctc cagcagcagt ccctgttcga 28440

ccgcccgccg cagtccgccc gcgtccatcc cgtcaccagc cctgccgccg gtggtgcggg 28500

tgagctggac agcggtgtgg aacgcgcgca gagtacgcag gtcacgcacg tcaccgatga 28560

agacccggcc acccggcgcc agccgggaca ccgcggcctc gatcaccgac gccagatact 28620

gcgcaccggg gaagtactgc accaccgagt tgatcaccac ggtgtcgaag tagccctccg 28680

gcaggccccg cgtgtcatcg gcccgctggc aggcgagccg cacccgttcc cgcagcaccg 28740

gatcggcctg cacatgcccg tccagcgcat cgatcaccac acccgacaga tcggtggccc 28800

agtactcctc acacccggcc gccagacgcg acatcagcag accggtcccc accccgatct 28860

ccagaacccg ccgcccgccc agaccccgga tccgctccac cgtggcatca cgccactccc 28920

gcatctcctc cagcgggatc gccgcaccgt cataactgct gtcccagccc gagaaatcct 28980

ccccgaacac agaagacccc ggcccgccat acaacgagtc ataaatctcc cgccactcac 29040

ccacctgctc atccaccaca cccgacacac caccgtcagg aacagcccca tcaccggaga 29100

cagagccgga gacgtcaccg gaggtgagac cggtgggcac gacatacagg gcgagctgtt 29160

tgccgcgggt gtcgtcctca tgcacggtgg ccgcggcctg cgcgacggcg ggatggccgg 29220

cggcggtggt ctcgatctcg ccgagttcga tccggaaccc gcggatcttg acctggtcgt 29280

cggcgcgccc gacgaactcc agctcaccgg aggtgttcca gcgcaccacg tccccggtcc 29340

ggtacatgcg tgaacccgcc ggcccgtacg gatccgccac gaaccgtgac gcggtcagac 29400

cagcacggtt cacataccca cgcgccagac ccgcacccgc cacatacaac tcacccgcaa 29460

cacccaccgg caccacacgc aacccagcat ccaggacata tcccagcgcc ctatctacgg 29520

gtcggccgat ggataccacc gggtttccgc cactgacact gtcgacgtga cacgtcgtgg 29580

cgtgcaccgt cgtctccgtc ggcccgtaga tattgaccac ccggacaccc ggaaccagcc 29640

cggacgccag ctcagcagtc cgcgccggca acgcctcacc accgaacacc acccggcgca 29700

gcaccaaacc ctcagccccc gccggatcct gcgccaccac acccgccaac gcgtcaaaag 29760

ccgacggcgt ctggcacagc accgtcacct gctcagccac cagcagacgc aacaactcca 29820

ccggcgaccg actcgtcaga tgcggcacca ccaccagaca cccgccatgc agcaacgccc 29880

cccacaactc aaacaccgag aagtcgaacg tgtacgaatg gaacaaagcc cacacatcat 29940

ccggcgcaaa cccgaaccag tgcccgccct cacccacgtc gaaaagccgc accacatttc 30000

cgtgcgacgt catcacgccc ttcgggcgcc ccgtcgaacc cgacgtataa atcacatgca 30060

ccggatgcgc cggcgacaac accgcacccc gatccgcatc cgacggatcc gacaccccgc 30120

acccggccag ctcacccagc accgccgggt catccagcga cacgtactcg acatcatcga 30180

caaccggcag accagccacg accgcagagg acgtcaaagc caacacggga cggctgtcct 30240

cgagcatgta ggcgacgcgt tcggccgggt agtccggatc caccggcaga tacgccgccc 30300

ccgccttcac caccgccagc accgccacca ccagatcgac accacgcggc aacgccagag 30360

cgaccacccg ctccggaccc acaccacgcc ccagcaacca atgcgccagc cggttcgccc 30420

gctcgttcaa caccccatac gacacacgaa catccccgaa aacaaccgca gtcgcccccg 30480

gaacagacgc agcacacacc tcaaaaagcc cagggagggt cgccgtcgat atcgcatcgg 30540

tggcaccact ccattccgtc aaaattcgac gctcttcctc tgcgtccaaa aactccagca 30600

ggctaatcgg ctgggaggaa cgactcggat ccgtgaactc cttcaggagg cgcatgaacc 30660
tctcgctgtg agcctggagt tcttgatagc tatagaggtc ggaattcgcc tctatatcga 30720
tacgaagctc gcttccgtcc cgcctgtcgt aaatggcgat ggtgaggtca tcaacaactc 30780
ccagagacag attcttcgct actgcattag cgtcaccgaa agttatattg tagtcgaacg 30840
gcatgaagtt gacccgtggg ccaatcattc ctcgaatgcc cgccggaaga ccgagatctc 30900
gcgcaatatc ctcgctgcgg tagcgctgat gcctgagcag atctgctatc tcgcggcaca 30960
cttgcccctc aagatcagaa acagttgttt cctgactcac agtcaatttg aggggaagca 31020
ggttggacat cattcccgcg acctgcttca aatccgcacc gggacgcgct gcggcagcaa 31080
atccgaggag gaaatcaacc tgccctgtgg ccctgtaaac gtaagctgcc gccgccgccg 31140
tcagagttac agcccatcct cggagatccc ggtcacctcg gaccgcgtcg ccgccatgga 31200
catcagagaa agtcatgctt acacggtgga actttccaga ggccggtgcg gtacgtcccg 31260
ccaactccac gggttccggg aggtcagcaa ggcgccccat ccagaattcg cggtcccgcg 31320
caaggtcctc ggaggctcgg taggaggcat cgtcgtccag cagcagcgcg acagagtcgt 31380
cgccaccaag aactccctcg ttgcgagaaa ggcgattata gctgtctgcc acccttctgg 31440
cgacgactcc catggcaaag gcgtccatgg cgatgtggtg gtatccctga taccaaacaa 31500
ccctgttccg ggaaatccgg aacagggcgt atgagaaaac aggaccgaca gttatatcta 31560
tggaccgccc caggtcttcc ctcatccacc gctctgcgac ggaaagcgga tcatcaacat 31620
cacttaaatc aataaccgga agatcccagt cctccttgcg ctcaactacc tgctcgggcc 31680
cttcttgccc ttccgtgaat cgaacgtgca acgtttcagt ctgtgcgacc acggtgcgca 31740
gcgccctttc gaaaagggtc aggtcaatgt cgcccgaaat atccaggtac tcaccgactc 31800
gataaagagg gttctcaagg tcctgctgct gactgaacca gatctcacgc tgagcggcag 31860
acaaaagttt cgagttgatc tgcatgactg aaaagccccc tgtgccaatt tgacggtgca 31920
gtcatttggt caactcgggc gcatccccga gcaacacgac agggctcacc cctctgccgc 31980
gccgaagatc atcatcacaa ggccagccac catcacacca cccctttcca gccaacctgc 32040
gaaatgtcac agggggatgc gatcaagcca atcctggttc tgatggttaa ctggcgacgg 32100
tatgtggaag ccgcttgccg tagtacccgt agcaggtgcg gaccttccct gcacgccacc 32160
atccggttct gcggaccatg tcggtcacag cagacatgga aaatctgcca ggaatggcca 32220
tagctcagcc cggcccatag tcatccacag cagatgagcc ccagctggat gtaactcaac 32280
cctcccgaac ggaggcggta tcgagagact gcaacgctag caacgttggc ttgattgccc 32340
aggaccacag tgtcaggatc gccgtggcgt cgtgcctgtt cagggaaaca acatggggat 32400
agtgaagcgg gggcatcagc caagggggcc ctactgccac gctggttaga cgcctcacgg 32460
tttcaagctg tcagcgatga ggtcaacggc catttgtaat ccccagtggc ggccaaatgt 32520
tctacgccct catggccgaa tcggcgtccc cagcggcggg acggtctgtc ccagggcctc 32580

cgtgaagtgc aacttcccag tgcgggacgg gtgcagatgc agaggttttc ccgacgcccg 32640
acccactcca gtgtctccag cgagtcctgg acctgacggg ttatggagga ctgggtctcc 32700
tcccaatcgc cgaacgtctt gtccgcgggg tactcggccc gcttgcgtcg gccccgtagg 32760
ttcgcggcat cacgtccggt tcgccgaggt gggcaccagg tccgccttgg cgatgcgaac 32820
cgtcgcctcg ctgccgccct tggcctcggg atccgctggc agacaggtgt ggatcgttat 32880
gccgtagtgc cggccgactt cgacgatctc agggttgcgg accgcgatgt tcgcaatgtg 32940
ctcggtcgtg acgatcttct cgttgtccgt cagcgcatag ctcgggaccc ctccgatccg 33000
ccgcaaagtg gcatccagac aagccacgat cgtcggcagc gccttttcga acaccgggat 33060
gaccacccgg aatctcgacc aggccagccg agcgcaccac aaagacgtac gtcttccgcc 33120
aatgcgtggc ccttcacccc agtcctactg cagccagagg ccggctcggc gatcttctct 33180
gatgcgatga aaggagcggg acctcccact cctgccgggc gcgcaggatg ctcggctgag 33240
gcagaagtcc gaactcgggg agccctcgat tggtactggg ccgttgacgt actcaccgag 33300
gccggagccg gggttcacct cacccacccc ctgggggtga aggcgtttgc gtaccgccgg 33360
gtcaagaatg atgagcgaga cgccgccgac ctgaccgacc tgctgcggct gggccggcta 33420
ccggttgcgg gttcgatgct ggcagtgtac gtgcggcagt gcttgaagaa cggcgacacc 33480
gtggaagtca gcgcttcgtg ctggtggaag ttctcggcgg acaccatcac ccagcacggc 33540
gagtacctgt tggccttcgt cgatggtcac gtgtgtgtcg gcgccttcga gatcgttggg 33600
gcggagcccg atgagaccga aggcgagaag tacgtcttcg atcttcttcg ccctgcagca 33660
cggttccagt gggcgctggg gaggaagctt ccactgccgc ccggtcgcaa cccgtgcgca 33720
ttctgaccgg ccaggccctg cgcgagttcc tcgtccgacc cgcggtaagg gatctggggc 33780
ggctgcgccg gcggtgagct cgatggaacc cggtgatgtc tcgagcgcat cctcgccttc 33840
accttgacga ccgcgcggcg ggtggtgcga tccccgccgg tcaagcccgc cgcagtgatc 33900
cgccggtgca cgatgtccgc acctatccgc cctttcgagg cggtcagcaa ctcctcgatc 33960
ttgtccatga attcgtcgat cgggcgggcc cggtgctgac gctccgcggg acgctcgccc 34020
gcctcacgca acttcacata gcgagccacc gtgtggtggt cgacgccagc cagctcggcc 34080
gcggcgcggt gactccccgt gaggtgatac gcccccaagc ttgccactgc ggtccggccg 34140
caagcgcggc gtcgaggccg aggagctgtg cggctttggc aggggtgggt gccagctaag 34200
gggtcacggg tctcgggctc acacaggccg ttggtccagg tggcataggc gctcagcgct 34260
gcggggtagt actcgcgcag cagagaccgg agctggttgg aggtcagcgc cggtcccagg 34320
tcgcgtcctg ctgggccagg acggcgatgg cgcgggccat gtcggagtcg ttagggaggg 34380
ggcggcggcg tgcatgtcgg ttcgcagaag gttcgccagg gtcagggcgc cgccggggcg 34440
gttttcttgc gggagacaga gtgccggtcg cggtagcggg ccgcagctag gcccgtgacc 34500

ggctggcctc ctgggtgtcg ggtgaactca ccgacaatga ggcggtgttg gtggtcgatg 34560

agacggggga cgaggagtcc tccaccgact gcgtgggcgc cgcccgccag tactccggcg 34620

cgctgggggc ttgtacgtga ataactgtca gtcttgatct gtggggtcgt cctccgcaga 34680

tgcaaggcgg gctctcagct gctcaagtgt gcgtgcgggg gctccgggta tcggcgtgac 34740

ggcgatccgg tagaggtcga ggatccggtg gccatcctgg aactggttgg acaggttggt 34800

gcgtgtgacg cccagcagtt gggccaggag agtgctggtc gccgccttgc ggcggcgcag 34860

cagggctgtg aggagccggt ggaagtggtc caggctgctg gtttgcgggt gaaggtagct 34920

gcgcgggcgg tggaaacggc gctggaaggc cgcctcggcc agggcgtccc agtacggctc 34980

cgagactgcc gcgaggtgcg cgaagtccgc gcgtggcata ccggtcaggg ccgggtcgat 35040

gagcattgtg gtcagggcgg gatcgatggg ccgacgggtc gtgggagctt cggcaggact 35100

gggcggggca ggagccaagg tgtagttcca gtctccgtgg aatgcctctg gcgcgatcgg 35160

cagggcctgg aactcggcgg gtgtgacagt cgtgccgagc gggtagctgc cggtgtccag 35220

ctcggcgccg atggacagtc cgttcgcggt cgtcgtcgcg gcgatggtct ccacgacgac 35280

ctggtagctg gtcagtggct ggccgcgcca gttggcggtg atgtggcaga acatccggtg 35340

ctcgatcttg ttccacttcg aagtgcccgg tgggaagtga cagaccgtga tttccaggcc 35400

gctctcctgc gcgaaggcgt gaaggtattt cttccatgtc cagcggcggg catcgttgga 35460

gccgccggca tccgcagtga tcagtaaccg ggttgcgttc gggtggtcgg tccgtccgcg 35520

gcgccgccac cagcggcgga tggactccac cgcgaactcg gcggtgtcgt ggtcggtgcc 35580

gacgttgacc cagccggtgt tggtggccag gtcgtagatc ccgtagggga tggcgaccgg 35640

catttcgtcg gtgacgaagg tgtggcagtc caccttgatc gggcccttgc cggggcgcca 35700

ggtgcgtccg ggccggtcgc ggttgccgat ccactccttg gccttggtgt cgacgctgat 35760

caccggctgg tggctgtcga ggaaggcggt ggcggtggcg tcgcggtccg ggtggcgggc 35820

gccctccgtg gtcttggcgg tgccctgcag gctgtagccc atcgcatgca gcaggcgccc 35880

gacggtcgtt gcgccgaccg ggtgtccctg tgcggtcagt gtcgaggcaa gcgtccgcag 35940

cgaaagcgtg gtccagcgca gcggggagac cgggtcgcca cgggtacgcg gctcgatcag 36000

ggcctccaac gccggccgca gtccggtgtc ggtcgtcgtc agcggcttgc gtccagcccc 36060

tagagcccgg ttgcggcacg tcgtcgccgg atgcgcgtcc agctcggcga tgccacgtgc 36120

gatggtggcg gtgctcgtgc cggaagcggc agccaccgcg gcgattccgc cgtggccgag 36180

cgcaacagcc tcgctggcca ggtagaaccg tcgacgccgc tcgtcgaggt ggggcaggat 36240

ccgatcgaac ttggccttca accgggctgt ggggatgcga agttgcggaa ccggagtcat 36300

gctccagcct cccaccacgg ctcaccccac agatcaagtc tgacagttat tcacgtacaa 36360

gccctgggcg gaatcgggct gtgccaggtc gccgtgcacc tcacctacgc cacgccgcgc 36420

ggccacgcgc tgatcgaccg ggagttgtac ctgcccgccg cctggcgcca ggacgaggag 36480

cgccgcctgc tgcggcacgt gcccgacgag gtctttttcg ccaccaagcc ccagctcgcc 36540
gccgtcatgc tcacccgtgc ccgcgaactg ggcgtgtgcg cccgctggtt cgccggcgac 36600
gaggtctacg gcagcctcga gctgcgacga accgcccgga tgctcggctt cgactacgcc 36660
ctggccgtca aggccgacca caccgccacc acctccgcgg gccgcttcac cgccgcccga 36720
ctcgcggcga aggtcccggc gaagtcctgg atgcggatgc gcaccggaca cgggttgaag 36780
ggcgaccggc actacgactg ggcgctgatc gaggtccgcc ccgacgacac ccccaccgac 36840
agtgaggccg gcgggcacgc cttcctcgtc atccgccgcc accgctacac ccgcgaactg 36900
tccttctacc gctgccactc caccaccccc atcagcctcg ccgacctcgt caacatcatc 36960
tgcaccaggt ggaagatcga agaggacttc cagggcgcaa agggcctcac cggcctcgac 37020
cagggccagg tcgcctgctg gaactcctgg atgcactgga gcctgatcag cctgatcgcc 37080
gccgcggtcc tggccatcac ccgcgcccgc accgccgccc cggccgccgg catcgcgctc 37140
gtccccgcca gcccccgcga actgctcgcc gtgctccgcg ccacggccct tccggcgccc 37200
cgccgccacc tcgggcacgt cctgcactgg tccgcctggc gccgccatca ccagcaccaa 37260
gccgtccagg cccaccgccg ctggaacaac gtcaccgcag aagcgacaag gtgaccccac 37320
gacgaagatc acgacttaca gcgactgagc gtcagtgctc tagccagagg atcgcggccg 37380
gggcggcttt atatccgccc ggcctctact cggctgccat gacgagcccc acgaccatac 37440
acgtgtctat ccggaggcct cagtggcctg atggcagatg tcatagcccc aggctcaaac 37500
agaaccagcg gaaccaccca gtgggtctga tccgtcgacc ctgcgggcac gcgacatccg 37560
cctcgccagt gcggtctcgt cgatccgagg atcctgcgcg gcgtacgcga ttgacttgcc 37620
cgtggcgatg acgatctgca cagccacctc atggaactcc gcctcgaagt cccgtcscat 37680
cttgcctaca atgatcctcc acttcccctg caaccagaat ttctatgagg gaagctctct 37740
tcgaatcacc gatggaaccg tacaggcatg accagatatc gagctgacgt atcggcttcg 37800
gcgtctacag acgacttccc catgattaca gccttcccgt tggaactggt gaatgagaac 37860
tgggcccagg aggcatccaa gttcgtgagc ccgtcaagaa ggaaggatgg gttgaacgcg 37920
atgtcgatac tttcaccttc aagtgaagct gccatgcgcg acgtggctcg atcgtcatct 37980
gccccaccag cttgtagaac caattctccg tcggtgaaat caagcgcaac cgagctgttt 38040
cgctccagga cgagagaaac cctcttcaat gcctcaacaa ggcgagagac ctcaaccgcc 38100
ccgaaagatg tgaactcctt ggggaacaac ttttcatagt ctgggagggt cccctccagt 38160
aggcggcaag tcatctgccg tgtacccgca tggagcccga gaagattccc ttcatcgctc 38220
aatgcaattt ttactaggtc tcccttgttg agggattttg caaaatccag gagtgtccga 38280
gccggcacca ataccgaact agatgatggt gtgccttcag gcttccatgc gagctctcga 38340
atcgcgaagc ggtagcggtc ggtggcattc agcttcatga tctccccatc gaaattcact 38400

ccgatgccag tgaggactgg cagggcatca tcgcggctag ccacgacggc aacctgagag 38460

acagcgcgtg cgaactggtc accttctacc gatccacaga cggtgggcag cttcgggagc 38520

gttggatact catccaatgg cagcgcgttg aggacaaagc tgttcgagtc gcattgcacg 38580

tacaccttgg tctggtcaac attgaactca atcggcacat ctggaagaac tttcgcgatg 38640

tccgcgagtc ggcgacctgg aatcaggacc gttcccgatt cctccacatt cgctttgagc 38700

tcaaccctcg tggatacctc atagtcgaaa cctgacaacg tcagtgtgtc accttcggca 38760

tttaccacga caccagagag gatagggaca gacggccgga cggcgagccc acgggcaacc 38820

cagccgattc cctccgccag aacttcccgc tcgacctgaa accgcatgga atacgcctcc 38880

gatggcttgc tgcacgtcgc gacggagtcc gctccgccgc ctgcggccca gtatggcgca 38940

tcacaccgtc cacaggcggt gccgcggggg gccagcctcc agggatcatc agccacccgg 39000

gccggataat cgcctgcgct gcagcacttc atggtcgcgc tcgctccatg gacggcacct 39060

tttcgcgcct accggttcat tttcggccat agttgaatgc atacaccatg ggtcccccat 39120

gcgcttccat ccgcacgaca gcggctctgt agacccctca accggacgtc tggtcggagg 39180

gtcgaccctc gcagagcggc ggctgaaaat accccttcaa gaggcggccg catcggcttg 39240

gctgaaaaga agtggttgag cccgatgcga tttggcgacg atcatcttga tcgccggggc 39300

gacgctgctt gctgtctcac ccctcacggt ccctgcacaa tccatgcccg atccttttgt 39360

tcgggatccg ccccagggag tgcgttcttc atgcctgcca gctcgacatc atcgccggat 39420

tcgcgacatg aggtgtgagt ccactgggct ggctcacttc acgtcacgtc ccgacatggc 39480

ggccctaggc ggccgcagca ggcgatggag tatctccgac gacgtgccct atcaaaaact 39540

ctgacgacga gcaaaatgcc gccacatgca gatcactctc gattttccat ggcctttgac 39600

tgcgccatga tcttgcgacc aaccttttcg gggagctgag ccgtgttcga tcgcatatcc 39660

cacgacaaca ggaaaattcc cacagcaggt accctccctg ggctttttga ggtgtgtgct 39720

gcgtctgttc cgggggcgac tgcggttgtt ttcggggatg ttcgtgtgtc gtatggggtg 39780

ttgaacgagc gggcgaaccg gctggcgcat tggttgctgg ggcgtggtgt gggtccggag 39840

cgggtggtcg ctctggcgtt gccgcgtggt gtcgatctgg tggtggcggt gctggcggtg 39900

gtgaaggcgg gggcggcgta tctgccggtg gatccggact acccggccga acgcgtcgcc 39960

tacatgctcg aggacagccg tcccgtgttg gctttgacgt cctctgcggt cgtggctggt 40020

ctgccggttg tcgatgatgt cgagtacgtg tcgctggatg acccggcggt gctgggtgag 40080

ctggccgggt gcggggtgtc ggatccgtcg gatgcggatc ggggtgcggt gttgtcgccg 40140

gcgcatccgg tgcatgtgat ttatacgtcg ggttcgacgg ggcgcccgaa gggcgtgatg 40200

acgtcgcacg gaaatgtggt gcggcttttc gacgtgggtg agggcgggca ctggttcggg 40260

tttgcgccgg atgatgtgtg ggctttgttc cattcgtaca cgttcgactt ctcggtgttt 40320

gagttgtggg gggcgttgct gcatggcggg tgtctggtgg tggtgccgca tctgacgagt 40380

cggtcgccgg tggagttgtt gcgtctgctg gtggctgagc aggtgacggt gctgtgccag 40440

acgccgtcgg cttttgacgc gttggcgggt gtggtggcgc aggatccggc gggggctgag 40500

ggtttggtgc tgcgccgggt ggtgttcggt ggtgaggcgt tgccggcgcg gactgctgag 40560

ctggcgtccg ggctggttcc gggtgtccgg gtggtcaata tctacgggcc gacggagacg 40620

acggtgcacg ccacgacgtg tcacgtcgac agtgtcagtg gcggaaaccc ggtggtatcc 40680

atcggccgac ccgtggacaa agcccgtgcc tacgtgttgg acgacgagct ctttcccgtg 40740

gctcctggtg ttgcgggtga gttgtatgtg gcgggtgcgg gtctggcgcg tgggtatgtg 40800

aaccgtgctg gtctgaccgc gtcgcggttc gtggcggatc cgtacgggcc ggcgggttca 40860

cgcatgtacc ggaccgggga cgtggtgcgc tggaacacct ccggtgagct ggagttcgtc 40920

gggcgcgccg acgaccaggt caagatccgc gggttccgga tcgaactcgg cgagatcgag 40980

accaccgccg ccggccatcc cgccgtcgcg caggccgcgg ccaccgtgca tgaggacgac 41040

acccgcggca aacagctcgc cctgtatgtc gtgcccaccg gtctcacctc cggtgacgtc 41100

tccggctctg tctccggtga tggggctgtt cctgacggtg gtgtgtcggg tgtggtggat 41160

gagcaggtgg gtgagtggcg ggaratttat gactcgttgt atggcgggcc ggggtcttct 41220

gtgttcgggg aggatttctc gggctgggac agcagttatg acggtgcggc gatcccgctg 41280

gaggagatgc gggagtggcg tgatgccacg gtggagcgga tccggggtct gggcgggcgg 41340

cgggttctgg agatcggggt ggggaccggt ctgctgatgt cgcgtctggc ggccgggtgt 41400

gaggagtact gggccaccga tctgtcgggt gtggtgatcg atgcgctgga cgggcatgtg 41460

caggccgatc cggtgctgcg ggaacgggtg cggctcgcct gccagcgggc cgatgacacg 41520

cggggcctgc cggagggcta cttcgacacc gtggtgatca actcggtggt gcagtacttc 41580

cccggtgcgc agtatctggc gtcggtgatc gaggccgcgg tgtcccggct ggcgccgggt 41640

ggccgggtct tcatcggtga cgtgcgtgac ctgcgtactc tgcgcgcgtt ccacaccgct 41700

gtccagctca cccgcaccac cggcggcagg gctggtgacg ggatggacgc gggcggactg 41760

cggcgggcgg tcgaacaggg actgctgctg gagaacgaac tgctgctgga cccggagttc 41820

ttcaccgccg tgggccgcac gctgcccgcg gtgtcggcgg tcgaggtgcg cctcaaacac 41880

ggccaggccc acaacgaact cacccgccac cgctacgacg tcatcctcca caccacaaac 41940

acagaaacag acacagcaac agaggaagaa gcagaggcag cgcggcccac gaaggcagag 42000

gcagaaacgc cggcactggt cgaacggatc agctggaaca ctctgtccgg tggtctggac 42060

ggcctcgatg acctgctgcg gtctcgtggg gctgcgccgt tgcgggtgac gggtataccc 42120

aacgcccggc tggcgggcga gtacgccgcg ctgcgggtcc tggagaacgg cggcaccctc 42180

accgaggccg tcaccgcgct ggccggcccc cgcggcatcg accccgaaca cctccaccag 42240

ctcgccgccg ccaccggcta ccacgccgtg ctccagcccg cccccgcccc cgacacctac 42300

aacaccctcc tcctgcccct cgatattttc gacggcacgg catggtccgc caccgccacc 42360
gccaccgcca ccgatctccg ggaaacctcc gccccggacc acaccgcgga gacgtccttc 42420
caggcactgg cgaacaaccc ggccgcctcc cgggacacca gcacactgat cacacaggtc 42480
cgtgaccacc tgcgcaccaa actccccgac cacatggtgc ccgccgccat cgtcgtcctc 42540
gaacggctcc cgctgaccgc cagcggcaaa ctcgaccgcc gggccctgcc cgcacccgac 42600
ctcggcaccc acaccaccgg ccgcgccccc cgcagccccc gcgaggaaat cctcgccggc 42660
ctgttcgccg aagtcctcgg cctgcccgcc gtcggcatcg acgacagctt cttcgacctc 42720
ggcgggcact ccctcctcgc cacccgcctc atcagccgca tccgcgccat tcttggcgtg 42780
gagataccga tccgggatct attcgaagcc cccaccgtcg ccggcctggc caccctcctg 42840
gacgagaacc gggccgtccg cccgacactt acccccgcag cccgtccgga acgaataccc 42900
ctgtcctccg cgcagaaccg cctgtggttc ctccaccgcc tcgaaggtat gggtgcagcc 42960
gcgtacaacg ttccgatggc gttgcggctg accggttcgg tcatgcccga ggtgctgcgt 43020
ctggcgctgg ctgatgtggt ggagcggcac gagagcctgc ggaccgtctt ccccgaaacc 43080
gacggggtgc cctgtcagca catcctgtct gtggtggagg cccggccggt gctgcacgtc 43140
gtccaaacga gcgaggaagg gctggccgaa gcggtctcga cagcatcgca gtatgccttc 43200
gatctctctg cggagttgcc ggtgcgggcg tggctgttcg cactcgcccc cgaggagcac 43260
gtgctcgtgc tggtggtgca ccacatcgca ggagacggct ggtcactgtc ccccttgttc 43320
cgtgacctga cgaccgccta cgcagcccgg gccgacggcc gcacgccggg ctgggcgccg 43380
ctgccggtgc agtacgcgga ctacacgctg tggcagaacg acctcctcgg cgaccactcc 43440
gacaccggca gcctgatcgc ccggcagctg gagtactggc gcaccaccct caccggcctg 43500
cccgaacagg tcaccctgcc caccgaccgg ccgcggccgg ccaccgccac ctaccaaggc 43560
gccctgcacg acttcgcctg ggacgccgaa ctccaccaag gcctgataga cctggcgcgt 43620
tccaccggta cgacggtgtt catggtgctg caggcggggt tggcggcgtt gatgagccgt 43680
ctgggtgcgg gggacgacat ccccctcggc agtccgatcg ccggccgtac cgacgaggcc 43740
ctcgacgacc tggtcgggtt cttcgtcaac accctcgtcc tgcgcacgga tacttccggc 43800
aaccccacct tccgtgaact cctggcccgg gtacgcgaga ccgacctggc cgcctacgcc 43860
caccaggacg tccccttcga acacctcgtc gaaatcctca accccgaacg ctccctcgcc 43920
caccaccccc tcttccaagt catgctcgcc ctccaaaacg cccccgaagg gcagttcaaa 43980
cttcctggcc tgcaagcacg ctttgagacc acccacacgc agaccgcgaa gttcgacctg 44040
ttcttcaacg tccacgaata ccgcgcaacg gacggcgggc ctggggggct ctatggttct 44100
gtggagttca gtacggatct gtttgaccgg gtgtcggtgg aggtggtgct ggagcggttg 44160
cggcgggtgc tggtgtcggt ggcggcggat ccggatgtga cggtgggtgc tctgccggtg 44220
ctgagcgggc aggaggagca ccggctggtg gcggagtgga acgacaccgc cctggaggtg 44280

ccgccggcca gcctgccgga actgttccag gcccaggctg ccgccacacc ggaggcgacc 44340

gcggtcgtct tcgaggatgt ccgcatctcg tatggggagt tgaacgagcg ggcgaaccgg 44400

ctggcgcact atctgattgg gcagggcgcg ggtccggaac ggatcgtggc gctggccctg 44460

ccccgcggca ccgacctggt gatcgcggtg ctggcggtac tcaaggccgg cgcggcgtat 44520

ctgccggtgg atccggacta cccggccgaa cgcatcaccc acatgctcac cgacacccgc 44580

cccacactcc tgctgaccac ctccgacgcg acagccggcc tgccccacac cgaagggatc 44640

ccccaggttc tcctggacga ctcggccgtc atcgagaccg tggcggtgtt gtcggcggat 44700

gatccgtcgg acacggaccg cggtgtggtg ctggaggggt cgcatccggc gtatgtgatc 44760

tacacctccg gttcgacggg tgtgccgaag ggggtggtga tgccgtcggg tggtctggtg 44820

aatctgctgc actggcatca ctcggtgatt gcggggcggg cgggtgcgcg gacggcgcag 44880

ttcacgacga tcagtttcga tgtgtcggcg caggagatct tgtcggcgct ggtgttcggc 44940

aaggagttgt gggttccggg tgaggaggtc cggcgcagtg ggagggggct ggcgcggtgg 45000

ctgcaggagc atgcggtcga ggagttgttc gctccggcgc tggtgattga cgcggtcgcg 45060

caggcggccg gtgagctggg gctggtcctg cccgcgctgc ggcatgtggc ccaggcaggt 45120

gaggcgctgg tgccgggggc ggcgatgcgc cggttcttcc gggagcgtcc gcacatccgg 45180

ctgcacaacc actacggccc tgccgaaaca cacgttgtaa ctgctcaccc cctgccaaac 45240

cgaattgcag actgggcaac ttcagtcccc atcggccgtc cgctgcccaa cacccgcgtc 45300

tacgtgctgg atgcggggtt gcgtgtggtg ccggtgggtg ttgcgggtga gttgtatgtg 45360

gcgggtgcgg gtctggcgcg tgggtatgtg aaccgtgctg gtctgaccgc gtcgcggttc 45420

gtggcggatc cgtacgggcc ggcgggttca cgcatgtacc ggaccgggga cgtggtgcgc 45480

tggaacacct ccggtgagct ggagttcgtc gggcgcgccg acgaccaggt caagatccgc 45540

gggttccgga tcgaactcgg cgagatcgag accaccgccg ccgagcatcc cgccgtcgcg 45600

caggccgcgg ccaccgtgca tgaggacgac acccgcggca aacagctcgc cctctatgtc 45660

gtacccaccg gcatcacctc cggtgacgtc cccgcctcgg gtggtgtggt ggatgcgggt 45720

acggtccggg agtacctgcg cacgaaactg cccgactaca tggtgcccgc cgcggtggtg 45780

gtgctggagc agctgccact gaccgcgagc gggaagctcg accgccgggc cctgcccaca 45840

ccggagttcg ctgccgaacc cgccggacgc accgcacgca gcccccgcga ggagatcctc 45900

gccggcctgt tcgccgaagt cctcggcctg cccgccgtcg gcatcgacga cagcttcttc 45960

gacctcggcg gacactccct cctcgccacc cgcctcatca gccgcatccg cgccaccctc 46020

aacaccgaag tccccgtccg cgccctcttc gaagccccca ccgtcgccgc actgactgtg 46080

ctgctggagg ctggcagcag cgacgtggtc cgcaggaaac tgactcccgc agcccgtccg 46140

gagcggatac cgctgtcctc cgcgcagagc cgcctgtggt tcctccaccg cctcgagggg 46200

ccgagtgcga cctacaacat gccgatggcg ttgcggctga ccggtccggt catgcctgag 46260

gtcctgcgtc tggcgctggc tgatgtggtg gagcggcacg agagcctgcg gaccgtcttc 46320

cccgaaaccg acggcgtgcc ctgccagcac atcctggccg gggccgaagc ccaacccgtg 46380

ctggaggttg tagaggtcgg tgaggacgga ctcgaagaag cactggccgg tgccgcccgc 46440

tacccgctcg atctctccgc cgagttgccg gtgcgtggct ggttgttcgg gttggggccg 46500

accgagcacg tgctcgttct ggtagtgcat cacattgcgg gggatggctg gtcgctggca 46560

cctctctccc gtgacctggt caccgcttac gcggcccgtg ccgacggccg cacgccgggc 46620

tgggcgccgc tgccggtgca gtacgcggac tacacgctgt ggcagaacga cctcctcggc 46680

gaccagtccg atggggacag tctggtcgcc cggcagctgg agtactggcg caccaccctc 46740

accggcctgc ccgaacaggt caccctgccc accgaccggc cgcggccggc caccgccacc 46800

taccaaggcg ccctgcacga cttcgcctgg gacgccgaac tccaccaagg cctgatagac 46860

ctggcgcgtt ccaccggtac gaccgtgttc atggtgctgc aggcggggtt ggcggcgttg 46920

atgagccgtc tgggtgcggg ggacgacatc cccctcggca gtccgatcgc cggccgtacc 46980

gacgaggccc tcgacgacct ggtcgggttc ttcgtcaaca ccctcgtcct gcgcacggat 47040

acttccggca accccacctt ccgtgaactc ctggcccggg tacgcgagac cgacctggcc 47100

gcctacgccc accaggacgt ccccttcgaa cacctcgtcg aaatcctcaa ccccgaacgc 47160

tccctcgccc accaccccct cttccaggtg gccctcgccc tccacaacac accacccggc 47220

aacttcaccc tccccgacat caatattcat ggtcagcgcc tctcgactgg tacgtcgcgg 47280

tttgatgtgt cgctgcattt tgtcgagtgg cagcgtgagg atggtgcggc ttcggggctt 47340

ggtggcttcg tggagttcag tacggatctg tttgaccggg tgtcggtgga ggtggtgctg 47400

gagcggttgc ggcgggtgct ggtgtcggtg gcggcggatc cggatgtgac ggtgggtgct 47460

ctgccggtgc tgagcgggca ggaggagcac cggctggtgg cggagtggaa cgacaccgtg 47520

ctggaggtgc cgccggccag cctgccggaa ctgttccagg cccaggctgc cgaggcgccg 47580

gaggcgaccg cggtcgtctt cgagaccgag cgactctcct accgcgagct gaacgagcgg 47640

gcgaaccggc tggcgcacta cctgatcaaa cagggcgcgg gaccggaacg gatcgtggcg 47700

ctggccctgc cccgcggcac cgacctggtg atcgcggtcc tggcggtact caaggccgga 47760

gcggcgtatc tgcccgtgga cccggactac cccgccgaac gcatcaccca catgctgcac 47820

gatgccgccc cggccctcat ggtcaccacc agcggcgtgg ccgccggcct gccccacacc 47880

gaaggcgtca cctccgtcct cgtggatgct ccggccgtcg tcgaggcggt ggcggtgttg 47940

tcggcggatg atccgtcgga cacggaccgc ggtgtggtgc tggaggggtc gcatccggca 48000

tacgtgatct acacctccgg ttcgacgggt gtgccgaagg gagtgctggt tccccatgcg 48060

ggactggcga acctgacggc agcggaacgg gctgcgctgg acctgtccgc cggcagccgg 48120

gtcctgcaac tggcctcggt cggattcgac gcggcggtgc tggaactgag catggcgttc 48180

ggcagcggcg ccaccctggt catcgcgcca caaggccggc tgctgggcga cgacctggcc 48240

gcgctcctcg cccgccagga gatcacccac acactgatca cccccagcgc gctcgccacc 48300

ctgcccgaaa cggacctgcc gcacctgcgg acactgctga ccggagcgga agcctgcccg 48360

ccggaactgg tggcccgctg gtcgcccggc aggcgcttca tcaacgccta cggccccacc 48420

gaagccagcg tcgtcgccac ctggagcgat cccctcaccc aggacaccgc ccccatcggc 48480

cgtccgctgc ccaacacccg cgtctacgtc ctggatgcgg ggttgcgtgt ggtgccggtg 48540

ggtgttgcgg gtgagttgta tgtggcgggt gcgggtctgg cgcgtgggta tgtgaaccgt 48600

gctggtctga ccgcgtcgcg gttcgtggcg gatccgtacg ggccggcggg ttcacgcatg 48660

taccggaccg gggacgtggt gcgctggaac acctccggtg agctggagtt cgtcgggcgc 48720

gccgacgacc aggtcaagat ccgcgggttc cggatcgaac tcggcgagat cgagaccacc 48780

gccgccggcc atcccgccgt cgcgcaggcc gcggccaccg tgcatgagga cgacacccgc 48840

ggcaaacagc tcgccctgta tgtcgtgccc accggtctca cctccggtga cgtctccggc 48900

tctgtctccg gtgatggggc tgttcctgac ggtggtgtgt cgggtgtggt ggatgagcag 48960

gtgggtgagt ggcgggagat ttatgactcg ttgtatggcg ggccggggtc ttctgtgttc 49020

ggggaggatt tctcgggctg ggacagcagt tatgacggtg cggcgatccc gctggaggag 49080

atgcgggagt ggcgtgatgc cacggtggag cggatccggg gtctgggcgg gcggcgggtt 49140

ctggagatcg gggtggggac cggtctgctg atgtcgcgtc tggcggccgg gtgtgaggag 49200

tactgggcca ccgatctgtc gggtgtggtg atcgatgcgc tggacggcca tgtgcaggcc 49260

gatccggtgc tgcgggaacg ggtgcggctc gcctgccagc gggccgatga cacgcggggc 49320

ctgccggagg gctacttcga caccgtggtg atcaactcgg tggtgcagta cttccccggt 49380

gcgcagtatc tggcgtcggt gatcgaggcc gcggtgtccc ggctggcgcc gggtggccgg 49440

gtcttcatcg gtgacgtgcg tgacctgcgt actctgcgcg cgttccacac cgctgtccag 49500

ctcacccgca ccaccggcgg cagggctggt gacgggatgg acgcgggcgg actgcggcgg 49560

gcggtcgaac agggactgct gctggagaac gaactgctgc tggacccgga gttcttcacc 49620

gccgtgggcc gcacgctgcc cgcggtgtcg gcggtcgagg tgcgcctcaa acacggccag 49680

gcccacaacg aactcacccg ccaccgctac gacgtcatcc tccacaccac aaacacagaa 49740

acagacacag caacagagga agaagcagag gcagcgcggc ccacgaaggc agaggcagaa 49800

acgccggcac tggtcgaacg gatcagctgg aacactctgt ccggtggtct ggacggcctc 49860

gatgacctgc tgcggtctcg tggggctgcg ccgttgcggg tgacgggtat acccaacgcc 49920

cggctggcgg gcgagtacgc cgcgctgcgg gtcctggaga acggcggcac cctcaccgag 49980

gccgtcaccg cgctggccgg cccccgcggc atcgaccccg aacacctcca ccagctcgcc 50040

gccgccaccg gctaccacgc cgtgctccag cccgcccccg cccccgacac ctacaacacc 50100

ctcctcctgc ccctcgatat tttcgacggc acggcatggt ccgccaccgc caccgccacc 50160
gccaccgatc tccgggaaac ctccgccccg gaccacaccg cggagacgtc cttccaggca 50220
ctggcgaaca acccggccgc ctcccgggac accagcacac tgatcacaca ggtccgtgac 50280
cacctgcgca ccaaactccc cgaccacatg gtgcccgccg ccatcgtcgt cctcgaacgg 50340
ctcccgctga ccgccagcgg caaactcgac cgccgggccc tgcccgcacc cgacctcggc 50400
acccacacca ccggccgcgc cccccgcagc ccccgcgagg aaatcctcgc cggcctgttc 50460
gccgaagtcc tcggcctgcc cgccgtcggc atcgacgaca gcttcttcga cctcggcggg 50520
gacagcatca tttcgatcca gctggtcagc cgagcccgca aggcagggct gaaggtctcg 50580
gccggtgacg tgctccgcta caagaccgtc tcggcactcg ctgccgtcgc ccaagatcta 50640
cacgctgagg cctcctacat cagcgatgac ggacttggtg acgttcctca gacccccatc 50700
atgcgttggg cgttcaaaca agaagacctc gttcatgggc tacatcaggc gatgctcttg 50760
gagacgcccg ccggtctgca gcaagctcag ctggtcgcca tcacccagac gctcctggac 50820
catcacgaaa tgcttcgtgc ccgtttagtc gtgaggagag gcgaagaaag cgtcctgcgg 50880
gtggccgaag ccggggcagt ccgcgcggat gacctcattc agcaagtggc cgtcgccgca 50940
aactccggag aaaatctgcg cgattcgata tccgcagagt tctctgccgc acgggatcgc 51000
ctttccccgg aatccggcag catgctccag ttggtgtggt tcaacagtgg acccaatcac 51060
gctggtaggc tgctcatcgt gatacaccat ctggtcgtcg acggcgtatc atggcgcatc 51120
ctcctttctg acgtggcaga cgcctgggaa gacatctcgg cggggcgtgc acccgttctc 51180
gaaccacgtc gcacgtcgtt caagcgctgg gcaaatgccc tgatctcgga ggcacatgcc 51240
cctaaacgtg ttgaggagat gcctctatgg atgggaaccc tcaccgattc tgggtgggaa 51300
tttgcttcga agcctaagga cgaaaaggaa gcgagtacgt ccgcgcagct caccctcact 51360
ctttcccctg agaagacaat tccgatcctc acagaggtcc ctgcggtctt ccacgggaaa 51420
atcaacgacg tcttgctaac agcctttacg atcgccgccg tcgaatggcg aagggtccac 51480
cgagcgacgt acgccggcac cgatgtactg ctgaatctgg aaggacacgg acgggagcac 51540
ttcgctgaag gtttcgatgt ttcccggact gtgggctggt ttaccagtat gtttcctgtg 51600
cggctcgacc caggcgaata cgatcgcgat gaggtccgga tcggcgggcc tgcactcggt 51660
aaagtcctga agaccgtcaa ggagcagttg cgatcccagc cggacaacgg cctcgggttc 51720
ggcctcctcc gttacctcaa taccgagact gcaacgaaac tgtgcggact tgcagtgccg 51780
caggtcagct tcaactacct cgggcgtttc ggcgcctccg gtggtgccgg atgggtccag 51840
gcacccgaga gtgacatcgt ggccaacggg cagcacggcc cgctcgctca cctgctggag 51900
gtgaatgcgc tgactcgtga cggcgtagac ggaccagagt tgatcgcgac atggtcgtgg 51960
actcctggga ggttcacgga agctgaggtc ctagatttcg cggagaggtg gttcagcgtt 52020
ctgggttctc tcacaatcca cgcgtcggcc ccagacgcag gcggatacac accatccgat 52080

gtacccctcg tctcactatc gcaagaacaa atcgaactgc tcgaatccga atggagggca 52140

tcggaatgaa ggatccaggc ctggaggata ttcttccact cactcccctc caagaaggaa 52200

tattattcca tgcagccttc gatgccgagg gacaggatcc gtacgtcata cagttgacac 52260

tcgaatttca aggcccattg aaggccaagg atctgcggtc agcggcacag tcgttgctgg 52320

agcggcattc aaacctccgt gccagctttc gctaccacgg cctggaccgc cctgtccaac 52380

tcatacctcg gcatgccgaa gccccctggg aggagatcga cctcagcaac ctgtccgccg 52440

cccttcgtga ccaggaggta tcgaaagctg tcgctgctga cctgcagcgc cggttcgatc 52500

tcaccaaagg gccgtccatc cgcttcacct tggtccgact cgcagaagac ctccacaagt 52560

tcgtgatcac ctctcaccac atcctcatgg atggctggtc gataccggtg ctgtttcgcg 52620

agttgttcgc ttgttatgca gccggaggcg caacgtcgat gcttcccccg gcgcagccct 52680

tccgtaacta cctcgcgtgg tcggccaagc aggaccgcag ctccgcggag caagcctgga 52740

aggaagctct ttccggcctc gatcatccca cccgtctcgc tccccacagt cctgcacgca 52800

gtgttctgct accgcagcag gtgtctctca gcctgggtga ggaccttacg gagcagttgg 52860

gggcctgggc ccgcagtcga gacttcactc tgagccaggt tgtgcaggga gcttggggcg 52920

tgctactggg caggctcacc ggccagagcg atgttgtgtt cggtgccacg gtcgctgggc 52980

gcccgccgga agtagccgga gtggaatcga tgatcggtct cttcatcaac actctgcccg 53040

tcagactacg catcgttccc gacgaatcgt ttacggtatt gctgcgccgt catcagcgcg 53100

accaggagcg cctttttccc caccatcacc tcggcctcac tgatattcag cacgcagcag 53160

gcgtcggtca gttattcgac agtatcgtta tttttgagaa ctaccccaca aaaatcggct 53220

ccgaaaaaac aggcagcggt tcgctccgtg tgaccggatt cgaggcacac gacgccactc 53280

actatccgat aacgctgttc gtcattccag gtaagcgact gaacttccgt ttggactaca 53340

atgcggagta tttcgaccac gccacagtcc agacgatgct agaacggtta cgtcgtctac 53400

tggcatcggt agtgtccgat cccgaaccac aactaagcgg tctcaccttg ctcggcacgg 53460

aggaggaaca ccgcatactg acggagtgga acgacactcg actagaggtg ccggcggcgt 53520

ctctgttcga actgttccaa gcgcaggcgg tatccactcc ggatgcgacg gcagtcgtct 53580

ttgaggacac gagcgtcacc tacgaggagc tgaatgcgcg ggccaaccga cttgcgcact 53640

ggctgattgg ggagggcgtc ggaccagagc gagtggtcgc tctggccatc ccccggagtg 53700

tcgacctggt cgtggcggtg ctggcggtgt tgaaggccgg agcggcctac ctaccgatcg 53760

atccggagta tccgcctgag cgcatcgcgc acatgctgac agactctcgt cccacgatgc 53820

tggtgacctc gtccaaagcg gcgcaggacc tgccgaccgt ctctggcact cggtcagtgt 53880

gtctggatgg ccctgccctg atgtggtctc ttggaaccag caaggcgacg aatccgaccc 53940

acgtcgaccg cggcaaagcg gtcgatgccc ggcatccggc gtatgtgatc tacacgtcgg 54000

gctcgacggg gcggccgaag ggagtggtcg tacccacagg cagcgtggtt aacctgctca 54060

ccgccatggg cgattggttt cccgtgaccg gcaaggactg cctgcttgcc gtcacgacgt 54120

tcgccttcga catcgctact ctcgaattac ttctccccct gctcaacggt gcccggctgg 54180

tgctgacgaa ccgtgaaacg gtgctggaat cgtccgcatt ggcagaagtg atcgcacggc 54240

acagcgtgac gatcatgcag gcaaccccca ctttctggaa tgaattcgcc gccaacgagc 54300

ccgaagcact cacaggcatt cggattctca ccggcggtga agccttgagc gaaggtctgg 54360

ccggcagact ccaggcattg gccgacgatg tcaccaacgt gtacgggccc acggagacca 54420

ccatctggtc aacggccgcg gcgatcaccg cagctactgg cgttccgccg atcgggcgtc 54480

ccctggcgaa cacccaggca tatgtgctgg acgaaagcct tcggccagtg ccacccggtg 54540

ttcccgggga cctgtacctg gccggcgccg gtgtggccca gggatatcac aaccgccccg 54600

ggctaactgc gcaacgtttt gttgccaatc cctatggccc aataggcgcc cggatgtacc 54660

agacgggaga cgtggttcgg tgggatctcg acggctacct ggagttcctc ggtcggtctg 54720

acaaccagat aaagattcgc ggttttcgca tcgaacctgg agagatcgtc gcagcgcttg 54780

agaggcatcc acatgttgcc gaggcggccg tcgcggtgca ggaaagaaag aggaatcaga 54840

agggcctggt cgcctacgtc actgccagtg atgggcaaga aattgacacc actgaattac 54900

gcaatcacct gtcggatatc ctagcccgac acgccatacc ttccgccttc gttgtcatga 54960

gtgaactgcc gcgcacttcg aacgggaaac tcgacagttc ggctctacca gcacctacgc 55020

aagaggcaac agcacaagga agagcccctc gaagccctca ggaagaaatc ctgtgtgaac 55080

ttttcgctga ggtgctggag attcctcgcg tcggagtcga cgatgacttc ttcgaactcg 55140

gcggtcactc cctcctggct attcggctca tcagtcgcat tcgcgatgcc ctcggtagta 55200

atctgacgat acgaagcctc ttcgaagcac ccacggtact ggcactcgcg cagcgcctag 55260

acatggacac cgcagacaat gcgttcgacg tgatgcttcc gatgaagtcc aacggcagca 55320

acccaccaat cttctgcata catcccggcg gtggaatcgg atggatttac gcccggctga 55380

tcgcgtttct cgggcacgaa cagccagtct acgcgattca agctcggggg ctggccaagg 55440

aggaaccgct ccctgagaca atcgaggaaa tggctgagga ctacgtcagg gagatccggt 55500

ctatccaggc atctggtccg taccatcttg tcggatactc cttcggcggt ttggtggcgc 55560

aggccatcgc aacgagactt cagcaggacg gagacaaggt cggagtactg ggcttgatcg 55620

aggcgtatcc cgccgaagga atggccggca gtgaacgtcc agagatttca gagcaagaaa 55680

tcctggaagt gatcgcagag agcattgaca aggccgagga aattaaggcg aaagatcgcg 55740

cctttgagcg gatggagacg gaaggcatca ccttcgagca actcattgaa ggtgctcgcg 55800

cccgcggtag tgtcctgtcg gctttggata ggcgccatgt cacggccatg gccgacatcc 55860

atgccaactg tctacatctc aggcacaact tcaccccttc ctgttacgac ggtgatgcac 55920

ttctcttcag gtcgacactg actccgaata caccttcccc ggatacatgg cgaccgttta 55980

tcggcggccg gatcgaaacg agggaaatcg ccgccctaca ccaccagatg cttcatcctg 56040

aaccactcgc tgccataggg aaaatcctgg cggaagagct ccgaaaaacc caccacgaat 56100

acgaggaaaa atcgaaatga acaacccatt cgaagaccct gaaggcacct acaaggtact 56160

gatcaacgga gaggcccagt attcgctatg gccaacgtcg atcgacgtcc ctgcgggttg 56220

gaccatcgtt catggtccgg acagtcagca ggcctgcctc gatttcgtcg aggaaaactg 56280

gactgacatg cgccccaaga gcctaatcga agctatgaac gacacggtca gctgatcgct 56340

cgtgatacct gggccgggtc ggcccgcggg ctcacgctcc cggccggacc cggcctctct 56400

ggctagcgac catcccactg aaaggaacag attatgcagc tcacggccga tcaggtcgaa 56460

aagtacaaga gtgacggtta cgtccttctt gaaggtgcct tctctccgga ggaggtgcat 56520

gtcatgaggc aagccctgaa gaaggaccag gaggtgcagg gaccccatcg aattttggag 56580

gaagacggcc gcaccgtacg agcgctgtac gcttcgcaca cacggcagtc agtattcgac 56640

cagctcagcc gctcggatcg actgcttgga cccgcgacgc agctcttaga gtgcgacctc 56700

tacatacatc agttcaagat aaatacgaag cgggcgttcg gtggtgattc ctgggcctgg 56760

catcaggact tcatcgtctg gcgggatacg gatgggctgc cggcgccacg cgccgtgaat 56820

gtcggagtat tcctcagtga cgtcaccgag ttcaacggac cggtagtgtt cctcagtggc 56880

tcgcatcaac gcggcactgt cgagcgcaag gctcgggaaa cgtctcgatc cgaccaacat 56940

gtcgacccgg acgactactc gatgacacca gccgagctat cacagatggt ggaaaagcat 57000

ccaatggtga gcccgaaggc cgcctctggt tcagtcatgc tttttcatcc ggagatcatc 57060

cacggatcgg ctcccaacat ctctcccttc gcgagggatc tgctgatcat cacgtacaac 57120

gacgtcgcga atgcaccgaa gcccgcaggt gagccgcgtc ctgagtacgt catcgggcga 57180

gacaccacgc ctctggtctc caggagcgga ccgctgcatg aggccgccga atcgaggctg 57240

gcatgagcga taggtctccg tctgtggtaa cccctggtgt ggcgtccggc ggcatcactg 57300

ccagagccgc ggtcctggaa tccttccaga agccgcttac cgtacgacag tttcccgtac 57360

ctgcccccag tccgggagaa atcctggtcg acgtacgtta cggcgggatc tgcggaacag 57420

acctccacct gcagctaggg caccttccga tccctgttcc gcttgttctg ggccatgaag 57480

ggttgggcag catccggagg ctcgggacgg aaggtctcac ggacgccaat gggaccgaac 57540

tgcgtatcgg cgacacggtg atgtgggcct cgtccatcgc ctgcggctct tgtggcccat 57600

gtcggcaaca tcgagagccc acgctctgcg aatcacgccg gacatatgga gtcaatcggc 57660

aagtcgaggg cgattccggt ctgttcggcg cctggtccga aaccattctt ctccatccag 57720

gcgcgacagt ggtgcggctt ccgcagtctg tggatccctt ggctgccatg tctctggcct 57780

gcgccggccc cacgctaatc cacgcccttt acgaacgccg acccgtaaga gtaggagaga 57840

ccgtcatcgt tcagggcagt ggccccgtcg gcatggcagc cgccgcgctg gctcaactgt 57900

ccggcgccgc catggtcatc ctgctcggtg ggccacaaca gcgtctggac ctcgcccgac 57960

agtgcggtat cggggacgtc cacctgaaca tcgccgatag gtcggacacc acatcggcac 58020

tcaacgaggc ccgcgagatg acgcgaggag gactcggtgc cgatttggtc atcgagtgtg 58080

caggtgtacc tgaggccgtc gcccaggggg tctatctggc ccggcgcggt gggtcctatc 58140

tcgtcgtggg ccagtacacg gacagcggag agacgctgtt caaccctcat cagttggtct 58200

accggcaact agaggtcgtg ggttcgtggg ccttcaccgg agctcacctg gtccactacg 58260

tcaatctcct gccctctctg ctcgagcgat tcgatctacg ccgtctggtg acggagttcc 58320

ctctcggtga ggtaaacgac gcgatggtag ctgtcggcac cggcgaggtg gtgaaggcag 58380

tactggaaag ccggcacctt cccacggtcg atacctgagc agtgatcact ttggaggcag 58440

aaatatgcca attgccacgg tgaacgagac ccaattgcgc tacgacatcc acgggtcggg 58500

agagcccgtc gtgatggtga tgggcagcgg aagcggcggc cgtgtttggg agatgcatca 58560

ggtgcctgct ctggtggccg acggctacca ggtcgttaca ttcgacgatc gcggcgtctc 58620

gcaaccggag gatatcgagc cctacggtct cgacgacatg gtggccgacg tggcagcgct 58680

cgtcgatcac ctgagtctcg ggccctgtcg attcgttggt acgtctctcg gagcgctgac 58740

cgtgcaggaa ctggccatac ggcgtccgga cctggtatcc gagtctgttt tgatggccac 58800

tcgcggacgt actgacacac tgagcggggc catctccaag gccgagatcg atctcatcga 58860

tgagagtgca aagattcccg ttcgctatca ggcagttgtt caggctttgc tgaacctgtc 58920

gcggaaaact ctacgaaacg aacaagaact gcgggactgg atcgacatcc tggagatgtc 58980

ggggcctcag ccctcagggc tgcggtcaca actggaggca tcgttgatcg aggaccgact 59040

ggaggctctc aggtctacgc gtgctcagac tctggtcatt gggttccagg acgacctcat 59100

cactccgcca cacctcaacc gccaagtggc tgaggccatc cccggtagta cctacatcga 59160

agtggccgat tgcggccatt acggatacct ggagcaacct gatgtcgtca acaagcacat 59220

actcgacttc ttctccaagg gatgactcga aggatgcgct atggcgtcgt gattctccct 59280

gaacacagct gggccagagc ccaggacctg tggcgaaacg tggaggatct tggtttcgat 59340

cacgcttgga catatgacca tttgcagtgg cgatggctga gcgacaggcc ctggtttggc 59400

gccataccga ccttgaccgc cgcagccgtg gtcacatcac ggatcggact gggcacactc 59460

gtggccagca ccaaactgcg ggacccagtc atgctcgcca aggagatcat gacccttgac 59520

gacatctcgg gcggccgaat gctgtgcggc gtaggttcag gcggccccga tcgggatctc 59580

ttgcaggcct acgaactcac gcgacgccag ttgaccgccc gctacgggga gttcatagag 59640

ttgttggact cactgctgcg ccaggagcca gtagtcttcg agggcaccta ttacacgtgc 59700

cgcaacacgt tgctgcagcc agcctgcctc cagcgacctc gcgcccctct ctgtgtggcg 59760

gcggccggcc cgcagggaat gcggctcgca gctcggttcg cggatatctg ggtgacgatg 59820

ggggcgccga acatgttcga cgaggctccg tatgccgact cggccatgct catcaaggat 59880

caggtcgctg cgctggagca gacgtgccac gaggttggac gtgatcctgc gagtctcagg 59940
aggctcctgg tcaccggccc ctcgatcggc ggcgtcctcg actcggtcgg ctctttccag 60000
gacgcggcgg ggactttcca ggaggtaggt atcaccgacc tcgtcgtcca ctggccccgc 60060
cctgactttc cataccgagg cgaccccgcg gtcatggagg acatcgcaag tatcctgccg 60120
accgcaccag ggaaattgta aacccgcagg ggtgtgcgga attcctccca tcaagtggaa 60180
agcagagcaa ggtcggggcc ttgccggcag acggatcggg caatataccg gacttcaccc 60240
cgggccacgt cgcgctggcc ggagaaacag tcatccgcga atggcgcagt actcagccct 60300
caagtcgatc ggggcgcatc tgctccggcc atccggggcg gccaggctcg cgaacagtcc 60360
agtcaagttg gggagtggag caacaccgcc cacgtctgtg ggcaccgtcg atatgtccac 60420
catcttcggc tctggcgtcc aagccgtcga cagcagatgg atgactcttg atccatacag 60480
agccctccga ggtccagctg acgccgggtg cgagtgacgg ccacgtaggc gaggcgtgcg 60540
tccgtgtcgc tcaccgcttc cgggagcggg cggccgctgg cgtcctgctg gtcgctgtcc 60600
ggcggtgggg gaagtcgtcg gcggttctga cttcgggtca ctcccggccc tttgccttgt 60660
gagccgtgga caccgtcacc tcggcgttgt tctcgtcggt cagcgcgtcg acggcggcga 60720
tgacagcttc gggaccatgg gtgtcgacga gttcaacgaa cggctggagg tctcggccga 60780
cgggatcgta ctcggcgtag tcttccaact cgccccagga ggcgaagagg accagtcagg 60840
aggggaggtg cgacggccgt ctttgaggtc cctggcggcg agcgccagtg caacgagtgt 60900
ctgtccgccc ctggccagag ccaccggacg gccaggctgc tcggtgatca ggcagggtcg 60960
ggagacaggc acctactgcc tgtgtccgcc tggtatgcga gagttcagga cctggtcgtg 61020
agcagccgcg cgagggcatg accctcggta ctccgagatc cgacgccggc tccgtgtgtg 61080
gtgggggtcg agcgccgccg cgtgccggac gtctgttccg cccttcccgg ggcccctgcg 61140
cggtcaggcc gcagtgctcg cggccttcgg ccgggcggcg ttcacctggc ccatggctcg 61200
gtcgatcgtg atctcgatga ccacccgctc cgggtccgga gccggcgtcc gcccgtaccg 61260
gtccgcgtag cgggccacgg cgtcggcgac cgctgccgcg tcggtcagga cttcggccgt 61320
gccttccagg gtggcccagc ggcccccatc gacctgacag acggctgcgc gggcttcgcc 61380
ggggcgggac gcctggatgt tggcgacctt cctgcttgat ttgcgtgtga taacgcgtgc 61440
gactcccgcg tcgacatcaa gggtcacgcc gacgggcact acatgcggac ttccgtcgga 61500
gcgaagcgtg gtgagggtgc acaagtggta ctcattccag aacgctgagt actcagggat 61560
ctggctgaat atgtcgctag gcatacccgt gagcgtaacg cggactactg cacagattgg 61620
cgcagaagtg acctcttcca cacacctgcc cgcaagacca tgcctcggtg agatgcgcac 61680
tttgcggctg aatgcccacg ttatgatccc gctaaatatc tcaaaaacat aacggaagcg 61740
tgtgtcggtc gggttggcat gaccaatatg accgcaagtg aatcagcagt tcagcgccgt 61800

gatcgggaat cgggaatggt cgatttgatg agttttgcca gaggtggcga agtcttcctg 61860
tcgtgccttg aggtgtagct tttcggcctt gatctcatgc gggcctgacg tagggtccaa 61920
gactgttccc acagggcttc attcgttcgc ttgaccgttc gcgctagcga tttcacgagt 61980
tcggagaggc tcttgcgtgt cctcgcggct cgctagccga ggtcggcgtg caccgtgtgc 62040
ggtgcacctc tcggtgcttg cgatcacctc catgagcgcc gtttggggac tcgaacatcg 62100
ttgacccgtg gtggctggcg cccggctctc agcaagagcc ccgatcggcc ggtacgaagg 62160
gattgctcaa tgtctccggg agacagtcgt cgcgtcgctg cggaatccac ggttcccgcg 62220
cgggagaagg taagctgtcg gcggttgggg cggtggcgta cagctgcgag gcggaccgtg 62280
gagcgcgacc tccaccgccg actgcggcag gagctgcgcg acctcggcat ccacccaccc 62340
ctggacgtag aggaactggc caaggcgctc ggcgagaggc gcggacgccc gatcgtcctg 62400
cgtcccttcc cgctggagaa gccagggccc tcggggctgt ggatcgatac gccgcagatg 62460
gacgtgatcc tctaccagca ggagacgacc cggctgcacc agcggcagat catcctccac 62520
gagatcctcc acatcctcgt cgccgagtgg gaagaggacc aggccgagga ggcgcccgag 62580
gaatccccgg acgacttcgt cgagggttgg gcgaccctca ttcccgtact cgatcccaaa 62640
ctgatccggc gggtcgcccg ccgttgctcc tatgaggacg aagaggagtg ctcggtcgag 62700
ctcgcggcca cgatcatcct cgagtggtcc tcagtgctcg acgaactccc tccgctctcg 62760
gaagacccgg aggtacgtcg tgtgcagtcc gccctgggcg accggcgagg gtggctgtag 62820
tccgtgagcg agatactcct gctggtcatc gcggccgccg tcgtgtggag gctctacctg 62880
tggtcccggg ccccgcatga cgctcccacg aggtcggtcg ccctctgcct gctgagtgcg 62940
gggctgtcgt accccgtggc catgccgggc ggcaccaccg gcatcgacac cgtggccggg 63000
cacggcacgg cgaagctgct gcagaacgtc cttctcctgc tgacggtcta cttcctgatg 63060
tgcttctacc tgtactcggc cgacgggtcg gtggcgcgac gacgtgcgcg gtgggaagcc 63120
gtcgtcgtgt ccgtggtcgc tgtggcgatc atcctggcgg ccgtgaccgt gccgcacgag 63180
gacttcgccg gctcgttcag cacggcggac atgacgatcc cgcagatcgc cttcttctac 63240
gccggcgcgg gtctgtacct gatgtacgcg ctcggcgctg cgggacggtg gaccgtccgt 63300
tatgcccgca tgtcgagcag gccgcacgcc accgggctgt ggatgaccgc gatcggtctc 63360
ggcgccatgg cggtggcctg cgccgtccgc gcggtcttcg tcgccgtccg gtggagcggc 63420
ggaaccgttc cggaccgact catggctggc gtggcgttct ggctcgtcgt gtcgatcctg 63480
ctcttcgtcg cgggtgtcac ctattcggcg acccgctcca ggatcacggc cacccggctg 63540
tggctacggc gtcgccgtga tcaccgtcgg cttagcccgc tgtggcaact gctggccgag 63600
gtctacccgg agaacgaact gcgccccgcc tcccgagggc tgtgggaccg gtggcgtgcg 63660
cgcggcgtgc accgtcgcta ccaccggcgg atagtggaat gccgggacgg tctggtcgac 63720
atcagcccct atctggtcga cgagaacggc gacgccgacc tgctccgcct ggagccggcg 63780

gaactcgcga gcagactgcg gcaggccgcc gacatgatcc ggcagggttc cccggcgcct 63840

ggacaggcgg tgccgctcgc ggtgccgaag gaggacgacc gggacgcgga cgtccgtcag 63900

ctgatcgcgg tgtcggaagc actccggctg accgcgtgac cggggaagaa cgagggagac 63960

gtaacgagat gctgatcacc gccagccggg tcctgaccgg ctccgggacg cacctggagg 64020

acggcgccgt cctcgtcgaa ggcgactcga tcgccgcagt cgggcggcgc gcagaacttg 64080

ccgaccgcgt cggcgcggat gggaagcacc tggcgtttcc cgacgcgaca ctgctgcccg 64140

gactgatcga cgcccacgtg catctcgcgt tcgacggcgg cggcgacccg gtggccaccc 64200

tcgacgagtc gagcgacgag aagctgttgg aggacatgcg ccgacgcgcg gagcaactcc 64260

tgtccagcgg ggtgacgacg gtccgggacc tgggcgaccg ccacggcctg gccctccgcc 64320

tggatgaaga gatcagccag ggcggcactt ccggcccgcg catcgtggcc gcgggcacac 64380

cggccactcc tcctggaggg cattgtcact tcctgggtgg cgaggtctca ggcgtggacc 64440

aggtccgcga tctcgtacgg cgcaacatcg cggcgggcgc cggcgtgatc aaggcgatgg 64500

tcaccggagg cggcctgacc aaggacgggc cgaagagctg gcagagccag ttcagtccgg 64560

acgaactcca ggccttggtc gacgaggccc accaggccga cgtgccggtg gccgctcacg 64620

cgcacggtac ggacggcatc accgcggcag tcgaagcagg cgtcgacacg atcgagcact 64680

gcacctggat gaccgccgac ggctttgacc tccgccagga cgtcctgaag cagatcatcg 64740

atcgggacat cgcggtctgc ccggccgtca gccctcactg ggagatgctg ccccgcttct 64800

tcggggagga gcgggcggcg gccatgttcg atctcgtacg gcagatggcg gaggccggcg 64860

ccaagctcat cgctggcacc gacgcagggg tccagcgcgc cgggttcgac ggcctggtcc 64920

ccgccctttc cttctacgcg caccttggcc tgtcgaacag caggatcctc gacatggcga 64980

ccgccgacgc ggccggtgca ctagggctgg gcgagacgac gggccggatc gctccgaggt 65040

tccgcgcgga cctgctggtg atcgacggag accctctgga agacctcagt gcgctaaaga 65100

tggtccgggc cgtggtcgcc gctggccgcc tcctcgagcc gggcaggacg gcagaacagc 65160

agtagcaacc gtgcctgtcc gcaggccgtg agctgcggac aggcgctact cgttcgcgcc 65220

cgtttccttc tcctccagtt ccgccaccaa ctcgttgacg agtgccatcg ccttcggcga 65280

caggccctgc gcgcccaggc cacgagcctt gacccggccc acggccccct tcttggcggc 65340

tgccagtagc tggaggccct catagacctg agcggccacg gcgtcgtccg gcttgaagta 65400

catcctcgag acaccgatga cgtcggccag tgcgtccaga acctcagggg cagcggtctc 65460

agccacgccc gtacggagat gccggaagcc ctcctcggtg aggatctgct ccccggcgct 65520

ggcgttcacc gcgcttgcca tctccgcatc cgacggtggc cctggaggac tgggatacga 65580

gctgttcagc agcagggtga tcttctcggt cagcgatccc ggagcagcgt cagcggctgg 65640

ggccctcttc gcctccgacg gcgcagatcc ctcgaccggc ataccctgcg ccttggcgat 65700

cgaccggtct tccgcccgcc gcagttcgac ctcgctgacg ccgtatgccg cagccagagc 65760

cgggatgtac ttgtccttga gccgccgctc accgcgctcg gcgcctgcca ctggcccggc 65820

gctcttggtt ccgatcagcg tggccgtctc gtactggtag tagccggcat cgcaccgcag 65880

atcagtcaga tcgggcggcc cggaccgcgg gaacagatca tctacatcgt ggccgacagc 65940

acgcgccagt gcaggcagct tctcagggtc cggagtctgg gcaccgcttt cccagctagc 66000

gaccgccgag tcggatacgc ccagcacctc gcccacggca gcttgggatt tctcggcggc 66060

tcgccgcacc gtacgtagac ggctccggtc gaagtgacga gcagacatcg aaccctcgtt 66120

ttttgtgcaa gctggaggtc gtccagtgtg ttgacttttt cttcaggcgg gaatagcttc 66180

agcttagcga agggcgggtg tggtttgcac ccctgctcga tcttcgtgtt cgcgccctgt 66240

cgaggctctc gggctgaggg agggagcgag gggagcgcaa ccgtgacgtc ctgtgcgtga 66300

agcgcctcag tgggaagcgg cgccggcgag ttcgcctctc gggcgtcgga aagtgcgcga 66360

cttcgctccg gaagagggcg gagtcgtact tcggtcactc catgcccttg aacggtacgc 66420

gcgagtggct gtcgcgtgag gagtgtcgta tgtctctgct tcaccaaacg gtcgtcggta 66480

aagccgtccg gctcgaatgc aaggcgcctg cagagattcc gtcgacgctg atcctcatgc 66540

tctgcagcag cttagaaaac aggtgcgggc gtggattccc gtcgaggact gtgtcctgac 66600

gcccaccaac ccagcgatgt atgtcgagtt ggcgtcgaat cggctcagta cccgctgtca 66660

gctggagtgt taaccatcct ctgcatatgt tgctgctccc tcggcggaac agccgaaccg 66720

aagcctctca agggagaaac gcgaaaaccc catccaacgt caagtccgcg gatttcaggg 66780

ggaaatgacg cggcgcccag gagttagcag ctcccgggcg ccaagcccgc caccctgagg 66840

gcgcggattc ataaccgggg cgagtcgg 66868

<210> 2
<211> 412
<212> PRT
<213> Streptomyces DSM 22643

<400> 2

Met Ser Glu Asn Leu Thr Leu Gly Asp Arg Leu Arg Ile Ala Arg Arg
1               5                   10                  15

Thr Arg Lys Leu Ser Ala Ala Gln Leu Ala Gln Lys Val Ala Val Ser
            20                  25                  30

Pro Ser Tyr Val Gln Lys Leu Glu Ser Gly Ala Arg Lys Ala Ser Pro
        35                  40                  45

Ser Leu Val Leu Ala Leu Ala Lys Ala Leu Arg Phe Gly Pro Glu Val
    50                  55                  60

Leu Thr Gly Gln Pro Tyr Tyr Gly Glu Pro Glu Ala Glu Asp Gly Val

65                  70                  75                  80

His Ala Val Ile Pro Glu Leu Arg Arg Leu Leu Leu Cys Tyr Asp Ser
                85                  90                  95

Pro Asp Asp Leu Glu Ile Ala Pro Arg Ala Leu Pro Val Leu Ala Ser
            100                 105                 110

Glu Val Asp Gln Val Ala Ala Leu Arg Arg Asp Ala Arg Tyr Ala Pro
        115                 120                 125

Met Gly Pro Leu Leu Pro Pro Ile Ile Thr Glu Leu Thr His Val Ala
    130                 135                 140

Leu Gly Gly Asn Asn Gly Asp Arg Gly Lys Ala Phe Trp His Leu Ala
145                 150                 155                 160

Arg Ala Tyr Arg Ala Val Asn Ser Leu Ala His Lys Met Gly His His
                165                 170                 175

Asp Leu Ser Asn Thr Ala Leu Glu Arg Val Arg Trp Ala Ala Asp Arg
            180                 185                 190

Ser Gly Asp Pro Leu Met Gln Phe Thr Ala Gly Tyr Leu Val Ala Gly
        195                 200                 205

Ala Met Leu Arg Gln Gly Ala Tyr Ser Pro Ala Arg Arg Lys Leu Leu
    210                 215                 220

Gly Leu Arg Thr Glu Leu Glu Arg Phe Gln Pro Glu His Ser Phe Thr
225                 230                 235                 240

Glu Asp Ala Leu Ala Val Asp Gly Ala Leu Leu Leu Lys Leu Ala Val
                245                 250                 255

Leu Glu Ala Arg Glu Asn Asn Ser Asp Arg Ala Asp Ala Tyr Leu Arg
            260                 265                 270

Glu Ala Glu Gln Val Ala Thr Met Ala Gly Asn Arg Asp Ser Leu Ala
        275                 280                 285

Tyr Glu Met Ser Phe Gly Pro Thr Asn Ile Arg Ile His Glu Val His
    290                 295                 300

Ala Met Ile Asp Thr Gly Asp Thr Glu Gln Ala Leu Ala Arg Leu Val
305                 310                 315                 320

Glu Trp Ser Pro Val Ser Gly Gly Glu Trp Ala Pro Pro Ser Thr Thr
                325                 330                 335

Val Gly Glu Arg Ser Ser His His Phe Ile Asp Val Ala Ser Ala Lys
            340                 345                 350

Leu Ala Glu Gly Asp Arg Asp Gly Ala Phe Ala Asp Leu Lys Arg Ala
        355                 360                 365

Arg Lys Val Ala Pro Asn His Thr Arg Phe His Pro Ser Val Arg Glu
    370                 375                 380

Thr Thr Ala Ala Leu Leu Arg Met Asp Ala His Pro Ser Asn Glu Leu
385                 390                 395                 400

Ser Ala Phe Gly Ser Trp Thr Gly Ile Ser Thr Thr
                405                 410

<210>  3
<211>  146
<212>  PRT
<213>  Streptomyces DSM 22643

<400>  3

Met Ser Asp Asp Asn Pro Ala Ala Arg Ala Asn Pro Pro Arg Trp Met
1               5                   10                  15

Pro Ile Gly Glu Glu Pro Glu Leu Cys Ser Ala Gly Glu Trp Trp Asp
            20                  25                  30

Ala Val Arg Ala Val Glu Ala Val Gly Arg Arg Ala Ile Glu Ile Leu
        35                  40                  45

Gly Glu Gly Asp Glu Pro Val Gly Pro Val Ile Leu Asp His Gly Gly
    50                  55                  60

Pro Glu Pro Arg Leu Tyr Phe Leu Val Pro Val Gly Thr Ala Ala Arg
65                  70                  75                  80

Trp Glu Glu Pro Gly Thr Val Ala Leu Gly Gln Lys Cys His Val Val
                85                  90                  95

Val Pro Ser Ala Glu Ser Thr Thr Pro Pro Gly Met His Trp His Val
            100                 105                 110

Phe Pro Gln Gly Pro Arg Ser Leu Thr Arg Pro Asp Ala Leu Arg Arg
        115                 120                 125

Ala Leu Ser Gln Ala Arg Arg Glu Arg Arg Gly Pro Ala Glu Glu Ala
    130                 135                 140

Ala Cys
145

<210> 4
<211> 64
<212> PRT
<213> Streptomyces DSM 22643

<400> 4

Met Ala Leu Ser Ile Ser Leu Val Leu Val Leu Gly Ile Val Val Val
1               5                   10                  15

Leu Leu Val Arg Ser Lys Ala Val Lys Pro Gly Pro Ala Ile Val Cys
            20                  25                  30

Val Leu Phe Gly Phe Phe Leu Ala Ser Thr Ser Ile Ala Pro Asn Ile
        35                  40                  45

Asn Arg Phe Val Thr Gly Val Ala Asp Met Ile Gly Gln Ile Ser Phe
    50                  55                  60

<210> 5
<211> 334
<212> PRT
<213> Streptomyces DSM 22643

<400> 5

Met Arg Leu Val Tyr His Pro Ala Gly His Asp Asp Asp Leu Arg Thr
1               5                   10                  15

Ala Leu Val Glu Leu Gln Ala Gly Arg Trp Lys Ser Ala Arg Gly Leu
            20                  25                  30

Leu Trp Glu Thr Gly Thr His Trp Pro Leu Arg Thr Ser Arg Thr Gln
        35                  40                  45

Leu Leu Ala Val Ala Ala Ala Arg Ser Asp Val Val Asp Val Trp Leu
    50                  55                  60

Val Glu Glu Pro Asp Ser Tyr Asp Ala Gln Leu Met Ala Val Arg Val
65                  70                  75                  80

Ala Val Glu Arg Ala Leu Arg Ala Gln Arg Gln Gln His Pro Arg Thr
                85                  90                  95

Leu Glu Phe Glu Thr Arg Ala Arg Arg Ala Ala Leu Leu Ala Ala Arg
            100                 105                 110

Arg Ala Pro His Asp Pro Val Pro Trp Val Cys Leu Val Ala Leu Ala
        115                 120                 125

Gln Ile Asp Thr Gln Gln Leu Arg Gln Glu His Arg Met Arg Pro Asn
    130                 135                 140

Glu Pro Met Leu Pro Ser Gly Pro Trp Gly Leu Leu Tyr Glu Val Asn
145                 150                 155                 160

Gln Arg Asp Pro Tyr Asn Arg Glu Ala Tyr His Arg Val Leu Gln Phe
                165                 170                 175

Leu Leu Ala Leu Glu Gly Pro Trp Gly Ala Ser Leu Ala Ala Val Phe
            180                 185                 190

Asp Phe Gly Arg Ser Val Ala Ser Gln Arg Pro Val Gly Ser Pro Leu
        195                 200                 205

Leu Leu Leu Pro Ala Tyr Ala Gln Ile Glu Gln Arg Arg Gln Ser Arg
    210                 215                 220

Ala Asp Pro Leu Trp Arg Arg Gln Trp Ala Gln Glu Ser Thr Leu Asp
225                 230                 235                 240

Tyr Thr Leu Ala Ala Phe His Asp Trp Phe His Lys Ala Pro Val Gly
                245                 250                 255

Gln His Ser Val Ala Asp Leu Asn Leu Leu Ala Tyr Ala Leu Trp Ala
            260                 265                 270

Gly Ala Gln Tyr Leu Glu Ala Ala Glu Val Phe Glu Val Met Gly Pro
        275                 280                 285

Tyr Ala Ala Arg Glu Pro Trp Ala Ser Val His Glu Gly Ala Ala Gly
    290                 295                 300

Pro Asp Pro Gly Glu Ala Leu Leu Leu Arg Ala Arg Ala Glu Ser Leu
305                 310                 315                 320

Ser Tyr Ala Arg Asn His Arg Pro Arg Ala Gly Pro His Leu
                325                 330

<210>  6
<211>  506
<212>  PRT
<213>  Streptomyces DSM 22643

<400>  6

Val Ser Arg Leu Ser Pro Asn His Arg Ala Thr Ser Arg Lys Pro Asp
1               5                   10                  15

Asp Ala Tyr Leu Arg Glu Leu Gly Tyr Glu Pro Val Leu Thr Arg Arg

20                  25                  30

Met Gly Pro Phe Gly Asn Phe Ala Ile Ser Phe Ser Val Ile Ser Val
        35                  40                  45

Leu Ser Gly Cys Met Thr Leu Tyr Gly Phe Gly Leu Asn Thr Gly Gly
    50                  55                  60

Pro Ser Val Met Leu Trp Gly Trp Leu Ile Val Gly Ala Met Val Thr
65                  70                  75                  80

Phe Ile Gly Ala Ala Leu Ala Glu Val Thr Ser Ala Tyr Pro Thr Ser
                85                  90                  95

Gly Ala Leu Tyr Tyr Gln Ala Glu Gln Leu Gly Gly Arg Lys Trp Gly
            100                 105                 110

Trp Tyr Thr Gly Trp Leu Asn Leu Leu Gly Leu Leu Gly Ala Ile Ala
        115                 120                 125

Gly Ile Asp Tyr Gly Ala Ala Leu Phe Thr Gly Ala Leu Leu Asn Leu
    130                 135                 140

Gln Trp Gly Phe Glu Pro Thr Pro Gly Gly Thr Met Val Ile Phe Leu
145                 150                 155                 160

Cys Ile Leu Ala Leu Asn Leu Phe Gly Val Arg Leu Val Ser Ile Leu
                165                 170                 175

Asn Ser Ile Ser Val Trp Trp His Leu Gly Gly Val Thr Val Ile Val
            180                 185                 190

Gly Ala Leu Ala Ile Val Pro Ser His His Gln Ser Ala Asp Phe Val
        195                 200                 205

Phe Gly Glu Phe Val Asn Asn Thr Gly Trp Ser Ser Pro Leu Tyr Val
    210                 215                 220

Ala Val Leu Gly Leu Leu Leu Ala Gln Tyr Thr Phe Cys Gly Tyr Asp
225                 230                 235                 240

Ala Ser Ala His Leu Ser Glu Glu Thr Thr Asp Ala Gln Val Ser Ala
                245                 250                 255

Ser Arg Gly Ile Ile His Ala Ile Gly Trp Ser Trp Leu Ala Gly Phe
            260                 265                 270

Val Leu Leu Ala Gly Leu Thr Phe Ala Ile Gln Asp Tyr Ala Gly Thr
        275                 280                 285

Val Gly Thr Ala Thr Gly Val Pro Pro Ala Gln Ile Phe Leu Asp Ala
    290                 295                 300

Leu Gly Val Ala Gly Ala Lys Ala Leu Leu Leu Val Val Ile Ile Ala
305                 310                 315                 320

Gln Leu Cys Cys Gly Asn Ala Glu Thr Ala Ala Ala Ser Arg Met Val
                325                 330                 335

Phe Ala Phe Ser Arg Asp Gly Ala Leu Pro Gly Ser His Leu Trp Arg
            340                 345                 350

Gln Val Asp Arg Arg Thr Gly Thr Pro Arg Met Ala Val Leu Leu Ala
        355                 360                 365

Val Val Cys Ala Ala Val Leu Ala Leu Pro Ser Leu Tyr Ser Pro Val
    370                 375                 380

Ala Tyr Ala Ala Ile Thr Ser Ile Asn Val Val Gly Ile Thr Pro Ala
385                 390                 395                 400

Tyr Ala Ile Pro Ile Tyr Leu Arg Ile Lys Asn His Asp Arg Phe Arg
                405                 410                 415

Pro Gly Pro Trp Asn Leu Gly Asn Trp Gly Val Ala Val Gly Thr Ile
            420                 425                 430

Ala Val Val Trp Val Val Phe Val Thr Val Leu Phe Cys Leu Pro Gln
        435                 440                 445

Thr Arg Pro Ala Glu Gly Gly Leu Val Ser Val Glu Thr Phe Asn Tyr
    450                 455                 460

Ala Pro Ile Ala Leu Leu Val Val Leu Val Leu Ala Trp Gly Trp Trp
465                 470                 475                 480

Arg Lys Gln Gly Ser Ser Tyr Glu Val Pro Ala Gln Asn Phe Asp Arg
                485                 490                 495

Ser Thr Ala Thr Tyr Glu Asp Glu Val Val
            500                 505

<210>  7
<211>  479
<212>  PRT
<213>  Streptomyces DSM 22643

<400>  7

Val Met Thr Arg Thr Thr Thr Gly Asn Gly Thr Val Ala Arg Leu Ala
1               5                   10                  15

Ala Ala Pro Gly Pro Glu Gln Gly Gly Arg Ser Gly Lys Gly Ile Ser
            20                  25                  30

Leu Ser Asp Leu Arg Asn Leu Val Lys Ala Gly Ala Ile Asp Thr Val
        35                  40                  45

Leu Leu Ala Val Pro Asp Leu Gln Gly Arg Leu Lys Gly Lys Arg Tyr
    50                  55                  60

Asp Ala Asn His Phe Leu Lys Arg Val Ala His Asp Gly Ala Glu Val
65                  70                  75                  80

Cys Ala Tyr Val Leu Ala Thr Asp Val Asp Met Ser Pro Ala Asp Gly
                85                  90                  95

Phe Ala Leu Thr Ser Trp Glu Thr Gly Tyr Gln Asp Leu Ser Val Gln
            100                 105                 110

Pro Ala Leu Ser Thr Leu Cys Val Val Pro Trp Leu Pro Arg Thr Val
        115                 120                 125

Ala Val Leu Gly Asp Ala Val His His Asp Gly Thr Leu Ile Asp Ile
    130                 135                 140

Ala Pro Arg Gln Ile Leu Leu Gln Gln Leu Thr Arg Leu Ser Arg His
145                 150                 155                 160

Gly Leu His Pro Lys Val Gly Ile Glu Thr Glu Phe Val Leu Tyr Lys
                165                 170                 175

Gly Thr Tyr Ala Asp Ala Glu Gln Ala Gly Tyr Gln Gly Leu Arg Pro
            180                 185                 190

Leu Thr Thr Glu Asn Leu Asp Tyr Ala Leu Asp His Asp Pro Val Ser
        195                 200                 205

Asp Arg Tyr Leu Arg Arg Leu Gln Arg Ala Leu Ala Gly Ala Gly Met
    210                 215                 220

Pro Val Glu Ala Ile Lys Thr Glu Ala Gly Pro Gly Gln Val Glu Val
225                 230                 235                 240

Thr Phe Pro Tyr Gly Gly Ala Leu Thr Ala Cys Asp Arg His Pro Leu
                245                 250                 255

Phe Lys His Ala Val Arg Thr Leu Gly Ser Arg Ala Gly Leu Ala Pro

260                 265                 270

Thr Phe Met Ala Ala Pro Glu Thr Gly Arg Ala Asn Gly Leu His Leu
        275                 280                 285

His Val Ser Leu Trp Ser Lys Ala Ile Ser Gln Leu His Glu Pro Gly
    290                 295                 300

Thr Glu His Glu Leu Ser Gln Val Gly Gln His Ala Ile Ala Gly Leu
305                 310                 315                 320

Leu Ala Gly Leu Pro Glu Leu Gly Pro Phe Tyr Ala Pro Ser Val Asn
                325                 330                 335

Ser Tyr Lys Arg Phe Thr Pro Gly Ser Phe Ala Pro Thr Thr Phe Thr
            340                 345                 350

Trp Gly Arg Asp Asn Arg Thr Cys Ala Val Arg Val Val Gly Arg Gly
        355                 360                 365

Glu Gly Leu His Leu Glu Ile Arg Val Pro Gly Ala Asp Ala Asn Pro
    370                 375                 380

Tyr Leu Ala Leu Ser Ala Val Leu Ala Ala Met Asp His Gly Leu Glu
385                 390                 395                 400

Arg Lys Pro Ala Leu Gly Pro Glu Ala Thr Gly Asn Ala Tyr Arg Ala
                405                 410                 415

Ser Gly Thr Glu Ala Pro Val Pro Ser Thr Leu Gly Leu Ala Leu Thr
            420                 425                 430

Val Phe Gln Asp Ser Ala Leu Ala Arg Gln Ala Phe Gly Thr Glu Val
        435                 440                 445

Val Glu His Tyr Ala Arg Leu Ala Gly Leu Glu Leu Ala His Asp Glu
    450                 455                 460

Arg Leu Val Thr Asp Ala Glu Arg Gln Arg Trp Leu Ala Arg Ala
465                 470                 475

<210>  8
<211>  297
<212>  PRT
<213>  Streptomyces DSM 22643

<400>  8

Val Thr Asp Met Pro Ser Ala Leu His Gln Leu Val Glu Ser Val Thr
1               5                   10                  15

Asp Met Tyr Glu Val Val Ala Glu His Ala Arg Pro Gly Ala Ile Arg
            20                  25                  30

Pro Ser Val Trp Glu Val Asn Gly Pro Gly Gly Gln Arg Trp Phe Gly
        35                  40                  45

Lys Val His Ala Gly Pro Lys Leu His Arg Arg Glu Val Thr Ala Tyr
    50                  55                  60

Gln Lys Trp Thr Val Ala Leu Arg Ala Asp His Ala Pro Glu Leu Val
65                  70                  75                  80

Ala Ala Asp Thr Leu Thr Arg Thr Val Leu Val Thr Ala Val Pro Gly
                85                  90                  95

His Gly Leu Asp Thr Leu Arg Leu Pro Ala Glu Gln Glu His Ala Ala
            100                 105                 110

Tyr Val Gln Ala Gly Glu Leu Leu Ala Arg Phe His Thr Ala Ala Ala
        115                 120                 125

Asp Glu Pro Met Pro Glu Thr Ala Asp Glu Ala Trp Asp Glu Ala Val
    130                 135                 140

Ala Arg Leu Leu Asp Arg Thr Ala Thr His Ala Ser Glu His Asp Leu
145                 150                 155                 160

Ala Leu Val Arg Thr Leu Ala Lys Glu Ala Pro Pro Arg Leu Pro Pro
                165                 170                 175

Val Ser Gln His Gly Asp Tyr Met Pro Lys Asn Trp Met Trp Asp Glu
            180                 185                 190

Thr Glu Gln Arg Leu Arg Val Ile Asp Phe Glu Arg Ala Glu Leu Arg
        195                 200                 205

Thr Pro Ala Tyr Arg Asp Leu Ser Arg Leu Arg Tyr Arg Ile Leu Cys
    210                 215                 220

His Arg Pro Asp Leu Asp Ala Ala Phe His His Gly Tyr Gly Arg Pro
225                 230                 235                 240

Leu Thr Glu Glu Glu Gln Ile Ala Cys Arg Ala Tyr Gly Ala Leu Asp
                245                 250                 255

Ala Leu Asp Ser Leu Asp Trp Gly Ile Lys His Arg Asp Ile Gly Leu
            260                 265                 270

Val Asp Glu Ala Gln Thr Met Leu Glu Asn Leu Arg Arg Glu Thr Gly
        275                 280                 285

Lys Arg Val Trp Gly Gly Trp Arg Ala
    290                 295

<210>  9
<211>  7794
<212>  PRT
<213>  Streptomyces DSM 22643

<220>
<221>  DOMAIN
<222>  (9)..(1493)
<223>  Module 1

<220>
<221>  DOMAIN
<222>  (9)..(336)
<223>  Module 1; domain C1

<220>
<221>  DOMAIN
<222>  (481)..(939)
<223>  Module 1; domain A1`

<220>
<221>  DOMAIN
<222>  (940)..(1365)
<223>  Module 1; domain MT in domain A1

<220>
<221>  DOMAIN
<222>  (1366)..(1406)
<223>  Module 1; domain A1``

<220>
<221>  DOMAIN
<222>  (1429)..(1493)
<223>  Module 1; domain T1

<220>
<221>  DOMAIN
<222>  (1517)..(2569)
<223>  Module  2

<220>
<221>  DOMAIN
<222>  (1517)..(1858)
<223>  Module  2; domain C2

<220>
<221>  DOMAIN
<222>  (2003)..(2482)
<223>  Module  2; domain A2

<220>
<221>  DOMAIN
<222>  (2505)..(2569)
<223>  Module  2; domain T2

<220>

<220>
<221>  DOMAIN
<222>  (2591)..(4102)
<223>  Module  3

<220>
<221>  DOMAIN
<222>  (2591)..(2932)
<223>  Module  3; domain C3

<220>
<221>  DOMAIN
<222>  (3077)..(3536)
<223>  Module  3; domain A3`

<220>
<221>  DOMAIN
<222>  (3537)..(3974)
<223>  Module  3; domain MT3 in domain A3

<220>
<221>  DOMAIN
<222>  (3975)..(4015)
<223>  Module  3; domain  A3``

<220>
<221>  DOMAIN
<222>  (4038)..(4102)
<223>  Module  3; domain  T3

<220>
<221>  DOMAIN
<222>  (4124)..(5170)
<223>  Module  4

<220>
<221>  DOMAIN
<222>  (4124)..(4465)
<223>  Module  4; domain C4

<220>
<221>  DOMAIN
<222>  (4612)..(5083)
<223>  Module  4; domain A4

<220>
<221>  DOMAIN
<222>  (5106)..(5170)
<223>  Module  4; domain T4

<220>
<221>  DOMAIN
<222>  (5193)..(6245)
<223>  Module  5

<220>
<221>  DOMAIN
<222>  (5193)..(5534)
<223>  Module  5; domain C5

<220>
<221>  DOMAIN
<222>  (5679)..(6158)
<223>  Module  5; domain A5

<220>

<221>  DOMAIN
<222>  (6181)..(6245)
<223>  Module  5; domain T5

<220>
<221>  DOMAIN
<222>  (6268)..(7312)
<223>  Module  6

<220>
<221>  DOMAIN
<222>  (6268)..(6609)
<223>  Module  6; domain C6

<220>
<221>  DOMAIN
<222>  (6754)..(7225)
<223>  Module  6; domain A6

<220>
<221>  DOMAIN
<222>  (7248)..(7312)
<223>  Module  6; domain T6

<220>
<221>  DOMAIN
<222>  (7336)..(7677)
<223>  Module  7

<220>
<221>  DOMAIN
<222>  (7336)..(7677)
<223>  Module  7; domain C7

<400>  9

Met Gln Ile Asn Ser Lys Leu Leu Ser Ala Ala Gln Arg Glu Ile Trp
1               5                   10                  15

Phe Ser Gln Gln Gln Asp Leu Glu Asn Pro Leu Tyr Arg Val Gly Glu
            20                  25                  30

Tyr Leu Asp Ile Ser Gly Asp Ile Asp Leu Thr Leu Phe Glu Arg Ala
        35                  40                  45

Leu Arg Thr Val Val Ala Gln Thr Glu Thr Leu His Val Arg Phe Thr
    50                  55                  60

Glu Gly Gln Glu Gly Pro Glu Gln Val Val Glu Arg Lys Glu Asp Trp
65                  70                  75                  80

Asp Leu Pro Val Ile Asp Leu Ser Asp Val Asp Asp Pro Leu Ser Val
                85                  90                  95

Ala Glu Arg Trp Met Arg Glu Asp Leu Gly Arg Ser Ile Asp Ile Thr
            100                 105                 110

Val Gly Pro Val Phe Ser Tyr Ala Leu Phe Arg Ile Ser Arg Asn Arg

115                 120                 125

Val Val Trp Tyr Gln Gly Tyr His His Ile Ala Met Asp Ala Phe Ala
    130                 135                 140

Met Gly Val Val Ala Arg Arg Val Ala Asp Ser Tyr Asn Arg Leu Ser
145                 150                 155                 160

Arg Asn Glu Gly Val Leu Gly Gly Asp Asp Ser Val Ala Leu Leu Leu
                165                 170                 175

Asp Asp Asp Ala Ser Tyr Arg Ala Ser Glu Asp Leu Ala Arg Asp Arg
            180                 185                 190

Glu Phe Trp Met Gly Arg Leu Ala Asp Leu Pro Glu Pro Val Glu Leu
        195                 200                 205

Ala Gly Arg Thr Ala Pro Ala Ser Gly Lys Phe His Arg Val Ser Met
    210                 215                 220

Thr Phe Ser Asp Val His Gly Gly Asp Ala Val Arg Gly Asp Arg Asp
225                 230                 235                 240

Leu Arg Gly Trp Ala Val Thr Leu Thr Ala Ala Ala Ala Ala Tyr Val
                245                 250                 255

Tyr Arg Ala Thr Gly Gln Val Asp Phe Leu Leu Gly Phe Ala Ala Ala
            260                 265                 270

Ala Arg Pro Gly Ala Asp Leu Lys Gln Val Ala Gly Met Met Ser Asn
        275                 280                 285

Leu Leu Pro Leu Lys Leu Thr Val Ser Gln Glu Thr Thr Val Ser Asp
    290                 295                 300

Leu Glu Gly Gln Val Cys Arg Glu Ile Ala Asp Leu Leu Arg His Gln
305                 310                 315                 320

Arg Tyr Arg Ser Glu Asp Ile Ala Arg Asp Leu Gly Leu Pro Ala Gly
                325                 330                 335

Ile Arg Gly Met Ile Gly Pro Arg Val Asn Phe Met Pro Phe Asp Tyr
            340                 345                 350

Asn Ile Thr Phe Gly Asp Ala Asn Ala Val Ala Lys Asn Leu Ser Leu
        355                 360                 365

Gly Val Val Asp Asp Leu Thr Ile Ala Ile Tyr Asp Arg Arg Asp Gly
    370                 375                 380

Ser Glu Leu Arg Ile Asp Ile Glu Ala Asn Ser Asp Leu Tyr Ser Tyr
385                 390                 395                 400

Gln Glu Leu Gln Ala His Ser Glu Arg Phe Met Arg Leu Leu Lys Glu
                405                 410                 415

Phe Thr Asp Pro Ser Arg Ser Ser Gln Pro Ile Ser Leu Leu Glu Phe
            420                 425                 430

Leu Asp Ala Glu Glu Glu Arg Arg Ile Leu Thr Glu Trp Ser Gly Ala
        435                 440                 445

Thr Asp Ala Ile Ser Thr Ala Thr Leu Pro Gly Leu Phe Glu Val Cys
    450                 455                 460

Ala Ala Ser Val Pro Gly Ala Thr Ala Val Val Phe Gly Asp Val Arg
465                 470                 475                 480

Val Ser Tyr Gly Val Leu Asn Glu Arg Ala Asn Arg Leu Ala His Trp
                485                 490                 495

Leu Leu Gly Arg Gly Val Gly Pro Glu Arg Val Val Ala Leu Ala Leu
            500                 505                 510

Pro Arg Gly Val Asp Leu Val Val Ala Val Leu Ala Val Val Lys Ala
        515                 520                 525

Gly Ala Ala Tyr Leu Pro Val Asp Pro Asp Tyr Pro Ala Glu Arg Val
    530                 535                 540

Ala Tyr Met Leu Glu Asp Ser Arg Pro Val Leu Ala Leu Thr Ser Ser
545                 550                 555                 560

Ala Val Val Ala Gly Leu Pro Val Val Asp Asp Val Glu Tyr Val Ser
                565                 570                 575

Leu Asp Asp Pro Ala Val Leu Gly Glu Leu Ala Gly Cys Gly Val Ser
            580                 585                 590

Asp Pro Ser Asp Ala Asp Arg Gly Ala Val Leu Ser Pro Ala His Pro
        595                 600                 605

Val His Val Ile Tyr Thr Ser Gly Ser Thr Gly Arg Pro Lys Gly Val
    610                 615                 620

Met Thr Ser His Gly Asn Val Val Arg Leu Phe Asp Val Gly Glu Gly
625                 630                 635                 640

Gly His Trp Phe Gly Phe Ala Pro Asp Asp Val Trp Ala Leu Phe His
                645                 650                 655

Ser Tyr Thr Phe Asp Phe Ser Val Phe Glu Leu Trp Gly Ala Leu Leu
            660                 665                 670

His Gly Gly Cys Leu Val Val Val Pro His Leu Thr Ser Arg Ser Pro
        675                 680                 685

Val Glu Leu Leu Arg Leu Leu Val Ala Glu Gln Val Thr Val Leu Cys
    690                 695                 700

Gln Thr Pro Ser Ala Phe Asp Ala Leu Ala Gly Val Val Ala Gln Asp
705                 710                 715                 720

Pro Ala Gly Ala Glu Gly Leu Val Leu Arg Arg Val Val Phe Gly Gly
                725                 730                 735

Glu Ala Leu Pro Ala Arg Thr Ala Glu Leu Ala Ser Gly Leu Val Pro
            740                 745                 750

Gly Val Arg Val Val Asn Ile Tyr Gly Pro Thr Glu Thr Thr Val His
        755                 760                 765

Ala Thr Thr Cys His Val Asp Ser Val Ser Gly Gly Asn Pro Val Val
    770                 775                 780

Ser Ile Gly Arg Pro Val Asp Arg Ala Leu Gly Tyr Val Leu Asp Ala
785                 790                 795                 800

Gly Leu Arg Val Val Pro Val Gly Val Ala Gly Glu Leu Tyr Val Ala
                805                 810                 815

Gly Ala Gly Leu Ala Arg Gly Tyr Val Asn Arg Ala Gly Leu Thr Ala
            820                 825                 830

Ser Arg Phe Val Ala Asp Pro Tyr Gly Pro Ala Gly Ser Arg Met Tyr
        835                 840                 845

Arg Thr Gly Asp Val Val Arg Trp Asn Thr Ser Gly Glu Leu Glu Phe
    850                 855                 860

Val Gly Arg Ala Asp Asp Gln Val Lys Ile Arg Gly Phe Arg Ile Glu
865                 870                 875                 880

Leu Gly Glu Ile Glu Thr Thr Ala Ala Gly His Pro Ala Val Ala Gln
                885                 890                 895

Ala Ala Ala Thr Val His Glu Asp Asp Thr Arg Gly Lys Gln Leu Ala
            900                 905                 910

Leu Tyr Val Val Pro Thr Gly Leu Thr Ser Gly Asp Val Ser Gly Ser
        915                 920                 925

Val Ser Gly Asp Gly Ala Val Pro Asp Gly Gly Val Ser Gly Val Val
    930                 935                 940

Asp Glu Gln Val Gly Glu Trp Arg Glu Ile Tyr Asp Ser Leu Tyr Gly
945                 950                 955                 960

Gly Pro Gly Ser Ser Val Phe Gly Glu Asp Phe Ser Gly Trp Asp Ser
                965                 970                 975

Ser Tyr Asp Gly Ala Ala Ile Pro Leu Glu Glu Met Arg Glu Trp Arg
            980                 985                 990

Asp Ala Thr Val Glu Arg Ile Arg Gly Leu Gly Gly Arg Arg Val Leu
        995                 1000                1005

Glu Ile Gly Val Gly Thr Gly Leu Leu Met Ser Arg Leu Ala Ala
    1010                1015                1020

Gly Cys Glu Glu Tyr Trp Ala Thr Asp Leu Ser Gly Val Val Ile
    1025                1030                1035

Asp Ala Leu Asp Gly His Val Gln Ala Asp Pro Val Leu Arg Glu
    1040                1045                1050

Arg Val Arg Leu Ala Cys Gln Arg Ala Asp Asp Thr Arg Gly Leu
    1055                1060                1065

Pro Glu Gly Tyr Phe Asp Thr Val Val Ile Asn Ser Val Val Gln
    1070                1075                1080

Tyr Phe Pro Gly Ala Gln Tyr Leu Ala Ser Val Ile Glu Ala Ala
    1085                1090                1095

Val Ser Arg Leu Ala Pro Gly Gly Arg Val Phe Ile Gly Asp Val
    1100                1105                1110

Arg Asp Leu Arg Thr Leu Arg Ala Phe His Thr Ala Val Gln Leu
    1115                1120                1125

Thr Arg Thr Thr Gly Gly Arg Ala Gly Asp Gly Met Asp Ala Gly
    1130                1135                1140

Gly Leu Arg Arg Ala Val Glu Gln Gly Leu Leu Leu Glu Asn Glu

1145                1150                1155

Leu Leu  Leu Asp Pro Glu Phe  Phe Thr Ala Val Gly  Arg Thr Leu
    1160                1165                1170

Pro Ala  Val Ser Ala Val Glu  Val Arg Leu Lys His  Gly Gln Ala
    1175                1180                1185

His Asn  Glu Leu Thr Arg His  Arg Tyr Asp Val Ile  Leu His Thr
    1190                1195                1200

Thr Asn  Thr Glu Thr Asp Thr  Glu Ala Pro Ala Pro  Thr Pro Ala
    1205                1210                1215

Pro Val  Glu Gly Ile Ser Trp  Asn Ser Val Pro Gly  Gln Leu Thr
    1220                1225                1230

Gly Leu  Glu Glu Ile Leu Arg  Ser Arg Gly Ala Ala  Pro Leu Arg
    1235                1240                1245

Val Thr  Gly Ile Pro Asn Ala  Arg Leu Ala Gly Glu  Tyr Ala Ala
    1250                1255                1260

Leu Arg  Val Leu Glu Asn Gly  Gly Thr Leu Thr Glu  Ala Val Thr
    1265                1270                1275

Ala Leu  Ala Gly Pro Arg Gly  Ile Asp Pro Glu His  Leu His Gln
    1280                1285                1290

Leu Ala  Ala Ala Thr Gly Tyr  His Ala Val Leu Gln  Pro Ala Pro
    1295                1300                1305

Ala Pro  Asp Thr Tyr Asn Thr  Leu Leu Leu Pro Leu  Asp Ile Phe
    1310                1315                1320

Asp Gly  Thr Ala Trp Ser Ala  Thr Ala Thr Ala Thr  Ala Thr Asp
    1325                1330                1335

Leu Arg  Glu Thr Ser Ala Pro  Asp His Thr Ala Glu  Thr Ser Phe
    1340                1345                1350

Gln Ala  Leu Ala Asn Asn Pro  Ala Ala Ser Arg Asp  Thr Ser Thr
    1355                1360                1365

Leu Ile  Thr Gln Val Arg Asp  His Leu Arg Thr Lys  Leu Pro Asp
    1370                1375                1380

His Met  Val Pro Ala Ala Ile  Val Val Leu Glu Arg  Leu Pro Leu
    1385                1390                1395

Thr Ala Ser Gly Lys Leu Asp Arg Arg Ala Leu Pro Ala Pro Asp
    1400                1405                1410

Leu Gly Thr His Thr Thr Gly Arg Ala Pro Arg Ser Pro Arg Glu
    1415                1420                1425

Glu Ile Leu Ala Gly Leu Phe Ala Glu Val Leu Gly Leu Pro Ala
    1430                1435                1440

Val Gly Ile Asp Asp Ser Phe Phe Asp Leu Gly Gly His Ser Leu
    1445                1450                1455

Leu Ala Thr Arg Leu Ile Ser Arg Ile Arg Ala Ile Leu Gly Val
    1460                1465                1470

Glu Ile Pro Ile Arg Asp Leu Phe Glu Ala Pro Thr Val Ala Gly
    1475                1480                1485

Leu Ala Val Leu Leu Glu Thr Gly Ser Ser Asp Val Val Arg Arg
    1490                1495                1500

Lys Leu Thr Pro Ala Ala Arg Pro Glu Arg Ile Pro Leu Ser Ser
    1505                1510                1515

Ala Gln Ser Arg Leu Trp Phe Leu His Arg Leu Glu Gly Pro Ser
    1520                1525                1530

Ala Thr Tyr Asn Ile Pro Met Ala Leu Arg Leu Ser Gly Arg Leu
    1535                1540                1545

Asp Arg Glu Ala Leu Gln Thr Ala Leu Ala Asp Val Val Glu Arg
    1550                1555                1560

His Glu Ser Leu Arg Thr Val Phe Pro Glu Thr Asp Gly Val Pro
    1565                1570                1575

Cys Gln His Ile Leu Ala Gly Ala Glu Ala Gln Pro Val Leu Glu
    1580                1585                1590

Thr Arg Gln Thr Ser Glu Glu Glu Leu Ala Asp Thr Ile Leu Ile
    1595                1600                1605

Val Ser Arg His Ala Phe Asp Leu Ser Thr Glu Leu Pro Val Arg
    1610                1615                1620

Ala Trp Leu Phe Ala Leu Ala Pro Glu Glu His Val Leu Val Leu
    1625                1630                1635

Val Val His His Ile Ala Gly Asp Gly Trp Ser Leu Ser Pro Leu
    1640                1645                1650

Phe Arg Asp Leu Thr Val Ala Tyr Ala Ala Arg Ala Glu Gly Gly
    1655                1660                1665

Val Pro Gly Trp Ser Ala Leu Pro Val Gln Tyr Ala Asp Tyr Thr
    1670                1675                1680

Leu Trp Gln Asn Asp Leu Leu Gly Asp Gln Ser Asp Thr Gly Ser
    1685                1690                1695

Leu Val Ala Arg Gln Leu Glu Tyr Trp Arg Thr Thr Leu Thr Gly
    1700                1705                1710

Leu Pro Glu Gln Val Thr Leu Pro Thr Asp Arg Pro Arg Pro Ala
    1715                1720                1725

Met Ala Ser Gln Arg Gly Glu Val Phe Glu Phe Ser Trp Asp Ala
    1730                1735                1740

Glu Leu His Gln Gly Leu Ile Asp Leu Ala Arg Ser Thr Gly Thr
    1745                1750                1755

Thr Val Phe Met Val Leu Gln Ala Gly Leu Ala Ala Leu Met Ser
    1760                1765                1770

Arg Leu Gly Ala Gly Asp Asp Ile Pro Leu Gly Ser Pro Ile Ala
    1775                1780                1785

Gly Arg Thr Asp Glu Ala Leu Asp Asp Leu Val Gly Phe Phe Val
    1790                1795                1800

Asn Thr Leu Val Leu Arg Thr Asp Thr Ser Gly Asn Pro Thr Phe
    1805                1810                1815

Arg Glu Leu Leu Ala Arg Val Arg Glu Thr Asp Leu Ala Ala Tyr
    1820                1825                1830

Ala His Gln Asp Val Pro Phe Glu His Leu Val Glu Ile Leu Asn
    1835                1840                1845

Pro Glu Arg Ser Leu Ala His His Pro Leu Phe Gln Val Met Leu
    1850                1855                1860

Ala Leu Gln Asn Thr Pro Glu Arg Arg Phe Ala Leu Ser Gly Leu
    1865                1870                1875

Glu Ala Lys Glu Trp Pro Ile Ala Thr His Thr Ala Arg Phe Asp
    1880                1885                1890

Leu Phe Leu Gly Leu Thr Glu Arg Arg Ser Glu Ser Gly Leu Pro
    1895                1900                1905

Gly Gly Leu Glu Val Phe Val Glu Phe Ser Thr Asp Leu Phe Asp
    1910                1915                1920

Arg Val Ser Val Glu Val Val Leu Glu Arg Leu Arg Arg Val Leu
    1925                1930                1935

Val Ser Val Ala Ala Asp Pro Asp Val Thr Val Gly Ala Leu Pro
    1940                1945                1950

Val Leu Ser Gly Gln Glu Glu His Arg Leu Val Ala Glu Trp Asn
    1955                1960                1965

Asp Thr Ala Leu Glu Val Pro Pro Ala Ser Leu Pro Glu Leu Phe
    1970                1975                1980

Gln Ala Gln Ala Ala Glu Ala Pro Glu Ala Thr Ala Val Val Phe
    1985                1990                1995

Glu Thr Glu Arg Leu Ser Tyr Arg Glu Leu Asn Glu Arg Ala Asn
    2000                2005                2010

Arg Leu Ala His Tyr Leu Ile Gly Gln Gly Ala Gly Pro Glu Arg
    2015                2020                2025

Ile Val Ala Leu Ala Leu Pro Arg Gly Thr Asp Leu Val Ile Ala
    2030                2035                2040

Val Leu Ala Val Leu Lys Ala Gly Ala Ala Tyr Leu Pro Val Asp
    2045                2050                2055

Pro Asp Tyr Pro Ala Glu Arg Ile Thr His Met Leu Thr Asp Thr
    2060                2065                2070

Arg Pro Thr Leu Leu Leu Thr Thr Ser Asp Val Ala Ala Gly Leu
    2075                2080                2085

Pro Arg Thr Glu Gly Val Thr Ser Val Leu Leu Asp Asp Pro Ala
    2090                2095                2100

Val Thr Glu Thr Val Ala Leu Cys Ala Val Ser Asp Pro Thr Asp
    2105                2110                2115

Ser Glu Arg Gly Val Val Leu Glu Gly Ser His Pro Ala Tyr Val

2120                 2125                 2130

Ile Tyr  Thr Ser Gly Ser Thr  Gly Val Pro Lys Gly  Val Val Met
    2135                 2140                 2145

Pro Ser  Gly Gly Leu Val Asn  Leu Leu His Trp His  His Ser Val
    2150                 2155                 2160

Ile Ala  Gly Arg Ala Gly Ala  Arg Thr Ala Gln Phe  Thr Thr Ile
    2165                 2170                 2175

Ser Phe  Asp Val Ser Ala Gln  Glu Ile Leu Ser Ala  Leu Val Phe
    2180                 2185                 2190

Gly Lys  Glu Leu Trp Val Pro  Gly Glu Glu Val Arg  Arg Ser Gly
    2195                 2200                 2205

Glu Gly  Leu Ala Arg Trp Leu  Gln Glu His Ala Val  Glu Glu Leu
    2210                 2215                 2220

Phe Ala  Pro Ala Leu Val Ile  Asp Ala Val Ala Gln  Ala Ala Gly
    2225                 2230                 2235

Glu Leu  Gly Leu Val Leu Pro  Ala Leu Arg His Val  Ala Gln Gly
    2240                 2245                 2250

Gly Glu  Ala Leu Val Pro Gly  Ala Ala Met Arg Arg  Phe Phe Arg
    2255                 2260                 2265

Glu Arg  Pro His Ile Arg Leu  His Asn His Tyr Gly  Pro Thr Glu
    2270                 2275                 2280

Thr His  Ala Val Thr Ala Tyr  Val Leu Pro Glu Arg  Val Ala Asp
    2285                 2290                 2295

Trp Pro  Val Ser Ala Ala Pro  Val Gly Gly Pro Val  Ala Asn Asp
    2300                 2305                 2310

Arg Val  Tyr Val Leu Asp Ala  Ala Leu Arg Ala Val  Pro Val Gly
    2315                 2320                 2325

Val Ala  Gly Glu Leu Tyr Val  Ala Gly Ala Gly Leu  Ala Arg Gly
    2330                 2335                 2340

Tyr Val  Asn Arg Ala Gly Leu  Thr Ala Ser Arg Phe  Val Ala Asp
    2345                 2350                 2355

Pro Tyr  Gly Pro Ala Gly Ser  Arg Met Tyr Arg Thr  Gly Asp Val
    2360                 2365                 2370

Val Arg Trp Asn Thr Ser Gly Glu Leu Glu Phe Val Gly Arg Ala
    2375                2380                2385

Asp Asp Gln Val Lys Ile Arg Gly Phe Arg Ile Glu Leu Gly Glu
    2390                2395                2400

Ile Glu Thr Thr Ala Ala Gly His Pro Ala Val Ala Gln Ala Ala
    2405                2410                2415

Ala Thr Val His Glu Asp Asp Thr Arg Gly Lys Gln Leu Ala Leu
    2420                2425                2430

Tyr Ile Val Pro Thr Gly Thr Gly Gly Val Val Asp Ala Gly Thr
    2435                2440                2445

Val Arg Glu Tyr Leu Arg Thr Lys Leu Pro Asp Tyr Met Val Pro
    2450                2455                2460

Ala Ala Val Val Val Leu Glu Gln Leu Pro Leu Thr Ala Ser Gly
    2465                2470                2475

Lys Leu Asp Arg Arg Ala Leu Pro Thr Pro Glu Phe Ala Ala Glu
    2480                2485                2490

Pro Ala Gly Arg Thr Ala Arg Ser Pro Arg Glu Glu Ile Leu Ala
    2495                2500                2505

Gly Leu Phe Ala Glu Val Leu Gly Leu Pro Ala Val Gly Ile Asp
    2510                2515                2520

Asp Ser Phe Phe Asp Leu Gly Gly His Ser Leu Leu Ala Thr Arg
    2525                2530                2535

Leu Ile Ser Arg Ile Arg Ala Thr Leu Asn Thr Glu Val Pro Val
    2540                2545                2550

Arg Ala Leu Phe Glu Ala Pro Thr Val Ala Gly Leu Ala Thr Leu
    2555                2560                2565

Leu Asp Glu Asn Arg Ala Val Arg Pro Thr Leu Thr Pro Ala Ala
    2570                2575                2580

Arg Pro Glu Arg Ile Pro Leu Ser Ser Ala Gln Asn Arg Leu Trp
    2585                2590                2595

Phe Leu His Arg Leu Glu Gly Pro Ser Ala Thr Tyr Asn Met Pro
    2600                2605                2610

Met Ala Leu Arg Leu Ser Gly Arg Leu Asp Arg Glu Ala Leu His
    2615                2620                2625

Thr Ala Leu Ala Asp Val Ala Glu Arg His Glu Ser Leu Arg Thr
    2630                2635                2640

Val Phe Pro Glu Thr Asp Gly Val Pro Cys Gln His Ile Leu Ala
    2645                2650                2655

Gly Ala Glu Ala Gln Pro Val Leu Glu Val Ala Asp Ile Ala Glu
    2660                2665                2670

Asp Gln Leu Asp Glu Ala Leu Ala Gly Ala Ala Arg Tyr Pro Phe
    2675                2680                2685

Asp Leu Ser Ala Glu Leu Pro Val Arg Ala Trp Leu Phe Ala Leu
    2690                2695                2700

Ala Pro Glu Glu His Val Leu Val Leu Val Val His His Ile Ala
    2705                2710                2715

Gly Asp Gly Trp Ser Leu Ser Pro Leu Phe Arg Asp Leu Thr Val
    2720                2725                2730

Ala Tyr Ala Ala Arg Ala Asp Gly Arg Thr Pro Gly Trp Ser Ala
    2735                2740                2745

Leu Pro Val Gln Tyr Ala Asp Tyr Thr Leu Trp Gln Asn Asp Leu
    2750                2755                2760

Leu Gly Asp Gln Ser Asp Gly Asp Ser Leu Val Ala Arg Gln Leu
    2765                2770                2775

Glu Tyr Trp Arg Thr Thr Leu Thr Gly Leu Pro Glu Gln Val Thr
    2780                2785                2790

Leu Pro Thr Asp Arg Pro Arg Pro Ala Thr Ala Thr Tyr Gln Gly
    2795                2800                2805

Ala Leu His Asp Phe Ala Trp Asp Ala Glu Leu His Gln Gly Leu
    2810                2815                2820

Ile Asp Leu Ala Arg Ser Thr Gly Thr Thr Val Phe Met Val Leu
    2825                2830                2835

Gln Ala Gly Leu Ala Ala Leu Met Ser Arg Leu Gly Ala Gly Asp
    2840                2845                2850

Asp Ile Pro Leu Gly Ser Pro Ile Ala Gly Arg Thr Asp Glu Ala
    2855                2860                2865

Leu Asp Asp Leu Val Gly Phe Phe Val Asn Thr Leu Val Leu Arg
    2870                2875                2880

Thr Asp Thr Ser Gly Asn Pro Thr Phe Arg Glu Leu Leu Ala Arg
    2885                2890                2895

Val Arg Glu Thr Asp Leu Ala Ala Tyr Ala His Gln Asp Val Pro
    2900                2905                2910

Phe Glu His Leu Val Glu Ile Leu Asn Pro Glu Arg Ser Pro Ala
    2915                2920                2925

His His Pro Leu Phe Gln Val Ala Leu Ala Leu His Asn Thr Pro
    2930                2935                2940

Pro Arg Asn Phe Thr Leu Pro Asp Ile Asn Met Arg Ser Glu Arg
    2945                2950                2955

Ala Glu Thr Arg Thr Ser Arg Phe Asp Leu Ser Phe His Phe Phe
    2960                2965                2970

Glu Ser Asn Glu Leu Gly Asp Leu Pro Ala Gly Leu Gly Gly Phe
    2975                2980                2985

Val Glu Phe Ser Thr Asp Leu Phe Asp Arg Val Ser Val Glu Val
    2990                2995                3000

Val Leu Glu Arg Leu Arg Arg Val Leu Val Ser Val Ala Ala Asp
    3005                3010                3015

Pro Asp Val Thr Val Gly Ala Leu Pro Val Leu Ser Gly Gln Glu
    3020                3025                3030

Glu His Arg Leu Val Ala Glu Trp Asn Asp Thr Ala Leu Glu Val
    3035                3040                3045

Pro Pro Ala Ser Leu Pro Glu Leu Phe Gln Ala Gln Ala Ala Ala
    3050                3055                3060

Thr Pro Glu Ala Thr Ala Val Val Phe Glu Asp Val Arg Ile Ser
    3065                3070                3075

Tyr Arg Val Leu Asn Glu Arg Ala Asn Arg Leu Ala His Tyr Leu
    3080                3085                3090

Ile Lys Gln Gly Ala Gly Pro Glu Arg Ile Val Ala Leu Ala Leu

3095                3100                3105

Pro Arg Gly Thr Asp Leu Val Ile Ala Val Leu Ala Val Leu Lys
    3110                3115                3120

Ala Gly Ala Ala Tyr Leu Pro Val Asp Pro Asp Tyr Pro Ala Glu
    3125                3130                3135

Arg Ile Thr His Met Leu Thr Asp Thr Arg Pro Thr Leu Leu Leu
    3140                3145                3150

Thr Thr Ser Asp Ala Thr Ala Asp Leu Pro His Ile Glu Gly Ile
    3155                3160                3165

Pro Gln Val Leu Leu Asp Asp Pro Val Val Val Glu Ala Val Ala
    3170                3175                3180

Val Leu Ser Ala Asp Asp Pro Ser Asp Thr Asp Arg Gly Val Leu
    3185                3190                3195

Asp Ala Ser His Pro Ala Tyr Val Ile Tyr Thr Ser Gly Ser Thr
    3200                3205                3210

Gly Gln Pro Lys Gly Val Leu Ile Pro His Gln Asn Val Thr Arg
    3215                3220                3225

Leu Phe Ala Ala Thr Asp His Trp Phe Gly Phe Gly Ala Asp Asp
    3230                3235                3240

Val Trp Thr Leu Phe His Ser Tyr Ala Phe Asp Phe Ser Val Trp
    3245                3250                3255

Glu Leu Trp Gly Ala Leu Leu His Gly Gly Arg Leu Val Ile Val
    3260                3265                3270

Ser His Leu Thr Ser Arg Ser Pro Val Glu Leu Leu Arg Leu Leu
    3275                3280                3285

Val Ala Glu Gly Val Thr Val Leu Asn Gln Thr Pro Ser Ala Phe
    3290                3295                3300

Ser Gln Leu Gln Gln Ala Ala Gln Glu Asn Ser Ala Leu Asp Arg
    3305                3310                3315

Arg Leu Ser Leu Arg Arg Val Val Phe Gly Gly Glu Ala Leu Asp
    3320                3325                3330

Thr Ser Arg Leu Lys Pro Trp Phe Glu Ala His Leu Asp Thr Glu
    3335                3340                3345

Pro Val Leu Val Asn Met Tyr Gly Ile Thr Glu Thr Thr Val His
    3350                3355                3360

Val Thr Tyr Arg Glu Leu Asn Arg Gly Ser Ala Phe Ala Ser Asp
    3365                3370                3375

Gly Ser Ser Ala Ile Gly Thr Ala Ile Pro Asp Leu Arg Val Tyr
    3380                3385                3390

Val Leu Asp Ala Gly Leu Arg Val Val Pro Val Gly Val Ala Gly
    3395                3400                3405

Glu Leu Tyr Val Ala Gly Ala Gly Leu Ala Arg Gly Tyr Val Asn
    3410                3415                3420

Arg Ala Gly Leu Thr Ala Ser Arg Phe Val Ala Asp Pro Tyr Gly
    3425                3430                3435

Pro Ala Gly Ser Arg Met Tyr Arg Thr Gly Asp Val Val Arg Trp
    3440                3445                3450

Asn Thr Ser Gly Glu Leu Glu Phe Val Gly Arg Ala Asp Asp Gln
    3455                3460                3465

Val Lys Ile Arg Gly Phe Arg Ile Glu Leu Gly Glu Ile Glu Thr
    3470                3475                3480

Thr Ala Ala Gly His Pro Ala Val Ala Gln Ala Ala Ala Thr Val
    3485                3490                3495

His Glu Asp Asp Thr Arg Gly Lys Gln Leu Ala Leu Tyr Val Val
    3500                3505                3510

Pro Thr Gly Leu Thr Ser Gly Asp Val Ser Gly Ser Val Ser Gly
    3515                3520                3525

Asp Gly Ala Val Pro Asp Gly Gly Val Ser Gly Val Val Asp Glu
    3530                3535                3540

Gln Val Gly Glu Trp Arg Glu Ile Tyr Asp Ser Leu Tyr Gly Gly
    3545                3550                3555

Pro Gly Ser Ser Val Phe Gly Glu Asp Phe Ser Gly Trp Asp Ser
    3560                3565                3570

Ser Tyr Asp Gly Ala Ala Ile Pro Leu Glu Glu Met Arg Glu Trp
    3575                3580                3585

Arg Asp Ala Thr Val Glu Arg Ile Arg Gly Leu Gly Gly Arg Arg
    3590                3595                3600

Val Leu Glu Ile Gly Val Gly Thr Gly Leu Leu Met Ser Arg Leu
    3605                3610                3615

Ala Ala Gly Cys Glu Glu Tyr Trp Ala Thr Asp Leu Ser Gly Val
    3620                3625                3630

Val Ile Asp Ala Leu Asp Gly His Val Gln Ala Asp Pro Val Leu
    3635                3640                3645

Arg Glu Arg Val Arg Leu Ala Cys Gln Arg Ala Asp Asp Thr Arg
    3650                3655                3660

Gly Leu Pro Glu Gly Tyr Phe Asp Thr Val Val Ile Asn Ser Val
    3665                3670                3675

Val Gln Tyr Phe Pro Gly Ala Gln Tyr Leu Ala Ser Val Ile Glu
    3680                3685                3690

Ala Ala Val Ser Arg Leu Ala Pro Gly Gly Arg Val Phe Ile Gly
    3695                3700                3705

Asp Val Arg Asp Leu Arg Thr Leu Arg Ala Phe His Thr Ala Val
    3710                3715                3720

Gln Leu Thr Arg Thr Thr Gly Gly Arg Ala Gly Asp Gly Met Asp
    3725                3730                3735

Ala Gly Gly Leu Arg Arg Ala Val Glu Gln Gly Leu Leu Leu Glu
    3740                3745                3750

Asn Glu Leu Leu Leu Asp Pro Glu Phe Phe Thr Ala Val Gly Arg
    3755                3760                3765

Thr Leu Pro Ala Val Ser Ala Val Glu Val Arg Leu Lys His Gly
    3770                3775                3780

Gln Ala His Asn Glu Leu Thr Arg His Arg Tyr Asp Val Ile Leu
    3785                3790                3795

His Thr Thr Asn Thr Glu Thr Asp Thr Ala Thr Glu Glu Glu Ala
    3800                3805                3810

Glu Ala Ala Arg Pro Thr Lys Ala Glu Ala Glu Thr Pro Ala Leu
    3815                3820                3825

Val Glu Arg Ile Ser Trp Asn Thr Leu Ser Gly Gly Leu Asp Gly
    3830                3835                3840

Leu Asp Asp Leu Leu Arg Ser Arg Gly Ala Ala Pro Leu Arg Val
    3845                3850                3855

Thr Gly Ile Pro Asn Ala Arg Leu Ala Gly Glu Tyr Ala Ala Leu
    3860                3865                3870

Arg Val Leu Glu Asn Gly Gly Thr Leu Thr Glu Ala Val Thr Ala
    3875                3880                3885

Leu Ala Gly Pro Arg Gly Ile Asp Pro Glu His Leu His Gln Leu
    3890                3895                3900

Ala Ala Ala Thr Gly Tyr His Ala Val Leu Gln Pro Ala Pro Ala
    3905                3910                3915

Pro Asp Thr Tyr Asn Thr Leu Leu Leu Pro Leu Asp Ile Phe Asp
    3920                3925                3930

Gly Thr Ala Trp Ser Ala Thr Ala Thr Ala Thr Ala Thr Asp Leu
    3935                3940                3945

Arg Glu Thr Ser Ala Pro Asp His Thr Ala Glu Thr Ser Phe Gln
    3950                3955                3960

Ala Leu Ala Asn Asn Pro Ala Ala Ser Arg Asp Thr Ser Thr Leu
    3965                3970                3975

Ile Thr Gln Val Arg Asp His Leu Arg Thr Lys Leu Pro Asp His
    3980                3985                3990

Met Val Pro Ala Ala Ile Val Val Leu Glu Arg Leu Pro Leu Thr
    3995                4000                4005

Ala Ser Gly Lys Leu Asp Arg Arg Ala Leu Pro Ala Pro Asp Leu
    4010                4015                4020

Gly Thr His Thr Thr Gly Arg Ala Pro Arg Ser Pro Arg Glu Glu
    4025                4030                4035

Ile Leu Ala Gly Leu Phe Ala Glu Val Leu Gly Leu Pro Ala Val
    4040                4045                4050

Gly Ile Asp Asp Ser Phe Phe Asp Leu Gly Gly His Ser Leu Leu
    4055                4060                4065

Ala Thr Arg Leu Ile Ser Arg Ile Arg Ala Thr Leu Asn Thr Glu

4070                4075                4080

Val Pro Val Arg Asp Leu Phe Glu Ala Pro Thr Val Ala Gly Leu
    4085                4090                4095

Ala Thr Leu Leu Asp Glu Asn Arg Ala Val Arg Pro Thr Leu Thr
    4100                4105                4110

Pro Ala Ala Arg Pro Glu Arg Ile Pro Leu Ser Ser Ala Gln Ser
    4115                4120                4125

Arg Leu Trp Phe Leu His Arg Leu Glu Gly Pro Ser Ala Thr Tyr
    4130                4135                4140

Asn Met Pro Met Ala Leu Arg Leu Ser Gly Arg Leu Asp Arg Glu
    4145                4150                4155

Ala Leu His Thr Ala Leu Ala Asp Val Val Glu Arg His Glu Ser
    4160                4165                4170

Leu Arg Thr Val Phe Pro Glu Thr Asp Gly Val Pro Cys Gln Gln
    4175                4180                4185

Ile Leu Ala Gly Ala Glu Ala Gln Pro Val Leu Glu Val Ala Asp
    4190                4195                4200

Ile Ala Glu Asp Gln Leu Asp Gln Ala Leu Ala Gly Ala Ala Arg
    4205                4210                4215

Tyr Pro Phe Asp Leu Ser Val Glu Leu Pro Val Arg Ala Trp Leu
    4220                4225                4230

Phe Ala Leu Ala Arg Glu Glu His Val Leu Val Leu Val Val His
    4235                4240                4245

His Ile Ala Ser Asp Gly Trp Ser Leu Ala Pro Leu Ser Arg Asp
    4250                4255                4260

Leu Val Thr Ala Tyr Ala Ala Arg Ala Asp Gly Arg Val Pro Gly
    4265                4270                4275

Trp Ala Pro Leu Pro Val Gln Tyr Ala Asp Tyr Thr Leu Trp Gln
    4280                4285                4290

Asn Asp Leu Leu Gly Asp His Ser Asp Thr Gly Ser Leu Ile Ala
    4295                4300                4305

Arg Gln Leu Glu Tyr Trp Arg Thr Thr Leu Thr Gly Leu Pro Glu
    4310                4315                4320

Gln Val Thr Leu Pro Thr Asp Arg Pro Arg Pro Ala Met Ala Ser
    4325                4330                4335

Gln Arg Gly Glu Val Phe Glu Phe Ser Trp Asp Ala Glu Leu His
    4340                4345                4350

Gln Gly Leu Val Asp Leu Ala Arg Ser Thr Gly Thr Thr Val Phe
    4355                4360                4365

Met Val Leu Gln Ala Gly Leu Ala Ala Leu Met Ser Arg Leu Gly
    4370                4375                4380

Ala Gly Asp Asp Ile Pro Leu Gly Ser Pro Ile Ala Gly Arg Thr
    4385                4390                4395

Asp Glu Ala Leu Asp Asp Leu Val Gly Phe Phe Val Asn Thr Leu
    4400                4405                4410

Val Leu Arg Thr Asp Thr Ser Gly Asn Pro Thr Phe Arg Glu Leu
    4415                4420                4425

Leu Ala Arg Val Arg Glu Thr Asp Leu Ala Ala Tyr Ala His Gln
    4430                4435                4440

Asp Val Pro Phe Glu His Leu Val Glu Ile Leu Asn Pro Glu Arg
    4445                4450                4455

Ser Leu Ala His His Pro Leu Phe Gln Val Met Leu Ala Leu Gln
    4460                4465                4470

Asn Thr Pro Glu Arg Ser Phe Ala Leu Pro Gly Thr Val Asn Val
    4475                4480                4485

Gly Ala Val Pro Val Ala Pro Thr Gly Thr Ser Arg Phe Asp Val
    4490                4495                4500

Ser Leu His Phe Val Glu Trp Gln Arg Glu Asp Gly Ala Ala Ser
    4505                4510                4515

Gly Leu Gly Gly Phe Val Glu Phe Ser Thr Asp Leu Phe Asp Arg
    4520                4525                4530

Val Ser Val Glu Val Val Leu Glu Arg Leu Arg Arg Val Leu Val
    4535                4540                4545

Ser Val Ala Ala Asp Pro Asp Val Thr Val Gly Ala Leu Pro Val
    4550                4555                4560

Leu Ser Gly Gln Glu Glu His Arg Leu Val Ala Glu Trp Asn Asp
    4565                4570                4575

Thr Val Leu Glu Val Pro Pro Ala Ser Leu Pro Glu Leu Phe Gln
    4580                4585                4590

Ala Gln Ala Ala Glu Ala Pro Glu Ala Thr Ala Val Val Phe Glu
    4595                4600                4605

Thr Glu Arg Leu Ser Tyr Arg Glu Leu Asn Glu Arg Ala Asn Arg
    4610                4615                4620

Leu Ala His Tyr Leu Ile Lys Gln Gly Ala Gly Pro Glu Arg Ile
    4625                4630                4635

Val Ala Leu Ala Leu Pro Arg Gly Thr Asp Leu Val Ile Ala Val
    4640                4645                4650

Leu Ala Val Leu Lys Ala Gly Ala Ala Tyr Leu Pro Val Asp Pro
    4655                4660                4665

Asp Tyr Pro Ala Glu Arg Ile Thr His Met Leu His Asp Ala Ala
    4670                4675                4680

Pro Ala Leu Met Val Thr Thr Ser Gly Val Ala Ala Gly Leu Pro
    4685                4690                4695

His Thr Glu Gly Val Thr Ser Val Leu Val Asp Ala Pro Ala Val
    4700                4705                4710

Val Glu Ala Val Ala Val Leu Ser Ala Asp Asp Pro Ser Asp Thr
    4715                4720                4725

Asp Arg Gly Val Val Leu Glu Gly Ser His Pro Ala Tyr Val Ile
    4730                4735                4740

Tyr Thr Ser Gly Ser Thr Gly Val Pro Lys Gly Val Leu Val Pro
    4745                4750                4755

His Ala Gly Leu Ala Asn Leu Thr Ala Ala Glu Arg Ala Ala Leu
    4760                4765                4770

Asp Leu Ser Ala Gly Ser Arg Val Leu Gln Leu Ala Ser Val Gly
    4775                4780                4785

Phe Asp Ala Ala Val Leu Glu Leu Ser Met Ala Phe Gly Ser Gly
    4790                4795                4800

Ala Thr Leu Val Ile Ala Pro Gln Gly Arg Leu Leu Gly Asp Asp
    4805                4810                4815

Leu Ala Ala Leu Leu Ala Arg Gln Glu Ile Thr His Thr Leu Ile
    4820                4825                4830

Thr Pro Ser Ala Leu Ala Thr Leu Pro Glu Thr Asp Leu Pro His
    4835                4840                4845

Leu Arg Thr Leu Leu Thr Gly Ala Glu Ala Cys Pro Pro Glu Leu
    4850                4855                4860

Val Ala Arg Trp Ser Pro Gly Arg Arg Phe Ile Asn Ala Tyr Gly
    4865                4870                4875

Pro Thr Glu Ala Ser Val Val Ala Thr Trp Ser Asp Pro Leu Thr
    4880                4885                4890

Gln Asp Thr Ala Pro Ile Gly Arg Pro Leu Pro Asn Thr Arg Val
    4895                4900                4905

Tyr Val Leu Asp Ala Gly Leu Arg Val Val Pro Val Gly Val Ala
    4910                4915                4920

Gly Glu Leu Tyr Val Ala Gly Ala Gly Leu Ala Arg Gly Tyr Val
    4925                4930                4935

Asn Arg Ala Gly Leu Thr Ala Ser Arg Phe Val Ala Asp Pro Tyr
    4940                4945                4950

Gly Pro Ala Gly Ser Arg Met Tyr Arg Thr Gly Asp Val Val Arg
    4955                4960                4965

Trp Asn Thr Ser Gly Glu Leu Glu Phe Val Gly Arg Ala Asp Asp
    4970                4975                4980

Gln Val Lys Ile Arg Gly Phe Arg Ile Glu Leu Gly Glu Ile Glu
    4985                4990                4995

Thr Thr Ala Ala Gly His Pro Ala Val Ala Gln Ala Ala Ala Thr
    5000                5005                5010

Val His Glu Asp Asp Thr Arg Gly Lys Gln Leu Ala Leu Tyr Ile
    5015                5020                5025

Val Pro Thr Gly Ile Thr Ser Gly Asp Val Pro Ala Ser Gly Gly
    5030                5035                5040

Val Val Asp Ala Gly Thr Val Arg Glu Tyr Leu Arg Thr Arg Leu

5045                5050                5055

Pro Asp Tyr Met Val Pro Ala Ala Val Val Val Leu Glu Arg Leu
    5060                5065                5070

Pro Leu Thr Ala Ser Gly Lys Leu Asp Arg Arg Ala Leu Pro Thr
    5075                5080                5085

Pro Glu Phe Ala Ala Glu Pro Ala Gly Arg Thr Ala Arg Ser Pro
    5090                5095                5100

Arg Glu Glu Ile Leu Ala Gly Leu Phe Ala Glu Val Leu Gly Leu
    5105                5110                5115

Pro Ala Val Gly Ile Asp Asp Ser Phe Phe Asp Leu Gly Gly His
    5120                5125                5130

Ser Leu Leu Ala Thr Arg Leu Ile Ser Arg Ile Arg Ala Ile Leu
    5135                5140                5145

Gly Ala Glu Ile Pro Ile Arg Ala Leu Phe Glu Thr Pro Thr Val
    5150                5155                5160

Ala Gly Leu Ala Ala Leu Leu Glu Thr Ser Ser Glu Ser Val Arg
    5165                5170                5175

Ser Ala Leu Val Pro Val Thr Arg Pro Glu Arg Met Pro Leu Ser
    5180                5185                5190

Ser Ala Gln Ser Arg Leu Trp Phe Leu His Arg Leu Glu Gly Pro
    5195                5200                5205

Ser Ala Thr Tyr Asn Val Pro Met Ala Leu Arg Leu Thr Gly Pro
    5210                5215                5220

Val Met Pro Glu Val Leu Arg Leu Ala Leu Ala Asp Val Val Glu
    5225                5230                5235

Arg His Glu Ser Leu Arg Thr Val Phe Pro Glu Thr Asp Gly Ile
    5240                5245                5250

Pro His Gln Arg Ile Leu Thr Gly Ala Glu Ala Arg Pro Val Leu
    5255                5260                5265

Glu Val Ala Glu Val Gly Glu Asp Gly Leu Glu Glu Ala Leu Ala
    5270                5275                5280

Gly Ala Ala Arg Tyr Pro Phe Asp Leu Ser Ala Glu Leu Pro Val
    5285                5290                5295

Arg Ala Trp Leu Phe Ala Leu Ala Pro Glu Glu His Val Leu Val
    5300                5305                5310

Leu Val Val His His Ile Ala Gly Asp Gly Trp Ser Leu Ser Pro
    5315                5320                5325

Leu Phe Arg Asp Leu Thr Val Ala Tyr Ala Ala Arg Ala Asp Gly
    5330                5335                5340

Arg Thr Pro Gly Trp Ser Ala Leu Pro Val Gln Tyr Ala Asp Tyr
    5345                5350                5355

Thr Leu Trp Gln Asn Asp Leu Leu Gly Asp His Ser Asp Thr Gly
    5360                5365                5370

Ser Leu Ile Ala Arg Gln Leu Glu Tyr Trp Arg Thr Thr Leu Thr
    5375                5380                5385

Gly Leu Pro Glu Gln Val Thr Leu Pro Thr Asp Arg Pro Arg Pro
    5390                5395                5400

Ala Thr Ala Thr Tyr Gln Gly Asp Ser Leu Tyr Phe Lys Trp Asp
    5405                5410                5415

Ala Glu Leu His Gln Gly Leu Ile Asp Leu Ala Arg Ser Thr Gly
    5420                5425                5430

Thr Thr Val Phe Met Val Leu Gln Ala Gly Leu Ala Ala Leu Met
    5435                5440                5445

Ser Arg Leu Gly Ala Gly Asp Asp Ile Pro Leu Gly Ser Pro Ile
    5450                5455                5460

Ala Gly Arg Thr Asp Glu Ala Leu Asp Asp Leu Val Gly Phe Phe
    5465                5470                5475

Val Asn Thr Leu Val Leu Arg Thr Asp Thr Ser Gly Asn Pro Thr
    5480                5485                5490

Phe Arg Glu Leu Leu Ala Arg Val Arg Glu Thr Asp Leu Ala Ala
    5495                5500                5505

Tyr Ala His Gln Asp Val Pro Phe Glu His Leu Val Glu Ile Leu
    5510                5515                5520

Asn Pro Glu Arg Ser Leu Ala His His Pro Leu Phe Gln Val Ser
    5525                5530                5535

Val Thr Leu Asp Asn Thr Pro Gly His Arg Leu Glu Leu Asp Gly
    5540                5545                5550

Leu Glu Ile Ser Val Glu Pro Val Gly Thr Arg Thr Ser Arg Phe
    5555                5560                5565

Asp Leu Ala Leu Asn Phe Tyr Gly Leu Val Gly Asp Ser Gly Gly
    5570                5575                5580

Pro Gly Gly Leu His Gly Ser Val Glu Phe Ser Thr Asp Leu Phe
    5585                5590                5595

Asp Arg Val Ser Val Glu Val Val Leu Glu Arg Leu Arg Arg Val
    5600                5605                5610

Leu Val Ser Val Ala Ala Asp Pro Asp Val Thr Val Gly Ala Leu
    5615                5620                5625

Pro Val Leu Ser Gly Gln Glu Glu His Arg Leu Val Ala Glu Trp
    5630                5635                5640

Asn Asp Thr Ala Leu Glu Val Pro Pro Ala Ser Leu Pro Glu Leu
    5645                5650                5655

Phe Gln Ala Gln Ala Ala Ala Thr Pro Glu Ala Thr Ala Val Val
    5660                5665                5670

Phe Glu Thr Glu Arg Leu Ser Tyr Arg Glu Leu Asn Glu Arg Ala
    5675                5680                5685

Asn Arg Leu Ala His Tyr Leu Ile Gly Gln Gly Ala Gly Pro Glu
    5690                5695                5700

Arg Ile Val Ala Leu Ala Leu Pro Arg Gly Thr Asp Leu Val Ile
    5705                5710                5715

Ala Val Leu Ala Val Leu Lys Ala Gly Ala Ala Tyr Leu Pro Val
    5720                5725                5730

Asp Pro Asp Tyr Pro Ala Glu Arg Ile Thr His Met Leu Thr Asp
    5735                5740                5745

Thr Arg Pro Thr Leu Leu Leu Thr Thr Ser Asp Val Ala Ala Gly
    5750                5755                5760

Leu Pro Arg Thr Glu Gly Val Thr Ser Val Leu Leu Asp Asp Pro
    5765                5770                5775

Ala Val Thr Glu Thr Val Ala Leu Cys Ala Val Ser Asp Pro Thr
    5780                5785                5790

Asp Ser Glu Arg Gly Val Val Leu Glu Gly Ser His Pro Ala Tyr
    5795                5800                5805

Val Ile Tyr Thr Ser Gly Ser Thr Gly Val Pro Lys Gly Val Val
    5810                5815                5820

Met Pro Ser Gly Gly Leu Val Asn Leu Leu His Trp His Pro Ser
    5825                5830                5835

Val Ile Ala Gly Arg Ala Gly Ala Arg Thr Ala Gln Phe Thr Thr
    5840                5845                5850

Ile Ser Phe Asp Val Ser Ala Gln Glu Ile Leu Ser Ala Leu Val
    5855                5860                5865

Phe Gly Lys Glu Leu Trp Val Pro Gly Glu Glu Val Arg Arg Ser
    5870                5875                5880

Gly Glu Gly Leu Ala Arg Trp Leu Gln Glu His Ala Val Glu Glu
    5885                5890                5895

Leu Phe Ala Pro Ala Leu Val Ile Asp Ala Val Ala Gln Ala Ala
    5900                5905                5910

Gly Glu Leu Gly Leu Val Leu Pro Ala Leu Arg His Val Ala Gln
    5915                5920                5925

Gly Gly Glu Ala Leu Val Pro Gly Ala Ala Met Arg Arg Phe Phe
    5930                5935                5940

Arg Glu Arg Pro His Ile Arg Leu His Asn His Tyr Gly Pro Thr
    5945                5950                5955

Glu Thr His Ala Val Thr Ala Tyr Val Leu Pro Glu Arg Val Ala
    5960                5965                5970

Asp Trp Pro Val Ser Ala Ala Pro Val Gly Gly Pro Val Ala Asn
    5975                5980                5985

Asp Arg Val Tyr Val Leu Asp Ala Ala Leu Arg Ala Val Pro Val
    5990                5995                6000

Gly Val Ala Gly Glu Leu Tyr Val Ala Gly Ala Gly Leu Ala Arg
    6005                6010                6015

Gly Tyr Val Asn Arg Ala Gly Leu Thr Ala Ser Arg Phe Val Ala

6020                6025                6030

Asp Pro Tyr Gly Pro Ala Gly Ser Arg Met Tyr Arg Thr Gly Asp
    6035                6040                6045

Val Val Arg Trp Asn Thr Ser Gly Glu Leu Glu Phe Val Gly Arg
    6050                6055                6060

Ala Asp Asp Gln Val Lys Ile Arg Gly Phe Arg Ile Glu Leu Gly
    6065                6070                6075

Glu Ile Glu Thr Thr Ala Ala Gly His Pro Ala Val Ala Gln Ala
    6080                6085                6090

Ala Ala Thr Val His Glu Asp Asp Thr Arg Gly Lys Gln Leu Ala
    6095                6100                6105

Leu Tyr Ile Val Pro Thr Gly Thr Gly Gly Val Val Asp Ala Gly
    6110                6115                6120

Thr Val Arg Glu Tyr Leu Arg Thr Lys Leu Pro Asp Tyr Met Val
    6125                6130                6135

Pro Ala Ala Val Val Val Leu Glu Gln Leu Pro Leu Thr Ala Ser
    6140                6145                6150

Gly Lys Leu Asp Arg Arg Ala Leu Pro Thr Pro Glu Phe Ala Ala
    6155                6160                6165

Glu Pro Ala Gly Arg Thr Ala Arg Ser Pro Arg Glu Glu Ile Leu
    6170                6175                6180

Ala Gly Leu Phe Ala Glu Val Leu Gly Leu Pro Ala Val Gly Ile
    6185                6190                6195

Asp Asp Ser Phe Phe Asp Leu Gly Gly His Ser Leu Leu Ala Thr
    6200                6205                6210

Arg Leu Ile Ser Arg Ile Arg Ala Ile Leu Gly Ala Glu Ile Pro
    6215                6220                6225

Ile Arg Ala Leu Phe Glu Thr Pro Thr Val Ala Gly Leu Ala Ala
    6230                6235                6240

Leu Leu Glu Thr Ser Ser Glu Ser Val Arg Ser Ala Leu Val Pro
    6245                6250                6255

Val Thr Arg Pro Glu Arg Met Pro Leu Ser Ser Ala Gln Ser Arg
    6260                6265                6270

Leu Trp Phe Leu His Arg Leu Glu Gly Pro Ser Ala Thr Tyr Asn
    6275                6280                6285

Met Pro Met Ala Leu Arg Leu Ser Gly Arg Leu Asp Arg Glu Ala
    6290                6295                6300

Leu His Thr Ala Leu Ala Asp Val Val Glu Arg His Glu Ser Leu
    6305                6310                6315

Arg Thr Val Phe Pro Glu Thr Asp Gly Val Pro Cys Gln Gln Ile
    6320                6325                6330

Leu Ala Gly Ala Glu Ala Gln Pro Val Leu Glu Val Ala Asp Ile
    6335                6340                6345

Ala Glu Asp Gln Leu Asp Gln Ala Leu Ala Gly Ala Ala Arg Tyr
    6350                6355                6360

Pro Phe Asp Leu Ser Val Glu Leu Pro Val Arg Ala Trp Leu Phe
    6365                6370                6375

Ala Leu Ala Arg Glu Glu His Val Leu Val Leu Val Val His His
    6380                6385                6390

Ile Ala Ser Asp Gly Trp Ser Leu Ala Pro Leu Ser Arg Asp Leu
    6395                6400                6405

Val Thr Ala Tyr Ala Ala Arg Ala Asp Gly Arg Val Pro Gly Trp
    6410                6415                6420

Ala Pro Leu Pro Val Gln Tyr Ala Asp Tyr Thr Leu Trp Gln Asn
    6425                6430                6435

Asp Leu Leu Gly Asp His Ser Asp Thr Gly Ser Leu Ile Ala Arg
    6440                6445                6450

Gln Leu Glu Tyr Trp Arg Thr Thr Leu Thr Gly Leu Pro Glu Gln
    6455                6460                6465

Val Thr Leu Pro Thr Asp Arg Pro Arg Pro Ala Met Ala Ser Gln
    6470                6475                6480

Arg Gly Glu Val Phe Glu Phe Ser Trp Asp Ala Glu Leu His Gln
    6485                6490                6495

Gly Leu Val Asp Leu Ala Arg Ser Thr Gly Thr Thr Val Phe Met
    6500                6505                6510

Val Leu Gln Ala Gly Leu Ala Ala Leu Met Ser Arg Leu Gly Ala
    6515                6520                6525

Gly Asp Asp Ile Pro Leu Gly Ser Pro Ile Ala Gly Arg Thr Asp
    6530                6535                6540

Glu Ala Leu Asp Asp Leu Val Gly Phe Phe Val Asn Thr Leu Val
    6545                6550                6555

Leu Arg Thr Asp Thr Ser Gly Asn Pro Thr Phe Arg Glu Leu Leu
    6560                6565                6570

Ala Arg Val Arg Glu Thr Asp Leu Ala Ala Tyr Ala His Gln Asp
    6575                6580                6585

Val Pro Phe Glu His Leu Val Glu Ile Leu Asn Pro Glu Arg Ser
    6590                6595                6600

Leu Ala His His Pro Leu Phe Gln Val Ser Val Thr Leu Asp Asn
    6605                6610                6615

Thr Pro Gly His Arg Leu Glu Leu Asp Gly Leu Glu Ile Ser Val
    6620                6625                6630

Glu Pro Val Gly Thr Arg Thr Ser Arg Phe Asp Leu Ala Leu Asn
    6635                6640                6645

Phe Tyr Gly Leu Val Gly Asp Ser Gly Gly Pro Gly Gly Leu His
    6650                6655                6660

Gly Ser Val Glu Phe Ser Thr Asp Leu Phe Asp Arg Val Ser Val
    6665                6670                6675

Glu Val Val Leu Glu Arg Leu Arg Arg Val Leu Val Ser Val Ala
    6680                6685                6690

Ala Asp Pro Asp Val Thr Val Gly Ala Leu Pro Val Leu Ser Gly
    6695                6700                6705

Gln Glu Glu His Arg Leu Val Ala Glu Trp Asn Asp Thr Ala Leu
    6710                6715                6720

Glu Val Pro Pro Ala Ser Leu Pro Glu Leu Phe Gln Ala Gln Ala
    6725                6730                6735

Ala Glu Ala Pro Glu Ala Thr Ala Val Val Phe Glu Thr Glu Arg
    6740                6745                6750

Leu Ser Tyr Arg Glu Leu Asn Glu Arg Ala Asn Arg Leu Ala His
    6755                6760                6765

Tyr Leu Ile Gly Gln Gly Ala Gly Pro Glu Arg Ile Val Ala Leu
    6770                6775                6780

Ala Leu Pro Arg Gly Thr Asp Leu Val Ile Ala Val Leu Ala Val
    6785                6790                6795

Leu Lys Ala Gly Ala Ala Tyr Leu Pro Val Asp Pro Asp Tyr Pro
    6800                6805                6810

Ala Glu Arg Ile Thr His Met Leu Thr Asp Thr Arg Pro Thr Leu
    6815                6820                6825

Leu Leu Thr Thr Ser Asp Val Ala Ala Gly Leu Pro Arg Thr Glu
    6830                6835                6840

Gly Val Thr Ser Val Leu Leu Asp Asp Pro Ala Val Thr Glu Thr
    6845                6850                6855

Val Ala Leu Cys Ala Val Ser Asp Pro Thr Asp Ser Glu Arg Gly
    6860                6865                6870

Val Val Leu Glu Gly Ser His Pro Ala Tyr Val Ile Tyr Thr Ser
    6875                6880                6885

Gly Ser Thr Gly Val Pro Lys Gly Val Leu Val Pro His Ala Gly
    6890                6895                6900

Leu Ala Asn Leu Thr Ala Ala Glu Arg Ala Ala Leu Asp Leu Ser
    6905                6910                6915

Ala Gly Ser Arg Val Leu Gln Leu Ala Ser Val Gly Phe Asp Ala
    6920                6925                6930

Ala Val Leu Glu Leu Ser Met Ala Phe Gly Ser Gly Ala Thr Leu
    6935                6940                6945

Val Ile Ala Pro Gln Gly Arg Leu Leu Gly Asp Asp Leu Ala Ala
    6950                6955                6960

Leu Leu Ala Arg Gln Glu Ile Thr His Thr Leu Ile Thr Pro Ser
    6965                6970                6975

Ala Leu Ala Thr Leu Pro Glu Thr Asp Leu Pro His Leu Arg Thr
    6980                6985                6990

Leu Leu Thr Gly Ala Glu Ala Cys Pro Pro Glu Leu Val Ala Arg

6995                7000                7005

Trp Ser Pro Gly Arg Arg Phe Ile Asn Ala Tyr Gly Pro Thr Glu
    7010                7015                7020

Ala Ser Val Val Ala Thr Trp Ser Asp Pro Leu Thr Gln Asp Thr
    7025                7030                7035

Ala Pro Ile Gly Arg Pro Leu Pro Asn Thr Arg Val Tyr Val Leu
    7040                7045                7050

Asp Ala Gly Leu Arg Val Val Pro Val Gly Val Ala Gly Glu Leu
    7055                7060                7065

Tyr Val Ala Gly Ala Gly Leu Ala Arg Gly Tyr Val Asn Arg Ala
    7070                7075                7080

Gly Leu Thr Ala Ser Arg Phe Val Ala Asp Pro Tyr Gly Pro Ala
    7085                7090                7095

Gly Ser Arg Met Tyr Arg Thr Gly Asp Val Val Arg Trp Asn Thr
    7100                7105                7110

Ser Gly Glu Leu Glu Phe Val Gly Arg Ala Asp Asp Gln Val Lys
    7115                7120                7125

Ile Arg Gly Phe Arg Ile Glu Leu Gly Glu Ile Glu Thr Thr Ala
    7130                7135                7140

Ala Gly His Pro Ala Val Ala Gln Ala Ala Ala Thr Val His Glu
    7145                7150                7155

Asp Asp Thr Arg Gly Lys Gln Leu Ala Leu Tyr Ile Val Pro Thr
    7160                7165                7170

Gly Ile Thr Ser Gly Asp Val Pro Ala Ser Gly Gly Val Val Asp
    7175                7180                7185

Ala Gly Thr Val Arg Glu Tyr Leu Arg Thr Arg Leu Pro Asp Tyr
    7190                7195                7200

Met Val Pro Ala Ala Val Val Val Leu Glu Arg Leu Pro Leu Thr
    7205                7210                7215

Ala Ser Gly Lys Leu Asp Arg Arg Ala Leu Pro Thr Pro Glu Phe
    7220                7225                7230

Ala Ala Glu Pro Ala Gly Arg Thr Ala Arg Ser Pro Arg Glu Glu
    7235                7240                7245

Ile Leu Ala Gly Leu Phe Ala Glu Val Leu Gly Leu Pro Ala Val
    7250                7255                7260

Gly Ile Asp Asp Ser Phe Phe Asp Leu Gly Gly His Ser Leu Leu
    7265                7270                7275

Ala Thr Arg Leu Ile Ser Arg Ile Arg Ala Thr Leu Asn Thr Glu
    7280                7285                7290

Val Pro Val Arg Ala Leu Phe Glu Ala Pro Thr Val Ala Ala Leu
    7295                7300                7305

Thr Val Leu Leu Glu Thr Ser Asn Gly Glu Thr Val Arg Ser Thr
    7310                7315                7320

Leu Val Pro Ala Val Arg Pro Glu Arg Ile Pro Leu Ser Ser Ala
    7325                7330                7335

Gln Ser Arg Leu Trp Phe Leu His Arg Leu Glu Gly Pro Ser Ala
    7340                7345                7350

Thr Tyr Asn Ile Pro Met Ala Leu Arg Leu Ser Gly Arg Leu Asp
    7355                7360                7365

Arg Glu Ala Leu His Thr Ala Leu Thr Asp Val Val Glu Arg His
    7370                7375                7380

Glu Ser Leu Arg Thr Val Phe Pro Glu Thr Asp Ser Val Pro Tyr
    7385                7390                7395

Gln His Val Leu Ala Glu Val Glu Ala Arg Pro Val Leu His Val
    7400                7405                7410

Val Gln Thr Ser Glu Glu Gly Leu Ala Glu Ala Val Ser Ala Ala
    7415                7420                7425

Ser Gln Tyr Ala Phe Asp Leu Ser Ala Glu Leu Pro Val Arg Ala
    7430                7435                7440

Trp Leu Phe Ala Leu Ala Pro Glu Glu His Val Leu Val Leu Val
    7445                7450                7455

Val His His Ile Ala Gly Asp Gly Trp Ser Leu Ser Pro Leu Phe
    7460                7465                7470

Arg Asp Leu Thr Val Ala Tyr Ala Ala Arg Ala Asp Gly Arg Thr
    7475                7480                7485

Pro Gly Trp Ser Ala Leu Pro Val Gln Tyr Ala Asp Tyr Thr Leu
    7490                7495                7500

Trp Gln Asn Asp Leu Leu Gly Asp His Ser Asp Thr Gly Ser Leu
    7505                7510                7515

Ile Ala Arg Gln Leu Glu Tyr Trp Arg Thr Thr Leu Thr Gly Leu
    7520                7525                7530

Pro Glu Gln Val Thr Leu Pro Thr Asp Arg Pro Arg Pro Ala Thr
    7535                7540                7545

Ala Thr Tyr Gln Gly Asp Ser Leu Tyr Phe Lys Trp Asp Ala Glu
    7550                7555                7560

Leu His Gln Gly Leu Ile Asp Leu Ala Arg Ser Thr Gly Thr Thr
    7565                7570                7575

Val Phe Met Val Leu Gln Ala Gly Leu Ala Ala Leu Met Ser Arg
    7580                7585                7590

Leu Gly Ala Gly Asp Asp Ile Pro Leu Gly Ser Pro Ile Ala Gly
    7595                7600                7605

Arg Thr Asp Glu Ala Leu Asp Asp Leu Val Gly Phe Phe Val Asn
    7610                7615                7620

Thr Leu Val Leu Arg Thr Asp Thr Ser Gly Asn Pro Thr Phe Arg
    7625                7630                7635

Glu Leu Leu Ala Arg Val Arg Glu Thr Asp Leu Ala Ala Tyr Ala
    7640                7645                7650

His Gln Asp Val Pro Phe Glu His Leu Val Glu Ile Leu Asn Pro
    7655                7660                7665

Glu Arg Ser Leu Ala His His Pro Leu Phe Gln Val Met Leu Ala
    7670                7675                7680

Leu Gln Asn Thr Pro Glu Gly Gln Phe Lys Leu Pro Gly Leu Gln
    7685                7690                7695

Ala Arg Phe Glu Thr Thr His Thr Gln Thr Ala Lys Phe Asp Leu
    7700                7705                7710

Phe Phe Asn Phe Ser Glu Tyr Arg Ala Glu Asn Asn Ser Ala Arg
    7715                7720                7725

Gly Ile Asn Gly Val Ile Glu Tyr Asp Ser Ser Leu Phe Ser Val
    7730                7735                7740

Ala Thr Val Glu Lys Leu Thr Glu Arg Leu Leu Arg Val Leu Arg
    7745                7750                7755

Glu Val Ile Ala Asn Pro Asp Lys Arg Leu Arg Arg Ile Glu Val
    7760                7765                7770

Leu Thr Gln Gln Glu Arg Met Lys Leu Leu Asp Asn Leu Asn Lys
    7775                7780                7785

Trp Ser Gly Gly Asp Ser
    7790

<210> 10
<211> 110
<212> PRT
<213> Streptomyces 22643

<400> 10

Met Trp Cys Ala Arg Leu Ala Trp Ser Arg Phe Arg Val Val Ile Pro
1               5                   10                  15

Val Phe Glu Lys Ala Leu Pro Thr Ile Val Ala Cys Leu Asp Ala Thr
            20                  25                  30

Leu Arg Arg Ile Gly Gly Val Pro Ser Tyr Ala Leu Thr Asp Asn Glu
        35                  40                  45

Lys Ile Val Thr Thr Glu His Ile Ala Asn Ile Ala Val Arg Asn Pro
    50                  55                  60

Glu Ile Val Glu Val Gly Arg His Tyr Gly Ile Thr Ile His Thr Cys
65                  70                  75                  80

Leu Pro Ala Asp Pro Glu Ala Lys Gly Gly Ser Glu Ala Thr Val Arg
                85                  90                  95

Ile Ala Lys Ala Asp Leu Val Pro Thr Ser Ala Asn Arg Thr
            100                 105                 110

<210> 11
<211> 179
<212> PRT
<213> Streptomyces DSM 22643

<400> 11

Met Lys Gly Ala Gly Pro Pro Thr Pro Ala Gly Arg Ala Gly Cys Ser
1               5                   10                  15

Ala Glu Ala Glu Val Arg Thr Arg Gly Ala Leu Asp Trp Tyr Trp Ala
            20                  25                  30

Val Asp Val Leu Thr Glu Ala Gly Ala Gly Val His Leu Thr His Pro
        35                  40                  45

Leu Gly Val Lys Ala Phe Ala Tyr Arg Arg Val Lys Asn Asp Glu Arg
    50                  55                  60

Asp Ala Ala Asp Leu Thr Asp Leu Leu Arg Leu Gly Arg Leu Pro Val
65                  70                  75                  80

Ala Gly Ser Met Leu Ala Val Tyr Val Arg Gln Cys Leu Lys Asn Gly
                85                  90                  95

Asp Thr Val Glu Val Ser Ala Ser Cys Trp Trp Lys Phe Ser Ala Asp
            100                 105                 110

Thr Ile Thr Gln His Gly Glu Tyr Leu Leu Ala Phe Val Asp Gly His
        115                 120                 125

Val Cys Val Gly Ala Phe Glu Ile Val Gly Ala Glu Pro Asp Glu Thr
    130                 135                 140

Glu Gly Glu Lys Tyr Val Phe Asp Leu Leu Arg Pro Ala Ala Arg Phe
145                 150                 155                 160

Gln Trp Ala Leu Gly Arg Lys Leu Pro Leu Pro Pro Gly Arg Asn Pro
                165                 170                 175

Cys Ala Phe

<210>  12
<211>  186
<212>  PRT
<213>  Streptomyces DSM 22643

<400>  12

Val Ala Ser Leu Gly Ala Tyr His Leu Thr Gly Ser His Arg Ala Ala
1               5                   10                  15

Ala Glu Leu Ala Gly Val Asp His His Thr Val Ala Arg Tyr Val Lys
            20                  25                  30

Leu Arg Glu Ala Gly Glu Arg Pro Ala Glu Arg Gln His Arg Ala Arg
        35                  40                  45

Pro Ile Asp Glu Phe Met Asp Lys Ile Glu Glu Leu Leu Thr Ala Ser

50                  55                  60

Lys Gly Arg Ile Gly Ala Asp Ile Val His Arg Arg Ile Thr Ala Ala
65                  70                  75                  80

Gly Leu Thr Gly Gly Asp Arg Thr Thr Arg Arg Ala Val Val Lys Val
                85                  90                  95

Lys Ala Arg Met Arg Ser Arg His His Arg Val Pro Ser Ser Ser Pro
            100                 105                 110

Pro Ala Gln Pro Pro Gln Ile Pro Tyr Arg Gly Ser Asp Glu Glu Leu
        115                 120                 125

Ala Gln Gly Leu Ala Gly Gln Asn Ala His Gly Leu Arg Pro Gly Gly
    130                 135                 140

Ser Gly Ser Phe Leu Pro Ser Ala His Trp Asn Arg Ala Ala Gly Arg
145                 150                 155                 160

Arg Arg Ser Lys Thr Tyr Phe Ser Pro Ser Val Ser Ser Gly Ser Ala
                165                 170                 175

Pro Thr Ile Ser Lys Ala Pro Thr His Thr
            180                 185

<210>  13
<211>  550
<212>  PRT
<213>  Streptomyces DSM 22643

<400>  13

Met Thr Pro Val Pro Gln Leu Arg Ile Pro Thr Ala Arg Leu Lys Ala
1               5                   10                  15

Lys Phe Asp Arg Ile Leu Pro His Leu Asp Glu Arg Arg Arg Arg Phe
            20                  25                  30

Tyr Leu Ala Ser Glu Ala Val Ala Leu Gly His Gly Gly Ile Ala Ala
        35                  40                  45

Val Ala Ala Ala Ser Gly Thr Ser Thr Ala Thr Ile Ala Arg Gly Ile
    50                  55                  60

Ala Glu Leu Asp Ala His Pro Ala Thr Thr Cys Arg Asn Arg Ala Leu
65                  70                  75                  80

Gly Ala Gly Arg Lys Pro Leu Thr Thr Thr Asp Thr Gly Leu Arg Pro
                85                  90                  95

Ala Leu Glu Ala Leu Ile Glu Pro Arg Thr Arg Gly Asp Pro Val Ser
            100                 105                 110

Pro Leu Arg Trp Thr Thr Leu Ser Leu Arg Thr Leu Ala Ser Thr Leu
        115                 120                 125

Thr Ala Gln Gly His Pro Val Gly Ala Thr Thr Val Gly Arg Leu Leu
    130                 135                 140

His Ala Met Gly Tyr Ser Leu Gln Gly Thr Ala Lys Thr Thr Glu Gly
145                 150                 155                 160

Ala Arg His Pro Asp Arg Asp Ala Thr Ala Thr Ala Phe Leu Asp Ser
                165                 170                 175

His Gln Pro Val Ile Ser Val Asp Thr Lys Ala Lys Glu Trp Ile Gly
            180                 185                 190

Asn Arg Asp Arg Pro Gly Arg Thr Trp Arg Pro Gly Lys Gly Pro Ile
        195                 200                 205

Lys Val Asp Cys His Thr Phe Val Thr Asp Glu Met Pro Val Ala Ile
    210                 215                 220

Pro Tyr Gly Ile Tyr Asp Leu Ala Thr Asn Thr Gly Trp Val Asn Val
225                 230                 235                 240

Gly Thr Asp His Asp Thr Ala Glu Phe Ala Val Glu Ser Ile Arg Arg
                245                 250                 255

Trp Trp Arg Arg Arg Gly Arg Thr Asp His Pro Asn Ala Thr Arg Leu
            260                 265                 270

Leu Ile Thr Ala Asp Ala Gly Gly Ser Asn Asp Ala Arg Arg Trp Thr
        275                 280                 285

Trp Lys Lys Tyr Leu His Ala Phe Ala Gln Glu Ser Gly Leu Glu Ile
    290                 295                 300

Thr Val Cys His Phe Pro Pro Gly Thr Ser Lys Trp Asn Lys Ile Glu
305                 310                 315                 320

His Arg Met Phe Cys His Ile Thr Ala Asn Trp Arg Gly Gln Pro Leu
                325                 330                 335

Thr Ser Tyr Gln Val Val Val Glu Thr Ile Ala Ala Thr Thr Thr Ala
            340                 345                 350

Asn Gly Leu Ser Ile Gly Ala Glu Leu Asp Thr Gly Ser Tyr Pro Leu
        355                 360                 365

Gly Thr Thr Val Thr Pro Ala Glu Phe Gln Ala Leu Pro Ile Ala Pro
    370                 375                 380

Glu Ala Phe His Gly Asp Trp Asn Tyr Thr Leu Ala Pro Ala Pro Pro
385                 390                 395                 400

Ser Pro Ala Glu Ala Pro Thr Thr Arg Arg Pro Ile Asp Pro Ala Leu
                405                 410                 415

Thr Thr Met Leu Ile Asp Pro Ala Leu Thr Gly Met Pro Arg Ala Asp
            420                 425                 430

Phe Ala His Leu Ala Ala Val Ser Glu Pro Tyr Trp Asp Ala Leu Ala
        435                 440                 445

Glu Ala Ala Phe Gln Arg Arg Phe His Arg Pro Arg Ser Tyr Leu His
    450                 455                 460

Pro Gln Thr Ser Ser Leu Asp His Phe His Arg Leu Leu Thr Ala Leu
465                 470                 475                 480

Leu Arg Arg Arg Lys Ala Ala Thr Ser Thr Leu Leu Ala Gln Leu Leu
                485                 490                 495

Gly Val Thr Arg Thr Asn Leu Ser Asn Gln Phe Gln Asp Gly His Arg
            500                 505                 510

Ile Leu Asp Leu Tyr Arg Ile Ala Val Thr Pro Ile Pro Gly Ala Pro
        515                 520                 525

Ala Arg Thr Leu Glu Gln Leu Arg Ala Arg Leu Ala Ser Ala Glu Asp
    530                 535                 540

Asp Pro Thr Asp Gln Asp
545                 550

<210>  14
<211>  380
<212>  PRT
<213>  Streptomyces DSM 22643

<400>  14

Val Ala Glu Arg Asn Ser Leu Ala Gly Gln Val Glu Pro Ser Thr Pro
1               5                   10                  15

Leu Val Glu Val Gly Gln Asp Pro Ile Glu Leu Gly Leu Gln Pro Gly
            20                  25                  30

Cys Gly Asp Ala Lys Leu Arg Asn Arg Ser His Ala Pro Ala Ser His
        35                  40                  45

His Gly Ser Pro His Arg Ser Ser Leu Thr Val Ile His Val Gln Ala
    50                  55                  60

Leu Gly Gly Ile Gly Leu Cys Gln Val Ala Val His Leu Thr Tyr Ala
65                  70                  75                  80

Thr Pro Arg Gly His Ala Leu Ile Asp Arg Glu Leu Tyr Leu Pro Ala
                85                  90                  95

Ala Trp Ala Gln Asp Glu Glu Arg Arg Leu Leu Arg His Val Pro Asp
            100                 105                 110

Glu Val Phe Phe Ala Thr Lys Pro Gln Leu Ala Ala Val Met Leu Thr
        115                 120                 125

Arg Ala Arg Glu Leu Gly Val Cys Ala Arg Trp Phe Ala Gly Asp Glu
    130                 135                 140

Val Tyr Gly Ser Leu Glu Leu Arg Arg Thr Ala Arg Met Leu Gly Phe
145                 150                 155                 160

Asp Tyr Ala Leu Ala Val Lys Ala Asp His Thr Ala Thr Thr Ser Ala
                165                 170                 175

Gly Arg Phe Thr Ala Ala Arg Leu Ala Ala Lys Val Pro Ala Lys Ser
            180                 185                 190

Trp Met Arg Met Arg Thr Gly His Gly Leu Lys Gly Asp Arg His Tyr
        195                 200                 205

Asp Trp Ala Leu Ile Glu Val Arg Pro Asp Asp Thr Pro Thr Asp Ser
    210                 215                 220

Glu Ala Gly Gly His Ala Phe Leu Val Ile Arg Arg His Arg Tyr Thr
225                 230                 235                 240

Arg Glu Leu Ser Phe Tyr Arg Cys His Ser Thr Thr Pro Ile Ser Leu
                245                 250                 255

Ala Asp Leu Val Asn Ile Ile Cys Thr Arg Trp Lys Ile Glu Glu Asp
            260                 265                 270

Phe Gln Gly Ala Lys Gly Leu Thr Gly Leu Asp Gln Gly Gln Val Ala
        275                 280                 285

Cys Trp Asn Ser Trp Met His Trp Ser Leu Ile Ser Leu Ile Ala Ala
    290                 295                 300

Ala Val Leu Ala Ile Thr Arg Ala Arg Thr Ala Ala Pro Ala Ala Gly
305                 310                 315                 320

Ile Ala Leu Val Pro Ala Ser Pro Arg Glu Leu Leu Ala Val Leu Arg
                325                 330                 335

Ala Thr Ala Leu Pro Ala Pro Arg Arg His Leu Gly His Val Leu His
            340                 345                 350

Trp Ser Ala Trp Arg Arg His His Gln His Gln Ala Val Gln Ala His
        355                 360                 365

Arg Arg Trp Asn Asn Val Thr Ala Glu Ala Thr Arg
    370                 375                 380

<210>  15
<211>  428
<212>  PRT
<213>  Streptomyces DSM 22643

<400>  15

Met Lys Cys Cys Ser Ala Gly Asp Tyr Pro Ala Arg Val Ala Asp Asp
1               5                   10                  15

Pro Trp Arg Leu Ala Pro Arg Gly Thr Ala Cys Gly Arg Cys Asp Ala
            20                  25                  30

Pro Tyr Trp Ala Ala Gly Gly Gly Ala Asp Ser Val Ala Thr Cys Ser
        35                  40                  45

Lys Pro Ser Glu Ala Tyr Ser Met Arg Phe Gln Val Glu Arg Glu Val
    50                  55                  60

Leu Ala Glu Gly Ile Gly Trp Val Ala Arg Gly Leu Ala Val Arg Pro
65                  70                  75                  80

Ser Val Pro Ile Leu Ser Gly Val Val Val Asn Ala Glu Gly Asp Thr
                85                  90                  95

Leu Thr Leu Ser Gly Phe Asp Tyr Glu Val Ser Thr Arg Val Glu Leu
            100                 105                 110

Lys Ala Asn Val Glu Glu Ser Gly Thr Val Leu Ile Pro Gly Arg Arg
        115                 120                 125

Leu Ala Asp Ile Ala Lys Val Leu Pro Asp Val Pro Ile Glu Phe Asn

130                 135                 140

Val Asp Gln Thr Lys Val Tyr Val Gln Cys Asp Ser Asn Ser Phe Val
145                 150                 155                 160

Leu Asn Ala Leu Pro Leu Asp Glu Tyr Pro Thr Leu Pro Lys Leu Pro
                165                 170                 175

Thr Val Cys Gly Ser Val Glu Gly Asp Gln Phe Ala Arg Ala Val Ser
            180                 185                 190

Gln Val Ala Val Val Ala Ser Arg Asp Asp Ala Leu Pro Val Leu Thr
        195                 200                 205

Gly Ile Gly Val Asn Phe Asp Gly Glu Ile Met Lys Leu Asn Ala Thr
    210                 215                 220

Asp Arg Tyr Arg Phe Ala Ile Arg Glu Leu Ala Trp Lys Pro Glu Gly
225                 230                 235                 240

Thr Pro Ser Ser Ser Ser Val Leu Val Pro Ala Arg Thr Leu Leu Asp
                245                 250                 255

Phe Ala Lys Ser Leu Asn Lys Gly Asp Leu Val Lys Ile Ala Leu Ser
            260                 265                 270

Asp Glu Gly Asn Leu Leu Gly Leu His Ala Gly Thr Arg Gln Met Thr
        275                 280                 285

Cys Arg Leu Leu Glu Gly Thr Leu Pro Asp Tyr Glu Lys Leu Phe Pro
    290                 295                 300

Lys Glu Phe Thr Ser Phe Gly Ala Val Glu Val Ser Arg Leu Val Glu
305                 310                 315                 320

Ala Leu Lys Arg Val Ser Leu Val Leu Glu Arg Asn Ser Ser Val Ala
                325                 330                 335

Leu Asp Phe Thr Asp Gly Glu Leu Val Leu Gln Ala Gly Gly Ala Asp
            340                 345                 350

Asp Asp Arg Ala Thr Ser Arg Met Ala Ala Ser Leu Glu Gly Glu Ser
        355                 360                 365

Ile Asp Ile Ala Phe Asn Pro Ser Phe Leu Leu Asp Gly Leu Thr Asn
    370                 375                 380

Leu Asp Ala Ser Trp Ala Gln Phe Ser Phe Thr Ser Ser Asn Gly Lys
385                 390                 395                 400

Ala Val Ile Met Gly Lys Ser Ser Val Asp Ala Glu Ala Asp Thr Ser
                405                 410                 415

Ala Arg Tyr Leu Val Met Pro Val Arg Phe His Arg
            420                 425

<210>  16
<211>  4168
<212>  PRT
<213>  Streptomyces DSM 22643

<220>
<221>  DOMAIN
<222>  (42)..(1066)
<223>  Module 7

<220>
<221>  DOMAIN
<222>  (42)..(500)
<223>  Module 7; domain A7`

<220>
<221>  DOMAIN
<222>  (501)..(938)
<223>  Module 7; domain MT7 in domain A7

<220>
<221>  DOMAIN
<222>  (939)..(979)
<223>  Module 7; domain A7``

<220>
<221>  DOMAIN
<222>  (1002)..(1066)
<223>  Module 7; domain T7

<220>
<221>  DOMAIN
<222>  (1088)..(2148)
<223>  Module 8

<220>
<221>  DOMAIN
<222>  (1088)..(1430)
<223>  Module 8; domain C8

<220>
<221>  DOMAIN
<222>  (1575)..(2061)
<223>  Module 8; domain A8

<220>
<221>  DOMAIN
<222>  (2084)..(2148)
<223>  Module 8; domain T8

<220>
<221>  DOMAIN
<222>  (2172)..(4160)
<223>  Module 9

<220>
<221>  DOMAIN
<222>  (2172)..(2513)
<223>  Module 9; domain C9

<220>
<221>  DOMAIN
<222>  (2658)..(3098)
<223>  Module 9; domain A9`

<220>
<221>  DOMAIN
<222>  (3099)..(3536)
<223>  Module 9; domain MT9 in domain A9

<220>
<221>  DOMAIN
<222>  (3537)..(3577)
<223>  Module 9; domain A9``

<220>
<221>  DOMAIN
<222>  (3600)..(3663)
<223>  Module 9; domain T9

<220>
<221>  DOMAIN
<222>  (3679)..(4160)
<223>  Module 9; domain E9

<400>  16

Val Phe Asp Arg Ile Ser His Asp Asn Arg Lys Ile Pro Thr Ala Gly
1               5                   10                  15

Thr Leu Pro Gly Leu Phe Glu Val Cys Ala Ala Ser Val Pro Gly Ala
            20                  25                  30

Thr Ala Val Val Phe Gly Asp Val Arg Val Ser Tyr Gly Val Leu Asn
        35                  40                  45

Glu Arg Ala Asn Arg Leu Ala His Trp Leu Leu Gly Arg Gly Val Gly
    50                  55                  60

Pro Glu Arg Val Val Ala Leu Ala Leu Pro Arg Gly Val Asp Leu Val
65                  70                  75                  80

Val Ala Val Leu Ala Val Val Lys Ala Gly Ala Ala Tyr Leu Pro Val
                85                  90                  95

Asp Pro Asp Tyr Pro Ala Glu Arg Val Ala Tyr Met Leu Glu Asp Ser
            100                 105                 110

Arg Pro Val Leu Ala Leu Thr Ser Ser Ala Val Val Ala Gly Leu Pro
        115                 120                 125

Val Val Asp Asp Val Glu Tyr Val Ser Leu Asp Asp Pro Ala Val Leu
    130                 135                 140

Gly Glu Leu Ala Gly Cys Gly Val Ser Asp Pro Ser Asp Ala Asp Arg
145                 150                 155                 160

Gly Ala Val Leu Ser Pro Ala His Pro Val His Val Ile Tyr Thr Ser
                165                 170                 175

Gly Ser Thr Gly Arg Pro Lys Gly Val Met Thr Ser His Gly Asn Val
            180                 185                 190

Val Arg Leu Phe Asp Val Gly Glu Gly Gly His Trp Phe Gly Phe Ala
        195                 200                 205

Pro Asp Asp Val Trp Ala Leu Phe His Ser Tyr Thr Phe Asp Phe Ser
    210                 215                 220

Val Phe Glu Leu Trp Gly Ala Leu Leu His Gly Gly Cys Leu Val Val
225                 230                 235                 240

Val Pro His Leu Thr Ser Arg Ser Pro Val Glu Leu Leu Arg Leu Leu
                245                 250                 255

Val Ala Glu Gln Val Thr Val Leu Cys Gln Thr Pro Ser Ala Phe Asp
            260                 265                 270

Ala Leu Ala Gly Val Val Ala Gln Asp Pro Ala Gly Ala Glu Gly Leu
        275                 280                 285

Val Leu Arg Arg Val Val Phe Gly Gly Glu Ala Leu Pro Ala Arg Thr
    290                 295                 300

Ala Glu Leu Ala Ser Gly Leu Val Pro Gly Val Arg Val Val Asn Ile
305                 310                 315                 320

Tyr Gly Pro Thr Glu Thr Thr Val His Ala Thr Thr Cys His Val Asp
                325                 330                 335

Ser Val Ser Gly Gly Asn Pro Val Val Ser Ile Gly Arg Pro Val Asp
            340                 345                 350

Lys Ala Arg Ala Tyr Val Leu Asp Asp Glu Leu Phe Pro Val Ala Pro
        355                 360                 365

Gly Val Ala Gly Glu Leu Tyr Val Ala Gly Ala Gly Leu Ala Arg Gly
    370                 375                 380

Tyr Val Asn Arg Ala Gly Leu Thr Ala Ser Arg Phe Val Ala Asp Pro

385                 390                 395                 400

Tyr Gly Pro Ala Gly Ser Arg Met Tyr Arg Thr Gly Asp Val Val Arg
                405                 410                 415

Trp Asn Thr Ser Gly Glu Leu Glu Phe Val Gly Arg Ala Asp Asp Gln
            420                 425                 430

Val Lys Ile Arg Gly Phe Arg Ile Glu Leu Gly Glu Ile Glu Thr Thr
        435                 440                 445

Ala Ala Gly His Pro Ala Val Ala Gln Ala Ala Ala Thr Val His Glu
    450                 455                 460

Asp Asp Thr Arg Gly Lys Gln Leu Ala Leu Tyr Val Val Pro Thr Gly
465                 470                 475                 480

Leu Thr Ser Gly Asp Val Ser Gly Ser Val Ser Gly Asp Gly Ala Val
                485                 490                 495

Pro Asp Gly Gly Val Ser Gly Val Val Asp Glu Gln Val Gly Glu Trp
            500                 505                 510

Arg Glu Ile Tyr Asp Ser Leu Tyr Gly Gly Pro Gly Ser Ser Val Phe
        515                 520                 525

Gly Glu Asp Phe Ser Gly Trp Asp Ser Ser Tyr Asp Gly Ala Ala Ile
    530                 535                 540

Pro Leu Glu Glu Met Arg Glu Trp Arg Asp Ala Thr Val Glu Arg Ile
545                 550                 555                 560

Arg Gly Leu Gly Gly Arg Arg Val Leu Glu Ile Gly Val Gly Thr Gly
                565                 570                 575

Leu Leu Met Ser Arg Leu Ala Ala Gly Cys Glu Glu Tyr Trp Ala Thr
            580                 585                 590

Asp Leu Ser Gly Val Val Ile Asp Ala Leu Asp Gly His Val Gln Ala
        595                 600                 605

Asp Pro Val Leu Arg Glu Arg Val Arg Leu Ala Cys Gln Arg Ala Asp
    610                 615                 620

Asp Thr Arg Gly Leu Pro Glu Gly Tyr Phe Asp Thr Val Val Ile Asn
625                 630                 635                 640

Ser Val Val Gln Tyr Phe Pro Gly Ala Gln Tyr Leu Ala Ser Val Ile
                645                 650                 655

Glu Ala Ala Val Ser Arg Leu Ala Pro Gly Gly Arg Val Phe Ile Gly
            660                 665                 670

Asp Val Arg Asp Leu Arg Thr Leu Arg Ala Phe His Thr Ala Val Gln
        675                 680                 685

Leu Thr Arg Thr Thr Gly Gly Arg Ala Gly Asp Gly Met Asp Ala Gly
    690                 695                 700

Gly Leu Arg Arg Ala Val Glu Gln Gly Leu Leu Leu Glu Asn Glu Leu
705                 710                 715                 720

Leu Leu Asp Pro Glu Phe Phe Thr Ala Val Gly Arg Thr Leu Pro Ala
                725                 730                 735

Val Ser Ala Val Glu Val Arg Leu Lys His Gly Gln Ala His Asn Glu
            740                 745                 750

Leu Thr Arg His Arg Tyr Asp Val Ile Leu His Thr Thr Asn Thr Glu
        755                 760                 765

Thr Asp Thr Ala Thr Glu Glu Glu Ala Glu Ala Ala Arg Pro Thr Lys
    770                 775                 780

Ala Glu Ala Glu Thr Pro Ala Leu Val Glu Arg Ile Ser Trp Asn Thr
785                 790                 795                 800

Leu Ser Gly Gly Leu Asp Gly Leu Asp Asp Leu Leu Arg Ser Arg Gly
                805                 810                 815

Ala Ala Pro Leu Arg Val Thr Gly Ile Pro Asn Ala Arg Leu Ala Gly
            820                 825                 830

Glu Tyr Ala Ala Leu Arg Val Leu Glu Asn Gly Gly Thr Leu Thr Glu
        835                 840                 845

Ala Val Thr Ala Leu Ala Gly Pro Arg Gly Ile Asp Pro Glu His Leu
    850                 855                 860

His Gln Leu Ala Ala Ala Thr Gly Tyr His Ala Val Leu Gln Pro Ala
865                 870                 875                 880

Pro Ala Pro Asp Thr Tyr Asn Thr Leu Leu Leu Pro Leu Asp Ile Phe
                885                 890                 895

Asp Gly Thr Ala Trp Ser Ala Thr Ala Thr Ala Thr Ala Thr Asp Leu
            900                 905                 910

Arg Glu Thr Ser Ala Pro Asp His Thr Ala Glu Thr Ser Phe Gln Ala
        915                 920                 925

Leu Ala Asn Asn Pro Ala Ala Ser Arg Asp Thr Ser Thr Leu Ile Thr
    930                 935                 940

Gln Val Arg Asp His Leu Arg Thr Lys Leu Pro Asp His Met Val Pro
945                 950                 955                 960

Ala Ala Ile Val Val Leu Glu Arg Leu Pro Leu Thr Ala Ser Gly Lys
                965                 970                 975

Leu Asp Arg Arg Ala Leu Pro Ala Pro Asp Leu Gly Thr His Thr Thr
            980                 985                 990

Gly Arg Ala Pro Arg Ser Pro Arg Glu Glu Ile Leu Ala Gly Leu Phe
        995                 1000                1005

Ala Glu Val Leu Gly Leu Pro Ala Val Gly Ile Asp Asp Ser Phe
    1010                1015                1020

Phe Asp Leu Gly Gly His Ser Leu Leu Ala Thr Arg Leu Ile Ser
    1025                1030                1035

Arg Ile Arg Ala Ile Leu Gly Val Glu Ile Pro Ile Arg Asp Leu
    1040                1045                1050

Phe Glu Ala Pro Thr Val Ala Gly Leu Ala Thr Leu Leu Asp Glu
    1055                1060                1065

Asn Arg Ala Val Arg Pro Thr Leu Thr Pro Ala Ala Arg Pro Glu
    1070                1075                1080

Arg Ile Pro Leu Ser Ser Ala Gln Asn Arg Leu Trp Phe Leu His
    1085                1090                1095

Arg Leu Glu Gly Met Gly Ala Ala Ala Tyr Asn Val Pro Met Ala
    1100                1105                1110

Leu Arg Leu Thr Gly Ser Val Met Pro Glu Val Leu Arg Leu Ala
    1115                1120                1125

Leu Ala Asp Val Val Glu Arg His Glu Ser Leu Arg Thr Val Phe
    1130                1135                1140

Pro Glu Thr Asp Gly Val Pro Cys Gln His Ile Leu Ser Val Val
    1145                1150                1155

Glu Ala Arg Pro Val Leu His Val Val Gln Thr Ser Glu Glu Gly
    1160                1165                1170

Leu Ala Glu Ala Val Ser Thr Ala Ser Gln Tyr Ala Phe Asp Leu
    1175                1180                1185

Ser Ala Glu Leu Pro Val Arg Ala Trp Leu Phe Ala Leu Ala Pro
    1190                1195                1200

Glu Glu His Val Leu Val Leu Val Val His His Ile Ala Gly Asp
    1205                1210                1215

Gly Trp Ser Leu Ser Pro Leu Phe Arg Asp Leu Thr Thr Ala Tyr
    1220                1225                1230

Ala Ala Arg Ala Asp Gly Arg Thr Pro Gly Trp Ala Pro Leu Pro
    1235                1240                1245

Val Gln Tyr Ala Asp Tyr Thr Leu Trp Gln Asn Asp Leu Leu Gly
    1250                1255                1260

Asp His Ser Asp Thr Gly Ser Leu Ile Ala Arg Gln Leu Glu Tyr
    1265                1270                1275

Trp Arg Thr Thr Leu Thr Gly Leu Pro Glu Gln Val Thr Leu Pro
    1280                1285                1290

Thr Asp Arg Pro Arg Pro Ala Thr Ala Thr Tyr Gln Gly Ala Leu
    1295                1300                1305

His Asp Phe Ala Trp Asp Ala Glu Leu His Gln Gly Leu Ile Asp
    1310                1315                1320

Leu Ala Arg Ser Thr Gly Thr Thr Val Phe Met Val Leu Gln Ala
    1325                1330                1335

Gly Leu Ala Ala Leu Met Ser Arg Leu Gly Ala Gly Asp Asp Ile
    1340                1345                1350

Pro Leu Gly Ser Pro Ile Ala Gly Arg Thr Asp Glu Ala Leu Asp
    1355                1360                1365

Asp Leu Val Gly Phe Phe Val Asn Thr Leu Val Leu Arg Thr Asp
    1370                1375                1380

Thr Ser Gly Asn Pro Thr Phe Arg Glu Leu Leu Ala Arg Val Arg
    1385                1390                1395

Glu Thr Asp Leu Ala Ala Tyr Ala His Gln Asp Val Pro Phe Glu

1400                 1405                 1410

His Leu Val Glu Ile Leu Asn Pro Glu Arg Ser Leu Ala His His
    1415                 1420                 1425

Pro Leu Phe Gln Val Met Leu Ala Leu Gln Asn Ala Pro Glu Gly
    1430                 1435                 1440

Gln Phe Lys Leu Pro Gly Leu Gln Ala Arg Phe Glu Thr Thr His
    1445                 1450                 1455

Thr Gln Thr Ala Lys Phe Asp Leu Phe Phe Asn Val His Glu Tyr
    1460                 1465                 1470

Arg Ala Thr Asp Gly Gly Pro Gly Gly Leu Tyr Gly Ser Val Glu
    1475                 1480                 1485

Phe Ser Thr Asp Leu Phe Asp Arg Val Ser Val Glu Val Val Leu
    1490                 1495                 1500

Glu Arg Leu Arg Arg Val Leu Val Ser Val Ala Ala Asp Pro Asp
    1505                 1510                 1515

Val Thr Val Gly Ala Leu Pro Val Leu Ser Gly Gln Glu Glu His
    1520                 1525                 1530

Arg Leu Val Ala Glu Trp Asn Asp Thr Ala Leu Glu Val Pro Pro
    1535                 1540                 1545

Ala Ser Leu Pro Glu Leu Phe Gln Ala Gln Ala Ala Ala Thr Pro
    1550                 1555                 1560

Glu Ala Thr Ala Val Val Phe Glu Asp Val Arg Ile Ser Tyr Gly
    1565                 1570                 1575

Glu Leu Asn Glu Arg Ala Asn Arg Leu Ala His Tyr Leu Ile Gly
    1580                 1585                 1590

Gln Gly Ala Gly Pro Glu Arg Ile Val Ala Leu Ala Leu Pro Arg
    1595                 1600                 1605

Gly Thr Asp Leu Val Ile Ala Val Leu Ala Val Leu Lys Ala Gly
    1610                 1615                 1620

Ala Ala Tyr Leu Pro Val Asp Pro Asp Tyr Pro Ala Glu Arg Ile
    1625                 1630                 1635

Thr His Met Leu Thr Asp Thr Arg Pro Thr Leu Leu Leu Thr Thr
    1640                 1645                 1650

Ser Asp Ala Thr Ala Gly Leu Pro His Thr Glu Gly Ile Pro Gln
    1655                1660                1665

Val Leu Leu Asp Asp Ser Ala Val Ile Glu Thr Val Ala Val Leu
    1670                1675                1680

Ser Ala Asp Asp Pro Ser Asp Thr Asp Arg Gly Val Val Leu Glu
    1685                1690                1695

Gly Ser His Pro Ala Tyr Val Ile Tyr Thr Ser Gly Ser Thr Gly
    1700                1705                1710

Val Pro Lys Gly Val Val Met Pro Ser Gly Gly Leu Val Asn Leu
    1715                1720                1725

Leu His Trp His His Ser Val Ile Ala Gly Arg Ala Gly Ala Arg
    1730                1735                1740

Thr Ala Gln Phe Thr Thr Ile Ser Phe Asp Val Ser Ala Gln Glu
    1745                1750                1755

Ile Leu Ser Ala Leu Val Phe Gly Lys Glu Leu Trp Val Pro Gly
    1760                1765                1770

Glu Glu Val Arg Arg Ser Gly Glu Gly Leu Ala Arg Trp Leu Gln
    1775                1780                1785

Glu His Ala Val Glu Glu Leu Phe Ala Pro Ala Leu Val Ile Asp
    1790                1795                1800

Ala Val Ala Gln Ala Ala Gly Glu Leu Gly Leu Val Leu Pro Ala
    1805                1810                1815

Leu Arg His Val Ala Gln Ala Gly Glu Ala Leu Val Pro Gly Ala
    1820                1825                1830

Ala Met Arg Arg Phe Phe Arg Glu Arg Pro His Ile Arg Leu His
    1835                1840                1845

Asn His Tyr Gly Pro Ala Glu Thr His Val Val Thr Ala His Pro
    1850                1855                1860

Leu Pro Asn Arg Ile Ala Asp Trp Ala Thr Ser Val Pro Ile Gly
    1865                1870                1875

Arg Pro Leu Pro Asn Thr Arg Val Tyr Val Leu Asp Ala Gly Leu
    1880                1885                1890

Arg Val Val Pro Val Gly Val Ala Gly Glu Leu Tyr Val Ala Gly
    1895                1900                1905

Ala Gly Leu Ala Arg Gly Tyr Val Asn Arg Ala Gly Leu Thr Ala
    1910                1915                1920

Ser Arg Phe Val Ala Asp Pro Tyr Gly Pro Ala Gly Ser Arg Met
    1925                1930                1935

Tyr Arg Thr Gly Asp Val Val Arg Trp Asn Thr Ser Gly Glu Leu
    1940                1945                1950

Glu Phe Val Gly Arg Ala Asp Asp Gln Val Lys Ile Arg Gly Phe
    1955                1960                1965

Arg Ile Glu Leu Gly Glu Ile Glu Thr Thr Ala Ala Glu His Pro
    1970                1975                1980

Ala Val Ala Gln Ala Ala Ala Thr Val His Glu Asp Asp Thr Arg
    1985                1990                1995

Gly Lys Gln Leu Ala Leu Tyr Val Val Pro Thr Gly Ile Thr Ser
    2000                2005                2010

Gly Asp Val Pro Ala Ser Gly Gly Val Val Asp Ala Gly Thr Val
    2015                2020                2025

Arg Glu Tyr Leu Arg Thr Lys Leu Pro Asp Tyr Met Val Pro Ala
    2030                2035                2040

Ala Val Val Val Leu Glu Gln Leu Pro Leu Thr Ala Ser Gly Lys
    2045                2050                2055

Leu Asp Arg Arg Ala Leu Pro Thr Pro Glu Phe Ala Ala Glu Pro
    2060                2065                2070

Ala Gly Arg Thr Ala Arg Ser Pro Arg Glu Glu Ile Leu Ala Gly
    2075                2080                2085

Leu Phe Ala Glu Val Leu Gly Leu Pro Ala Val Gly Ile Asp Asp
    2090                2095                2100

Ser Phe Phe Asp Leu Gly Gly His Ser Leu Leu Ala Thr Arg Leu
    2105                2110                2115

Ile Ser Arg Ile Arg Ala Thr Leu Asn Thr Glu Val Pro Val Arg
    2120                2125                2130

Ala Leu Phe Glu Ala Pro Thr Val Ala Ala Leu Thr Val Leu Leu
    2135                2140                2145

Glu Ala Gly Ser Ser Asp Val Val Arg Arg Lys Leu Thr Pro Ala
    2150                2155                2160

Ala Arg Pro Glu Arg Ile Pro Leu Ser Ser Ala Gln Ser Arg Leu
    2165                2170                2175

Trp Phe Leu His Arg Leu Glu Gly Pro Ser Ala Thr Tyr Asn Met
    2180                2185                2190

Pro Met Ala Leu Arg Leu Thr Gly Pro Val Met Pro Glu Val Leu
    2195                2200                2205

Arg Leu Ala Leu Ala Asp Val Val Glu Arg His Glu Ser Leu Arg
    2210                2215                2220

Thr Val Phe Pro Glu Thr Asp Gly Val Pro Cys Gln His Ile Leu
    2225                2230                2235

Ala Gly Ala Glu Ala Gln Pro Val Leu Glu Val Val Glu Val Gly
    2240                2245                2250

Glu Asp Gly Leu Glu Glu Ala Leu Ala Gly Ala Ala Arg Tyr Pro
    2255                2260                2265

Leu Asp Leu Ser Ala Glu Leu Pro Val Arg Gly Trp Leu Phe Gly
    2270                2275                2280

Leu Gly Pro Thr Glu His Val Leu Val Leu Val Val His His Ile
    2285                2290                2295

Ala Gly Asp Gly Trp Ser Leu Ala Pro Leu Ser Arg Asp Leu Val
    2300                2305                2310

Thr Ala Tyr Ala Ala Arg Ala Asp Gly Arg Thr Pro Gly Trp Ala
    2315                2320                2325

Pro Leu Pro Val Gln Tyr Ala Asp Tyr Thr Leu Trp Gln Asn Asp
    2330                2335                2340

Leu Leu Gly Asp Gln Ser Asp Gly Asp Ser Leu Val Ala Arg Gln
    2345                2350                2355

Leu Glu Tyr Trp Arg Thr Thr Leu Thr Gly Leu Pro Glu Gln Val
    2360                2365                2370

Thr Leu Pro Thr Asp Arg Pro Arg Pro Ala Thr Ala Thr Tyr Gln

2375                2380                2385

Gly Ala Leu His Asp Phe Ala Trp Asp Ala Glu Leu His Gln Gly
    2390                2395                2400

Leu Ile Asp Leu Ala Arg Ser Thr Gly Thr Thr Val Phe Met Val
    2405                2410                2415

Leu Gln Ala Gly Leu Ala Ala Leu Met Ser Arg Leu Gly Ala Gly
    2420                2425                2430

Asp Asp Ile Pro Leu Gly Ser Pro Ile Ala Gly Arg Thr Asp Glu
    2435                2440                2445

Ala Leu Asp Asp Leu Val Gly Phe Phe Val Asn Thr Leu Val Leu
    2450                2455                2460

Arg Thr Asp Thr Ser Gly Asn Pro Thr Phe Arg Glu Leu Leu Ala
    2465                2470                2475

Arg Val Arg Glu Thr Asp Leu Ala Ala Tyr Ala His Gln Asp Val
    2480                2485                2490

Pro Phe Glu His Leu Val Glu Ile Leu Asn Pro Glu Arg Ser Leu
    2495                2500                2505

Ala His His Pro Leu Phe Gln Val Ala Leu Ala Leu His Asn Thr
    2510                2515                2520

Pro Pro Gly Asn Phe Thr Leu Pro Asp Ile Asn Ile His Gly Gln
    2525                2530                2535

Arg Leu Ser Thr Gly Thr Ser Arg Phe Asp Val Ser Leu His Phe
    2540                2545                2550

Val Glu Trp Gln Arg Glu Asp Gly Ala Ala Ser Gly Leu Gly Gly
    2555                2560                2565

Phe Val Glu Phe Ser Thr Asp Leu Phe Asp Arg Val Ser Val Glu
    2570                2575                2580

Val Val Leu Glu Arg Leu Arg Arg Val Leu Val Ser Val Ala Ala
    2585                2590                2595

Asp Pro Asp Val Thr Val Gly Ala Leu Pro Val Leu Ser Gly Gln
    2600                2605                2610

Glu Glu His Arg Leu Val Ala Glu Trp Asn Asp Thr Val Leu Glu
    2615                2620                2625

Val Pro Pro Ala Ser Leu Pro Glu Leu Phe Gln Ala Gln Ala Ala
    2630                2635                2640

Glu Ala Pro Glu Ala Thr Ala Val Val Phe Glu Thr Glu Arg Leu
    2645                2650                2655

Ser Tyr Arg Glu Leu Asn Glu Arg Ala Asn Arg Leu Ala His Tyr
    2660                2665                2670

Leu Ile Lys Gln Gly Ala Gly Pro Glu Arg Ile Val Ala Leu Ala
    2675                2680                2685

Leu Pro Arg Gly Thr Asp Leu Val Ile Ala Val Leu Ala Val Leu
    2690                2695                2700

Lys Ala Gly Ala Ala Tyr Leu Pro Val Asp Pro Asp Tyr Pro Ala
    2705                2710                2715

Glu Arg Ile Thr His Met Leu His Asp Ala Ala Pro Ala Leu Met
    2720                2725                2730

Val Thr Thr Ser Gly Val Ala Ala Gly Leu Pro His Thr Glu Gly
    2735                2740                2745

Val Thr Ser Val Leu Val Asp Ala Pro Ala Val Val Glu Ala Val
    2750                2755                2760

Ala Val Leu Ser Ala Asp Asp Pro Ser Asp Thr Asp Arg Gly Val
    2765                2770                2775

Val Leu Glu Gly Ser His Pro Ala Tyr Val Ile Tyr Thr Ser Gly
    2780                2785                2790

Ser Thr Gly Val Pro Lys Gly Val Leu Val Pro His Ala Gly Leu
    2795                2800                2805

Ala Asn Leu Thr Ala Ala Glu Arg Ala Ala Leu Asp Leu Ser Ala
    2810                2815                2820

Gly Ser Arg Val Leu Gln Leu Ala Ser Val Gly Phe Asp Ala Ala
    2825                2830                2835

Val Leu Glu Leu Ser Met Ala Phe Gly Ser Gly Ala Thr Leu Val
    2840                2845                2850

Ile Ala Pro Gln Gly Arg Leu Leu Gly Asp Asp Leu Ala Ala Leu
    2855                2860                2865

Leu Ala Arg Gln Glu Ile Thr His Thr Leu Ile Thr Pro Ser Ala
    2870                2875                2880

Leu Ala Thr Leu Pro Glu Thr Asp Leu Pro His Leu Arg Thr Leu
    2885                2890                2895

Leu Thr Gly Ala Glu Ala Cys Pro Pro Glu Leu Val Ala Arg Trp
    2900                2905                2910

Ser Pro Gly Arg Arg Phe Ile Asn Ala Tyr Gly Pro Thr Glu Ala
    2915                2920                2925

Ser Val Val Ala Thr Trp Ser Asp Pro Leu Thr Gln Asp Thr Ala
    2930                2935                2940

Pro Ile Gly Arg Pro Leu Pro Asn Thr Arg Val Tyr Val Leu Asp
    2945                2950                2955

Ala Gly Leu Arg Val Val Pro Val Gly Val Ala Gly Glu Leu Tyr
    2960                2965                2970

Val Ala Gly Ala Gly Leu Ala Arg Gly Tyr Val Asn Arg Ala Gly
    2975                2980                2985

Leu Thr Ala Ser Arg Phe Val Ala Asp Pro Tyr Gly Pro Ala Gly
    2990                2995                3000

Ser Arg Met Tyr Arg Thr Gly Asp Val Val Arg Trp Asn Thr Ser
    3005                3010                3015

Gly Glu Leu Glu Phe Val Gly Arg Ala Asp Asp Gln Val Lys Ile
    3020                3025                3030

Arg Gly Phe Arg Ile Glu Leu Gly Glu Ile Glu Thr Thr Ala Ala
    3035                3040                3045

Gly His Pro Ala Val Ala Gln Ala Ala Ala Thr Val His Glu Asp
    3050                3055                3060

Asp Thr Arg Gly Lys Gln Leu Ala Leu Tyr Val Val Pro Thr Gly
    3065                3070                3075

Leu Thr Ser Gly Asp Val Ser Gly Ser Val Ser Gly Asp Gly Ala
    3080                3085                3090

Val Pro Asp Gly Gly Val Ser Gly Val Val Asp Glu Gln Val Gly
    3095                3100                3105

Glu Trp Arg Glu Ile Tyr Asp Ser Leu Tyr Gly Gly Pro Gly Ser
    3110                3115                3120

Ser Val Phe Gly Glu Asp Phe Ser Gly Trp Asp Ser Ser Tyr Asp
    3125                3130                3135

Gly Ala Ala Ile Pro Leu Glu Glu Met Arg Glu Trp Arg Asp Ala
    3140                3145                3150

Thr Val Glu Arg Ile Arg Gly Leu Gly Gly Arg Arg Val Leu Glu
    3155                3160                3165

Ile Gly Val Gly Thr Gly Leu Leu Met Ser Arg Leu Ala Ala Gly
    3170                3175                3180

Cys Glu Glu Tyr Trp Ala Thr Asp Leu Ser Gly Val Val Ile Asp
    3185                3190                3195

Ala Leu Asp Gly His Val Gln Ala Asp Pro Val Leu Arg Glu Arg
    3200                3205                3210

Val Arg Leu Ala Cys Gln Arg Ala Asp Asp Thr Arg Gly Leu Pro
    3215                3220                3225

Glu Gly Tyr Phe Asp Thr Val Val Ile Asn Ser Val Val Gln Tyr
    3230                3235                3240

Phe Pro Gly Ala Gln Tyr Leu Ala Ser Val Ile Glu Ala Ala Val
    3245                3250                3255

Ser Arg Leu Ala Pro Gly Gly Arg Val Phe Ile Gly Asp Val Arg
    3260                3265                3270

Asp Leu Arg Thr Leu Arg Ala Phe His Thr Ala Val Gln Leu Thr
    3275                3280                3285

Arg Thr Thr Gly Gly Arg Ala Gly Asp Gly Met Asp Ala Gly Gly
    3290                3295                3300

Leu Arg Arg Ala Val Glu Gln Gly Leu Leu Leu Glu Asn Glu Leu
    3305                3310                3315

Leu Leu Asp Pro Glu Phe Phe Thr Ala Val Gly Arg Thr Leu Pro
    3320                3325                3330

Ala Val Ser Ala Val Glu Val Arg Leu Lys His Gly Gln Ala His
    3335                3340                3345

Asn Glu Leu Thr Arg His Arg Tyr Asp Val Ile Leu His Thr Thr

3350                3355                3360

Asn Thr Glu Thr Asp Thr Ala Thr Glu Glu Glu Ala Glu Ala Ala
    3365                3370                3375

Arg Pro Thr Lys Ala Glu Ala Glu Thr Pro Ala Leu Val Glu Arg
    3380                3385                3390

Ile Ser Trp Asn Thr Leu Ser Gly Gly Leu Asp Gly Leu Asp Asp
    3395                3400                3405

Leu Leu Arg Ser Arg Gly Ala Ala Pro Leu Arg Val Thr Gly Ile
    3410                3415                3420

Pro Asn Ala Arg Leu Ala Gly Glu Tyr Ala Ala Leu Arg Val Leu
    3425                3430                3435

Glu Asn Gly Gly Thr Leu Thr Glu Ala Val Thr Ala Leu Ala Gly
    3440                3445                3450

Pro Arg Gly Ile Asp Pro Glu His Leu His Gln Leu Ala Ala Ala
    3455                3460                3465

Thr Gly Tyr His Ala Val Leu Gln Pro Ala Pro Ala Pro Asp Thr
    3470                3475                3480

Tyr Asn Thr Leu Leu Leu Pro Leu Asp Ile Phe Asp Gly Thr Ala
    3485                3490                3495

Trp Ser Ala Thr Ala Thr Ala Thr Ala Thr Asp Leu Arg Glu Thr
    3500                3505                3510

Ser Ala Pro Asp His Thr Ala Glu Thr Ser Phe Gln Ala Leu Ala
    3515                3520                3525

Asn Asn Pro Ala Ala Ser Arg Asp Thr Ser Thr Leu Ile Thr Gln
    3530                3535                3540

Val Arg Asp His Leu Arg Thr Lys Leu Pro Asp His Met Val Pro
    3545                3550                3555

Ala Ala Ile Val Val Leu Glu Arg Leu Pro Leu Thr Ala Ser Gly
    3560                3565                3570

Lys Leu Asp Arg Arg Ala Leu Pro Ala Pro Asp Leu Gly Thr His
    3575                3580                3585

Thr Thr Gly Arg Ala Pro Arg Ser Pro Arg Glu Glu Ile Leu Ala
    3590                3595                3600

Gly Leu Phe Ala Glu Val Leu Gly Leu Pro Ala Val Gly Ile Asp
    3605                3610                3615

Asp Ser Phe Phe Asp Leu Gly Gly Asp Ser Ile Ile Ser Ile Gln
    3620                3625                3630

Leu Val Ser Arg Ala Arg Lys Ala Gly Leu Lys Val Ser Ala Gly
    3635                3640                3645

Asp Val Leu Arg Tyr Lys Thr Val Ser Ala Leu Ala Ala Val Ala
    3650                3655                3660

Gln Asp Leu His Ala Glu Ala Ser Tyr Ile Ser Asp Asp Gly Leu
    3665                3670                3675

Gly Asp Val Pro Gln Thr Pro Ile Met Arg Trp Ala Phe Lys Gln
    3680                3685                3690

Glu Asp Leu Val His Gly Leu His Gln Ala Met Leu Leu Glu Thr
    3695                3700                3705

Pro Ala Gly Leu Gln Gln Ala Gln Leu Val Ala Ile Thr Gln Thr
    3710                3715                3720

Leu Leu Asp His His Glu Met Leu Arg Ala Arg Leu Val Val Arg
    3725                3730                3735

Arg Gly Glu Glu Ser Val Leu Arg Val Ala Glu Ala Gly Ala Val
    3740                3745                3750

Arg Ala Asp Asp Leu Ile Gln Gln Val Ala Val Ala Ala Asn Ser
    3755                3760                3765

Gly Glu Asn Leu Arg Asp Ser Ile Ser Ala Glu Phe Ser Ala Ala
    3770                3775                3780

Arg Asp Arg Leu Ser Pro Glu Ser Gly Ser Met Leu Gln Leu Val
    3785                3790                3795

Trp Phe Asn Ser Gly Pro Asn His Ala Gly Arg Leu Leu Ile Val
    3800                3805                3810

Ile His His Leu Val Val Asp Gly Val Ser Trp Arg Ile Leu Leu
    3815                3820                3825

Ser Asp Val Ala Asp Ala Trp Glu Asp Ile Ser Ala Gly Arg Ala
    3830                3835                3840

Pro Val Leu Glu Pro Arg Arg Thr Ser Phe Lys Arg Trp Ala Asn
    3845                3850                3855

Ala Leu Ile Ser Glu Ala His Ala Pro Lys Arg Val Glu Glu Met
    3860                3865                3870

Pro Leu Trp Met Gly Thr Leu Thr Asp Ser Gly Trp Glu Phe Ala
    3875                3880                3885

Ser Lys Pro Lys Asp Glu Lys Glu Ala Ser Thr Ser Ala Gln Leu
    3890                3895                3900

Thr Leu Thr Leu Ser Pro Glu Lys Thr Ile Pro Ile Leu Thr Glu
    3905                3910                3915

Val Pro Ala Val Phe His Gly Lys Ile Asn Asp Val Leu Leu Thr
    3920                3925                3930

Ala Phe Thr Ile Ala Ala Val Glu Trp Arg Arg Val His Arg Ala
    3935                3940                3945

Thr Tyr Ala Gly Thr Asp Val Leu Leu Asn Leu Glu Gly His Gly
    3950                3955                3960

Arg Glu His Phe Ala Glu Gly Phe Asp Val Ser Arg Thr Val Gly
    3965                3970                3975

Trp Phe Thr Ser Met Phe Pro Val Arg Leu Asp Pro Gly Glu Tyr
    3980                3985                3990

Asp Arg Asp Glu Val Arg Ile Gly Gly Pro Ala Leu Gly Lys Val
    3995                4000                4005

Leu Lys Thr Val Lys Glu Gln Leu Arg Ser Gln Pro Asp Asn Gly
    4010                4015                4020

Leu Gly Phe Gly Leu Leu Arg Tyr Leu Asn Thr Glu Thr Ala Thr
    4025                4030                4035

Lys Leu Cys Gly Leu Ala Val Pro Gln Val Ser Phe Asn Tyr Leu
    4040                4045                4050

Gly Arg Phe Gly Ala Ser Gly Gly Ala Gly Trp Val Gln Ala Pro
    4055                4060                4065

Glu Ser Asp Ile Val Ala Asn Gly Gln His Gly Pro Leu Ala His
    4070                4075                4080

Leu Leu Glu Val Asn Ala Leu Thr Arg Asp Gly Val Asp Gly Pro
    4085                4090                4095

Glu Leu Ile Ala Thr Trp Ser Trp Thr Pro Gly Arg Phe Thr Glu
    4100                4105                4110

Ala Glu Val Leu Asp Phe Ala Glu Arg Trp Phe Ser Val Leu Gly
    4115                4120                4125

Ser Leu Thr Ile His Ala Ser Ala Pro Asp Ala Gly Gly Tyr Thr
    4130                4135                4140

Pro Ser Asp Val Pro Leu Val Ser Leu Ser Gln Glu Gln Ile Glu
    4145                4150                4155

Leu Leu Glu Ser Glu Trp Arg Ala Ser Glu
    4160                4165

<210>  17
<211>  1324
<212>  PRT
<213>  Streptomyces DSM 22643

<220>
<221>  DOMAIN
<222>  (13)..(1312)
<223>  Module 10

<220>
<221>  DOMAIN
<222>  (13)..(343)
<223>  Module 10; domain C10

<220>
<221>  DOMAIN
<222>  (484)..(951)
<223>  Module 10; domain A10

<220>
<221>  DOMAIN
<222>  (974)..(1038)
<223>  Module 10; domain T10

<220>
<221>  DOMAIN
<222>  (1060)..(1312)
<223>  Module 10; domain TE10

<400>  17

Met Lys Asp Pro Gly Leu Glu Asp Ile Leu Pro Leu Thr Pro Leu Gln
1               5                   10                  15

Glu Gly Ile Leu Phe His Ala Ala Phe Asp Ala Glu Gly Gln Asp Pro
            20                  25                  30

Tyr Val Ile Gln Leu Thr Leu Glu Phe Gln Gly Pro Leu Lys Ala Lys
        35                  40                  45

Asp Leu Arg Ser Ala Ala Gln Ser Leu Leu Glu Arg His Ser Asn Leu
    50                  55                  60

Arg Ala Ser Phe Arg Tyr His Gly Leu Asp Arg Pro Val Gln Leu Ile
65                  70                  75                  80

Pro Arg His Ala Glu Ala Pro Trp Glu Glu Ile Asp Leu Ser Asn Leu
                85                  90                  95

Ser Ala Ala Leu Arg Asp Gln Glu Val Ser Lys Ala Val Ala Ala Asp
            100                 105                 110

Leu Gln Arg Arg Phe Asp Leu Thr Lys Gly Pro Ser Ile Arg Phe Thr
        115                 120                 125

Leu Val Arg Leu Ala Glu Asp Leu His Lys Phe Val Ile Thr Ser His
    130                 135                 140

His Ile Leu Met Asp Gly Trp Ser Ile Pro Val Leu Phe Arg Glu Leu
145                 150                 155                 160

Phe Ala Cys Tyr Ala Ala Gly Gly Ala Thr Ser Met Leu Pro Pro Ala
                165                 170                 175

Gln Pro Phe Arg Asn Tyr Leu Ala Trp Ser Ala Lys Gln Asp Arg Ser
            180                 185                 190

Ser Ala Glu Gln Ala Trp Lys Glu Ala Leu Ser Gly Leu Asp His Pro
        195                 200                 205

Thr Arg Leu Ala Pro His Ser Pro Ala Arg Ser Val Leu Leu Pro Gln
    210                 215                 220

Gln Val Ser Leu Ser Leu Gly Glu Asp Leu Thr Glu Gln Leu Gly Ala
225                 230                 235                 240

Trp Ala Arg Ser Arg Asp Phe Thr Leu Ser Gln Val Val Gln Gly Ala
                245                 250                 255

Trp Gly Val Leu Leu Gly Arg Leu Thr Gly Gln Ser Asp Val Val Phe
            260                 265                 270

Gly Ala Thr Val Ala Gly Arg Pro Pro Glu Val Ala Gly Val Glu Ser
        275                 280                 285

Met Ile Gly Leu Phe Ile Asn Thr Leu Pro Val Arg Leu Arg Ile Val

290                 295                 300

Pro Asp Glu Ser Phe Thr Val Leu Leu Arg Arg His Gln Arg Asp Gln
305                 310                 315                 320

Glu Arg Leu Phe Pro His His His Leu Gly Leu Thr Asp Ile Gln His
                325                 330                 335

Ala Ala Gly Val Gly Gln Leu Phe Asp Ser Ile Val Ile Phe Glu Asn
            340                 345                 350

Tyr Pro Thr Lys Ile Gly Ser Glu Lys Thr Gly Ser Gly Ser Leu Arg
        355                 360                 365

Val Thr Gly Phe Glu Ala His Asp Ala Thr His Tyr Pro Ile Thr Leu
    370                 375                 380

Phe Val Ile Pro Gly Lys Arg Leu Asn Phe Arg Leu Asp Tyr Asn Ala
385                 390                 395                 400

Glu Tyr Phe Asp His Ala Thr Val Gln Thr Met Leu Glu Arg Leu Arg
                405                 410                 415

Arg Leu Leu Ala Ser Val Val Ser Asp Pro Glu Pro Gln Leu Ser Gly
            420                 425                 430

Leu Thr Leu Leu Gly Thr Glu Glu Glu His Arg Ile Leu Thr Glu Trp
        435                 440                 445

Asn Asp Thr Arg Leu Glu Val Pro Ala Ala Ser Leu Phe Glu Leu Phe
    450                 455                 460

Gln Ala Gln Ala Val Ser Thr Pro Asp Ala Thr Ala Val Val Phe Glu
465                 470                 475                 480

Asp Thr Ser Val Thr Tyr Glu Glu Leu Asn Ala Arg Ala Asn Arg Leu
                485                 490                 495

Ala His Trp Leu Ile Gly Glu Gly Val Gly Pro Glu Arg Val Val Ala
            500                 505                 510

Leu Ala Ile Pro Arg Ser Val Asp Leu Val Val Ala Val Leu Ala Val
        515                 520                 525

Leu Lys Ala Gly Ala Ala Tyr Leu Pro Ile Asp Pro Glu Tyr Pro Pro
    530                 535                 540

Glu Arg Ile Ala His Met Leu Thr Asp Ser Arg Pro Thr Met Leu Val
545                 550                 555                 560

Thr Ser Ser Lys Ala Ala Gln Asp Leu Pro Thr Val Ser Gly Thr Arg
                565                 570                 575

Ser Val Cys Leu Asp Gly Pro Ala Leu Met Trp Ser Leu Gly Thr Ser
            580                 585                 590

Lys Ala Thr Asn Pro Thr His Val Asp Arg Gly Lys Ala Val Asp Ala
        595                 600                 605

Arg His Pro Ala Tyr Val Ile Tyr Thr Ser Gly Ser Thr Gly Arg Pro
    610                 615                 620

Lys Gly Val Val Val Pro Thr Gly Ser Val Val Asn Leu Leu Thr Ala
625                 630                 635                 640

Met Gly Asp Trp Phe Pro Val Thr Gly Lys Asp Cys Leu Leu Ala Val
                645                 650                 655

Thr Thr Phe Ala Phe Asp Ile Ala Thr Leu Glu Leu Leu Leu Pro Leu
            660                 665                 670

Leu Asn Gly Ala Arg Leu Val Leu Thr Asn Arg Glu Thr Val Leu Glu
        675                 680                 685

Ser Ser Ala Leu Ala Glu Val Ile Ala Arg His Ser Val Thr Ile Met
    690                 695                 700

Gln Ala Thr Pro Thr Phe Trp Asn Glu Phe Ala Ala Asn Glu Pro Glu
705                 710                 715                 720

Ala Leu Thr Gly Ile Arg Ile Leu Thr Gly Gly Glu Ala Leu Ser Glu
                725                 730                 735

Gly Leu Ala Gly Arg Leu Gln Ala Leu Ala Asp Asp Val Thr Asn Val
            740                 745                 750

Tyr Gly Pro Thr Glu Thr Thr Ile Trp Ser Thr Ala Ala Ala Ile Thr
        755                 760                 765

Ala Ala Thr Gly Val Pro Pro Ile Gly Arg Pro Leu Ala Asn Thr Gln
    770                 775                 780

Ala Tyr Val Leu Asp Glu Ser Leu Arg Pro Val Pro Pro Gly Val Pro
785                 790                 795                 800

Gly Asp Leu Tyr Leu Ala Gly Ala Gly Val Ala Gln Gly Tyr His Asn
                805                 810                 815

Arg Pro Gly Leu Thr Ala Gln Arg Phe Val Ala Asn Pro Tyr Gly Pro
            820                 825                 830

Ile Gly Ala Arg Met Tyr Gln Thr Gly Asp Val Val Arg Trp Asp Leu
        835                 840                 845

Asp Gly Tyr Leu Glu Phe Leu Gly Arg Ser Asp Asn Gln Ile Lys Ile
    850                 855                 860

Arg Gly Phe Arg Ile Glu Pro Gly Glu Ile Val Ala Ala Leu Glu Arg
865                 870                 875                 880

His Pro His Val Ala Glu Ala Ala Val Ala Val Gln Glu Arg Lys Arg
                885                 890                 895

Asn Gln Lys Gly Leu Val Ala Tyr Val Thr Ala Ser Asp Gly Gln Glu
            900                 905                 910

Ile Asp Thr Thr Glu Leu Arg Asn His Leu Ser Asp Ile Leu Ala Arg
        915                 920                 925

His Ala Ile Pro Ser Ala Phe Val Val Met Ser Glu Leu Pro Arg Thr
    930                 935                 940

Ser Asn Gly Lys Leu Asp Ser Ser Ala Leu Pro Ala Pro Thr Gln Glu
945                 950                 955                 960

Ala Thr Ala Gln Gly Arg Ala Pro Arg Ser Pro Gln Glu Glu Ile Leu
                965                 970                 975

Cys Glu Leu Phe Ala Glu Val Leu Glu Ile Pro Arg Val Gly Val Asp
            980                 985                 990

Asp Asp Phe Phe Glu Leu Gly Gly  His Ser Leu Leu Ala  Ile Arg Leu
        995                 1000                 1005

Ile Ser  Arg Ile Arg Asp Ala  Leu Gly Ser Asn Leu  Thr Ile Arg
    1010                 1015                 1020

Ser Leu  Phe Glu Ala Pro Thr  Val Leu Ala Leu Ala  Gln Arg Leu
    1025                 1030                 1035

Asp Met  Asp Thr Ala Asp Asn  Ala Phe Asp Val Met  Leu Pro Met
    1040                 1045                 1050

Lys Ser  Asn Gly Ser Asn Pro  Pro Ile Phe Cys Ile  His Pro Gly
    1055                 1060                 1065

Gly Gly Ile Gly Trp Ile Tyr Ala Arg Leu Ile Ala Phe Leu Gly
    1070                1075                1080

His Glu Gln Pro Val Tyr Ala Ile Gln Ala Arg Gly Leu Ala Lys
    1085                1090                1095

Glu Glu Pro Leu Pro Glu Thr Ile Glu Glu Met Ala Glu Asp Tyr
    1100                1105                1110

Val Arg Glu Ile Arg Ser Ile Gln Ala Ser Gly Pro Tyr His Leu
    1115                1120                1125

Val Gly Tyr Ser Phe Gly Gly Leu Val Ala Gln Ala Ile Ala Thr
    1130                1135                1140

Arg Leu Gln Gln Asp Gly Asp Lys Val Gly Val Leu Gly Leu Ile
    1145                1150                1155

Glu Ala Tyr Pro Ala Glu Gly Met Ala Gly Ser Glu Arg Pro Glu
    1160                1165                1170

Ile Ser Glu Gln Glu Ile Leu Glu Val Ile Ala Glu Ser Ile Asp
    1175                1180                1185

Lys Ala Glu Glu Ile Lys Ala Lys Asp Arg Ala Phe Glu Arg Met
    1190                1195                1200

Glu Thr Glu Gly Ile Thr Phe Glu Gln Leu Ile Glu Gly Ala Arg
    1205                1210                1215

Ala Arg Gly Ser Val Leu Ser Ala Leu Asp Arg Arg His Val Thr
    1220                1225                1230

Ala Met Ala Asp Ile His Ala Asn Cys Leu His Leu Arg His Asn
    1235                1240                1245

Phe Thr Pro Ser Cys Tyr Asp Gly Asp Ala Leu Leu Phe Arg Ser
    1250                1255                1260

Thr Leu Thr Pro Asn Thr Pro Ser Pro Asp Thr Trp Arg Pro Phe
    1265                1270                1275

Ile Gly Gly Arg Ile Glu Thr Arg Glu Ile Ala Ala Leu His His
    1280                1285                1290

Gln Met Leu His Pro Glu Pro Leu Ala Ala Ile Gly Lys Ile Leu
    1295                1300                1305

Ala Glu Glu Leu Arg Lys Thr His His Glu Tyr Glu Glu Lys Ser

1310                    1315                    1320

Lys

<210>  18
<211>  72
<212>  PRT
<213>  Streptomyces DSM 22643

<400>  18

Met Asn Asn Pro Phe Glu Asp Pro Glu Gly Thr Tyr Lys Val Leu Ile
1               5                   10                  15

Asn Gly Glu Ala Gln Tyr Ser Leu Trp Pro Thr Ser Ile Asp Val Pro
            20                  25                  30

Ala Gly Trp Thr Ile Val His Gly Pro Asp Ser Gln Gln Ala Cys Leu
        35                  40                  45

Asp Phe Val Glu Glu Asn Trp Thr Asp Met Arg Pro Lys Ser Leu Ile
    50                  55                  60

Glu Ala Met Asn Asp Thr Val Ser
65                  70

<210>  19
<211>  270
<212>  PRT
<213>  Streptomyces DSM 22643

<400>  19

Met Gln Leu Thr Ala Asp Gln Val Glu Lys Tyr Lys Ser Asp Gly Tyr
1               5                   10                  15

Val Leu Leu Glu Gly Ala Phe Ser Pro Glu Glu Val His Val Met Arg
            20                  25                  30

Gln Ala Leu Lys Lys Asp Gln Glu Val Gln Gly Pro His Arg Ile Leu
        35                  40                  45

Glu Glu Asp Gly Arg Thr Val Arg Ala Leu Tyr Ala Ser His Thr Arg
    50                  55                  60

Gln Ser Val Phe Asp Gln Leu Ser Arg Ser Asp Arg Leu Leu Gly Pro
65                  70                  75                  80

Ala Thr Gln Leu Leu Glu Cys Asp Leu Tyr Ile His Gln Phe Lys Ile
                85                  90                  95

Asn Thr Lys Arg Ala Phe Gly Gly Asp Ser Trp Ala Trp His Gln Asp
            100                 105                 110

Phe Ile Val Trp Arg Asp Thr Asp Gly Leu Pro Ala Pro Arg Ala Val
        115                 120                 125

Asn Val Gly Val Phe Leu Ser Asp Val Thr Glu Phe Asn Gly Pro Val
    130                 135                 140

Val Phe Leu Ser Gly Ser His Gln Arg Gly Thr Val Glu Arg Lys Ala
145                 150                 155                 160

Arg Glu Thr Ser Arg Ser Asp Gln His Val Asp Pro Asp Asp Tyr Ser
                165                 170                 175

Met Thr Pro Ala Glu Leu Ser Gln Met Val Glu Lys His Pro Met Val
            180                 185                 190

Ser Pro Lys Ala Ala Ser Gly Ser Val Met Leu Phe His Pro Glu Ile
        195                 200                 205

Ile His Gly Ser Ala Pro Asn Ile Ser Pro Phe Ala Arg Asp Leu Leu
    210                 215                 220

Ile Ile Thr Tyr Asn Asp Val Ala Asn Ala Pro Lys Pro Ala Gly Glu
225                 230                 235                 240

Pro Arg Pro Glu Tyr Val Ile Gly Arg Asp Thr Thr Pro Leu Val Ser
                245                 250                 255

Arg Ser Gly Pro Leu His Glu Ala Ala Glu Ser Arg Leu Ala
            260                 265                 270

<210>  20
<211>  391
<212>  PRT
<213>  Streptomyces DSM 22643

<400>  20

Met Ser Asp Arg Ser Pro Ser Val Val Thr Pro Gly Val Ala Ser Gly
1               5                   10                  15

Gly Ile Thr Ala Arg Ala Ala Val Leu Glu Ser Phe Gln Lys Pro Leu
            20                  25                  30

Thr Val Arg Gln Phe Pro Val Pro Ala Pro Ser Pro Gly Glu Ile Leu
        35                  40                  45

Val Asp Val Arg Tyr Gly Gly Ile Cys Gly Thr Asp Leu His Leu Gln
    50                  55                  60

Leu Gly His Leu Pro Ile Pro Val Pro Leu Val Leu Gly His Glu Gly
65                  70                  75                  80

Leu Gly Ser Ile Arg Arg Leu Gly Thr Glu Gly Leu Thr Asp Ala Asn
                85                  90                  95

Gly Thr Glu Leu Arg Ile Gly Asp Thr Val Met Trp Ala Ser Ser Ile
            100                 105                 110

Ala Cys Gly Ser Cys Gly Pro Cys Arg Gln His Arg Glu Pro Thr Leu
        115                 120                 125

Cys Glu Ser Arg Arg Thr Tyr Gly Val Asn Arg Gln Val Glu Gly Asp
    130                 135                 140

Ser Gly Leu Phe Gly Ala Trp Ser Glu Thr Ile Leu Leu His Pro Gly
145                 150                 155                 160

Ala Thr Val Val Arg Leu Pro Gln Ser Val Asp Pro Leu Ala Ala Met
                165                 170                 175

Ser Leu Ala Cys Ala Gly Pro Thr Leu Ile His Ala Leu Tyr Glu Arg
            180                 185                 190

Arg Pro Val Arg Val Gly Glu Thr Val Ile Val Gln Gly Ser Gly Pro
        195                 200                 205

Val Gly Met Ala Ala Ala Ala Leu Ala Gln Leu Ser Gly Ala Ala Met
    210                 215                 220

Val Ile Leu Leu Gly Gly Pro Gln Gln Arg Leu Asp Leu Ala Arg Gln
225                 230                 235                 240

Cys Gly Ile Gly Asp Val His Leu Asn Ile Ala Asp Arg Ser Asp Thr
                245                 250                 255

Thr Ser Ala Leu Asn Glu Ala Arg Glu Met Thr Arg Gly Gly Leu Gly
            260                 265                 270

Ala Asp Leu Val Ile Glu Cys Ala Gly Val Pro Glu Ala Val Ala Gln
        275                 280                 285

Gly Val Tyr Leu Ala Arg Arg Gly Gly Ser Tyr Leu Val Val Gly Gln
    290                 295                 300

Tyr Thr Asp Ser Gly Glu Thr Leu Phe Asn Pro His Gln Leu Val Tyr
305                 310                 315                 320

Arg Gln Leu Glu Val Val Gly Ser Trp Ala Phe Thr Gly Ala His Leu
                325                 330                 335

Val His Tyr Val Asn Leu Leu Pro Ser Leu Leu Glu Arg Phe Asp Leu
            340                 345                 350

Arg Arg Leu Val Thr Glu Phe Pro Leu Gly Glu Val Asn Asp Ala Met
        355                 360                 365

Val Ala Val Gly Thr Gly Glu Val Val Lys Ala Val Leu Glu Ser Arg
    370                 375                 380

His Leu Pro Thr Val Asp Thr
385                 390

<210>  21
<211>  266
<212>  PRT
<213>  Streptomyces DSM 22643

<400>  21

Met Pro Ile Ala Thr Val Asn Glu Thr Gln Leu Arg Tyr Asp Ile His
1               5                   10                  15

Gly Ser Gly Glu Pro Val Val Met Val Met Gly Ser Gly Ser Gly Gly
            20                  25                  30

Arg Val Trp Glu Met His Gln Val Pro Ala Leu Val Ala Asp Gly Tyr
        35                  40                  45

Gln Val Val Thr Phe Asp Asp Arg Gly Val Ser Gln Pro Glu Asp Ile
    50                  55                  60

Glu Pro Tyr Gly Leu Asp Asp Met Val Ala Asp Val Ala Ala Leu Val
65                  70                  75                  80

Asp His Leu Ser Leu Gly Pro Cys Arg Phe Val Gly Thr Ser Leu Gly
                85                  90                  95

Ala Leu Thr Val Gln Glu Leu Ala Ile Arg Arg Pro Asp Leu Val Ser
            100                 105                 110

Glu Ser Val Leu Met Ala Thr Arg Gly Arg Thr Asp Thr Leu Ser Gly
        115                 120                 125

Ala Ile Ser Lys Ala Glu Ile Asp Leu Ile Asp Glu Ser Ala Lys Ile
    130                 135                 140

Pro Val Arg Tyr Gln Ala Val Val Gln Ala Leu Leu Asn Leu Ser Arg

145                 150                 155                 160

Lys Thr Leu Arg Asn Glu Gln Glu Leu Arg Asp Trp Ile Asp Ile Leu
                165                 170                 175

Glu Met Ser Gly Pro Gln Pro Ser Gly Leu Arg Ser Gln Leu Glu Ala
            180                 185                 190

Ser Leu Ile Glu Asp Arg Leu Glu Ala Leu Arg Ser Thr Arg Ala Gln
        195                 200                 205

Thr Leu Val Ile Gly Phe Gln Asp Asp Leu Ile Thr Pro Pro His Leu
    210                 215                 220

Asn Arg Gln Val Ala Glu Ala Ile Pro Gly Ser Thr Tyr Ile Glu Val
225                 230                 235                 240

Ala Asp Cys Gly His Tyr Gly Tyr Leu Glu Gln Pro Asp Val Val Asn
                245                 250                 255

Lys His Ile Leu Asp Phe Phe Ser Lys Gly
            260                 265

<210>  22
<211>  299
<212>  PRT
<213>  Streptomyces DSM 22643

<400>  22

Met Thr Arg Arg Met Arg Tyr Gly Val Val Ile Leu Pro Glu His Ser
1               5                   10                  15

Trp Ala Arg Ala Gln Asp Leu Trp Arg Asn Val Glu Asp Leu Gly Phe
            20                  25                  30

Asp His Ala Trp Thr Tyr Asp His Leu Gln Trp Arg Trp Leu Ser Asp
        35                  40                  45

Arg Pro Trp Phe Gly Ala Ile Pro Thr Leu Thr Ala Ala Ala Val Val
    50                  55                  60

Thr Ser Arg Ile Gly Leu Gly Thr Leu Val Ala Ser Thr Lys Leu Arg
65                  70                  75                  80

Asp Pro Val Met Leu Ala Lys Glu Ile Met Thr Leu Asp Asp Ile Ser
                85                  90                  95

Gly Gly Arg Met Leu Cys Gly Val Gly Ser Gly Gly Pro Asp Arg Asp
            100                 105                 110

Leu Leu Gln Ala Tyr Glu Leu Thr Arg Arg Gln Leu Thr Ala Arg Tyr
        115                 120                 125

Gly Glu Phe Ile Glu Leu Leu Asp Ser Leu Leu Arg Gln Glu Pro Val
    130                 135                 140

Val Phe Glu Gly Thr Tyr Tyr Thr Cys Arg Asn Thr Leu Leu Gln Pro
145                 150                 155                 160

Ala Cys Leu Gln Arg Pro Arg Ala Pro Leu Cys Val Ala Ala Ala Gly
                165                 170                 175

Pro Gln Gly Met Arg Leu Ala Ala Arg Phe Ala Asp Ile Trp Val Thr
            180                 185                 190

Met Gly Ala Pro Asn Met Phe Asp Glu Ala Pro Tyr Ala Asp Ser Ala
        195                 200                 205

Met Leu Ile Lys Asp Gln Val Ala Ala Leu Glu Gln Thr Cys His Glu
    210                 215                 220

Val Gly Arg Asp Pro Ala Ser Leu Arg Arg Leu Leu Val Thr Gly Pro
225                 230                 235                 240

Ser Ile Gly Gly Val Leu Asp Ser Val Gly Ser Phe Gln Asp Ala Ala
                245                 250                 255

Gly Thr Phe Gln Glu Val Gly Ile Thr Asp Leu Val Val His Trp Pro
            260                 265                 270

Arg Pro Asp Phe Pro Tyr Arg Gly Asp Pro Ala Val Met Glu Asp Ile
        275                 280                 285

Ala Ser Ile Leu Pro Thr Ala Pro Gly Lys Leu
    290                 295

<210>  23
<211>  178
<212>  PRT
<213>  Streptomyces DSM 22643

<400>  23

Val Arg Ile Ser Pro Arg His Gly Leu Ala Gly Arg Cys Val Glu Glu
1               5                   10                  15

Val Thr Ser Ala Pro Ile Cys Ala Val Val Arg Val Thr Leu Thr Gly
            20                  25                  30

Met Pro Ser Asp Ile Phe Ser Gln Ile Pro Glu Tyr Ser Ala Phe Trp

35                  40                  45

Asn Glu Tyr His Leu Cys Thr Leu Thr Thr Leu Arg Ser Asp Gly Ser
    50                  55                  60

Pro His Val Val Pro Val Gly Val Thr Leu Asp Val Asp Ala Gly Val
65                  70                  75                  80

Ala Arg Val Ile Thr Arg Lys Ser Ser Arg Lys Val Ala Asn Ile Gln
                85                  90                  95

Ala Ser Arg Pro Gly Glu Ala Arg Ala Ala Val Cys Gln Val Asp Gly
            100                 105                 110

Gly Arg Trp Ala Thr Leu Glu Gly Thr Ala Glu Val Leu Thr Asp Ala
        115                 120                 125

Ala Ala Val Ala Asp Ala Val Ala Arg Tyr Ala Asp Arg Tyr Gly Arg
    130                 135                 140

Thr Pro Ala Pro Asp Pro Glu Arg Val Val Ile Glu Ile Thr Ile Asp
145                 150                 155                 160

Arg Ala Met Gly Gln Val Asn Ala Ala Arg Pro Lys Ala Ala Ser Thr
                165                 170                 175

Ala Ala

<210>  24
<211>  216
<212>  PRT
<213>  Streptomyces DSM 22643

<400>  24

Met Ser Pro Gly Asp Ser Arg Arg Val Ala Ala Glu Ser Thr Val Pro
1               5                   10                  15

Ala Arg Glu Lys Val Ser Cys Arg Arg Leu Gly Arg Trp Arg Thr Ala
            20                  25                  30

Ala Arg Arg Thr Val Glu Arg Asp Leu His Arg Arg Leu Arg Gln Glu
        35                  40                  45

Leu Arg Asp Leu Gly Ile His Pro Pro Leu Asp Val Glu Glu Leu Ala
    50                  55                  60

Lys Ala Leu Gly Glu Arg Arg Gly Arg Pro Ile Val Leu Arg Pro Phe
65                  70                  75                  80

Pro Leu Glu Lys Pro Gly Pro Ser Gly Leu Trp Ile Asp Thr Pro Gln
                85                  90                  95

Met Asp Val Ile Leu Tyr Gln Gln Glu Thr Thr Arg Leu His Gln Arg
            100                 105                 110

Gln Ile Ile Leu His Glu Ile Leu His Ile Leu Val Ala Glu Trp Glu
        115                 120                 125

Glu Asp Gln Ala Glu Glu Ala Pro Glu Glu Ser Pro Asp Asp Phe Val
    130                 135                 140

Glu Gly Trp Ala Thr Leu Ile Pro Val Leu Asp Pro Lys Leu Ile Arg
145                 150                 155                 160

Arg Val Ala Arg Arg Cys Ser Tyr Glu Asp Glu Glu Glu Cys Ser Val
                165                 170                 175

Glu Leu Ala Ala Thr Ile Ile Leu Glu Trp Ser Ser Val Leu Asp Glu
            180                 185                 190

Leu Pro Pro Leu Ser Glu Asp Pro Glu Val Arg Arg Val Gln Ser Ala
        195                 200                 205

Leu Gly Asp Arg Arg Gly Trp Leu
    210                 215

<210>  25
<211>  371
<212>  PRT
<213>  Streptomyces DSM 22643

<400>  25

Val Ser Glu Ile Leu Leu Leu Val Ile Ala Ala Ala Val Val Trp Arg
1               5                   10                  15

Leu Tyr Leu Trp Ser Arg Ala Pro His Asp Ala Pro Thr Arg Ser Val
            20                  25                  30

Ala Leu Cys Leu Leu Ser Ala Gly Leu Ser Tyr Pro Val Ala Met Pro
        35                  40                  45

Gly Gly Thr Thr Gly Ile Asp Thr Val Ala Gly His Gly Thr Ala Lys
    50                  55                  60

Leu Leu Gln Asn Val Leu Leu Leu Leu Thr Val Tyr Phe Leu Met Cys
65                  70                  75                  80

Phe Tyr Leu Tyr Ser Ala Asp Gly Ser Val Ala Arg Arg Arg Ala Arg

                85                  90                  95

Trp Glu Ala Val Val Val Ser Val Val Ala Val Ala Ile Ile Leu Ala
            100                 105                 110

Ala Val Thr Val Pro His Glu Asp Phe Ala Gly Ser Phe Ser Thr Ala
        115                 120                 125

Asp Met Thr Ile Pro Gln Ile Ala Phe Phe Tyr Ala Gly Ala Gly Leu
    130                 135                 140

Tyr Leu Met Tyr Ala Leu Gly Ala Ala Gly Arg Trp Thr Val Arg Tyr
145                 150                 155                 160

Ala Arg Met Ser Ser Arg Pro His Ala Thr Gly Leu Trp Met Thr Ala
                165                 170                 175

Ile Gly Leu Gly Ala Met Ala Val Ala Cys Ala Val Arg Ala Val Phe
            180                 185                 190

Val Ala Val Arg Trp Ser Gly Gly Thr Val Pro Asp Arg Leu Met Ala
        195                 200                 205

Gly Val Ala Phe Trp Leu Val Val Ser Ile Leu Leu Phe Val Ala Gly
    210                 215                 220

Val Thr Tyr Ser Ala Thr Arg Ser Arg Ile Thr Ala Thr Arg Leu Trp
225                 230                 235                 240

Leu Arg Arg Arg Arg Asp His Arg Arg Leu Ser Pro Leu Trp Gln Leu
                245                 250                 255

Leu Ala Glu Val Tyr Pro Glu Asn Glu Leu Arg Pro Ala Ser Arg Gly
            260                 265                 270

Leu Trp Asp Arg Trp Arg Ala Arg Gly Val His Arg Arg Tyr His Arg
        275                 280                 285

Arg Ile Val Glu Cys Arg Asp Gly Leu Val Asp Ile Ser Pro Tyr Leu
    290                 295                 300

Val Asp Glu Asn Gly Asp Ala Asp Leu Leu Arg Leu Glu Pro Ala Glu
305                 310                 315                 320

Leu Ala Ser Arg Leu Arg Gln Ala Ala Asp Met Ile Arg Gln Gly Ser
                325                 330                 335

Pro Ala Pro Gly Gln Ala Val Pro Leu Ala Val Pro Lys Glu Asp Asp
            340                 345                 350

Arg Asp Ala Asp Val Arg Gln Leu Ile Ala Val Ser Glu Ala Leu Arg
        355                 360                 365

Leu Thr Ala
    370

<210>  26
<211>  398
<212>  PRT
<213>  Streptomyces DSM 22643

<400>  26

Met Leu Ile Thr Ala Ser Arg Val Leu Thr Gly Ser Gly Thr His Leu
1               5                   10                  15

Glu Asp Gly Ala Val Leu Val Glu Gly Asp Ser Ile Ala Ala Val Gly
            20                  25                  30

Arg Arg Ala Glu Leu Ala Asp Arg Val Gly Ala Asp Gly Lys His Leu
        35                  40                  45

Ala Phe Pro Asp Ala Thr Leu Leu Pro Gly Leu Ile Asp Ala His Val
    50                  55                  60

His Leu Ala Phe Asp Gly Gly Gly Asp Pro Val Ala Thr Leu Asp Glu
65                  70                  75                  80

Ser Ser Asp Glu Lys Leu Leu Glu Asp Met Arg Arg Arg Ala Glu Gln
                85                  90                  95

Leu Leu Ser Ser Gly Val Thr Thr Val Arg Asp Leu Gly Asp Arg His
            100                 105                 110

Gly Leu Ala Leu Arg Leu Asp Glu Glu Ile Ser Gln Gly Gly Thr Ser
        115                 120                 125

Gly Pro Arg Ile Val Ala Ala Gly Thr Pro Ala Thr Pro Pro Gly Gly
    130                 135                 140

His Cys His Phe Leu Gly Gly Glu Val Ser Gly Val Asp Gln Val Arg
145                 150                 155                 160

Asp Leu Val Arg Arg Asn Ile Ala Ala Gly Ala Gly Val Ile Lys Ala
                165                 170                 175

Met Val Thr Gly Gly Gly Leu Thr Lys Asp Gly Pro Lys Ser Trp Gln
            180                 185                 190

Ser Gln Phe Ser Pro Asp Glu Leu Gln Ala Leu Val Asp Glu Ala His
        195                 200                 205

Gln Ala Asp Val Pro Val Ala Ala His Ala His Gly Thr Asp Gly Ile
    210                 215                 220

Thr Ala Ala Val Glu Ala Gly Val Asp Thr Ile Glu His Cys Thr Trp
225                 230                 235                 240

Met Thr Ala Asp Gly Phe Asp Leu Arg Gln Asp Val Leu Lys Gln Ile
                245                 250                 255

Ile Asp Arg Asp Ile Ala Val Cys Pro Ala Val Ser Pro His Trp Glu
            260                 265                 270

Met Leu Pro Arg Phe Phe Gly Glu Glu Arg Ala Ala Ala Met Phe Asp
        275                 280                 285

Leu Val Arg Gln Met Ala Glu Ala Gly Ala Lys Leu Ile Ala Gly Thr
    290                 295                 300

Asp Ala Gly Val Gln Arg Ala Gly Phe Asp Gly Leu Val Pro Ala Leu
305                 310                 315                 320

Ser Phe Tyr Ala His Leu Gly Leu Ser Asn Ser Arg Ile Leu Asp Met
                325                 330                 335

Ala Thr Ala Asp Ala Ala Gly Ala Leu Gly Leu Gly Glu Thr Thr Gly
            340                 345                 350

Arg Ile Ala Pro Arg Phe Arg Ala Asp Leu Leu Val Ile Asp Gly Asp
        355                 360                 365

Pro Leu Glu Asp Leu Ser Ala Leu Lys Met Val Arg Ala Val Val Ala
    370                 375                 380

Ala Gly Arg Leu Leu Glu Pro Gly Arg Thr Ala Glu Gln Gln
385                 390                 395

<210> 27
<211> 300
<212> PRT
<213> Streptomyces DSM 22643

<400> 27

Met Ser Ala Arg His Phe Asp Arg Ser Arg Leu Arg Thr Val Arg Arg
1               5                   10                  15

Ala Ala Glu Lys Ser Gln Ala Ala Val Gly Glu Val Leu Gly Val Ser
            20                  25                  30

Asp Ser Ala Val Ala Ser Trp Glu Ser Gly Ala Gln Thr Pro Asp Pro
        35                  40                  45

Glu Lys Leu Pro Ala Leu Ala Arg Ala Val Gly His Asp Val Asp Asp
    50                  55                  60

Leu Phe Pro Arg Ser Gly Pro Pro Asp Leu Thr Asp Leu Arg Cys Asp
65                  70                  75                  80

Ala Gly Tyr Tyr Gln Tyr Glu Thr Ala Thr Leu Ile Gly Thr Lys Ser
                85                  90                  95

Ala Gly Pro Val Ala Gly Ala Glu Arg Gly Glu Arg Arg Leu Lys Asp
            100                 105                 110

Lys Tyr Ile Pro Ala Leu Ala Ala Ala Tyr Gly Val Ser Glu Val Glu
        115                 120                 125

Leu Arg Arg Ala Glu Asp Arg Ser Ile Ala Lys Ala Gln Gly Met Pro
    130                 135                 140

Val Glu Gly Ser Ala Pro Ser Glu Ala Lys Arg Ala Pro Ala Ala Asp
145                 150                 155                 160

Ala Ala Pro Gly Ser Leu Thr Glu Lys Ile Thr Leu Leu Leu Asn Ser
                165                 170                 175

Ser Tyr Pro Ser Pro Pro Gly Pro Pro Ser Asp Ala Glu Met Ala Ser
            180                 185                 190

Ala Val Asn Ala Ser Ala Gly Glu Gln Ile Leu Thr Glu Glu Gly Phe
        195                 200                 205

Arg His Leu Arg Thr Gly Val Ala Glu Thr Ala Ala Pro Glu Val Leu
    210                 215                 220

Asp Ala Leu Ala Asp Val Ile Gly Val Ser Arg Met Tyr Phe Lys Pro
225                 230                 235                 240

Asp Asp Ala Val Ala Ala Gln Val Tyr Glu Gly Leu Gln Leu Leu Ala
                245                 250                 255

Ala Ala Lys Lys Gly Ala Val Gly Arg Val Lys Ala Arg Gly Leu Gly
            260                 265                 270

Ala Gln Gly Leu Ser Pro Lys Ala Met Ala Leu Val Asn Glu Leu Val
        275                 280                 285

Ala Glu Leu Glu Glu Lys Glu Thr Gly Ala Asn Glu
    290                 295                 300

<210>  28
<211>  29
<212>  DNA
<213>  Artificial sequence

<220>
<223>  Description of Artificial  Sequence: Primer for amplification of
       the mps-1 gene

<400>  28
gccgccatat gatgcagctc acggccgat                                        29

<210>  29
<211>  29
<212>  DNA
<213>  Artificial Sequence

<220>
<223>  Description of Artificial Sequence: Primer for amplification of
       the mps-1 gene

<400>  29
ggtcaggatc ctcatgccag cctcgattc                                        29

<210>  30
<211>  31
<212>  DNA
<213>  Artificial Sequence

<220>
<223>  Description of Artificial Sequence: Primer for amplification of A
       domains A2, A5 and A8

<400>  30
ccgaccatat ggatccggat gtgacggtgg g                                     31

<210>  31
<211>  31
<212>  DNA
<213>  Artificial Sequence

<220>
<223>  Description of Artificial Sequence: Primer for amplification  of
       A domains A2, A5 and A8

<400>  31
accgggaatt cgccgacggc gggcaggccg a                                     31